# EUROPEAN PATENT APPLICATION

(11) **EP 4 556 007 A1**
(43) Date of publication of application: **21.05.2025**
(21) Application number: 23839043.9
(22) Date of filing: 14.07.2023
(51) Int. Cl.: A61K 31/437, A61K 31/4985, A61K 31/4365, C07D 239/70, C07D 279/32, A61P 25/00, A61P 25/28

(54) **TPK AGONIST AND METHOD FOR TREATING NEURODEGENERATIVE DISEASES USING SAME**

(30) Priority: 14.07.2022 CN 202210839749
(71) Applicant: Shanghai Raising Pharmaceutical Co., Ltd., Pudong New District Shanghai 201321 (CN)
(72) Inventor: MENG, Lijuan, Shanghai 201321 (CN); JING, Hefeng, Shanghai 201321 (CN); ZHANG, Huan, Shanghai 201321 (CN)
(74) Representative: Michalski Hüttermann & Partner Patentanwälte mbB
(86) International application number: PCT/CN2023/107391
(87) International publication number: WO 2024/012554

(57) **Abstract**

The present disclosure belongs to the field of biomedicine, and specifically relates to a method for preventing or treating neurodegenerative diseases or alleviating symptoms of neurodegenerative diseases. Said method comprises administering an effective amount of a thiamine pyrophosphokinase (TPK) agonist to an individual in need of said agonist.

## Description

### FIELD OF THE INVENTION

The present invention belongs to the field of biomedicine, and specifically relates to a method for the prophylaxis or treatment of neurodegenerative diseases or alleviating symptoms of neurodegenerative diseases. Said method comprises administering a prophylactically or therapeutically effective amount of a thiamine pyrophosphokinase (TPK) agonist to a subject in need thereof.

### BACKGROUND OF THE INVENTION

Alzheimer's disease (AD) is the most common degenerative disease of the central nervous system. Due to the large number of patients, prolonged disease course, long survival period with loss of self-care ability, and lack of effective prophylactical and therapeutic drugs, it imposes enormous economic and psychological burdens on individuals, families, and society as a whole. According to reports, China's expenditures on AD prevention, treatment, and care in 2015 reached as high as 167.74 billion US dollars, and it is estimated that by 2030, these expenditures will rise to 507.49 billion US dollars. In 2018, global expenditures on dementia-related diseases, predominantly AD, exceeded one trillion US dollars, accounting for over 1% of the global gross domestic product (GDP). AD is the only disease among the top ten major global diseases that lacks effective prophylactical and therapeutic drugs. It has become a significant disease severely affecting the healthcare systems and sustainable economic development of major global economies, including China.

AD is a disease involving multiple pathophysiological alterations, including neuronal loss, glial cell activation, characteristic extracellular β-amyloid protein (Aβ) deposition forming senile plaques, and intracellular hyperphosphorylated Tau protein causing neurofibrillary tangles. Additionally, synaptic loss, impaired cerebral glucose metabolism, oxidative stress, and other pathological changes are consistently observed in AD brains. Reduced cerebral glucose metabolism in patients is closely associated with cognitive dysfunction. Due to unclear pathogenesis, AD still lacks effective treatment methods.

### SUMMARY OF THE INVENTION

The inventors of the present application discovered that glucose metabolism disorders may be an early pre-clinical feature of AD. The intracellular glucose metabolism process in AD patients is mainly characterized by a significant decrease in the activities of three key enzymes (pyruvate dehydrogenase, α-ketoglutarate dehydrogenase, and transketolase) that rely on thiamine diphosphate (TDP) as a coenzyme. A multi-center clinical study has proven that the decrease in TDP levels in AD patients is a specific and common phenomenon with good diagnostic value, while there is no thiamine metabolism abnormality in patients with vascular dementia and frontotemporal dementia. Clinical and experimental studies further indicate that the decrease in TDP is the cause of cerebral glucose metabolism disorders. The inventors of the present application have found that among the four known genes related to thiamine metabolism, only the expression of thiamine pyrophosphokinase (TPK), a key enzyme that converts thiamine into the bioactive TDP, is significantly inhibited, and this inhibition of TPK expression is specific to AD. Therefore, TPK agonists can be used for the prophylaxis or treatment of neurodegenerative diseases (especially Alzheimer's disease).

In one aspect, the present invention provides a method for the prophylaxis or treatment of neurodegenerative diseases or alleviating symptoms of neurodegenerative diseases, which comprises administering to a subject in need thereof a prophylactically or therapeutically effective amount of a thiamine pyrophosphokinase (TPK) agonist.

In another aspect, the present invention provides use of a TPK agonist in the manufacture of a medicament for the prophylaxis or treatment of neurodegenerative diseases or alleviating symptoms of neurodegenerative diseases.

In another aspect, the present invention provides a TPK agonist for use in the prophylaxis or treatment of neurodegenerative diseases or alleviating symptoms of neurodegenerative diseases.

The neurodegenerative disease is preferably Alzheimer's disease; more preferably, the Alzheimer's disease is one in which the subject has decreased TPK enzyme activity, decreased TPK expression level, and/or decreased TDP level.

In another aspect, the present invention relates to TPK agonists with a novel structures.

### DETAILED DESCRIPTION OF THE INVENTION

### Definition

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps.

As used herein, when describing a divalent group that connects two other groups, it is understood that the divalent group can be connected to the two groups in any direction. For example, if the other two groups connected by the divalent group -CONR- are (Group 1) and (Group 2) respectively, then both (Group 1)-CONR-(Group 2) and (Group 2)-CONR-(Group 1) are included.

As used herein, the term "alkylene" refers to a saturated divalent hydrocarbyl, preferably refers to a saturated divalent hydrocarbyl having 1, 2, 3, 4, 5 or 6 carbon atoms, e.g., methylene, ethylene, propylene or butylene.

As used herein, the term "alkyl" is defined as a straight or branched saturated aliphatic hydrocarbon. In some embodiments, alkyl has 1-12, e.g., 1-6, carbon atoms. For example, as used herein, the term "C₁₋₆ alkyl" refers to a linear or branched group having 1-6 carbon atoms (such as methyl, ethyl, n-propyl, isopropyl, n-butyl, isobutyl, sec-butyl, *tert*-butyl, n-pentyl, or n-hexyl), which is optionally substituted with one or more (e.g., 1 to 3) suitable substituents such as halogen (in which case the group may be referred to as "haloalkyl") (e.g., CF₃, C₂F₅, CHF₂, CH₂F, CH₂CF₃, CH₂Cl or -CH₂CH₂CF₃ *etc*.). The term "C₁₋₄ alkyl" refers to a linear or branched aliphatic hydrocarbon chain having 1-4 carbon atoms (i.e., methyl, ethyl, n-propyl, isopropyl, n-butyl, isobutyl, sec-butyl or *tert*-butyl).

As used herein, the term "alkenyl" refers to a linear or branched monovalent hydrocarbyl having one or more double bonds and 2-6 carbon atoms ("C₂₋₆ alkenyl"). The alkenyl is e.g., CH=CH₂, -CH₂CH=CH₂, -C(CH₃)=CH₂, -CH₂-CH=CH-CH₃, 2-pentenyl, 3-pentenyl, 4-pentenyl, 2-hexenyl, 3-hexenyl, 4-hexenyl, 5-hexenyl, 2-methyl-2-propenyl and 4-methyl-3-pentenyl. When the compound of the present invention contains an alkenyl group, the compound may exist as the pure E (entgegen) form, the pure Z (zusammen) form, or any mixture thereof. The term "alkenylene" refers to a corresponding divalent group, including, e.g., "C₂₋₆ alkenylene", "C₂₋₄ alkenylene", *etc.,* and the specific examples thereof include but are not limited to: -CH=CH-, -CH₂CH=CH-, -C(CH₃)=CH-, butenylene, pentenylene, hexenylene, cyclopentenylene, cyclohexenylene, *etc.*

As used herein, the term "alkynyl" refers to a monovalent hydrocarbyl containing one or more triple bonds, preferably having 2, 3, 4, 5 or 6 carbon atoms, such as ethynyl, 2-propynyl, 2-butynyl, buta-1,3-diynyl, *etc.* The alkynyl group is optionally substituted with one or more (e.g., 1 to 3) same or different substituents. The term "alkynylene" refers to a corresponding divalent group, including e.g., "C₂₋₈ alkynylene", "C₂₋₆ alkynylene", "C₂₋₄ alkynylene", *etc.* Examples thereof include, but are not limited to, 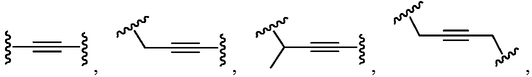 and the like, and the alkynylene group is optionally substituted with one or more (e.g., 1 to 3) same or different substituents.

As used herein, the term "fused ring" refers to a ring system formed by two or more ring structures sharing two adjacent atoms with each other.

As used herein, the term "spiro" refers to a ring system formed by two or more ring structures sharing one ring atom with each other.

As used herein, the term "bridged ring" refers to a ring system formed by two or more ring structures sharing two atoms which are not directly linked with each other.

As used herein, the terms "cyclic hydrocarbylene", "cyclic hydrocarbyl" and "hydrocarbon ring" refer to a saturated (i.e., "cycloalkylene" and "cycloalkyl") or unsaturated (i.e., having one or more double and/or triple bonds in the ring) monocyclic or polycyclic (including spiro ring, fused ring or bridged ring) hydrocarbon ring having e.g., 3-10 (suitably having 3-8, and more suitably having 3-6) ring carbon atoms, including but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), and the like.

As used herein, the term "cycloalkyl" refers to a saturated, monocyclic or polycyclic (e.g., bicyclic) hydrocarbon ring (e.g., monocyclic, such as cyclopropyl, cyclobutyl, cyclopentyl, cyclohexyl, cycloheptyl, cyclooctyl, or cyclononyl, or bicyclic, including spiro, fused or bridged cyclic system (such as bicyclo[1.1.1]pentyl, bicyclo[2.2.1]heptyl, bicyclo[3.2.1]octyl or bicyclo[5.2.0]nonyl, or decahydronaphthalene *etc*.)), which is optionally substituted with one or more (e.g., 1 to 3) suitable substituents. The cycloalkyl has 3 to 15 carbon atoms. For example, the term "C₃₋₆ cycloalkyl" refers to a saturated, monocyclic or polycyclic (e.g., bicyclic) hydrocarbon ring having 3 to 6 ring forming carbon atoms (e.g., cyclopropyl, cyclobutyl, cyclopentyl, or cyclohexyl), which is optionally substituted with one or more (e.g., 1 to 3) suitable substituents, e.g., methyl substituted cyclopropyl.

As used herein, the term "heterocyclyl" refers to a saturated or unsaturated monovalent monocyclic or bicyclic group having 2, 3, 4, 5, 6, 7, 8 or 9 carbon atoms and one or more (e.g., one, two, three or four) heteroatom-containing groups in the ring, wherein the heteroatom-containing group is selected from O, S, N, S(=O), S(=O)₂, S(=O)(=NR^{Z}), NR^{Z} or P(=O)(R^{Z}), wherein R^{Z}, at each occurrence, independently represents a hydrogen atom or a C₁₋₆ alkyl or a halo-C₁₋₆ alkyl; said heterocycloalkyl may be attached to the remainder of the molecule through any one of said carbon atoms or a nitrogen atom (if present). In particular, a 3- to 10-membered heterocyclyl is a group having 3 to 10 carbon atoms and heteroatoms in the ring, such as but not limited to oxiranyl, aziridinyl, azetidinyl, oxetanyl, tetrahydrofuranyl, dioxolinyl, pyrrolidinyl, pyrrolidonyl, imidazolidinyl, pyrazolidinyl, pyrrolinyl, tetrahydropyranyl, piperidinyl, morpholinyl, dithianyl, thiomorpholinyl, piperazinyl or trithianyl.

As used herein, the term "heterocyclyl" encompasses fused ring structures, the attachment point with another group can be from any ring in the fused ring structure. As such, the heterocyclyl of the present invention also includes but is not limited to heterocyclyl-fused heterocyclyl, heterocyclyl-fused cycloalkyl, monoheterocyclyl-fused monoheterocyclyl, monoheterocyclyl-fused monocycloalkyl, such as 3-7-membered (mono)heterocyclyl-fused 3-7-membered (mono)heterocyclyl, 3-7-membered (mono)heterocyclyl-fused (mono)cycloalkyl, 3-7-membered (mono)heterocyclyl-fused C₄₋₆ (mono)cycloalkyl, and the examples include, but are not limited to, pyrrolidinyl-fused cyclopropyl, cyclopentyl-fused azacyclopropyl, pyrrolidinyl-fused cyclobutyl, pyrrolidinyl-fused pyrrolidinyl, pyrrolidinyl-fused piperidinyl, pyrrolidinyl-fused piperazinyl, piperidinyl-fused morpholinyl,

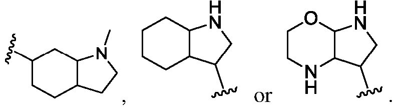

As used herein, the term "heterocyclyl" encompasses bridged heterocyclyl and spiro heterocyclyl.

As used herein, the term "bridged heterocycle" refers to a ring structure comprising one or more (e.g., 1, 2, 3 or 4) heteroatoms (e.g., oxygen, nitrogen, and/or sulfur atoms), formed by two saturated rings sharing two ring atoms which are not directly linked, including, but not limited to, 7-10-membered bridged heterocycle, 8-10-membered bridged heterocycle, 7-10-membered nitrogen-containing bridged heterocycle, 7-10-membered oxygen-containing bridged heterocycle, 7-10-membered sulfur-containing bridged heterocycle, and the like, such as 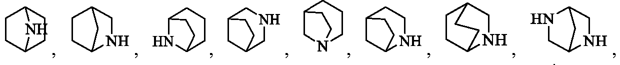 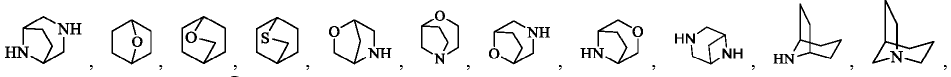 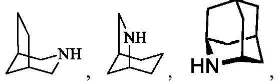 *etc.* The "nitrogen-containing bridged heterocycle", "oxygen-containing bridged heterocycle" and "sulfur-containing bridged heterocycle" optionally further comprise one or more additional heteroatoms selected from oxygen, nitrogen and sulfur.

As used herein, the term "spiro heterocycle" refers to a ring structure comprising one or more (e.g., 1, 2, 3 or 4) heteroatoms (e.g., oxygen atoms, nitrogen atoms, sulfur atoms) formed by two or more saturated rings sharing one ring atom, including, but not limited to, 5-10-membered spiro heterocycle, 6-10-membered spiro heterocycle, 6-10-membered nitrogen-containing spiro heterocycle, 6-10-membered oxygen-containing spiro heterocycle, and 6-10-membered sulfur-containing heterocycle, *etc.,* such as 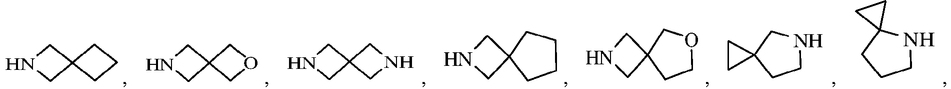 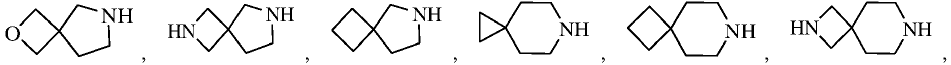 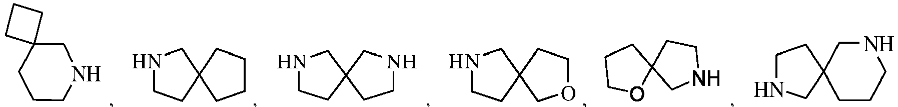 and 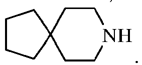 The "nitrogen-containing spiro heterocycle", "oxygen-containing spiro heterocycle" and "sulfur-containing spiro heterocycle" optionally further comprise one or more additional heteroatoms selected from oxygen, nitrogen and sulfur. The term "6-10-membered nitrogen-containing spiro heterocyclyl" refers to a spiro heterocyclyl group comprising a total of 6-10 ring atoms, and at least one of the ring atoms is a nitrogen atom.

As used herein, the terms "aryl(ene)" and "aromatic ring" refer to a monocyclic or fused-ring polycyclic aromatic group having a conjugated π electron system. For example, as used herein, the terms "C₆₋₁₀ aryl(ene)" and "C₆₋₁₀ aromatic ring" refer to an aromatic group containing 6 to 10 carbon atoms, such as phenyl(ene) (benzene ring) or naphthyl(ene) (naphthalene ring). Aryl(ene) or aromatic ring is optionally substituted with one or more (such as 1 to 3) suitable substituents (e.g., halogen, -OH, -CN, -NO₂, and C₁₋₆ alkyl, *etc*.). When an aryl(ene) group and an aromatic ring are fused rings, the fused ring can be a hydrocarbon ring, a heterocyclic ring or a heteroaromatic ring.

The term "aralkyl" preferably means aryl substituted alkyl, wherein the aryl and the alkyl are as defined herein. Normally, the aryl group may have 6-14 carbon atoms, and the alkyl group may have 1-6 carbon atoms. Exemplary aralkyl group includes, but is not limited to, benzyl, phenylethyl, phenylpropyl, phenylbutyl.

As used herein, the terms "heteroaryl(ene)" and "heteroaromatic ring" refer to a monocyclic, bicyclic or tricyclic aromatic ring system having 5, 6, 7, 8, 9, 10, 11, 12, 13 or 14 ring atoms, particularly 1 or 2 or 3 or 4 or 5 or 6 or 9 or 10 carbon atoms, and containing at least one heteroatom (such as O, N, or S), which can be same to different. Moreover, in each case, it can be benzo-fused. In particular, "heteroaryl(ene)" or "heteroaromatic ring" is selected from the group consisting of thienyl(ene) (ring), furyl(ene) (ring), pyrrolyl(ene) (ring), oxazolyl(ene) (ring), thiazolyl(ene) (ring), imidazolyl(ene) (ring), pyrazolyl(ene) (ring), isoxazolyl(ene) (ring), isothiazolyl(ene) (ring), oxadiazolyl(ene) (ring), triazolyl(ene) (ring), thiadiazolyl(ene) (ring) *etc.,* and benzo derivatives thereof; or pyridinyl(ene) (ring), pyridazinyl(ene) (ring), pyrimidinyl(ene) (ring), pyrazinyl(ene) (ring), triazinyl(ene) (ring), etc., and benzo derivatives thereof.

As used herein, the term "halo" or "halogen" are defined to include F, Cl, Br, or I.

As used herein, the term "alkylthio" means an alkyl group as defined above attached to a parent molecular moiety through a sulfur atom. Representative examples of C₁₋₆ alkylthio include, but are not limited to, methylthio, ethylthio, tert-butylthio and hexylthio.

As used herein, the term "nitrogen containing heterocycle" refers to a saturated or unsaturated monocyclic or bicyclic group having 2, 3, 4, 5, 6, 7, 8, 9, 10, 11, 12 or 13 carbon atoms and at least one nitrogen atom in the ring, which may further optionally comprise one or more (e.g., one, two, three or four) ring members selected from the group consisting of N, O, C=O, S, S=O and S(-O)₂. The nitrogen-containing heterocycle is connected with the rest of the molecule through a nitrogen atom. The nitrogen-containing heterocycle is preferably a saturated nitrogen-containing monocyclic ring. In particular, a 3- to 14-membered nitrogen-containing heterocycle is a group having 3-14 carbon atoms and heteroatoms (at least one of which is a nitrogen atom) in the ring, including but not limited to a 3-membered nitrogen-containing heterocycle (such as aziridinyl), a 4-membered nitrogen-containing heterocycle (such as azetidinyl), a 5-membered nitrogen-containing heterocycle (such as pyrrolyl, pyrrolidinyl (pyrrolidinyl ring), pyrrolinyl, pyrrolidonyl, imidazolyl, imidazolidinyl, imidazolinyl, pyrazolyl, pyrazolinyl), 6-membered nitrogen-containing heterocycle (such as piperidinyl (piperidinyl ring), morpholinyl, thiomorpholinyl, piperazinyl), 7-membered nitrogen-containing heterocycle, etc.

The term "substituted" means that one or more (e.g., one, two, three, or four) hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

If a substituent is described as being "optionally substituted," the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more of a list of substituents, one or more of the hydrogens on the nitrogen (to the extent there are any) may each be replaced with an independently selected optional substituent.

If substituents are described as being "independently selected" from a group, each substituent is selected independent of the other(s). Each substituent therefore may be identical to or different from the other substituent(s).

As used herein, the term "one or more" means one or more than one (e.g., 2, 3, 4, 5 or 10) as reasonable.

As used herein, unless specified, the point of attachment of a substituent can be from any suitable position of the substituent.

When a bond to a substituent is shown to cross a bond connecting two atoms in a ring, then such substituent may be bonded to any of the ring-forming atoms in that ring that are substitutable.

The present invention also includes all pharmaceutically acceptable isotopically labeled compounds, which are identical to those of the present invention except that one or more atoms are replaced by an atom having the same atomic number, but an atomic mass or mass number different from the atomic mass or mass number which predominates in nature. Examples of isotopes suitable for inclusion in the compound of the present invention include, but are not limited to, isotopes of hydrogen (such as deuterium (D, ²H), tritium (T, ³H)); carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopically labeled compounds of the present invention, for example those incorporating a radioactive isotope, are useful in drug and/or substrate tissue distribution studies (e.g., assays). The radioactive isotopes tritium, i.e., ³H, and carbon-14, i.e., ¹⁴C, are particularly useful for this purpose in view of their ease of incorporation and ready means of detection. Substitution with positron-emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-*d₆*, or DMSO-*d₆*.

It also should be understood that, certain compounds of the present invention can be used for the treatment in a free form, or where appropriate, in a form of a pharmaceutically acceptable derivative. In the present invention, the pharmaceutically acceptable derivative includes, but is not limited to a pharmaceutically acceptable salt, ester, solvate, metabolite or prodrug, which can directly or indirectly provide the compound of the present invention or a metabolite or residue thereof after being administered to a patient in need thereof. Therefore, "the compound of the present invention" mentioned herein also means to encompass various derivative forms of the compound as mentioned above.

A pharmaceutically acceptable salt of the compound of the present invention includes an acid addition salt and a base addition salt thereof.

A suitable acid addition salt is formed from an acid which forms a pharmaceutically acceptable salt. Specific examples include aspartate, benzoate, bicarbonate/carbonate, bisulfate/sulfate, fumarate, gluceptate, gluconate, glucuronate, hexafluorophosphate, hydrobromide/bromide, hydroiodide/iodide, maleate, malonate, methylsulfate, naphthylate, nicotinate, nitrate, orotate, oxalate, palmitate and the like.

A suitable base addition salt is formed from a base which forms a pharmaceutically acceptable salt. Specific examples include aluminum, arginine, choline, diethylamine, lysine, magnesium, meglumine, potassium, and the like.

For a review on suitable salts, see "Hand book of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

As used herein, the term "ester" refers to those derived from the compounds of the various formulae in the present application, which include physiologically-hydrolyzable esters (which may be hydrolyzed under physiological conditions to release the compounds of the present invention in the form of free acids or alcohols). The compound of the present invention itself may be an ester as well.

The compound of the present invention can exist as a solvate (preferably a hydrate), wherein the compound of the present invention contains a polar solvent, in particular water, methanol or ethanol for example, as a structural element of the crystal lattice of the compound. The amount of the polar solvent, in particular water, may exist in a stoichiometric or non-stoichiometric ratio.

The metabolite of the compound of the present invention, namely a substance formed *in vivo* upon administration of the compound of the present invention, is also included within the scope of the present invention. Such a product may result e.g., from the oxidation, reduction, hydrolysis, amidation, de-amidation, esterification, de-esterification, enzymolysis, and the like, of the administered compound. Accordingly, the present invention encompasses the metabolite of the compound of the present invention, including a compound produced by a method comprising contacting the compound of the present invention with a mammal for a period of time sufficient to result in a metabolic product thereof.

Also within the scope of the present invention is a prodrug of the compound of the invention, which is certain derivative of the compound of the invention that may have little or no pharmacological activity itself, but can, when administered into or onto the body, be converted into the compound of the invention having the desired activity, for example, by hydrolytic cleavage. In general, such prodrug will be a functional derivative of the compound which is readily converted *in vivo* into the compound with desired therapeutic activity. Further information on the use of the prodrug may be found in "Pro-drugs as Novel Delivery Systems, Vol. 14, ACS Symposium Series (T. Higuchi and V. Stella) and "Bioreversible Carriers in Drug Design," Pergamon Press, 1987 (edited by E. B. Roche, American Pharmaceutical Association). The prodrug in accordance with the invention can, for example, be produced by replacing appropriate functionalities present in the compound of the present invention with certain moieties known to those skilled in the art as "pro-moieties" as described, for example, in "Design of Prodrugs" by H. Bundgaard (Elsevier, 1985).

The present invention further encompasses the compound of the present invention having a protecting group. During any of the processes for preparation of the compound of the present invention, it may be necessary and/or desirable to protect sensitive or reactive groups on any of the molecules concerned, thereby resulting in the chemically protected form of the compound of the present invention. This may be achieved by means of conventional protecting groups, e.g., those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which is incorporated herein by reference. The protecting groups may be removed at a convenient subsequent stage using methods known from the art.

As used herein, the term "about" refers to a range within ±10%, preferably within ±5%, and more preferably within ±2% of the specified value.

As used herein, the term "effective amount" refers to the amount of a compound being administered which will relieve to some extent one or more of the symptoms of the disorder being treated.

As used herein, the term "prophylaxis" refers to administering a medicament beforehand to avert or forestall the appearance of one or more symptoms of a disease or disorder. The person of ordinary skill in the medical art recognizes that the term "prophylaxis" is not an absolute term. In the medical art, it is understood to refer to the prophylactic administration of a drug to substantially diminish the likelihood or seriousness of a condition, or symptom of the condition, and this is the sense intended in this disclosure. Prophylactic measures are divided between primary prophylaxis (to prevent the development of a disease) and secondary prophylaxis (whereby the disease has already developed, and the patient is protected against worsening of this process).

Unless otherwise indicated, the term "treating" or "treatment", as used herein, means reversing, alleviating, inhibiting the progress of, or preventing the disorder or condition to which such term applies, or one or more symptoms of such disorder or condition.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g., birds, amphibians, reptiles) and mammals, such as non-human primates, livestock and/or domesticated animals (such as sheep, dog, cat, cow, pig and the like).

As used herein, the term "decreased TPK enzyme activity" means that the TPK enzyme activity in the treated subject is lower than that in a normal subject.

As used herein, the term "decreased TPK expression level" means that the TPK mRNA/DNA level or protein expression level in the treated subject is lower than that in a normal subject.

As used herein, the term "decreased TDP level" means that the TDP level in the treated subject is lower than that in a normal subject.

In some embodiments, the present invention provides a method for the prophylaxis or treatment of neurodegenerative diseases or alleviating symptoms of neurodegenerative diseases, which comprises administering to a subject in need thereof a prophylactically or therapeutically effective amount of a thiamine pyrophosphokinase (TPK) agonist.

In a preferred embodiment, the neurodegenerative disease is Alzheimer's disease.

In a more preferred embodiment, the Alzheimer's disease is one in which the subject has decreased TPK enzyme activity, decreased TPK expression level, and/or decreased TDP level.

In some embodiments, the TPK agonist is a compound of Formula (I), or a pharmaceutically acceptable salt, ester, stereoisomer, polymorph, solvate, N-oxide, isotopically labeled compound, metabolite, or prodrug thereof: 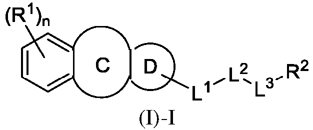 preferably, the TPK agonist is a compound of Formula (I), or a pharmaceutically acceptable salt, ester, stereoisomer, polymorph, solvate, N-oxide, isotopically labeled compound, metabolite, or prodrug thereof: 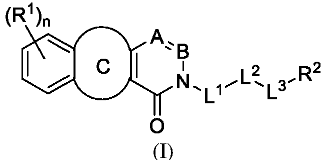 wherein:
A and B are each independently CR³ or N;
ring C and ring D are each independently C₃₋₁₀ hydrocarbon ring (e.g., C₃₋₆ hydrocarbon ring), 3- to 10-membered heterocycle, C₆₋₁₀ aromatic ring or 5- to 14-membered heteroaromatic ring; preferably, ring C is 5- to 6-membered heteroaromatic ring;
L¹, L² and L³ are each independently absent or are selected from the group consisting of -O-, -C(=O)-, -C(=O)O-, -NR-, -C(=O)NR-, -(S=O)NR-, -S(=O)₂NR-, -S-, -S(=O)-, -S(=O)₂-, -C₁₋₆ alkylene-, -C₂₋₆ alkenylene-, -C₂₋₆ alkynylene-, -C₃₋₆ cyclic hydrocarbylene-, -(3- to 10-membered heterocyclylene)-, -C₆₋₁₀ arylene-, -(5- to 14-membered heteroarylene)-, -W-C₁₋₆ alkylene-, -C₁₋₆ alkylene-W- and -W-C₁₋₆ alkylene-W'-, wherein the alkylene group is optionally further interrupted by one or more W; provided that at least one of L¹, L² and L³ is present;
W and W', at each occurrence, are each independently selected from the group consisting of -O-, -C(=O)-, -C(=O)O-, -NR-, -C(=O)NR-, -(S=O)NR-, -S(=O)₂NR-, -S-, -S(=O)- and -S(=O)₂-;
R¹, at each occurrence, is each independently selected from the group consisting of halogen, -OH, -NH₂, -CN, -NO₂, C₁₋₆ alkyl, haloC₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-OR^{a}, -C₁₋₆ alkylene-NR^{a}R^{b} and -O-C₁₋₆ alkylene-NR^{a}R^{b};
R² and R³, at each occurrence, are each independently selected from the group consisting of H, halogen, -OH, -NH₂, -CN, -NO₂, C₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-OR^{a}, -C₁₋₆ alkylene-NR^{a}R^{b} and -O-C₁₋₆ alkylene-NR^{a}R^{b};
R, R^{a} and R^{b}, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl;
n is 0, 1, 2, 3 or 4; preferably, n is 0, 1 or 2;
the above alkyl, alkylene, alkenyl, alkenylene, alkynyl, alkynylene, cyclic hydrocarbyl, cyclic hydrocarbylene, hydrocarbon ring, heterocyclyl, heterocyclylene, heterocycle, aryl, arylene, aromatic ring, heteroaryl, heteroarylene, heteroaromatic ring and aralkyl, at each occurrence, are each optionally substituted with one or more substituents independently selected from the group consisting of: halogen, -OH, =O, -NH₂, -CN, -NO₂, C₁₋₆ alkyl, haloC₁₋₆ alkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{c}, -OC(=O)R^{c}, -C(=O)OR^{c}, -OR^{c}, -SR^{c}, -S(=O)R^{c}, -S(=O)₂R^{c}, -S(=O)₂NR^{c}R^{d}, -NR^{c}R^{d}, -C(=O)NR^{c}R^{d}, -NR^{c}-C(=O)R^{d}, -NR^{c}-C(=O)OR^{d}, -NR^{c}-S(=O)₂-R^{d}, -NR^{c}-C(=O)-NR^{c}R^{d}, -C₁₋₆ alkylene-OR^{c}, -C₁₋₆ alkylene-NR^{c}R^{d} and -O-C₁₋₆ alkylene-NR^{c}R^{d}, the alkyl, alkylene, cyclic hydrocarbyl, heterocyclyl, aryl, heteroaryl and aralkyl are further optionally substituted with one or more substituents independently selected from the group consisting of: halogen, -OH, =O, -C(=O)O-*tert*-butyl, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -O-C₁₋₆ alkyl and -C₁₋₆ alkylene-O-C₁₋₆ alkyl; and
R^{c} and R^{d}, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl, the alkyl, cyclic hydrocarbyl, heterocyclyl, aryl, heteroaryl and aralkyl are further optionally substituted with one or more substituents independently selected from the group consisting of: halogen, -OH, =O, -C(=O)O-*tert*-butyl, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl and -C₁₋₆ alkylene-O-C₁₋₆ alkyl.

In some embodiments, ring C is C₃₋₆ hydrocarbon ring, 3- to 10-membered heterocycle, C₆₋₁₀ aromatic ring or 5- to 14-membered heteroaromatic ring.

In preferred embodiments, the TPK agonist is a compound of Formula (II), or a pharmaceutically acceptable salt, ester, stereoisomer, polymorph, solvate, N-oxide, isotopically labeled compound, metabolite, or prodrug thereof: 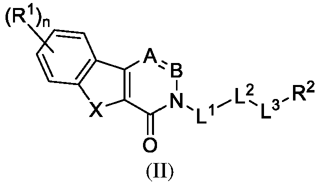 wherein:
X is -C(R)₂-, -NR-, -O- or -S-; preferably, X is -NR-, -O- or -S-; more preferably, X is -NH-, -N(CH₃)-, -O- or -S-; and
each of the remaining groups is as defined above.

In preferred embodiments, 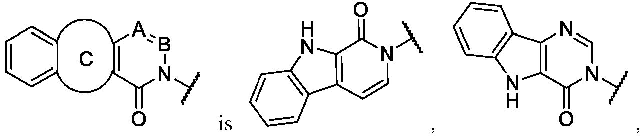 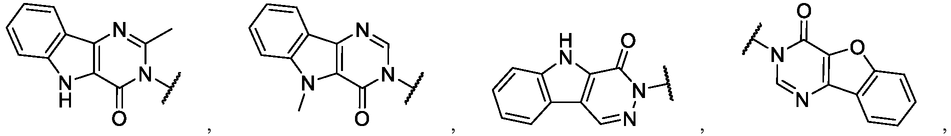 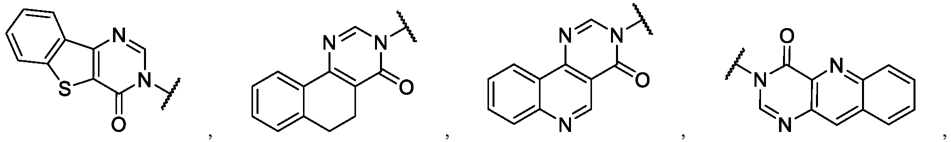 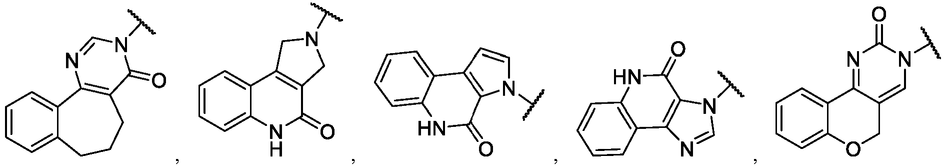 or

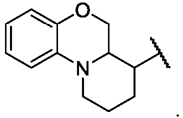

In preferred embodiments, 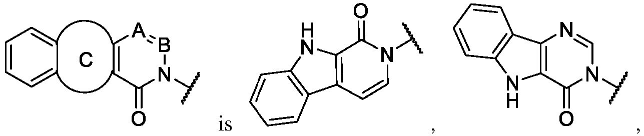 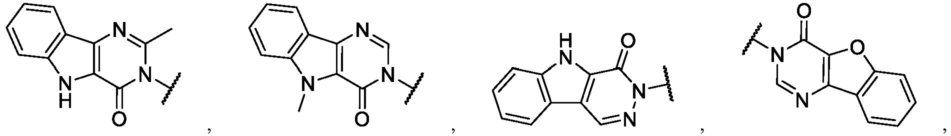 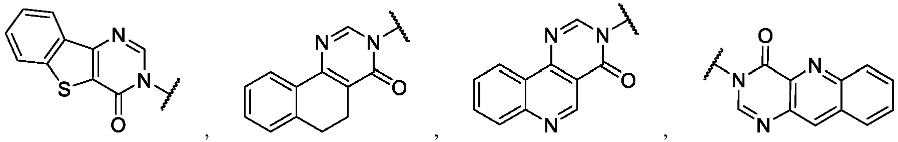 or

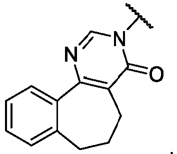

In preferred embodiments,

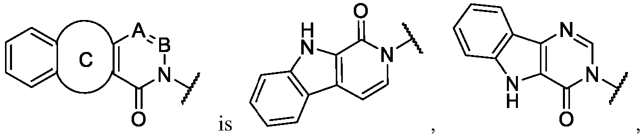

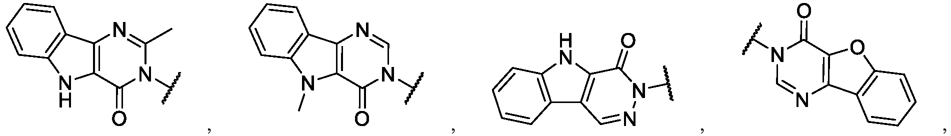

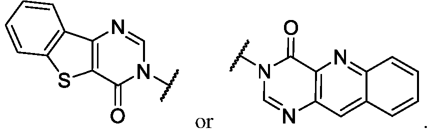

In preferred embodiments, R³, at each occurrence, is each independently H or C₁₋₆ alkyl (preferably methyl);
preferably, A and B are each independently CH, CCH₃ or N; and
more preferably, A is N and B is CH.

In preferred embodiments, L¹ and L³ are each independently absent or are -C(=O)-, -N(CH₃)-, -C₁₋₆ alkylene-, -W-C₁₋₆ alkylene- or -C₁₋₆ alkylene-W-, the alkylene group is optionally further interrupted by one or more W, and
W is -O-, -C(=O)-, -C(=O)O-, -NH-, -N(CH₃)-, -C(=O)NH- or -C(=O)N(CH₃)-.

In preferred embodiments, L¹ and L³ are each independently absent or are -C(=O)-, -N(CH₃)-, -C₁₋₆ alkylene- or -C₁₋₆ alkylene-W-, the alkylene group is optionally further interrupted by one or more W, and W is -O-, -C(=O)-, -C(=O)O-, -NH-, -N(CH₃)-, -C(=O)NH- or -C(=O)N(CH₃)-.

In preferred embodiments, L² is absent or is -(3- to 10-membered heterocyclylene)-;
preferably, L² is absent or is piperazinylene or piperidinylene.

In preferred embodiments, 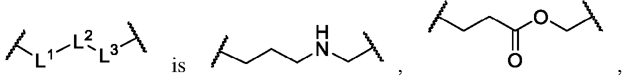 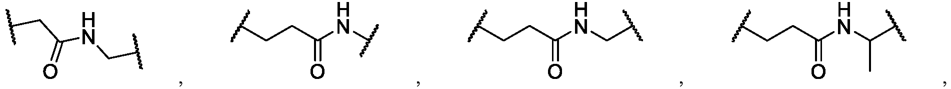 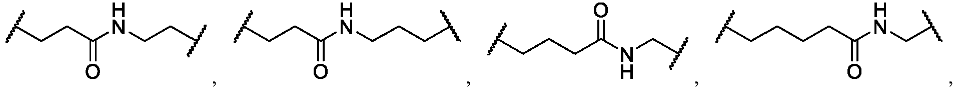 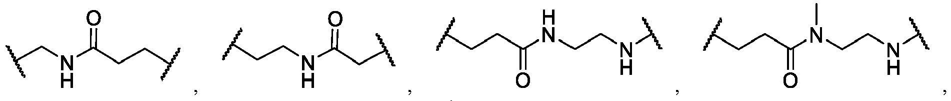 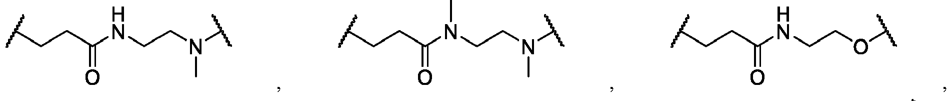 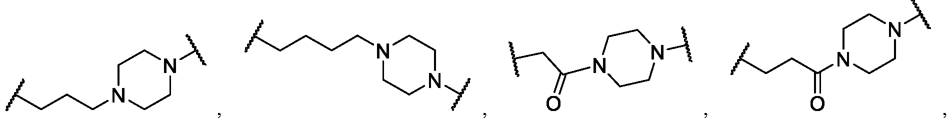 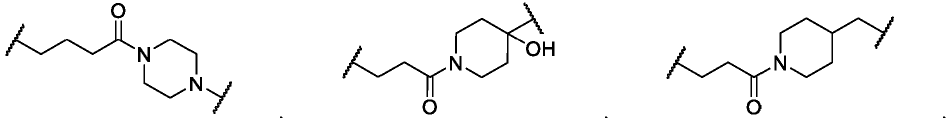 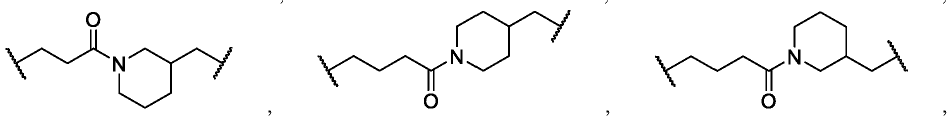 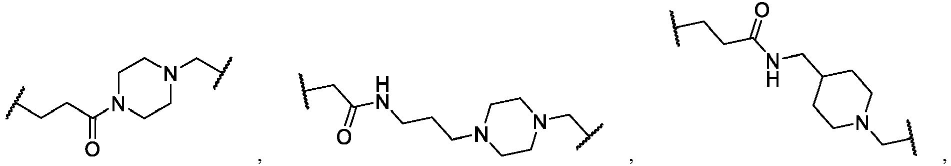 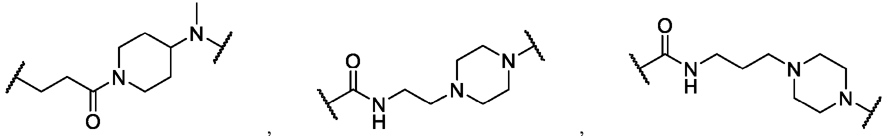 or

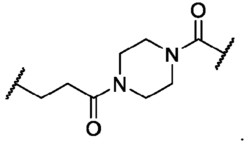

In preferred embodiments,

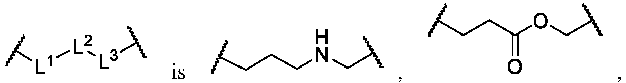

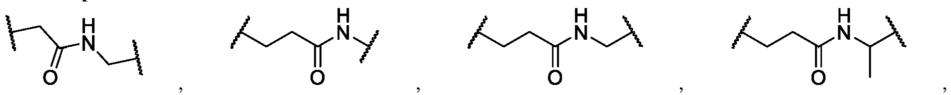

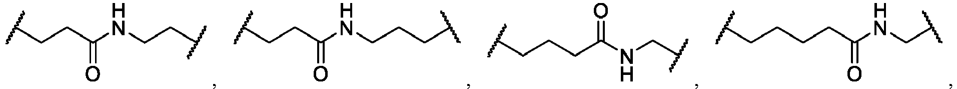

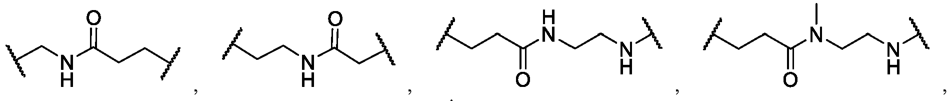

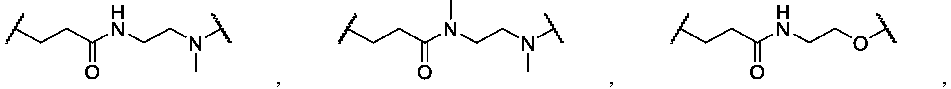

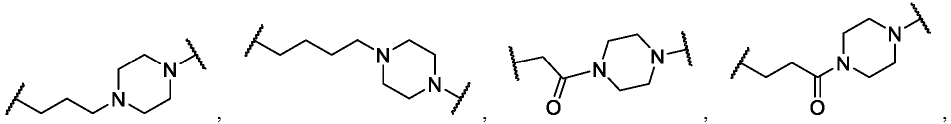

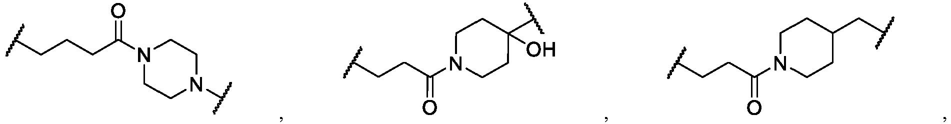

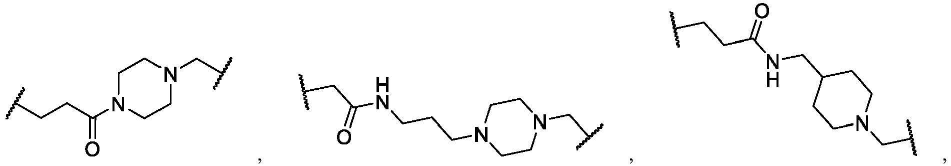

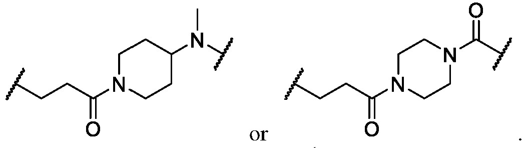

In preferred embodiments, R¹, at each occurrence, is each independently selected from the group consisting of halogen, -CN, C₁₋₆ alkyl, haloC₁₋₆ alkyl and C₁₋₆ alkoxy;
preferably, R¹, at each occurrence, is each independently selected from the group consisting of -F, -Cl, -Br, -CN, -CH₃, -CF₃ and -OCH₃.

In preferred embodiments, R² is independently selected from the group consisting of H, C₁₋₆ alkyl, C₆₋₁₀ aryl and 5- to 14-membered heteroaryl; the groups are optionally substituted with one or more substituents independently selected from the group consisting of: halogen, -OH, -NH₂, -CN, -NO₂, C₁₋₆ alkyl, haloC₁₋₆ alkyl, -N(C₁₋₆ alkyl)₂, C₁₋₆ alkoxy, haloC₁₋₆ alkoxy, -C(=O)-C₁₋₆ alkyl, -C(=O)OH, -C(=O)O-C₁₋₆ alkyl, -S-C₁₋₆ alkyl, -S(=O)₂-C₁₋₆ alkyl, -S(-O)₂-(3- to 10-membered heterocyclyl), -S(=O)₂NH₂ and -C(=O)NH₂;
preferably, R² is independently selected from the group consisting of isopropyl,

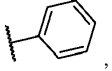

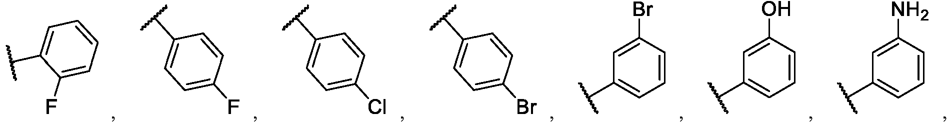

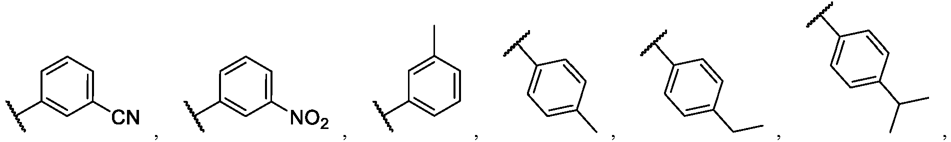

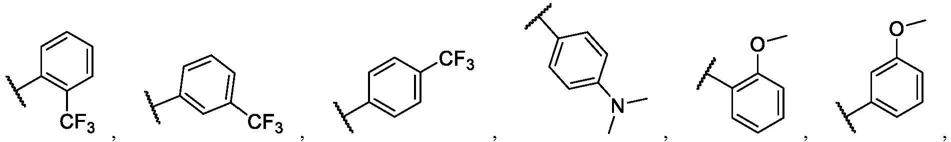

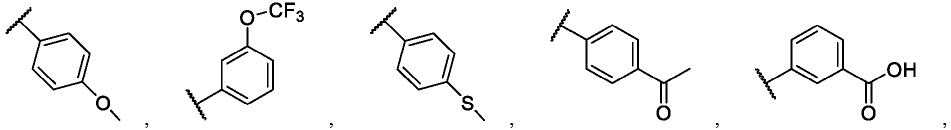

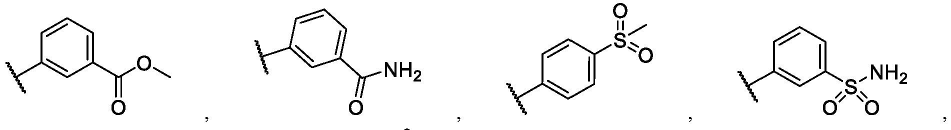

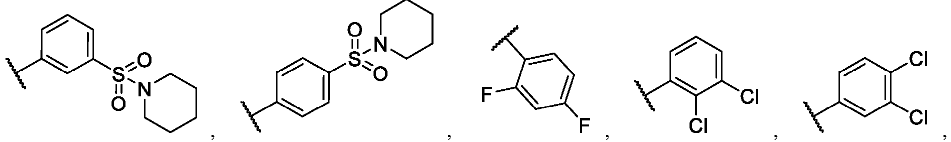

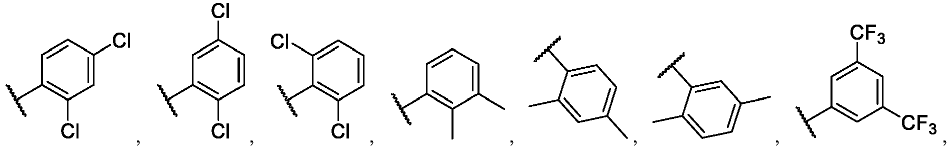

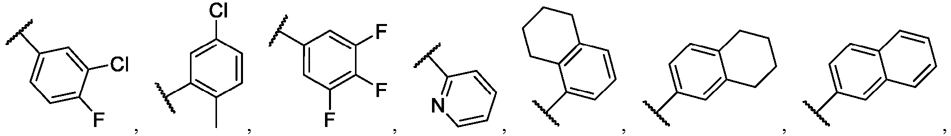

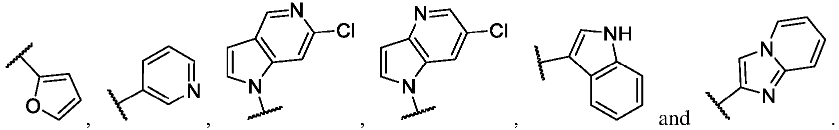

In preferred embodiments, the TPK agonist is a compound of Formula (III), or a pharmaceutically acceptable salt, ester, stereoisomer, polymorph, solvate, N-oxide, isotopically labeled compound, metabolite, or prodrug thereof: 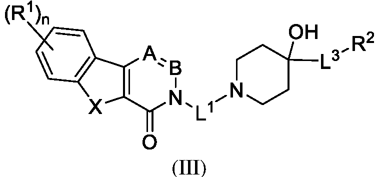 wherein each group is as defined above.

In some embodiments, the TPK agonist is a compound of Formula (IV), or a pharmaceutically acceptable salt, ester, stereoisomer, polymorph, solvate, N-oxide, isotopically labeled compound, metabolite, or prodrug thereof: 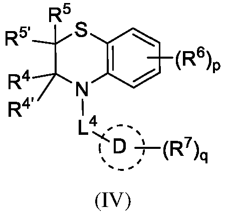 wherein:
ring D is absent or is C₃₋₆ hydrocarbon ring, 3- to 10-membered heterocycle, C₆₋₁₀ aromatic ring or 5-to 14-membered heteroaromatic ring;
L⁴ is selected from the group consisting of -O-, -C(=O)-, -C(=O)O-, -NR'-, -C(=O)NR'-, -(S=O)NR'-, -S(=O)₂NR'-, -S-, -S(=O)-, -S(=O)₂-, -C₁₋₆ alkylene-, -C₂₋₆ alkenylene-, -C₂₋₆ alkynylene-, -C₃₋₆ cyclic hydrocarbylene-, -(3- to 10-membered heterocyclylene)-, -C₆₋₁₀ arylene-, -(5- to 14-membered heteroarylene)-, -U-C₁₋₆ alkylene-, -C₁₋₆ alkylene-U-, -U-C₁₋₆ alkylene-U'- and -C₁₋₆ alkylene-U-C₁₋₆ alkylene-, wherein the alkylene group is optionally further interrupted by one or more U;
U and U', at each occurrence, are each independently selected from the group consisting of -O-, -C(=O)-, -C(=O)O-, -NR'-, -C(=O)NR'-, -(S=O)NR'-, -S(=O)₂NR'-, -S-, -S(=O)- and -S(=O)₂-;
R⁴, R^{4'}, R⁵, R^{5'}, R⁶ and R⁷, at each occurrence, are each independently selected from the group consisting of H, halogen, -OH, -NH₂, -CN, -NO₂, C₁₋₆ alkyl, haloC₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{e}, -OC(=O)R^{e}, -C(=O)OR^{e}, -OR^{e}, -SR^{e}, -S(=O)R^{e}, -S(-O)₂R^{e}, -S(=O)₂NR^{e}R^{f}, -NR^{e}R^{f}, -C(=O)NR^{e}R^{f}, -NR^{e}-C(=O)R^{f}, -NR^{e}-C(=O)OR^{f}, -NR^{e}-S(=O)₂-R^{f}, -NR^{e}-C(=O)-NR^{e}R^{f}, -C₁₋₆ alkylene-OR^{e}, -C₁₋₆ alkylene-NR^{e}R^{f}, -O-C₁₋₆ alkylene-NR^{e}R^{f} and -C₁₋₆ alkylene-OC(=O)-C₁₋₆ alkylene-C(=O)OR^{e}; or, R⁴ and R^{4'} or R⁵ and R^{5'} together form =O; or, R⁴, R^{4'}, R⁵, R^{5'} together with the group to which they are attached form C₃₋₆ hydrocarbon ring, 3- to 10-membered heterocycle, C₆₋₁₀ aromatic ring or 5- to 14-membered heteroaromatic ring;
R', R^{e} and R^{f}, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl;
p and q are each independently 1, 2, 3 or 4; preferably, p and q are each independently 1 or 2; provided that when ring D is absent, q is 1;
the above alkyl, alkylene, alkenyl, alkenylene, alkynyl, alkynylene, cyclic hydrocarbyl, cyclic hydrocarbylene, hydrocarbon ring, heterocyclyl, heterocyclylene, heterocycle, aryl, arylene, aromatic ring, heteroaryl, heteroarylene, heteroaromatic ring and aralkyl, at each occurrence, are each optionally substituted with one or more substituents independently selected from the group consisting of: halogen, -OH, =O, -NH₂, -CN, -NO₂, C₁₋₆ alkyl, haloC₁₋₆ alkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{g}, -OC(=O)R^{g}, -C(=O)OR^{g}, -OR^{g}, -SR^{g}, -S(=O)R^{g}, -S(=O)₂R³, -S(=O)₂NR^{g}R^{h}, -NR^{g}R^{h}, -C(=O)NR^{g}R^{h}, -NR^{g}-C(=O)R^{h}, -NR^{g}-C(=O)OR^{h}, -NR^{g}-S(=O)₂-R^{h}, -NR^{g}-C(=O)-NR^{g}R^{h}, -C₁₋₆ alkylene-OR^{g}, -C₁₋₆ alkylene-NR^{g}R^{h} and -O-C₁₋₆ alkylene-NR^{g}R^{h}, the alkyl, alkylene, cyclic hydrocarbyl, heterocyclyl, aryl, heteroaryl and aralkyl are further optionally substituted with one or more substituents independently selected from the group consisting of: halogen, -OH, =O, -C(=O)O-tert-butyl, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -O-C₁₋₆ alkyl and -C₁₋₆ alkylene-O-C₁₋₆ alkyl; and
R^{g} and R^{h}, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl, the alkyl, cyclic hydrocarbyl, heterocyclyl, aryl, heteroaryl and aralkyl are further optionally substituted with one or more substituents independently selected from the group consisting of: halogen, -OH, =O, -C(=O)O-tert-butyl, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl and -C₁₋₆ alkylene-O-C₁₋₆ alkyl.

In preferred embodiments, the TPK agonist is a compound of Formula (V), or a pharmaceutically acceptable salt, ester, stereoisomer, polymorph, solvate, N-oxide, isotopically labeled compound, metabolite, or prodrug thereof: 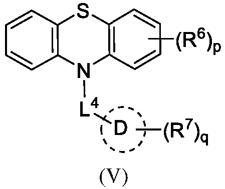 wherein each group is as defined above.

In preferred embodiments, the L⁴ is selected from the group consisting of -(CH₂)₂-, -(CH₂)₃-, -(CH₂)₆-, -(CH₂)₃-NH-, -(CH₂)₃-O-, -(CH₂)₄-O-, -(CH₂)₅-O-, -(CH₂)₆-O-, -C(=O)-CH₂-, -C(=O)-(CH₂)₂-, -(CH₂)₂-C(=O)NH-(CH₂)₂- and -CH₂-CH(OH)-CH₂-NH-.

In preferred embodiments, 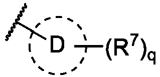 is -CN, -NH₂,

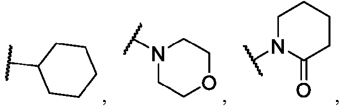

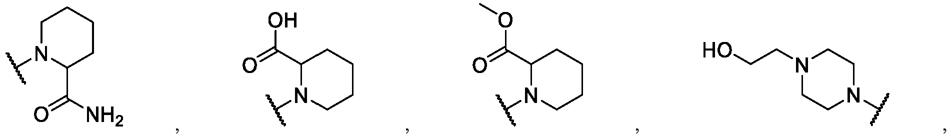

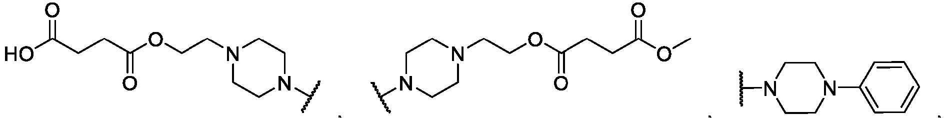

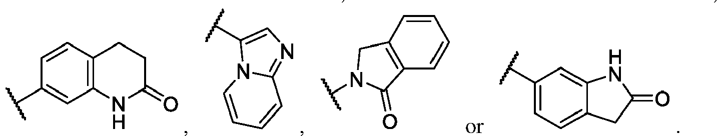

In some embodiments, the TPK agonist is selected from the group consisting of:

| **No.** | **Structure** |
|---|---|
| **1** | 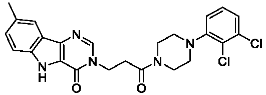 |
| **2** | 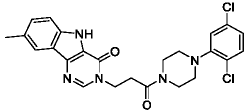 |
| **3** | 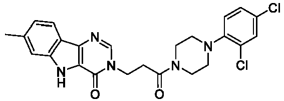 |
| **4** | 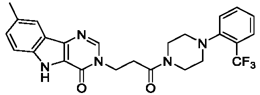 |
| **5** | 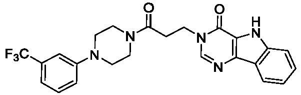 |
| **6** | 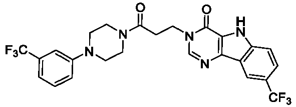 |
| **7** | 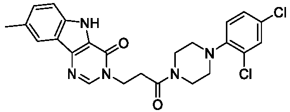 |
| **8** | 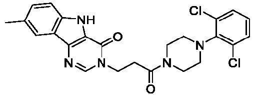 |
| **9** | 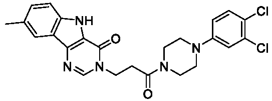 |
| **10** | 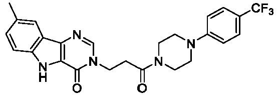 |
| **11** | 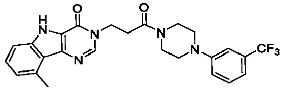 |
| **12** | 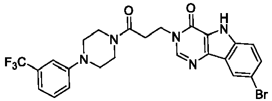 |
| **13** | 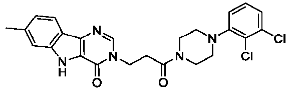 |
| **14** | 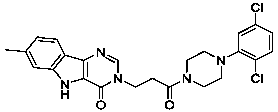 |
| **15** | 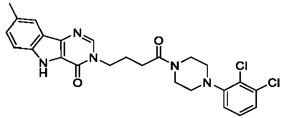 |
| **16** | 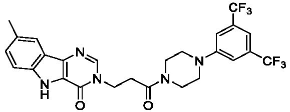 |
| **17** | 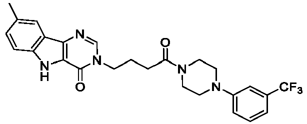 |
| **18** | 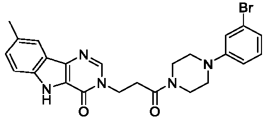 |
| **19** | 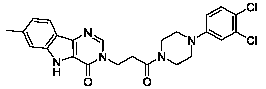 |
| **20** | 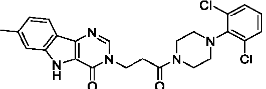 |
| **22** | 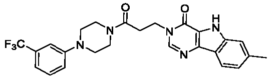 |
| **23** | 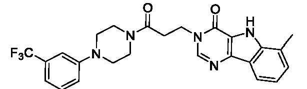 |
| **24** | 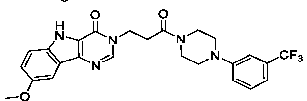 |
| **25** | 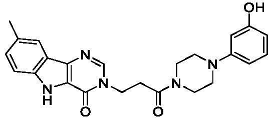 |
| **26** | 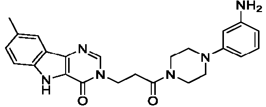 |
| **27** | 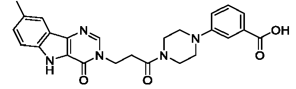 |
| **28** | 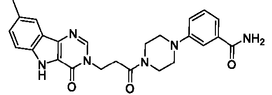 |
| **29** | 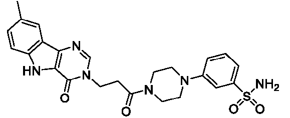 |
| **30** | 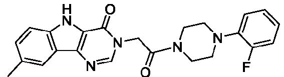 |
| **31** | 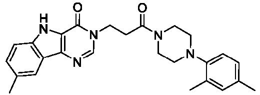 |
| **32** | 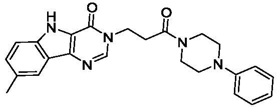 |
| **33** | 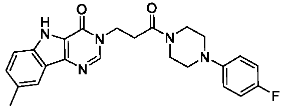 |
| **34** | 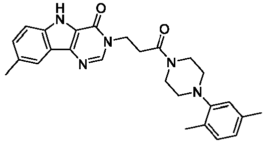 |
| **35** | 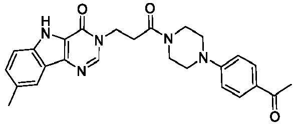 |
| **36** | 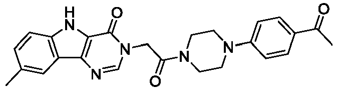 |
| **37** | 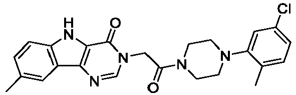 |
| **38** | 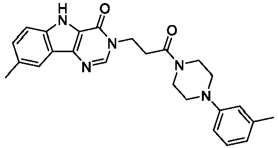 |
| **39** | 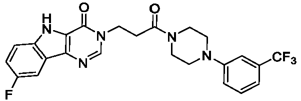 |
| **40** | 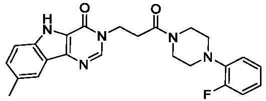 |
| **41** | 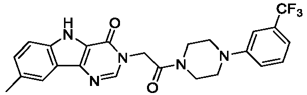 |
| **42** | 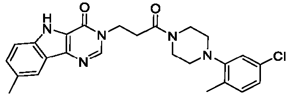 |
| **43** | 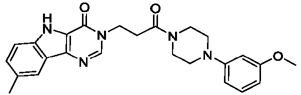 |
| **44** | 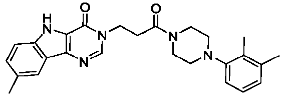 |
| **45** | 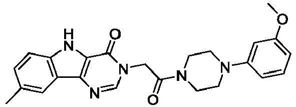 |
| **46** | 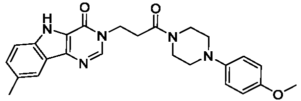 |
| **47** | 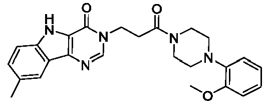 |
| **48** | 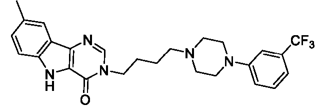 |
| **49** | 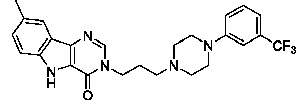 |
| **50** | 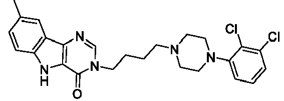 |
| **51** | 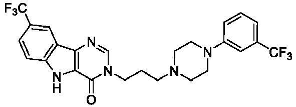 |
| **52** | 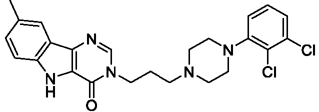 |
| **53** | 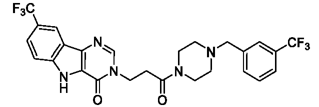 |
| **54** | 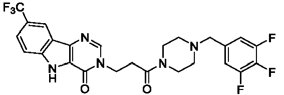 |
| **55** | 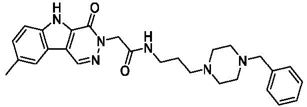 |
| **56** | 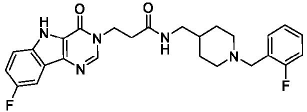 |
| **57** | 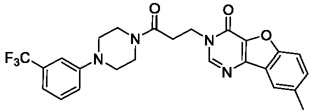 |
| **58** | 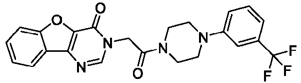 |
| **59** | 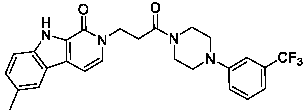 |
| **60** | 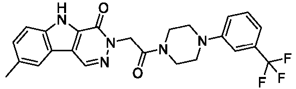 |
| **61** | 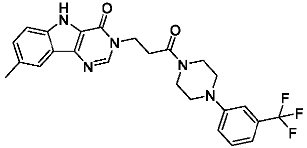 |
| **62** | 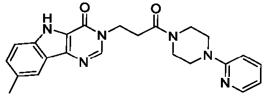 |
| **63** | 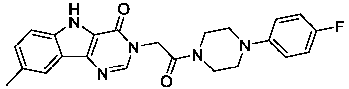 |
| **64** | 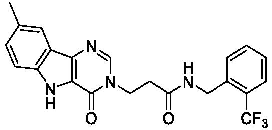 |
| **65** | 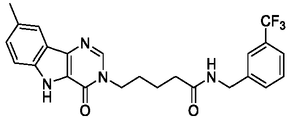 |
| **66** | 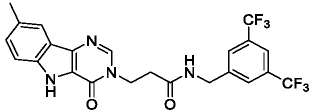 |
| **67** | 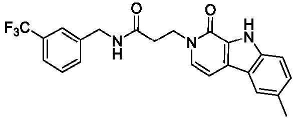 |
| **68** | 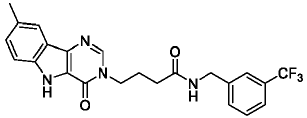 |
| **69** | 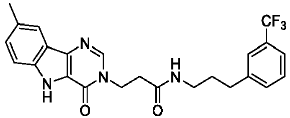 |
| **70** | 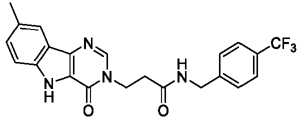 |
| **71** | 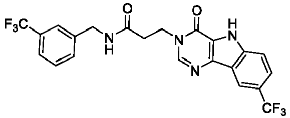 |
| **72** | 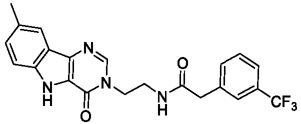 |
| **73** | 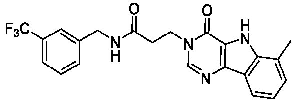 |
| **74** | 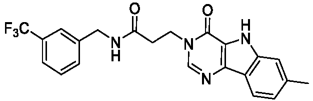 |
| **75** | 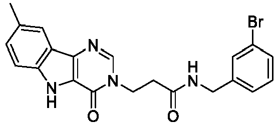 |
| **76** | 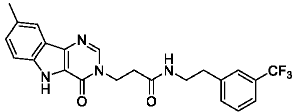 |
| **77** | 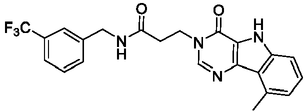 |
| **78** | 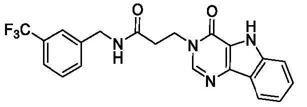 |
| **79** | 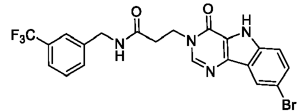 |
| **80** | 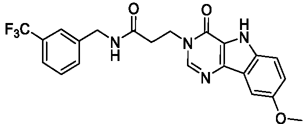 |
| **81** | 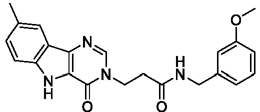 |
| **82** | 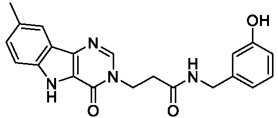 |
| **83** | 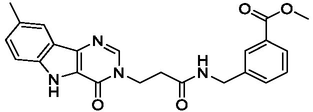 |
| **84** | 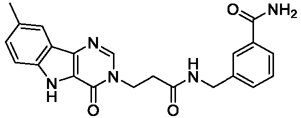 |
| **85** | 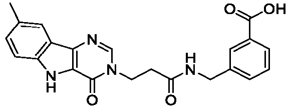 |
| **86** | 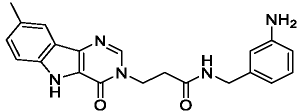 |
| **87** | 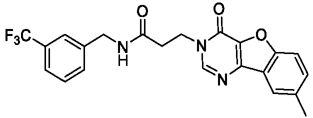 |
| **88** | 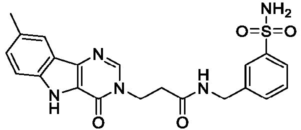 |
| **89** | 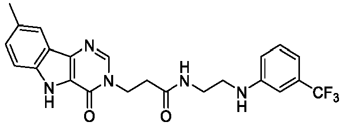 |
| **90** | 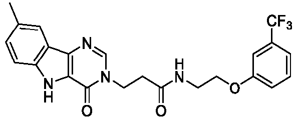 |
| **91** | 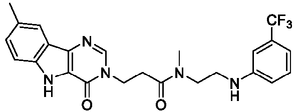 |
| **92** | 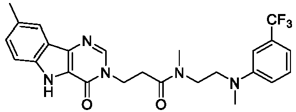 |
| **93** | 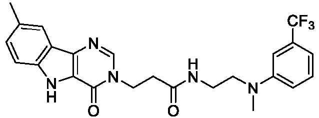 |
| **94** | 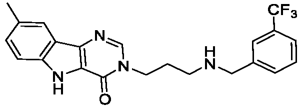 |
| **95** | 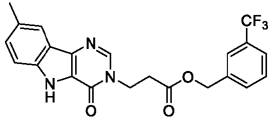 |
| **96** | 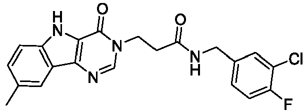 |
| **97** | 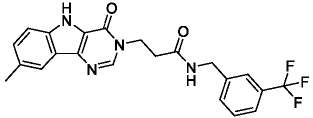 |
| **98** | 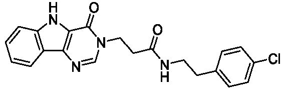 |
| **99** | 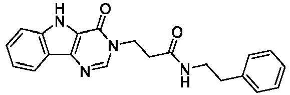 |
| **100** | 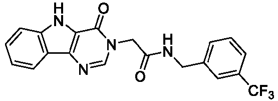 |
| **101** | 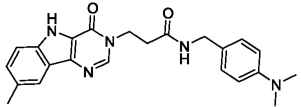 |
| **102** | 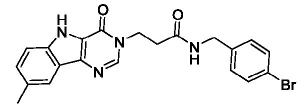 |
| **103** | 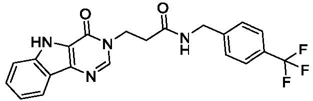 |
| **104** | 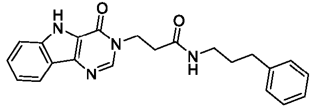 |
| **105** | 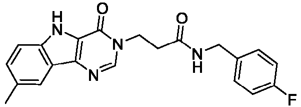 |
| **106** | 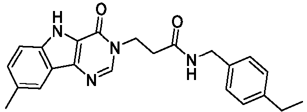 |
| **107** | 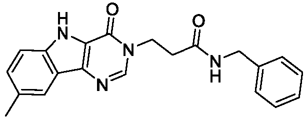 |
| **108** | 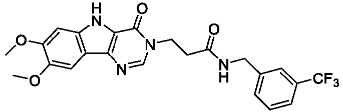 |
| **109** | 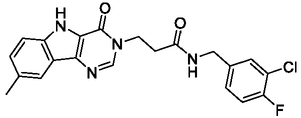 |
| **110** | 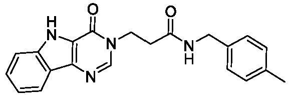 |
| **111** | 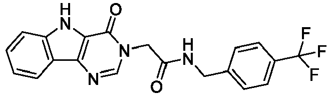 |
| **112** | 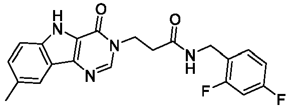 |
| **113** | 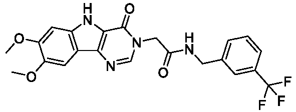 |
| **114** | 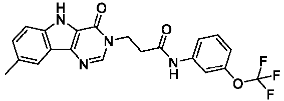 |
| **115** | 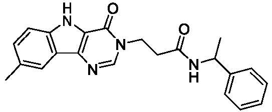 |
| **116** | 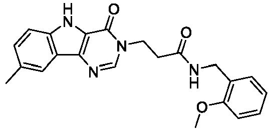 |
| **117** | 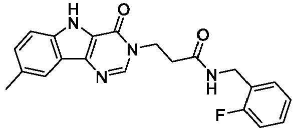 |
| **118** | 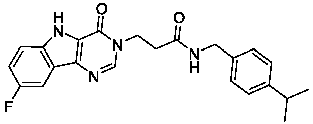 |
| **119** | 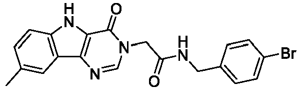 |
| **120** | 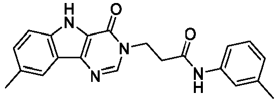 |
| **121** | 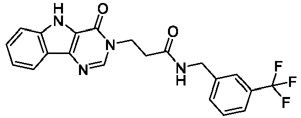 |
| **122** | 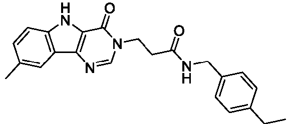 |
| **123** | 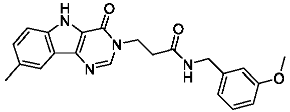 |
| **124** | 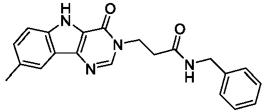 |
| **125** | 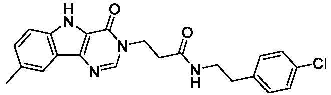 |
| **126** | 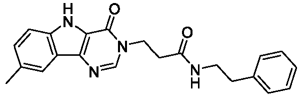 |
| **127** | 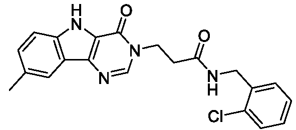 |
| **128** | 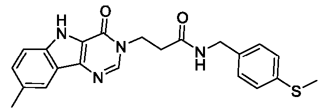 |
| **129** | 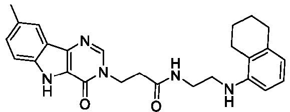 |
| **130** | 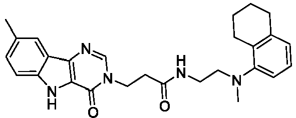 |
| **131** | 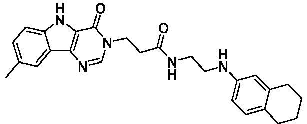 |
| **132** | 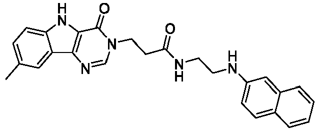 |
| **133** | 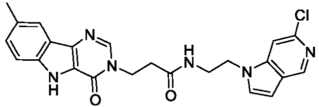 |
| **134** | 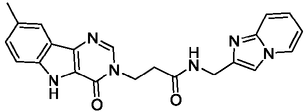 |
| **135** | 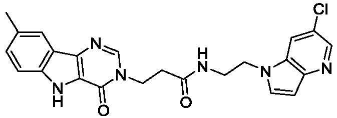 |
| **136** | 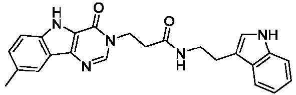 |
| **137** | 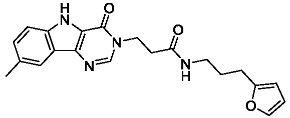 |
| **138** | 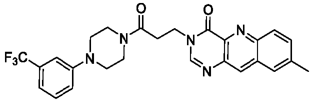 |
| **139** | 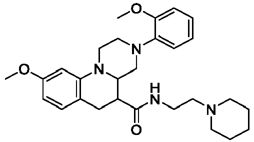 |
| **140** | 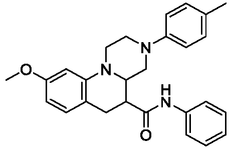 |
| **141** | 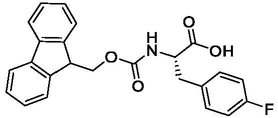 |
| **142** | 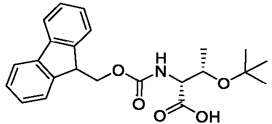 |
| **143** | 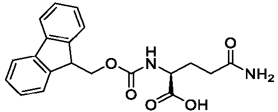 |
| **144** | 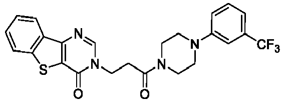 |
| **145** | 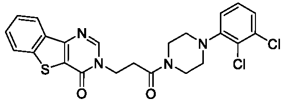 |
| **146** | 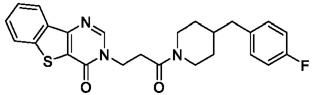 |
| **147** | 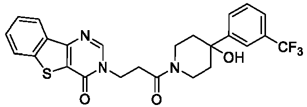 |
| **148** | 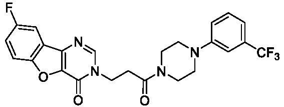 |
| **149** | 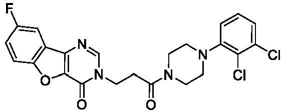 |
| **150** | 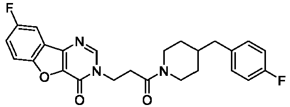 |
| **151** | 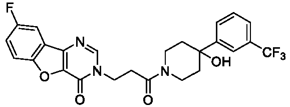 |
| **152** | 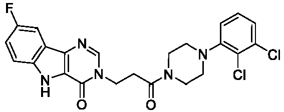 |
| **153** | 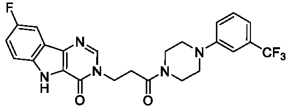 |
| **154** | 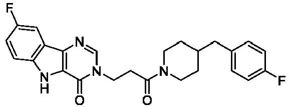 |
| **155** | 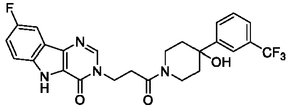 |
| **156** | 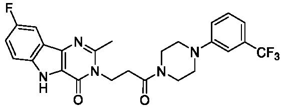 |
| **157** | 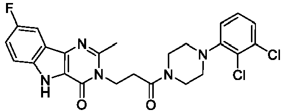 |
| **158** | 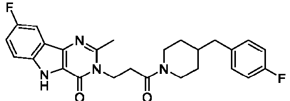 |
| **159** | 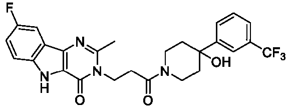 |
| **160** | 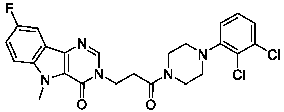 |
| **161** | 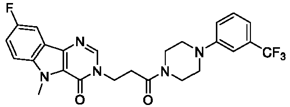 |
| **162** | 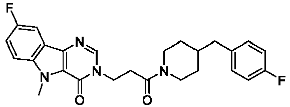 |
| **163** | 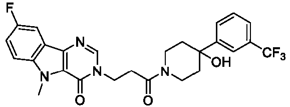 |
| **164** | 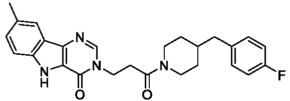 |
| **165** | 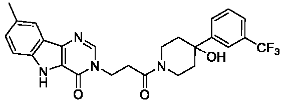 |
| **166** | 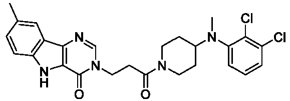 |
| **167** | 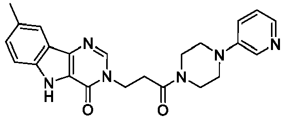 |
| **168** | 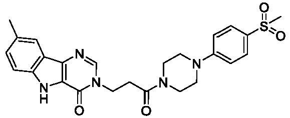 |
| **169** | 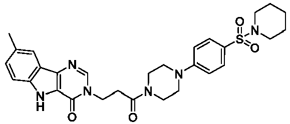 |
| **170** | 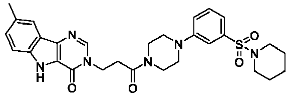 |
| **171** | 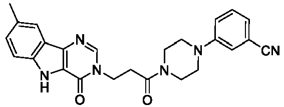 |
| **172** | 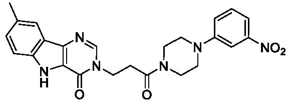 |
| **173** | 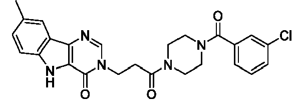 |
| **174** | 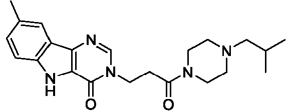 |
| **175** | 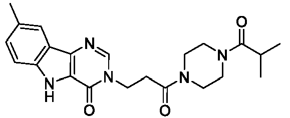 |
| **176** | 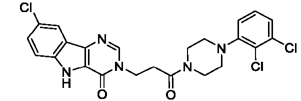 |
| **177** | 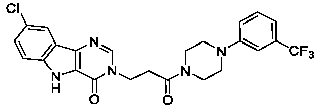 |
| **178** | 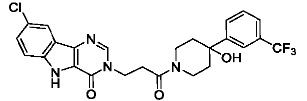 |
| **179** | 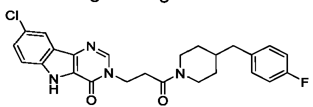 |
| **180** | 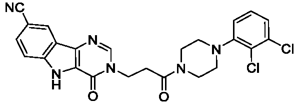 |
| **181** | 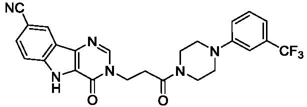 |
| **182** | 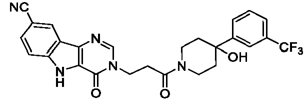 |
| **183** | 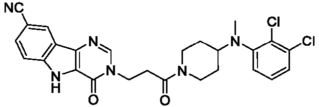 |
| **184** | 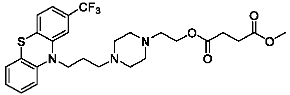 |
| **185** | 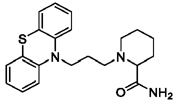 |
| **186** | 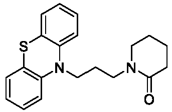 |
| **187** | 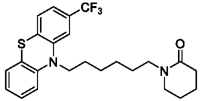 |
| **188** | 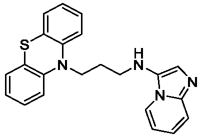 |
| **189** | 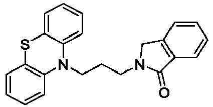 |
| **190** | 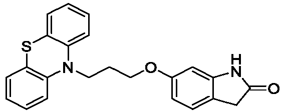 |
| **191** | 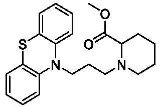 |
| **192** | 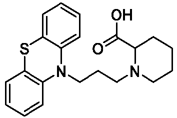 |
| **193** | 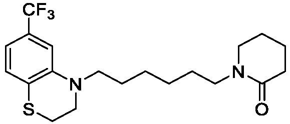 |
| **194** | 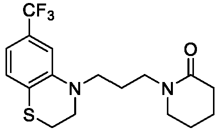 |
| **195** | 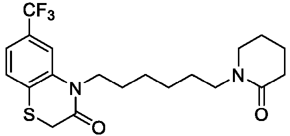 |
| **196** | 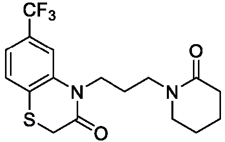 |
| **197** | 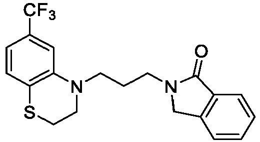 |
| **198** | 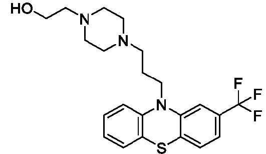 |
| **199** | 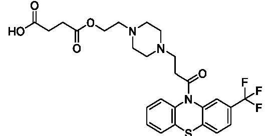 |
| **200** | 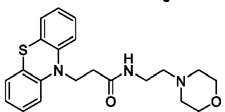 |
| **201** | 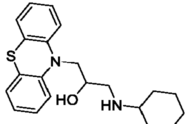 |
| **202** | 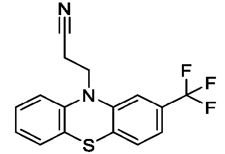 |
| **203** | 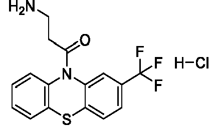 |
| **204** | 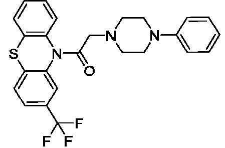 |
| **205** | 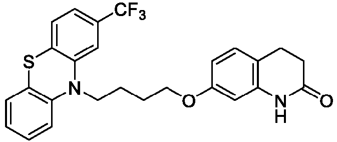 |
| **206** | 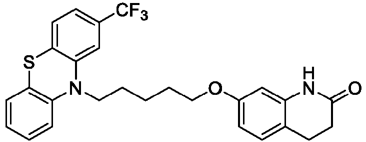 |
| **207** | 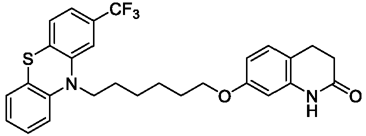 |
| **208** | 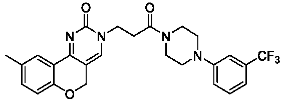 |
| **209** | 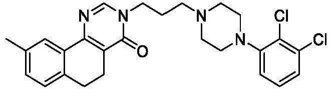 |
| **210** | 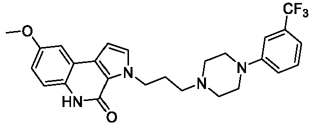 |
| **211** | 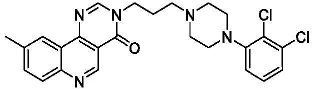 |
| **212** | 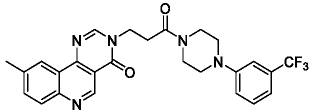 |
| **213** | 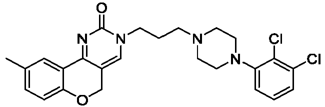 |
| **215** | 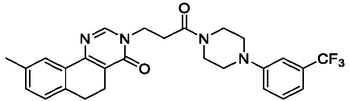 |
| **216** | 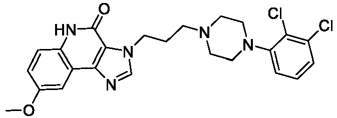 |
| **217** | 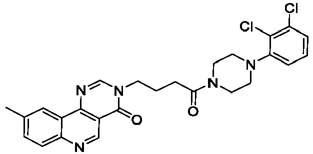 |
| **218** | 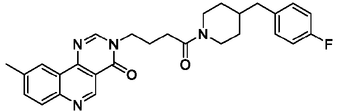 |
| **219** | 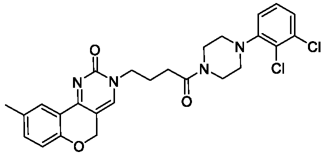 |
| **220** | 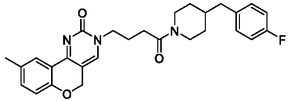 |
| **221** | 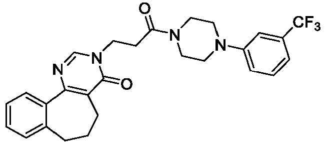 |
| **222** | 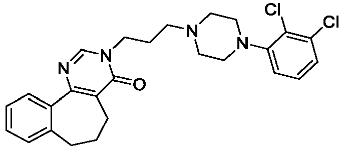 |
| **223** | 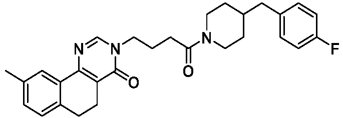 |
| **224** | 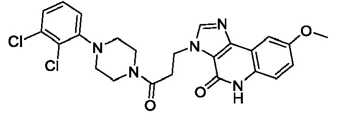 |
| **225** | 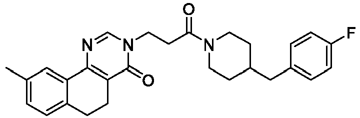 |
| **226** | 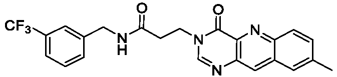 |
| **227** | 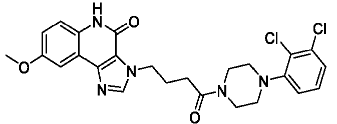 |
| **228** | 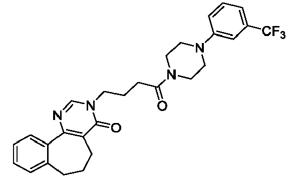 |
| **229** | 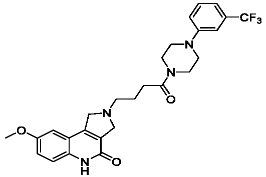 |
| **230** | 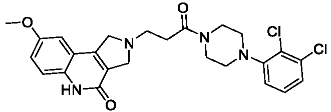 |
| **231** | 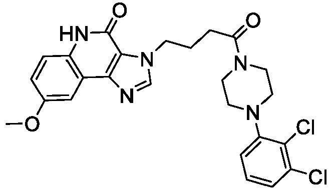 |
| **232** | 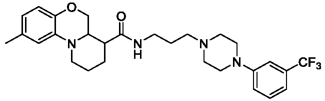 |
| **233** | 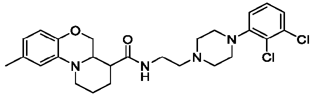 |
| **234** | 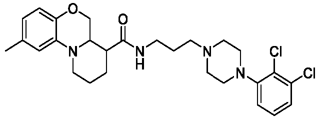 |
| **235** | 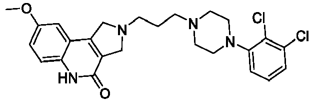 |

In preferred embodiments, the TPK agonist is administered in an amount of about 0.005 mg/day to about 5000 mg/day, e.g., in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

In preferred embodiments, the TPK agonist is administered in an amount of about 1 ng/kg to about 200 mg/kg, about 1 µg/kg to about 100 mg/kg or about 1 mg/kg to about 50 mg/kg body weight per day, e.g., is administered in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg or about 300 mg/kg body weight per day.

In preferred embodiments, the daily dose of the TPK agonist is administered at one time or is administered in two, three or four doses.

In preferred embodiments, the TPK agonist is administered continuously for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days or at least 50 days.

In preferred embodiments, the TPK agonist is administered for one or more (e.g., 1, 2, 3, 4, 5, 6, 7, 8, 9 or 10) courses of treatment, wherein each course of treatment lasts for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days or at least 50 days; and the interval between every two courses of treatment is 0, 1, 2, 3, 4, 5, 6, 7, 8, 9, 10 days, two weeks, three weeks, or four weeks.

In preferred embodiments, the TPK agonist is administered through injection (e.g., intravenous, intraarterial, subcutaneous, intraperitoneal, intramuscular injection, including dripping), or transdermal administration, or is administered via oral, buccal, nasal, transmucosal, or topical route, as an ophthalmic formulation, or via inhalation.

In preferred embodiments, the TPK agonist is administered in a dosage form selected from the group consisting of tablet, capsule, lozenge, hard candy, powder, spray, cream, salve, suppository, gel, paste, lotion, ointment, aqueous suspensions, injectable solution, elixir, and syrup.

In preferred embodiments, the method improves the following pathophysiological manifestations in the subject: abnormal cognitive behavior, neurodegenerative changes (e.g., progressive synaptic/neuronal loss and brain atrophy), β-amyloid deposition, abnormal phosphorylation of Tau and the resulting neurofibrillary tangles, glial cell activation and inflammation, and/or impaired cerebral glucose metabolism.

In preferred embodiments, the present disclosure further comprises administering one or more additional therapeutic agents.

In some embodiments, the present disclosure provides a compound, or a pharmaceutically acceptable salt, ester, stereoisomer, polymorph, solvate, N-oxide, isotopically labeled compound, metabolite, or prodrug thereof, the compound has the structure of Formula (III): 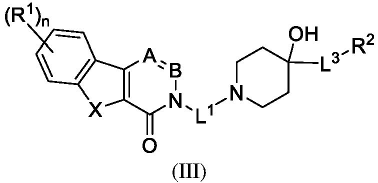
wherein each group is as defined above;
provided that -L³-R² is not H and methyl.

The present invention encompasses any combination of the above embodiments.

In some embodiments, the present disclosure provides a compound, or a pharmaceutically acceptable salt, ester, stereoisomer, polymorph, solvate, N-oxide, isotopically labeled compound, metabolite, or prodrug thereof, wherein the compound is selected from the group consisting of:

| **No.** | **Structure** |
|---|---|
| **1** | 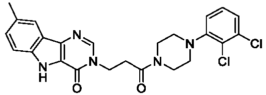 |
| **2** | 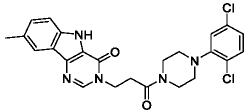 |
| **3** | 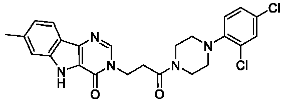 |
| **4** | 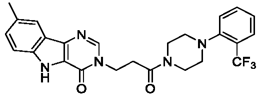 |
| **5** | 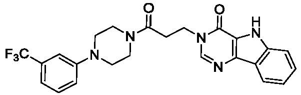 |
| **6** | 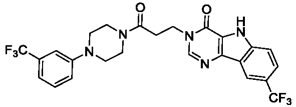 |
| **7** | 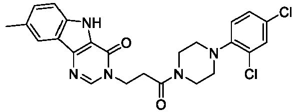 |
| **8** | 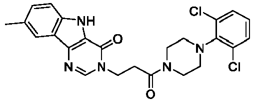 |
| **9** | 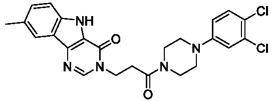 |
| **10** | 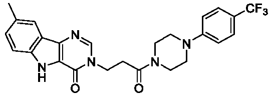 |
| **11** | 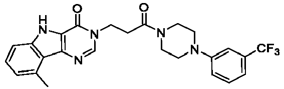 |
| **12** | 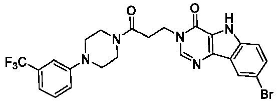 |
| **13** | 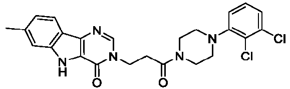 |
| **14** | 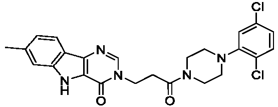 |
| **15** | 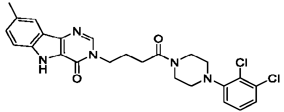 |
| **16** | 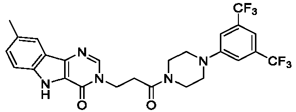 |
| **17** | 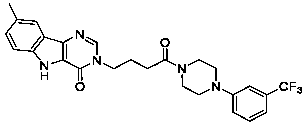 |
| **18** | 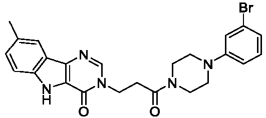 |
| **19** | 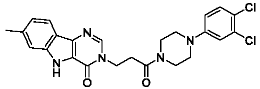 |
| **20** | 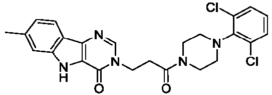 |
| **22** | 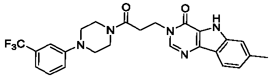 |
| **23** | 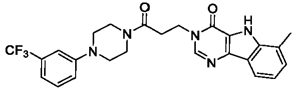 |
| **24** | 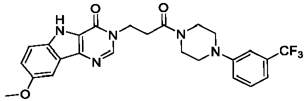 |
| **25** | 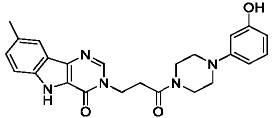 |
| **26** | 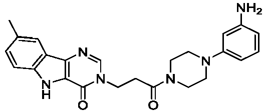 |
| **27** | 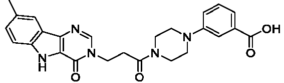 |
| **28** | 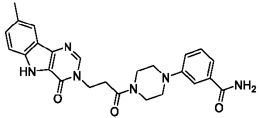 |
| **29** | 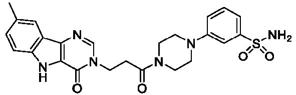 |
| **48** | 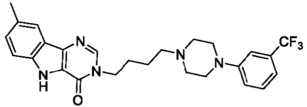 |
| **49** | 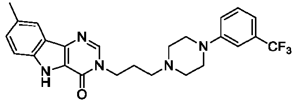 |
| **50** | 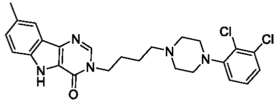 |
| **51** | 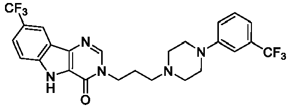 |
| **52** | 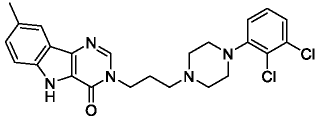 |
| **53** | 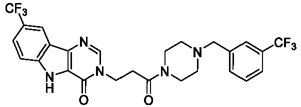 |
| **54** | 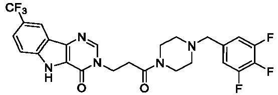 |
| **57** | 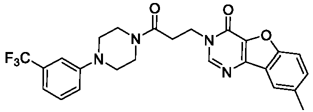 |
| **59** | 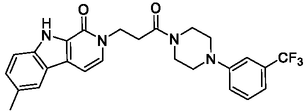 |
| **65** | 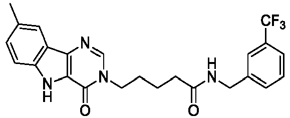 |
| **66** | 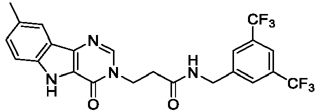 |
| **67** | 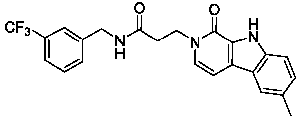 |
| **68** | 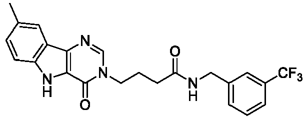 |
| **69** | 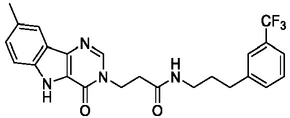 |
| **71** | 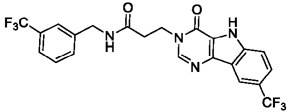 |
| **72** | 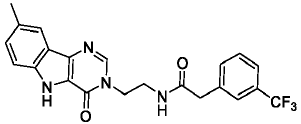 |
| **73** | 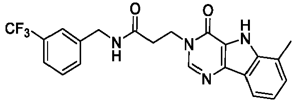 |
| **74** | 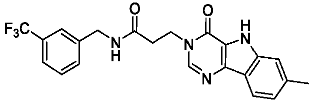 |
| **75** | 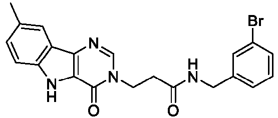 |
| **76** | 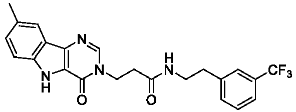 |
| **77** | 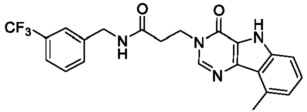 |
| **79** | 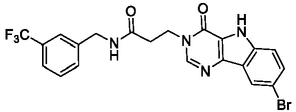 |
| **80** | 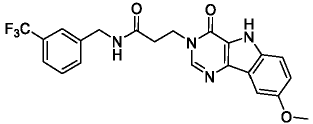 |
| **82** | 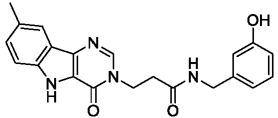 |
| **83** | 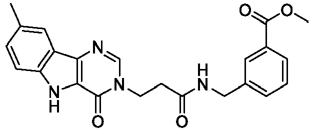 |
| **84** | 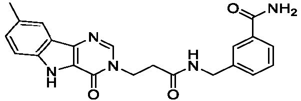 |
| **85** | 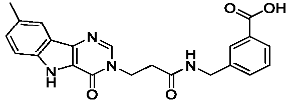 |
| **86** | 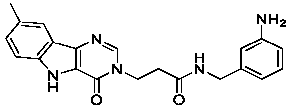 |
| **87** | 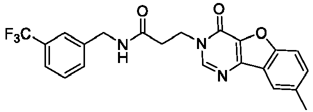 |
| **88** | 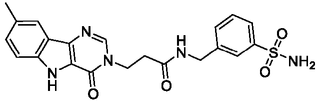 |
| **89** | 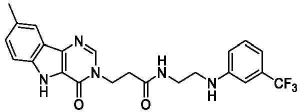 |
| **90** | 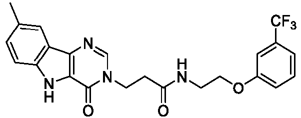 |
| **91** | 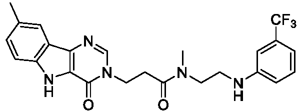 |
| **92** | 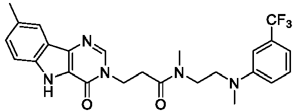 |
| **93** | 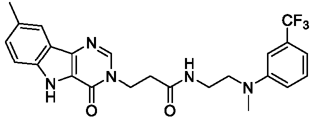 |
| **94** | 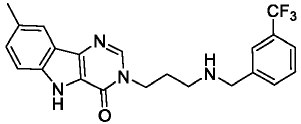 |
| **95** | 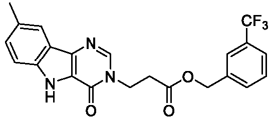 |
| **129** | 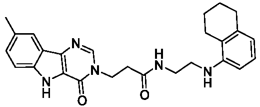 |
| **130** | 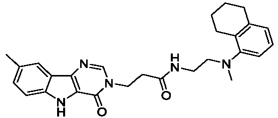 |
| **131** | 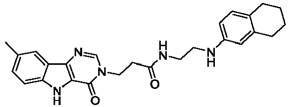 |
| **132** | 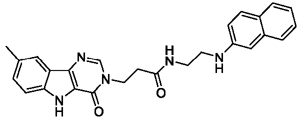 |
| **133** | 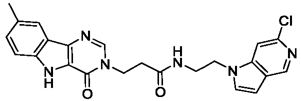 |
| **134** | 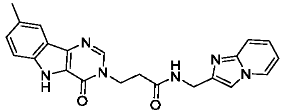 |
| **135** | 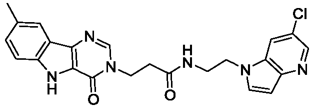 |
| **138** | 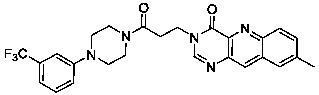 |
| **144** | 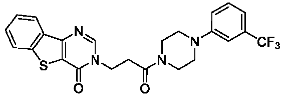 |
| **145** | 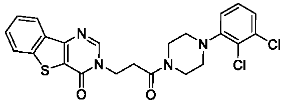 |
| **146** | 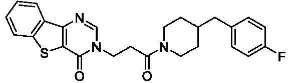 |
| **147** | 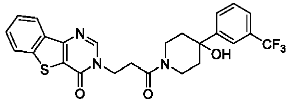 |
| **148** | 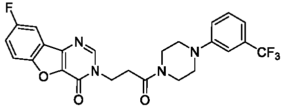 |
| **149** | 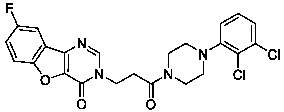 |
| **150** | 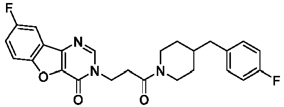 |
| **151** | 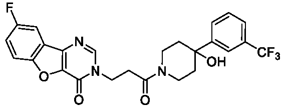 |
| **152** | 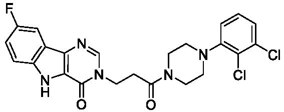 |
| **153** | 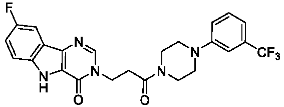 |
| **154** | 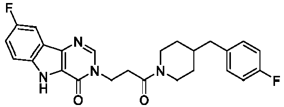 |
| **155** | 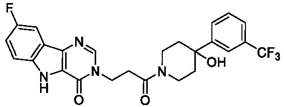 |
| **156** | 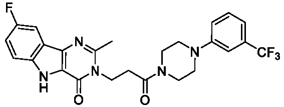 |
| **157** | 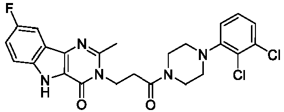 |
| **158** | 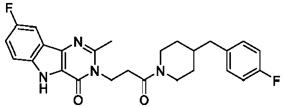 |
| **159** | 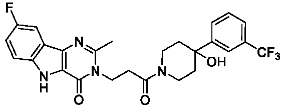 |
| **160** | 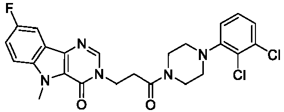 |
| **161** | 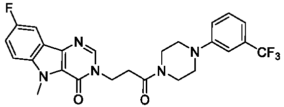 |
| **162** | 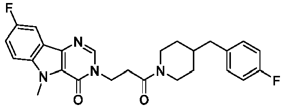 |
| **163** | 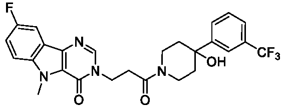 |
| **164** | 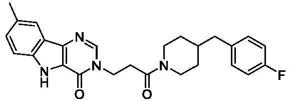 |
| **165** | 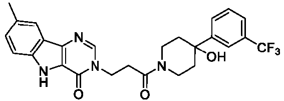 |
| **166** | 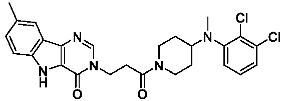 |
| **167** | 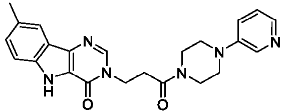 |
| **168** | 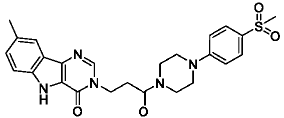 |
| **169** | 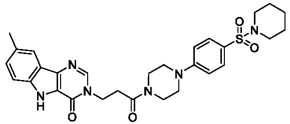 |
| **170** | 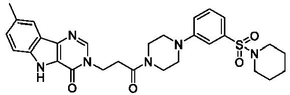 |
| **171** | 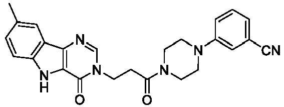 |
| **172** | 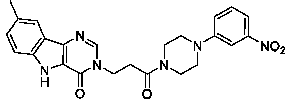 |
| **173** | 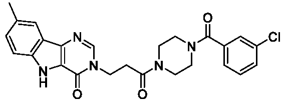 |
| **174** | 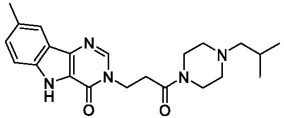 |
| **175** | 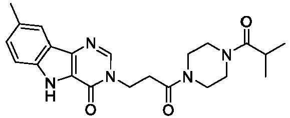 |
| **176** | 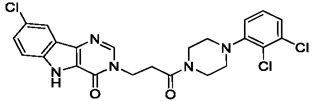 |
| **177** | 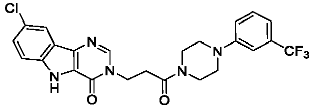 |
| **178** | 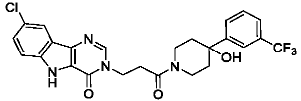 |
| **179** | 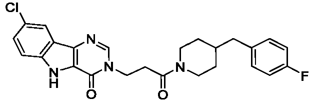 |
| **180** | 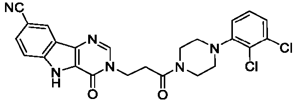 |
| **181** | 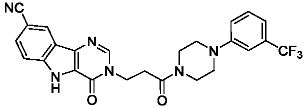 |
| **182** | 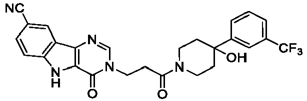 |
| **183** | 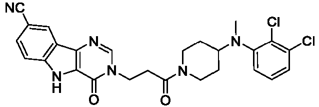 |
| **184** | 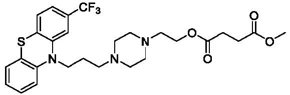 |
| **185** | 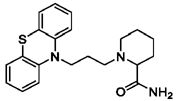 |
| **186** | 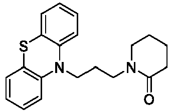 |
| **187** | 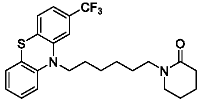 |
| **188** | 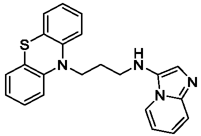 |
| **189** | 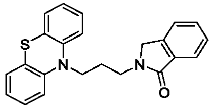 |
| **190** | 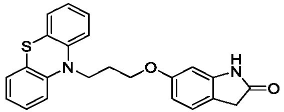 |
| **191** | 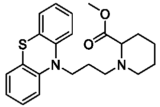 |
| **192** | 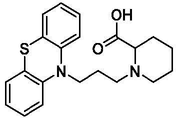 |
| **193** | 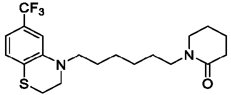 |
| **194** | 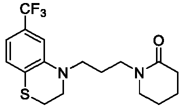 |
| **195** | 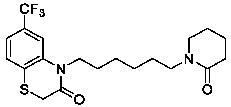 |
| **196** | 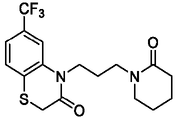 |
| **197** | 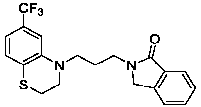 |
| **205** | 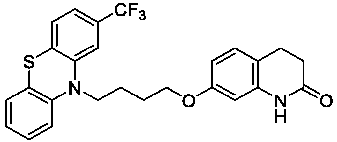 |
| **206** | 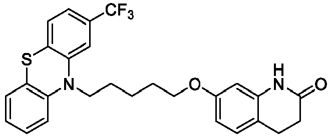 |
| **207** | 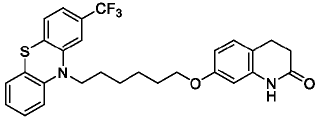 |
| **208** | 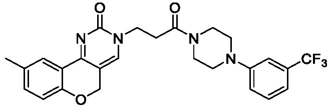 |
| **209** | 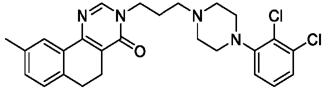 |
| **210** | 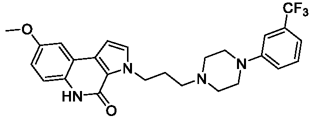 |
| **211** | 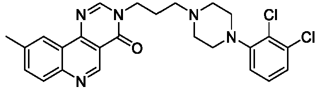 |
| **212** | 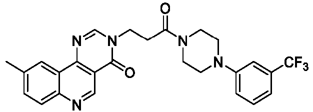 |
| **213** | 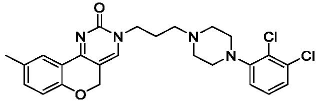 |
| **215** | 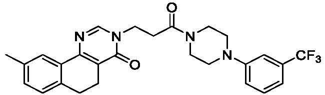 |
| **216** | 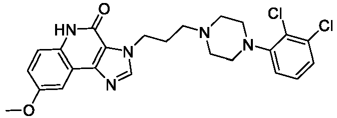 |
| **217** | 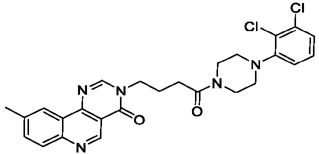 |
| **218** | 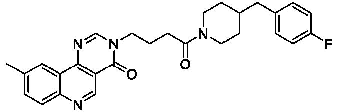 |
| **219** | 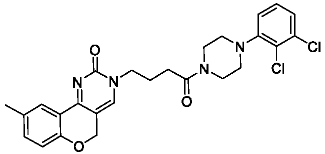 |
| **220** | 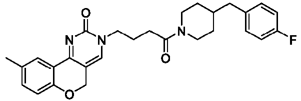 |
| **221** | 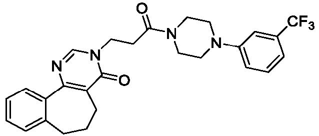 |
| **222** | 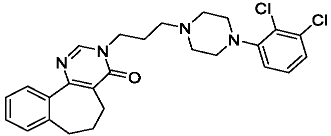 |
| **223** | 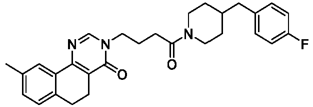 |
| **224** | 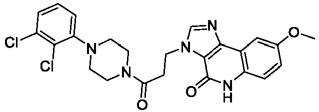 |
| **225** | 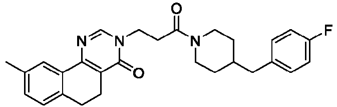 |
| **226** | 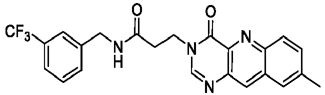 |
| **227** | 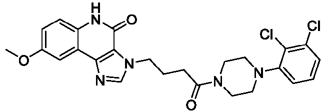 |
| **228** | 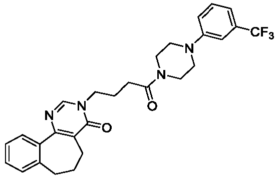 |
| **229** | 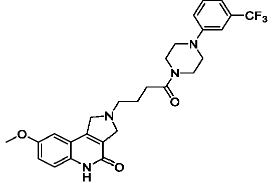 |
| **230** | 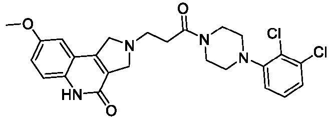 |
| **231** | 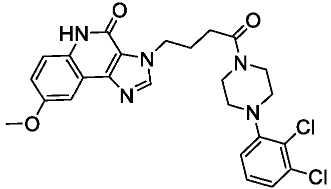 |
| **232** | 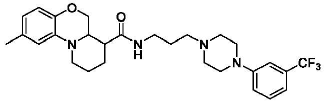 |
| **233** | 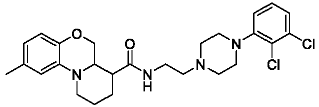 |
| **234** | 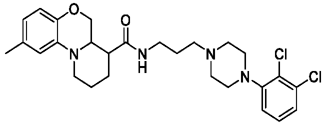 |
| **235** | 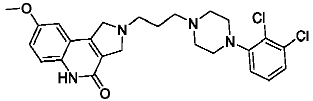 |

### Example

The present invention is further described below in conjunction with examples, but the provision of these examples is not intended to limit the scope of the present invention.

The abbreviations in the present invention have the following meanings:

| **Abbreviation** | **Meaning** |
|---|---|
| ACN | Acetonitrile |
| AcOH | Acetic acid |
| BOC | *tert*-Butoxycarbonyl |
| CDCl₃ | Deuterated chloroform |
| DCM | Dichloromethane |
| DMC | Dimethyl carbonate |
| DIPEA | *N,N*-Diisopropylethylamine |
| DMAP | 4-Dimethylaminopyridine |
| DMF | *N,N*-Dimethylformamide |
| DMF-DMA | N,N-Dimethylformamide dimethyl acetal |
| DMSO | Dimethyl sulfoxide |
| EA/EtOAc | Ethyl acetate |
| FA | Formic acid |
| HATU | O-(7-azabenzotriazol-1-yl)-N,N,N',N'-tetramethyluronium hexafluorophosphate |
| HNO₃ | Nitric acid |
| LC-MS | Liquid chromatography - mass spectrometry |
| LiHMDS | Lithium bis(trimethylsilyl)amide |
| LiOH | Lithium hydroxide |
| MeOH | Methanol |
| NaH | Sodium hydride |
| NMR | Nuclear magnetic resonance |
| Pd/C | Palladium/carbon |
| PE | Petroleum ether |
| PPA | Polyphosphoric acid |
| THF | Tetrahydrofuran |
| TLC | Thin layer chromatography |
| X-phos | 2-dicyclohexylphosphino-2',4',6'-triisopropylbiphenyl |

### Example 1: synthesis of 3-(3-(4-(2,3-dichlorophenyl)piperazin-1-yl)-3-oxopropyl)-8-methyl-3,5-dihydro-4H-pyrimido[5,4-b]in dol-4-one (Compound 1)

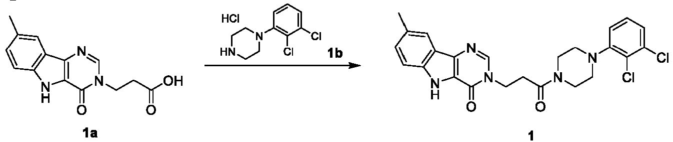

1a (100 mg, 0.37 mmol, 1.0 eq.), 1-(2,3-dichlorophenyl)piperazine hydrochloride (1b) (118 mg, 0.44 mmol, 1.2 eq.), HATU (280 mg, 0.74 mmol, 2.0 eq.) and dichloromethane (3 mL) were added into a reaction flask. Then DIPEA (0.2 mL, 1.11 mmol, 3.0 eq.) was added, and the reaction was carried out at room temperature overnight. The reaction was concentrated under reduced pressure. The residue was purified by 18C reverse-phase column chromatography (eluent: 0.1% formic acid aqueous solution:MeOH = 10% - 70%). The target fraction was collected and concentrated to obtain the title compound 1 (white solid).
LCMS: 484 [M+H]⁺
¹H NMR (400 MHz, DMSO-*d₆*) δ 11.99 (s, 1H), 8.32 (s, 1H), 7.76 (s, 1H), 7.41 (d, 1H), 7.26 (m, 3H), 7.00 (d, 1H), 4.30 (t, 2H), 3.64-3.54 (m, 4H), 2.95-2.83 (m, 6H), 2.44 (s, 3H).

### Example 2: synthesis of 3-(3-(4-(2,5-dichlorophenyl)piperazin-1-yl)-3-oxopropyl)-8-methyl-3,5-dihydro-4H-pyrimido[5,4-b]in dol-4-one (Compound 2)

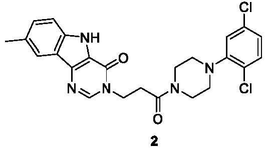

Except that 1b in Example 1 was replaced with 1-(2,5-dichlorophenyl)piperazine hydrochloride (2b) and the reaction solvent was replaced with DMF, the same synthetic route as in Example 1 was adopted to prepare the title compound 2 (off-white solid).
LCMS: 484 [M+1]⁺
¹H NMR (400 MHz, DMSO-*d₆*): δ 11.97 (s, 1H), 8.33 (s, 1H), 7.77 (s, 1H), 7.45-7.41 (m, 2H), 7.29 (d, 1H), 7.12-7.10 (m, 2H), 4.32 (t, 2H), 3.61-3.58 (m, 4H), 2.94-2.92 (m, 6H), 2.45 (s, 3H).

### Example 3: synthesis of 3-(3-(4-(2,4-dichlorophenyl)piperazin-1-yl)-3-oxopropyl)-7-methyl-3,5-dihydro-4H-pyrimido[5,4-b]in dol-4-one (Compound 3)

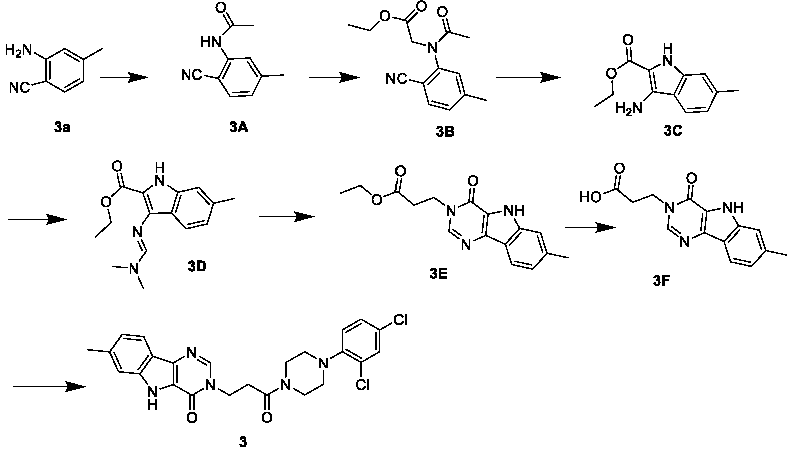

Step 1: 3a (10 g, 75.8 mmol, 1.0 eq), triethylamine (15.3 g, 151.6 mmol, 2.0 eq) and DCM (100 mL) were added into a 500 mL three-necked flask. The temperature was decreased in an ice bath, and acetyl chloride (7.1 g, 90.96 mmol, 1.2 eq) was added dropwise. The reaction was stirred at room temperature for 3 h, concentrated, and purified by column chromatography to obtain the yellow solid intermediate 3A (8.0 g, yield: 60.6%).

Step 2: 3A (9.0 g, 51.7 mmol, 1.0 eq) and THF (100 mL) were added into a 250 mL three-necked flask. The temperature was decreased in an ice bath, and NaH (3.1 g, 77.6 mmol, 1.5 eq) and ethyl bromoacetate (12.9 g, 0.405 mmol, 1.2 eq) were added. The reaction was stirred at room temperature for 2 hours, quenched with water, and extracted with EA. The organic phase was dried, concentrated, and column chromatography was carried out to obtain the white solid intermediate 3B (8.4 g, yield: 62.4%).

Step 3: 3B (8.4 g, 32.3 mmol, 1.0 eq) and THF (100 mL) were added into a 250 mL three-necked flask. The temperature was decreased in an ice bath, and potassium tert-butoxide (3.84 g, 32.3 mmol, 1.0 eq) was added. The reaction was stirred at room temperature for 2 hours, quenched with water, and extracted with EA. The organic phase was dried, concentrated, and column chromatography was carried out to obtain the white solid intermediate 3C (4.4 g, yield: 62.4%).

Step 4: 3C (2 g, 9.17 mmol, 1.0 eq), DMF (20 mL) and DMF-DMA (4.36 g, 36.68 mmol, 4.0 eq) were added into a 100 mL single-necked flask. The temperature was raised to 70°C and the reaction was carried out for 7 h. The reaction was concentrated to obtain the crude yellow solid 3D, which was directly used in the next reaction.

Step 5: 3D (crude product, 9.17 mmol, 1.0 eq), ethanol (20 mL) and ethyl β-alaninate hydrochloride (2.8 g, 18.34 mmol, 2.0 eq) were added into a 250 mL single-necked flask. The temperature was raised to 70°C and the reaction was carried out for 7 h. The reaction was cooled for crystallization and filtered to obtain the white solid intermediate 3E (1.8 g, yield: 65.6%).

Step 6: 3E (1.8 g, 6.02 mmol, 1.0 eq), methanol (20 mL) and LiOH (0.433 g, 18.06 mmol, 3.0 eq) were added into a 100 mL single-necked flask. The reaction was stirred at room temperature for 3 h, and concentrated to remove methanol. The pH was adjusted to 4 with dilute hydrochloric acid, and filtration was carried out to obtain the white solid intermediate 3F (0.9 g, yield: 55.3%).

Step 7: 3F (150 mg, 0.55 mmol, 1.0 eq.) was added into N,N-dimethylformamide (7 mL), and HATU (252 mg, 0.66 mmol, 1.2 eq.) was added. The reaction was carried out at room temperature for 1 h. 1-(2,4-dichlorophenyl)piperazine hydrochloride 3b (178 mg, 0.66 mmol, 1.2 eq.) and DIPEA (264 mg, 2.05 mmol, 3.7 eq.) were added, and the reaction was carried out at room temperature overnight. The reaction was concentrated under reduced pressure. The residue was triturated with methanol to prepare 140 mg of the title compound 3 (pale yellow solid), with a yield of 52%.
LCMS: 484 [M+H]⁺
¹H NMR (400 MHz, DMSO-*d₆*) δ 11.98 (s, 1H), 8.35 (s, 1H), 7.89 (d, 1H), 7.56 (d, 1H), 7.34 (s, 1H), 7.31 (dd, 1H), 7.09 (d, 1H), 7.04 (d, 1H), 4.34 (t, 2H), 3.65-3.57 (m, 4H), 2.94 (t, 2H), 2.91-2.86 (m, 4H), 2.49 (s, 3H).

### Example 4: synthesis of 8-methyl-3-(3-oxo-3-(4-(2-(trifluoromethyl)phenyl)piperazin-1-yl)propyl)-3,5-dihydro-4H-pyrimido[5 ,4-b]indol-4-one (Compound 4)

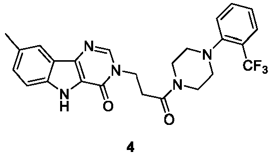

Except that 1b in Example 1 was replaced with 1-(2-(trifluoromethyl)phenyl)piperazine, the same synthetic route as in Example 1 was adopted to prepare the title compound 4 (white solid).
LCMS: 484 [M+1];
¹H NMR (400 MHz, DMSO-*d₆*) δ 11.99 (s, 1H), 8.31 (s, 1H), 7.78 (s, 1H), 7.64 (d, 1H), 7.52 (s, 1H), 7.41 (d, 1H), 7.31 (m, 3H), 4.31 (s, 2H), 3.52 (s, 4H), 2.91 (s, 2H), 2.72 (d, 4H), 2.45 (s, 3H).

### Example 5: synthesis of 3-(3-oxo-3-(4-(3-(trifluoromethyl)phenyl)piperazin-1-yl)propyl)-3,5-dihydro-4H-pyrimido[5,4-b]indol -4-one (Compound 5)

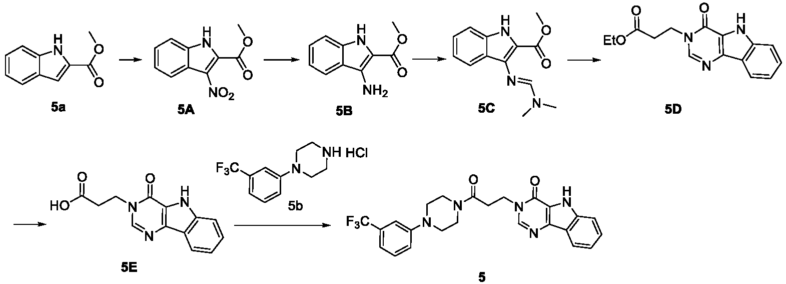

Step 1: 5a (5 g, 28.54 mmol) was added to DCM (100 mL). Then a mixed reagent of 65% HNO₃ (2.77 g, 28.54 mmol) and AcOH (17.14 g, 285.41 mmol) was added dropwise. The reaction solution was stirred at 40 °C for 2 hours. The reaction solution was slowly poured into a saturated aqueous sodium bicarbonate solution to quench the reaction and adjust the pH to 8 - 9. The reaction was extracted with dichloromethane. The organic phase was dried, filtered, and concentrated by rotary evaporation. Then it was purified by normal phase column chromatography (eluent: (petroleum ether:(ethyl acetate/dichloromethane (1:1)) = 5% - 25%)). The target product was collected to obtain the yellow solid intermediate 5A (2.2 g, yield: 35.0%).

Step 2: 5A (2.2 g, 9.99 mmol) was added to methanol (30 mL). Then 10% wt. Pd/C (220 mg) was added. The reaction solution was stirred at room temperature under the protection of a hydrogen balloon for 3 hours. The reaction was filtered, and the mother liquor was concentrated by rotary evaporation to obtain the brown solid intermediate 5B (1.7 g, yield: 89.5%).

Step 3: 5B (1.7 g, 8.94 mmol) was added to DMF (20 mL). Then DMF-DMA (4.26 g, 35.75 mmol) was added. The reaction solution was stirred at 80 °C for 3 hours. The reaction was diluted with water and extracted with ethyl acetate. The organic phase was dried, filtered, and concentrated by rotary evaporation to obtain the brown solid intermediate 5C (2 g, yield: 91.2%).

Step 4: 5C (1.8 g, 7.34 mmol) was added to MeOH (25 mL). Then ethyl β-alaninate hydrochloride (2.2 g, 14.68 mmol) was added. The reaction solution was stirred at 70 °C for 3 hours. The reaction solution was cooled to room temperature, and a large amount of solid precipitated. The reaction was filtered, and the filter cake was concentrated by rotary evaporation to obtain the brown solid intermediate 5D (1.3 g, yield: 62.1 %).

Step 5: 5D (500 mg, 1.75 mmol) was added to a mixed solvent of THF/MeOH/H₂O (6 mL/2 mL/2 mL). Then LiOH (126 mg, 5.26 mmol) was added. The reaction solution was stirred at room temperature for 2 hours. The reaction was diluted with water, and the pH was adjusted to 5 - 6 with a 1M aqueous hydrochloric acid solution until a large amount of solid precipitated. The reaction was filtered, and the filter cake was concentrated by rotary evaporation to obtain the yellow solid intermediate 5E (430 mg, yield: 95.4%).

Step 6: Except that 1b in Example 1 was replaced with 1-(3-(trifluoromethyl)phenyl)piperazine hydrochloride (5b) and 1a was replaced with 5E, the same synthetic route as in Example 1 was adopted to prepare the title compound 5 (white solid).
LCMS: 470 [M+H]⁺
¹H NMR (400 MHz, DMSO-*d₆*) δ 12.11 (s, 1H), 8.36 (s, 1H), 7.98 (d, 1H), 7.51 (d, 1H), 7.46 - 7.37 (m, 2H), 7.23 - 7.16 (m, 3H), 7.06 (d, 1H), 4.32 (t, 2H), 3.59-3.57 (m, 4H), 3.20-3.18 (m, 4H), 2.92 (t, 2H).

### Example 6: synthesis of 3-(3-oxo-3-(4-(3-(trifluoromethyl)phenyl)piperazin-1-yl)propyl)-8-(trifluoromethyl)-3,5-dihydro-4H-p yrimido[5,4-b]indol-4-one (Compound 6)

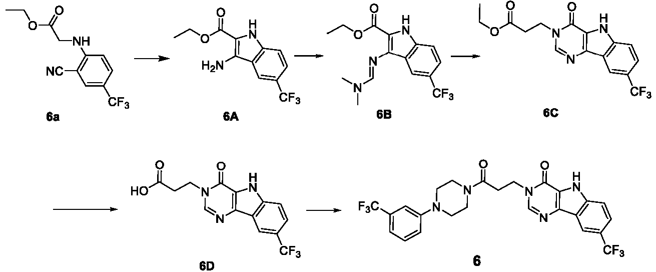

Step 1: Compound 6a (2.0 g, 7.35 mmol, 1.0 eq.) was dissolved in anhydrous tetrahydrofuran (30 mL). Potassium *tert*-butoxide (824 mg, 7.35 mmol, 1.0 eq.) was added in batches under an ice-bath. The reaction was carried out at room temperature overnight. The reaction was diluted with water (30 mL) and extracted with ethyl acetate (2×30 mL). The organic phases were combined, washed with saturated brine (20 mL), dried, filtered, and concentrated under reduced pressure to obtain a crude product. The crude product was separated and purified by a silica gel column (eluent: petroleum ether:ethyl acetate = 10:1 - 5:1). The target fraction was collected and concentrated to obtain the white solid 6A (1.0 g, yield: 50%).

Steps 2 - 4: Except that 3C in Example 3 was replaced with 6A, the same synthetic route as in Steps 4 to 6 of Example 3 was adopted to prepare Compound 6D.

Step 5: Except that 1a in Example 1 was replaced with 6D and 1b was replaced with 1-(3-(trifluoromethyl)phenyl)piperazine hydrochloride (5b), the same synthetic route as in Example 1 was adopted to prepare the title compound 6 (yellow solid).
LCMS: 538 [M+H]⁺
¹H NMR (400 MHz, DMSO-*d₆*) δ 12.61 (s, 1H), 8.44 (s, 1H), 8.29 (s, 1H), 7.75-7.69 (m, 2H), 7.39 (t, 1H), 7.18 (d, 1H), 7.15 (s, 1H), 7.06 (d, 1H), 4.33 (t, 2H), 3.60-3.56 (m, 4H), 3.21-3.17 (m, 4H), 2.93 (t, 2H).

### Example 7: synthesis of 3-(3-(4-(2,4-dichlorophenyl)piperazin-1-yl)-3-oxopropyl)-8-methyl-3,5-dihydro-4H-pyrimido[5,4-b]in dol-4-one (Compound 7)

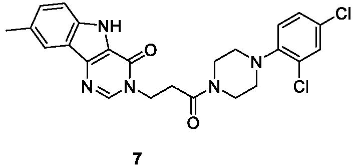

Except that 1b in Example 1 was replaced with 1-(2,4-dichlorophenyl)piperazine hydrochloride, the same synthetic route as in Example 1 was adopted to prepare the title compound 7 (yellow solid).
LCMS: 484 [M+1]⁺
¹H NMR (400 MHz, DMSO-*d₆*): δ 11.99 (s, 1H), 8.33 (s, 1H), 7.77 (s, 1H), 7.55-7.54 (m, 1H), 7.43 (d, 1H), 7.30-7.27 (m, 2H), 7.02 (d, 1H), 4.32 (t, 2H), 3.61-3.57 (m, 4H), 2.94 (t, 2H), 2.86 (br, 4H), 2.45 (s, 3H).

### Example 8: synthesis of 3-(3-(4-(2,6-dichlorophenyl)piperazin-1-yl)-3-oxopropyl)-8-methyl-3,5-dihydro-4H-pyrimido[5,4-b]in dol-4-one (Compound 8)

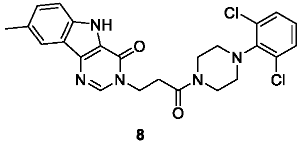

Except that 1b in Example 1 was replaced with 1-(2,6-dichlorophenyl)piperazine hydrochloride, the same synthetic route as in Example 1 was adopted to prepare the title compound 8 (white solid).
LCMS: 484 [M+1]⁺
¹H NMR (400 MHz, DMSO-*d₆*): δ 11.97 (s, 1H), 8.33 (s, 1H), 7.78 (s, 1H), 7.44-7.10 (m, 3H), 7.30 (d, 1H), 7.18 (t, 1H), 4.33 (t, 2H), 3.60 (br, 2H), 3.54 (br, 2H), 3.05 (br, 4H), 2.94 (t, 2H), 2.45 (s, 3H).

### Example 9: synthesis of 3-(3-(4-(3,4-dichlorophenyl)piperazin-1-yl)-3-oxopropyl)-8-methyl-3,5-dihydro-4H-pyrimido[5,4-b]in dol-4-one (Compound 9)

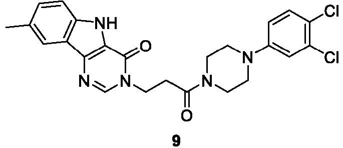

Except that 1b in Example 1 was replaced with 1-(3,4-dichlorophenyl)piperazine hydrochloride, the same synthetic route as in Example 1 was adopted to prepare the title compound 9 (off-white solid).
LCMS: 484 [M+1]⁺
¹H NMR (400 MHz, DMSO-*d₆*): δ 11.96 (s, 1H), 8.32 (s, 1H), 7.76 (s, 1H), 7.43 (d, 1H), 7.39 (d, 1H), 7.29 (d, 1H), 7.11 (s, 1H), 6.91-6.88 (m, 1H), 4.31 (t, 2H), 3.57-3.55 (m, 4H), 3.18-3.13 (m, 4H), 2.94 (t, 2H), 2.45 (s, 3H).

### Example 10: synthesis of 8-methyl-3-(3-oxo-3-(4-(4-(trifluoromethyl)phenyl)piperazin-1-yl)propyl)-3,5-dihydro-4H-pyrimido[5 ,4-b]indol-4-one (Compound 10)

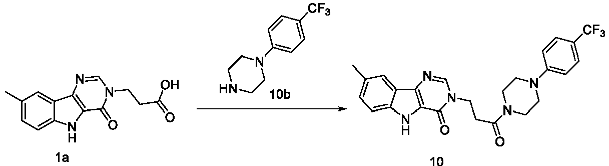

Except that 1b in Example 1 was replaced with 10b, the same synthetic route as in Example 1 was adopted to prepare the title compound 10 (white solid).
LCMS: 484 [M+1];
¹H NMR (400 MHz, DMSO-*d₆*) δ 11.97 (s, 1H), 8.32 (s, 1H), 7.75 (s, 1H), 7.48 (d, 2H), 7.41 (d, 1H), 7.27 (d, 1H), 7.01 (d, 2H), 4.30 (t, 2H), 3.58 (br, 4H), 3.25 (d, 4H), 2.92 (t, 2H), 2.43 (s, 3H).

### Example 11: synthesis of 9-methyl-3-(3-oxo-3-(4-(3-(trifluoromethyl)phenyl)piperazin-1-yl)propyl)-3,5-dihydro-4H-pyrimido[5 ,4-b]indol-4-one (Compound 11)

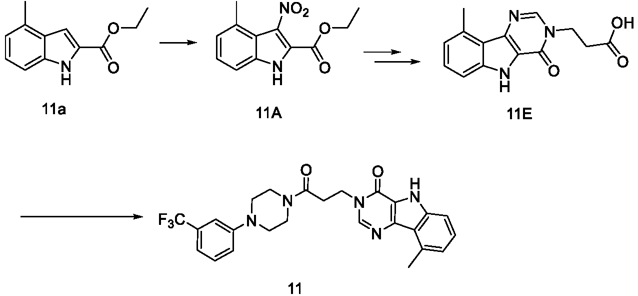

Step 1: 11a (1.0 g, 5.29 mmol, 1.0 eq), acetic acid (3.17 g, 52.9 mmol, 10.0 eq) and DCM (20 mL) were added to a 100 mL single-necked flask. The temperature was decreased in an ice-bath. 65% nitric acid (0.513 g, 5.29 mmol, 1.0 eq) was added. The reaction was stirred at room temperature for 12 h. A saturated sodium bicarbonate solution was added, and the layers were separated. The organic phase was dried and concentrated. It was separated and purified by a silica gel column (eluent: DCM:MeOH = 30:1 - 10:1) to obtain the pale yellow solid 11A.

Steps 2 - 5: Except that 5A in Example 5 was replaced with 11A, the same synthetic route as in Steps 2 to 5 of Example 5 was adopted to prepare Compound 11E.

Step 6: Except that 1b in Example 1 was replaced with 5b and 1a was replaced with 11E, the same synthetic route as in Example 1 was adopted to prepare the title compound 11 (white solid).
LCMS: 484 [M+1];
¹H NMR (400 MHz, DMSO-*d₆*) δ 12.04 (s, 1H), 8.34 (s, 1H), 7.39 (t, 1H), 7.31 (d, 2H), 7.21-7.13 (m, 2H), 7.07 (d, 1H), 6.95 (d, 1H), 4.31 (t, 2H), 3.65-3.52 (br, 4H), 3.20 (s, 4H), 2.92 (t, 2H), 2.81 (s, 3H).

### Example 12: synthesis of 8-bromo-3-(3-oxo-3-(4-(3-(trifluoromethyl)phenyl)piperazin-1-yl)propyl)-3,5-dihydro-4H-pyrimido[5 ,4-b]indol-4-one (Compound 12)

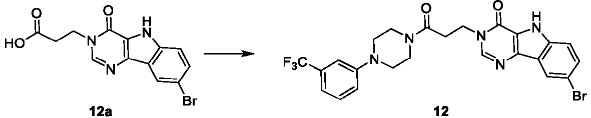

Except that 1b in Example 1 was replaced with 1-(3-(trifluoromethyl)phenyl)piperazine hydrochloride (5b) and 1a was replaced with 12a, the same synthetic route as in Example 1 was adopted to prepare the title compound 12 (white solid).
LCMS [M+H]⁺: 548, 550
¹H NMR (400 MHz, CD₃OD) δ 8.38 (s, 1H), 8.18 (s, 1H), 7.57 (dd, 1H), 7.48 (d, 1H), 7.34 (t, 1H), 7.14-7.03 (m, 3H), 4.46 (t, 2H), 3.76-3.62 (m, 4H), 3.18-3.11 (m, 4H), 3.05 (t, 2H).

### Example 13: synthesis of 3-(3-(4-(2,3-dichlorophenyl)piperazin-1-yl)-3-oxopropyl)-7-methyl-3,5-dihydro-4H-pyrimido[5,4-b]in dol-4-one (Compound 13)

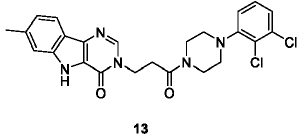

Except that 1a in Example 1 was replaced with 3F, the same synthetic route as in Example 1 was adopted to prepare the title compound 13 (pale yellow solid).
LCMS: 484 [M+H]⁺
¹H NMR (400 MHz, DMSO-*d₆*) δ 11.99 (s, 1H), 8.35 (s, 1H), 7.89 (d, 1H), 7.36-7.32 (m, 2H), 7.28 (t, 1H), 7.09 (d, 1H), 7.04 (d, 1H), 4.35 (t, 2H), 3.67-3.59 (m, 4H), 2.97-2.89 (m, 6H), 2.50 (s, 3H).

### Example 14: synthesis of 3-(3-(4-(2,5-dichlorophenyl)piperazin-1-yl)-3-oxopropyl)-7-methyl-3,5-dihydro-4H-pyrimido[5,4-b]in dol-4-one (Compound 14)

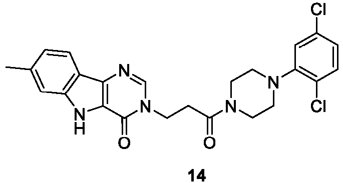

Except that 1a in Example 1 was replaced with 3F and 1b was replaced with 1-(2,5-dichlorophenyl)piperazine hydrochloride, the same synthetic route as in Example 1 was adopted to prepare the title compound 14 (pale yellow solid).
LCMS: 484 [M+H]⁺
¹H NMR (400 MHz, DMSO-*d₆*) δ 11.99 (s, 1H), 8.37 (s, 1H), 7.90 (d, 1H), 7.47 (d, 1H), 7.35 (s, 1H), 7.16-7.12 (m, 2H), 7.09 (d, 1H), 4.35 (t, 2H), 3.66-3.59 (m, 4H), 2.99-2.93 (m, 6H), 2.49 (s, 3H).

### Example 15: synthesis of 3-(4-(4-(2,3-dichlorophenyl)piperazin-1-yl)-4-oxobutyl)-8-methyl-3,5-dihydro-4H-pyrimido[5,4-b]ind ol-4-one (Compound 15)

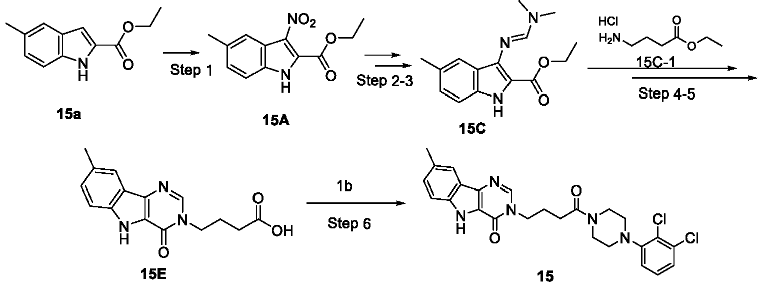

Step 1: Except that 11a in Step 1 of Example 11 was replaced with 15a, the same synthetic route as in Step 1 of Example 11 was adopted to prepare the compound 15A.

Steps 2 - 5: Except that 5A in Step 2 of Example 5 was replaced with 15A and ethyl β-alaninate hydrochloride in Step 4 was replaced with 15C-1, the same synthetic route as in Steps 2 - 5 of Example 5 was adopted to prepare the compound 15E.

Step 6: Except that 1a in Example 1 was replaced with 15E, the same synthetic route as in Example 1 was adopted to prepare the title compound 15 (white solid).
LCMS: 498 [M+H]⁺
¹H NMR (400 MHz, DMSO-*d₆*) δ 11.99 (s, 1H), 8.28 (s, 1H), 7.80 (s, 1H), 7.46-7.44 (m, 1H), 7.36-7.31 (m, 3H), 7.14-7.13 (m, 1H), 4.18-4.15 (m, 2H), 3.67-3.56 (m, 4H), 2.99-2.91 (m, 4H), 2.48-2.46 (m, 5H), 2.08-2.01 (m, 2H).

### Example 16: synthesis of 3-(3-(4-(3,5-bis(trifluoromethyl)phenyl)piperazin-1-yl)-3-oxopropyl)-8-methyl-3,5-dihydro-4H-pyrimi do[5,4-b]indol-4-one (Compound 16)

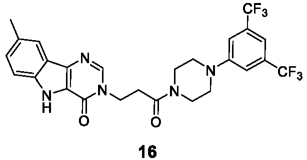

Except that 1b in Example 1 was replaced with 1-(3,5-bis(trifluoromethyl)phenyl)piperazine, the same synthetic route as in Example 1 was adopted to prepare the title compound 16 (white solid).
LCMS: 552 [M+1];
¹H NMR (400 MHz, DMSO-*d₆*) δ 11.97 (s, 1H), 8.31 (s, 1H), 7.74 (s, 1H), 7.44 (s, 2H), 7.40 (d, 1H), 7.33 - 7.22 (m, 2H), 4.30 (t, 2H), 3.58 (s, 4H), 3.33 (s, 4H), 2.93 (d, 2H), 2.43 (s, 3H).

### Example 17: synthesis of 8-methyl-3-(4-oxo-4-(4-(3-(trifluoromethyl)phenyl)piperazin-1-yl)butyl)-3,5-dihydro-4H-pyrimido[5, 4-b]indol-4-one (Compound 17)

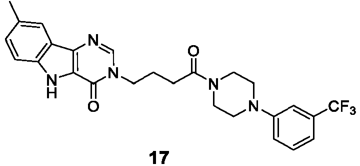

Except that 1a in Example 1 was replaced with 15E and 1b was replaced with 5b, the same synthetic route as in Example 1 was adopted to prepare the title compound 17 (white solid).
LCMS: 498 [M+H]⁺
¹H NMR (400 MHz, DMSO-*d₆*) δ 11.98 (s, 1H), 8.28 (s, 1H), 7.79 (s, 1H), 7.47-7.43 (m, 2H), 7.31-7.29 (m, 1H), 7.24-7.20 (m, 2H), 7.13-7.11 (m, 1H), 4.16 (t, 2H), 3.60-3.59 (m, 4H), 3.27-3.20 (m, 4H), 2.50-2.47 (m, 5H), 2.06-2.02 (m, 2H).

### Example 18: synthesis of 3-(3-(4-(3-bromophenyl)piperazin-1-yl)-3-oxopropyl)-8-methyl-3,5-dihydro-4H-pyrimido[5,4-b]indol-4-one (Compound 18)

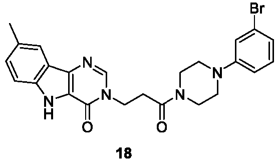

Except that 1b in Example 1 was replaced with 1-(3-bromophenyl)piperazine, the same synthetic method as in Example 1 was adopted to prepare the title compound 18 (white solid).
LCMS: 494, 496 [M+1]⁺
¹H NMR (400 MHz, DMSO-*d₆*) δ 11.96 (s, 1H), 8.31 (s, 1H), 7.75 (s, 1H), 7.41 (d, 1H), 7.27 (d, 1H), 7.13 (t, 1H), 7.06 (s, 1H), 6.97 - 6.84 (m, 2H), 4.29 (t, 2H), 3.59 - 3.50 (m, 4H), 3.13 (br, 4H), 2.90 (t, 2H), 2.44 (s, 3H).

### Example 19: synthesis of 3-(3-(4-(3,4-dichlorophenyl)piperazin-1-yl)-3-oxopropyl)-7-methyl-3,5-dihydro-4H-pyrimido[5,4-b]in dol-4-one (Compound 19)

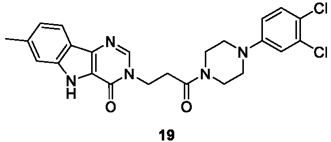

Except that 1-(2,4-dichlorophenyl)piperazine hydrochloride in Step 7 of Example 3 was replaced with 1-(3,4-dichlorophenyl)piperazine hydrochloride, the same synthetic route as in Example 3 was adopted to prepare the title compound 19 (pale yellow solid).
LCMS: 484 [M+H]⁺
¹H NMR (400 MHz, DMSO-*d₆*) δ 11.97 (s, 1H), 8.35 (s, 1H), 7.88 (d, 1H), 7.41 (d, 1H), 7.34 (s, 1H), 7.14 (d, 1H), 7.09 (d, 1H), 6.93 (dd, 1H), 4.34 (t, 2H), 3.62-3.56 (m, 4H), 3.21-3.15 (m, 4H), 2.95 (t, 2H), 2.49 (s, 3H).

### Example 20: synthesis of 3-(3-(4-(2,6-dichlorophenyl)piperazin-1-yl)-3-oxopropyl)-7-methyl-3,5-dihydro-4H-pyrimido[5,4-b]in dol-4-one (Compound 20)

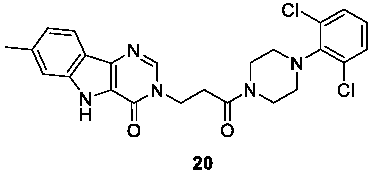

Except that 1-(2,4-dichlorophenyl)piperazine hydrochloride in Step 7 of Example 3 was replaced with 1-(2,6-dichlorophenyl)piperazine hydrochloride, the same synthetic route as in Example 3 was adopted to prepare the title compound 20 (pale yellow solid).
LCMS: 484 [M+H]⁺
¹H NMR (400 MHz, DMSO-*d₆*) δ 11.98 (s, 1H), 8.35 (s, 1H), 7.90 (d, 1H), 7.46-7.42 (m, 2H), 7.34 (s, 1H), 7.19 (t, 1H), 7.09 (d, 1H), 4.35 (t, 2H), 3.65-3.55 (m, 4H), 3.11-3.06 (m, 4H), 2.96 (t, 2H), 2.50 (s, 3H).

### Example 21: synthesis of 7-methyl-3-(3-oxo-3-(4-(3-(trifluoromethyl)phenyl)piperazin-1-yl)propyl)-3,5-dihydro-4H-pyrimido[5 ,4-b]indol-4-one (Compound 22)

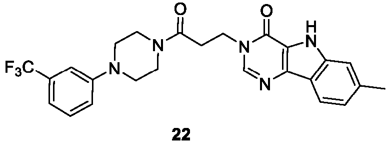

Except that 1-(2,4-dichlorophenyl)piperazine hydrochloride in Step 7 of Example 3 was replaced with 1-(3-(trifluoromethyl)phenyl)piperazine hydrochloride, the same synthetic route as in Example 3 was adopted to prepare the title compound 22 (white solid).
LCMS: 484 [M+1]⁺
¹H NMR (400 MHz, DMSO-*d₆*) δ 11.97 (s, 1H), 8.33 (s, 1H), 7.85 (d, 1H), 7.40 (t, 1H), 7.29 (s, 1H), 7.23 - 7.13 (m, 2H), 7.11 - 6.98 (m, 2H), 4.30 (s, 2H), 3.59 (d, 4H), 3.20 (d, 4H), 2.92 (s, 2H), 2.45 (s, 3H).

### Example 22: synthesis of 6-methyl-3-(3-oxo-3-(4-(3-(trifluoromethyl)phenyl)piperazin-1-yl)propyl)-3,5-dihydro-4H-pyrimido[5 ,4-b]indol-4-one (Compound 23)

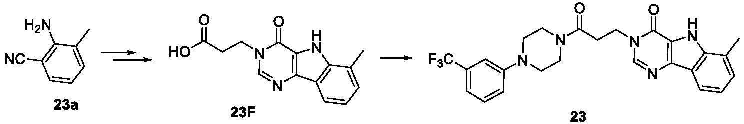

Except that 3a in Step 1 of Example 3 was replaced with 23a and 1-(2,4-dichlorophenyl)piperazine hydrochloride in Step 7 was replaced with 1-(3-(trifluoromethyl)phenyl)piperazine hydrochloride, the same synthetic route as in Example 3 was adopted to prepare the title compound 23 (white solid).
LCMS: 484 [M+H]⁺
¹H NMR (400 MHz, DMSO-*d₆*) δ 12.00 (s, 1H), 8.38 (s, 1H), 7.84 (d, 1H), 7.44 (t, 1H), 7.28 (d, 1H), 7.26-7.08 (m, 4H), 4.36 (t, 2H), 3.66-3.58 (m, 4H), 3.29-3.20 (m, 4H), 2.97 (t, 2H), 2.59 (s, 3H).

### Example 23: synthesis of 8-methoxy-3-(3-oxo-3-(4-(3-(trifluoromethyl)phenyl)piperazin-1-yl)propyl)-3,5-dihydro-4H-pyrimido [5,4-b]indol-4-one (Compound 24)

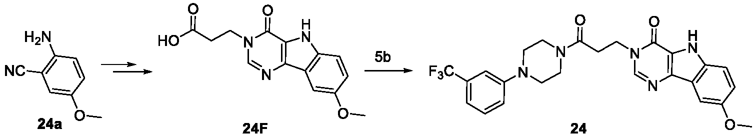

Except that 3a in Step 1 of Example 3 was replaced with 24a and 1-(2,4-dichlorophenyl)piperazine hydrochloride in Step 7 was replaced with 5b, the same synthetic route as in Example 3 was adopted to prepare the title compound 24 (white solid).
LCMS: 500 [M+H]⁺
¹H NMR (400 MHz, DMSO-*d₆*) δ 11.96 (s, 1H), 8.34 (s, 1H), 7.49-7.41 (m, 3H), 7.25-7.18 (m, 2H), 7.16-7.08 (m, 2H), 4.34 (t, 2H), 3.86 (s, 3H), 3.66-3.57 (m, 4H), 3.28-3.19 (m, 4H), 2.96 (t, 2H).

### Example 24: synthesis of 3-(3-(4-(3-hydroxyphenyl)piperazin-1-yl)-3-oxopropyl)-8-methyl-3,5-dihydro-4H-pyrimido[5,4-b]ind ol-4-one (Compound 25)

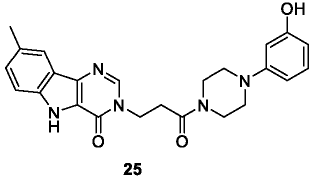

Except that 1b in Example 1 was replaced with 1-(3-hydroxyphenyl)piperazine, the same synthetic route as in Example 1 was adopted to prepare the title compound 25 (white solid).
LCMS: 432 [M+H]⁺
¹H NMR (400 MHz, DMSO-*d₆*) δ 11.97 (s, 1H), 9.15 (s, 1H), 8.32 (s, 1H), 7.76 (s, 1H), 7.41 (d, 1H), 7.27 (d, 1H), 6.98 - 6.94 (m, 1H), 6.34 (d, 1H), 6.28 (s, 1H), 6.21 (d, 1H), 4.31-4.27 (m, 2H), 3.59-3.50 (m, 4H), 3.05 - 3.00 (m, 4H), 2.92 - 2.88 (m, 2H), 2.44 (s, 3H).

### Example 25: synthesis of 3-(3-(4-(3-aminophenyl)piperazin-1-yl)-3-oxopropyl)-8-methyl-3,5-dihydro-4H-pyrimido[5,4-b]indol-4-one (Compound 26)

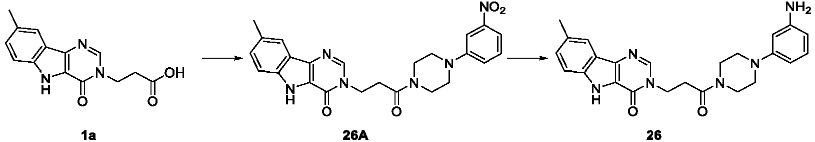

Step 1: Except that 1b in Example 1 was replaced with 1-(3-nitrophenyl)piperazine, the same synthetic route as in Example 1 was adopted to prepare the intermediate 26A (white solid).

Step 2: 26A (0.09 g, 0.2 mmol, 1.0 eq), Pd/C (0.1 g) and methanol (5 mL) were added to a 100 mL single-necked flask. The air was replaced with hydrogen, and the mixture was stirred at room temperature for 12 hours. After filtration, the filtrate was concentrated and purified by silica gel column chromatography (eluent: DCM:MeOH = 30:1 - 10:1) to prepare the title compound 26 (white solid).
LCMS: 431 [M+1];
¹H NMR (400 MHz, CD₃OD) δ 8.33 (s, 1H), 7.85 (s, 1H), 7.45 (d, 1H), 7.33 (d, 1H), 6.91 (t, 1H), 6.26 (dd, 3H), 4.45 (t, 2H), 3.72 - 3.66 (m, 2H), 3.63 - 3.59 (m, 2H), 3.05 - 2.97 (m, 6H), 2.48 (s, 3H).

### Example 26: synthesis of 3-(4-(3-(8-methyl-4-oxo-4,5-dihydro-3H-pyrimido[5,4-b]indol-3-yl)propanoyl)piperazin-1-yl)benzoic acid (compound 27)

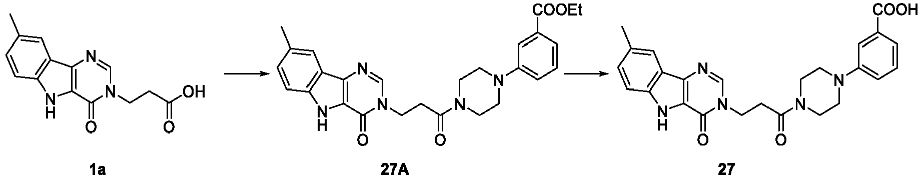

Step 1: Except that 1b in Example 1 was replaced with ethyl 3-(piperazin-1-yl)benzoate, the same synthetic route as in Example 1 was adopted to prepare the intermediate 27A (white solid).

Step 2: 27A (100 mg, 0.206 mmol, 1.0 eq), LiOH (7.4 mg, 0.309 mmol, 1.5 eq) and MeOH (20 mL) were added to a 100 mL single-necked flask. The mixture was stirred at room temperature for 2 hours, concentrated, and the pH was adjusted to 4 with dilute hydrochloric acid. After filtration, the filter cake was dried to prepare the title compound 27 (white solid).
LCMS: 460 [M+1];
¹H NMR (400 MHz, DMSO-*d₆*) δ 12.83 (s, 1H), 11.97 (s, 1H), 8.32 (s, 1H), 7.75 (s, 1H), 7.52 - 7.35 (m, 3H), 7.29 (dd, 2H), 7.17 (d, 1H), 4.30 (t, 2H), 3.68 - 3.50 (m, 4H), 3.14 (s, 4H), 2.91 (t, 2H), 2.43 (s, 3H).

### Example 27: synthesis of 3-(4-(3-(8-methyl-4-oxo-4,5-dihydro-3H-pyrimido[5,4-b]indol-3-yl)propanoyl)piperazin-1-yl)benzami de (compound 28)

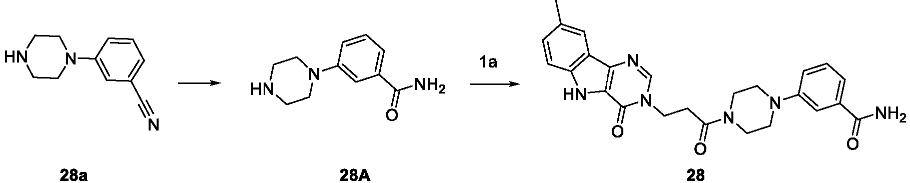

Step 1: 28a (200 mg, 1.07 mmol) was added to 90% H₂SO₄ (9 mL), and the reaction was carried out at room temperature for 2 days. The reaction solution was poured into ice-water to quench the reaction, and the pH was adjusted to 9 - 10 with ammonia water. After rotary evaporation, the residue was triturated with methanol and filtered to obtain the white solid intermediate 28A (0.1 g, yield: 45.6%).

Step 2: Except that 1b in Example 1 was replaced with 28A, the same synthetic route as in Example 1 was adopted to prepare the title compound 28 (white solid).
LCMS: 459 [M+H]⁺
¹H NMR (400 MHz, DMSO-*d₆*) δ 11.97 (s, 1H), 8.32 (s, 1H), 7.89 (s, 1H), 7.75 (s, 1H), 7.41- 7.39 (m, 2H), 7.30-7.23 (m, 4H), 7.07-7.04 (m, 1H), 4.32-4.28 (m, 2H), 3.61-3.56 (m, 4H), 3.20 -3.11 (m, 4H), 2.94-2.90 (m, 2H), 2.44 (s, 3H).

### Example 28: synthesis of 3-(4-(3-(8-methyl-4-oxo-4,5-dihydro-3H-pyrimido[5,4-b]indol-3-yl)propanoyl)piperazin-1-yl)benzene sulfonamide (compound 29)

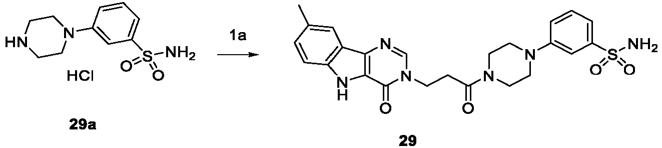

Except that 1b in Example 1 was replaced with 29a, the same synthetic route as in Example 1 was adopted to prepare the title compound 29 (white solid).
LCMS: 495 [M+1];
¹H NMR (400 MHz, DMSO-*d₆*) δ 11.95 (s, 1H), 8.31 (s, 1H), 7.74 (s, 1H), 7.44 - 7.30 (m, 3H), 7.30 - 7.14 (m, 4H), 7.11 (d, 1H), 4.29 (s, 2H), 3.58 (s, 4H), 3.21 - 3.13 (m, 4H), 2.91 (t, 2H), 2.42 (s, 3H).

### Example 29: synthesis of 8-methyl-3-(4-(4-(3-(trifluoromethyl)phenyl)piperazin-1-yl)butyl)-3,5-dihydro-4H-pyrimido[5,4-b]ind ol-4-one (Compound 48) formate

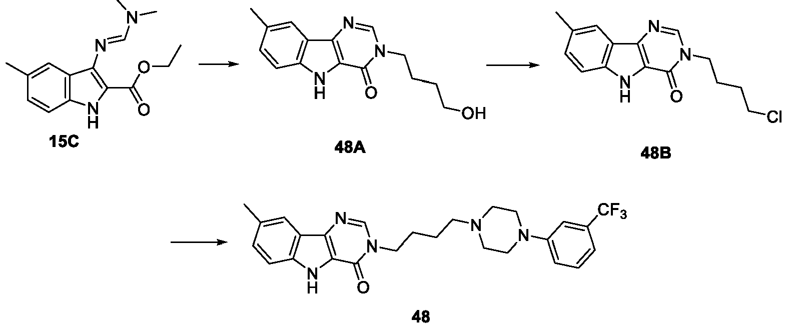

Step 1: 15C (for the synthetic route, see Example 15) (550 mg, 2.06 mmol, 1.0 eq.), 4-aminobutanol (550 mg, 6.18 mmol, 3.0 eq) and EtOH (8 mL) were added to a reaction flask. The reaction solution was purged with nitrogen three times and reacted at 85 °C for 12 hours. After TLC monitoring showed that the starting materials were completely reacted, the solvent was removed by rotary evaporation, and the residue was recrystallized from EtOAc (20 mL) to obtain the white solid 48A.

Step 2: 48A (270 mg, 1.0 mmol, 1.0 eq.) and toluene (5 mL) were added to a reaction flask. Then thionyl chloride (285 mg, 2.5 mmol, 2.5 eq.) was added dropwise to the reaction solution. The reaction solution was reacted at 45 °C for 3 hours. After LCMS monitoring showed that the starting materials were substantially reacted, the concentrated crude product 48B was directly used in the next step (280 mg, yield: 90%).

Step 3: 48B (280 mg, 0.9 mmol, 1.0 eq.), N,N-dimethylformamide (8 mL), potassium carbonate (478 mg, 3.6 mmol, 4.0 eq), potassium iodide (20 mg, 0.09 mmol, 0.1 eq) and 1-(3-(trifluoromethyl)phenyl)piperazine hydrochloride (266 mg, 0.99 mmol, 1.1 eq) were added to a reaction flask. The reaction solution was purged with nitrogen three times and reacted at 85 °C for 4 hours. After TLC monitoring showed that the starting materials were completely reacted, the mixture was filtered and the solvent was removed by rotary evaporation. The obtained crude product was purified by C18 reverse-phase column chromatography (eluent: 0.1% formic acid aqueous solution:MeOH = 10% - 70%). The target fraction was collected and concentrated to prepare the formate of the title compound 48 (white solid).
LCMS: 484 [M+H]⁺
¹H NMR (400 MHz, DMSO-*d₆*) δ 11.97 (s, 1H), 8.28 (s, 1H), 8.14 (s, 1H), 7.76 (s, 1H), 7.43-7.35 (m, 2H), 7.27(d, 1H), 7.18 (d, 1H), 7.13 (s, 1H), 7.04 (d, 1H), 4.10 (t, 2H), 3.23-3.14 (m, 4H), 2.55-2.50 (m, 4H), 2.43 (s, 3H), 2.39 (t, 2H), 1.83-1.64 (m, 2H), 1.59-1.42 (m, 2H).

### Example 30: synthesis of 8-methyl-3-(3-(4-(3-(trifluoromethyl)phenyl)piperazin-1-yl)propyl)-3,5-dihydro-4H-pyrimido[5,4-b]i ndol-4-one (Compound 49)

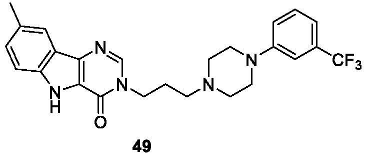

Except that 4-aminobutanol in Step 1 of Example 29 was replaced with 3-amino-1-propanol, the same synthetic route as in Example 29 was adopted to prepare the title compound 49 (yellow solid).
LCMS: 470 [M+H]⁺
¹H NMR (400 MHz, DMSO-*d₆*) δ 11.94 (s, 1H), 8.28 (s, 1H), 7.75 (s, 1H), 7.41-7.36 (m, 2H), 7.27 (d, 1H), 7.17 (d, 1H), 7.11 (s, 1H), 7.04 (d, 1H), 4.13 (t, 2H), 3.32 (br, 2H), 3.16 (br, 4H), 2.43-2.39 (m, 7H), 1.96-1.92 (m, 2H).

### Example 31: synthesis of 3-(4-(4-(2,3-dichlorophenyl)piperazin-1-yl)butyl)-8-methyl-3,5-dihydro-4H-pyrimido[5,4-b]indol-4-o ne (Compound 50)

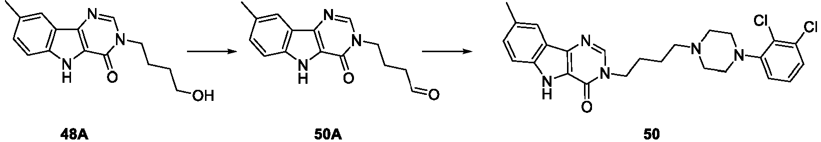

Step 1: 48A (270 mg, 1.0 mmol, 1.0 eq.) and DCM (5 mL) were added to a reaction flask. Then Dess-Martin periodinane (510 mg, 1.2 mmol, 1.2 eq.) was added to the reaction solution in batches. The reaction solution was reacted at room temperature for 1 hour. After LCMS monitoring showed that the starting materials were substantially reacted, the mixture was filtered, washed with saturated aqueous sodium bicarbonate solution, extracted with DCM. The organic phase was dried and concentrated to obtain a crude product, which was then separated by normal phase chromatography (eluent: PE:EA = 10:1 to 1:1) to obtain the pale yellow liquid intermediate 50A (250 mg, yield: 90%).

Step 2: 50A (250 mg, 0.9 mmol, 1.0 eq.) was added to a reaction flask and dissolved in MeOH (8 mL). Then 1-(2,3-dichlorophenyl)piperazine hydrochloride (359 mg, 1.35 mmol, 1.5 eq.) was added. After the reaction was stirred at room temperature under nitrogen for 1 hour, sodium triacetoxyborohydride (780 mg, 2.70 mmol, 3.0 eq.) was added in batches. After the reaction was stirred at room temperature under nitrogen for 12 hours, the reaction was completed. The solvent was removed by rotary evaporation to obtain a crude product. The crude product was purified by C18 reverse-phase column chromatography. The target fraction was collected and concentrated to prepare the title compound 50 (white solid).
LCMS: 484 [M+H]⁺
¹H NMR (400 MHz, DMSO-*d₆*) δ 11.97 (s, 1H), 8.29 (s, 1H), 7.76 (s, 1H), 7.41 (d, 1H), 7.31-7.25 (m, 3H), 7.10 (t, 1H), 4.10 (t, 2H), 2.94 (br, 4H), 2.50 (br, 4H), 2.44 (s, 3H), 2.37 (t, 2H), 1.77-1.74 (m, 2H), 1.54-1.38 (m, 2H).

### Example 32: synthesis of 8-(trifluoromethyl)-3-(3-(4-(3-(trifluoromethyl)phenyl)piperazin-1-yl)propyl)-3,5-dihydro-4H-pyrimi do[5,4-b]indol-4-one (Compound 51)

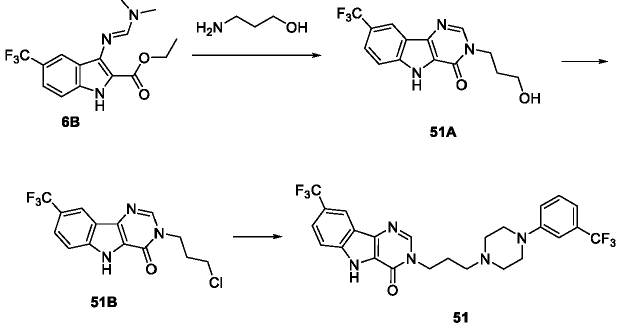

Except that 4-aminobutanol in Step 1 of Example 29 was replaced with 3-amino-1-propanol and 15C was replaced with 6B, the same synthetic route as in Example 29 was adopted to prepare the title compound 51 (pale yellow solid).
LCMS: 524 [M+H]⁺
¹H NMR (400 MHz, DMSO-*d₆*) δ 12.60 (s, 1H), 8.43 (s, 1H), 8.31 (s, 1H), 7.77-7.70 (m, 2H), 7.39 (t, 1H), 7.17 (dd, 1H), 7.09 (s, 1H), 7.05 (d, 1H), 4.20 (t, 2H), 3.18-3.08 (m, 4H), 2.51-2.46 (m, 4H), 2.42 (t, 2H), 2.03-1.92 (m, 2H).

### Example 33: synthesis of 3-(3-(4-(2,3-dichlorophenyl)piperazin-1-yl)propyl)-8-methyl-3,5-dihydro-4H-pyrimido[5,4-b]indol-4-one (Compound 52) diformate

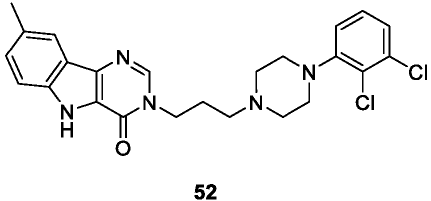

Except that 4-aminobutanol in Step 1 of Example 29 was replaced with 3-amino-1-propanol and 1-(3-(trifluoromethyl)phenyl)piperazine hydrochloride in Step 3 was replaced with 1-(2,3-dichlorophenyl)piperazine hydrochloride, the same synthetic route as in Example 29 was adopted to prepare the diformate of the title compound 52 (white solid).
LCMS: 470, 472 [M+H]⁺
¹H NMR (400 MHz, CD₃OD) δ 8.32 (s, 1H), 8.26 (br, 2H), 7.88 (s, 1H), 7.47 (d, 1H), 7.36 (d, 1H), 7.25-7.15 (m, 2H), 6.93 (d, 1H), 4.30 (t, 2H), 3.08-2.97 (m, 8H), 2.90 (t, 2H), 2.50 (s, 3H), 2.24-2.15 (m, 2H).

### Example 34: synthesis of 3-(3-oxo-3-(4-(3-(trifluoromethyl)benzyl)piperazin-1-yl)propyl)-8-(trifluoromethyl)-3,5-dihydro-4H-p yrimido[5,4-b]indol-4-one (Compound 53)

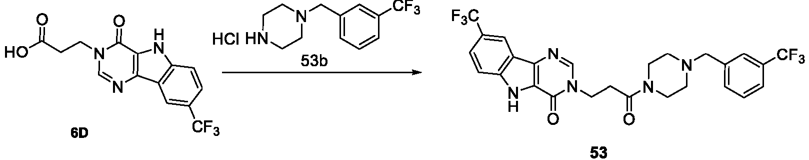

Except that 1a in Example 1 was replaced with 6D and 1b was replaced with 53b, the same synthetic route as in Example 1 was adopted to prepare the title compound 53 (pale yellow solid).
LCMS: 552 [M+H]⁺
¹H NMR (400 MHz, DMSO-*d₆*) δ 12.59 (br, 1H), 8.44 (s, 1H), 8.35 (s, 1H), 7.81-7.73 (m, 2H), 7.66-7.55 (m, 4H), 4.34 (t, 2H), 3.54 (s, 2H), 3.51-3.42 (m, 4H), 2.90 (t, 2H), 2.35-2.28 (m, 4H).

### Example 35: synthesis of 3-(3-oxo-3-(4-(3,4,5-trifluorobenzyl)piperazin-1-yl)propyl)-8-(trifluoromethyl)-3,5-dihydro-4H-pyrim ido[5,4-b]indol-4-one (Compound 54)

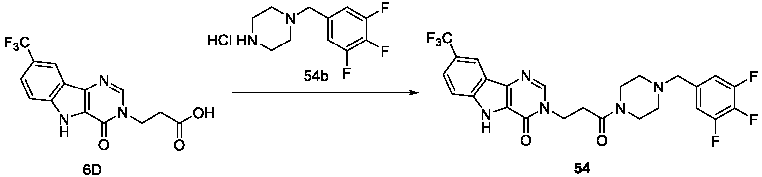

Except that 1-(2,4-dichlorophenyl)piperazine hydrochloride in Example 6 was replaced with 1-(3,4,5-trifluorobenzyl)piperazine hydrochloride, the same synthetic route as in Example 6 was adopted to prepare the title compound 54 (pale yellow solid).
LCMS: 538 [M+H]⁺
¹H NMR (400 MHz, DMSO-*d₆*) δ 12.59 (br, 1H), 8.44 (s, 1H), 8.34 (s, 1H), 7.82-7.71 (m, 2H), 7.28-7.17 (m, 2H), 4.33 (t, 2H), 3.54-3.40 (m, 6H), 2.90 (t, 2H), 2.38-2.25 (m, 4H).

### Example 36: synthesis of 8-methyl-3-(3-oxo-3-(4-(3-(trifluoromethyl)phenyl)piperazin-1-yl)propyl)benzofuro[3,2-d]pyrimidin-4(3H)-one (Compound 57)

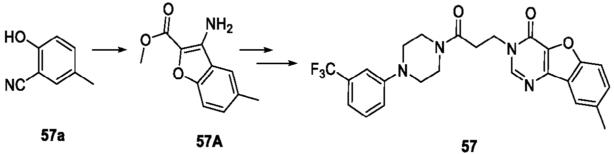

57a (3 g, 22.6 mmol, 1.0 eq), methyl bromoacetate (4.14 g, 27 mmol, 1.2 eq), potassium carbonate (6.24 g, 45.2 mmol, 2.0 eq) and DMF (20 mL) were heated to 70 °C and reacted for 5 h. After cooling and concentration, EA was added for extraction. The layers were separated, and the organic phase was dried and concentrated to obtain the white solid intermediate 57A (2.5 g, yield: 54%).

Except that 3C in Step 4 of Example 3 was replaced with 57A and 1-(2,4-dichlorophenyl)piperazine hydrochloride in Step 7 was replaced with 1-(3-(trifluoromethyl)phenyl)piperazine hydrochloride, the same synthetic route as in Steps 4 - 7 of Example 3 was adopted to prepare the title compound 57 (white solid).
LCMS: 485 [M+1];
¹H NMR (400 MHz, DMSO-*d₆*) δ 8.59 (s, 1H), 7.80 (s, 1H), 7.70 (d, 1H), 7.47 (d, 1H), 7.39 (d, 1H), 7.22 - 7.13 (m, 2H), 7.06 (d,1H), 4.30 (d, 2H), 3.66 - 3.49 (m, 4H), 3.19 (d, 4H), 2.92 (d, 2H), 2.47 (s, 3H).

### Example 37: synthesis of 6-methyl-2-(3-oxo-3-(4-(3-(trifluoromethyl)phenyl)piperazin-1-yl)propyl)-2,9-dihydro-1H-pyrido[3,4-b]indol-1-one (Compound 59)

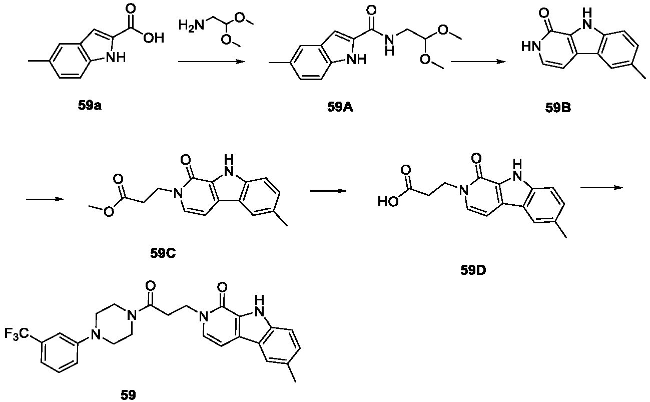

Step 1: 59a (5 g, 28.54 mmol) and triethylamine (8.66 g, 85.62 mmol) were added to DMF (50 mL). Then HATU (16.3 g, 42.78 mmol) was added. After the mixture was stirred at room temperature for 30 minutes, aminoacetaldehyde dimethyl acetal (3.6 g, 34.25 mmol) was added. The reaction solution was reacted at room temperature for 2 hours. The reaction solution was diluted with water and extracted with ethyl acetate. The organic phase was dried, filtered, and the solvent was removed by rotary evaporation. The residue was purified by normal phase column chromatography (eluent: (petroleum ether:ethyl acetate = 5% - 50%)). The target product was collected to obtain the white solid intermediate 59A (5 g, yield: 66.8%).

Step 2: 59A (4 g, 14.2 mmol) was added to polyphosphoric acid (40 mL). The reaction solution was stirred at 110 °C for 1 hour. The reaction was diluted with water and the pH was adjusted to 8 - 9 with saturated aqueous sodium bicarbonate solution. The reaction was extracted with ethyl acetate. The organic phase was dried, filtered, and the solvent was removed by rotary evaporation. The residue was purified by normal phase column chromatography (eluent: dichloromethane:methanol = 20:1). The target product was collected to obtain the yellow solid intermediate 59B (1.3 g, yield: 34.4%).

Step 3: 59B (1.3 g, 6.56 mmol) and potassium carbonate (1.81 g, 13.12 mmol) were added to DMF (20 mL). Then methyl 3-bromopropionate (1.2 g, 7.21 mmol) was added. The reaction solution was stirred at room temperature overnight. The reaction was diluted with water and extracted with ethyl acetate. The organic phase was dried, filtered, and the solvent was removed by rotary evaporation to obtain a crude product, which was purified by normal phase column chromatography (eluent: dichloromethane: methanol = 25:1). The target product was collected to obtain the yellow solid intermediate 59C (1.2 g, yield: 64.4%).

Steps 4 - 5: Except that 3E in Step 6 of Example 3 was replaced with 59C and 1-(2,4-dichlorophenyl)piperazine hydrochloride in Step 7 was replaced with 1-(3-(trifluoromethyl)phenyl)piperazine hydrochloride, the same synthetic route as in Steps 6 - 7 of Example 3 was adopted to prepare the title compound 59 (white solid).
LCMS: 483 [M+H]⁺
¹H NMR (400 MHz, DMSO-*d₆*) δ 11.83 (s, 1H), 7.76 (s, 1H), 7.41 - 7.35 (m, 3H), 7.21 (d, 1H), 7.14 - 7.11 (m, 2H), 7.06 (d, 1H), 6.96 (d, 1H), 4.28 (t, 2H), 3.60 - 3.55 (m, 4H), 3.17 - 3.16 (m, 4H), 2.86 (t, 2H), 2.41 (s, 3H).

### Example 38: synthesis of 5-(8-methyl-4-oxo-4,5-dihydro-3H-pyrimido[5,4-b]indol-3-yl)-N-(3-(trifluoromethyl)benzyl)pentana mide (compound 65)

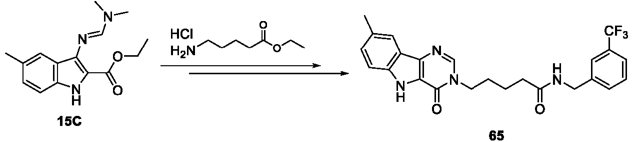

Except that 15C-1 in Step 4 of Example 15 was replaced with ethyl 5-aminopentanoate hydrochloride and 1b in Step 6 was replaced with meta-trifluoromethylbenzylamine, the same synthetic route as in Example 15 was adopted to prepare the title compound 65 (pale yellow solid).
LCMS: 457 [M+H]⁺
¹H NMR (400 MHz, DMSO-*d₆*) δ 11.96 (s, 1H), 8.44 (t, 1H), 8.26 (s, 1H), 7.76 (s, 1H), 7.62-7.48 (m, 4H), 7.41 (d, 1H), 7.27 (d, 1H), 4.33 (d, 2H), 4.07 (t, 2H), 2.44 (s, 3H), 2.21 (t, 2H), 1.73-1.68 (m, 2H), 1.60-1.54 (m, 2H).

### Example 39: synthesis of N-(3,5-bis(trifluoromethyl)benzyl)-3-(8-methyl-4-oxo-4,5-dihydro-3H-pyrimido[5,4-b]indol-3-yl)prop anamide (compound 66)

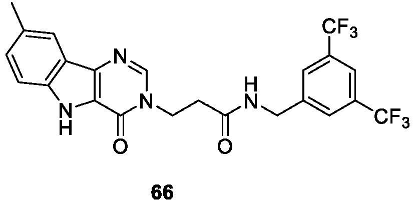

Except that 1b in Example 1 was replaced with 3,5-bis(trifluoromethyl)benzylamine, the same synthetic route as in Example 1 was adopted to prepare the title compound 66 (off-white solid).
LCMS: 497 [M+H]⁺
¹H NMR (400 MHz, DMSO-*d₆*) δ 11.96 (s, 1H), 8.64 (t, 1H), 8.15 (s, 1H), 7.94 (s, 1H), 7.90 (s, 2H), 7.74 (s, 1H), 7.40 (d, 1H), 7.28 (d, 1H), 4.42 (d, 2H), 4.29 (t, 2H), 2.73 (t, 2H), 2.44 (s, 3H).

### Example 40: synthesis of 3-(6-methyl-1-oxo-1,9-dihydro-2H-pyrido[3,4-b]indol-2-yl)-N-(3-(trifluoromethyl)benzyl)propanamid e (compound 67)

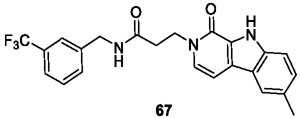

Except that 1-(3-(trifluoromethyl)phenyl)piperazine hydrochloride in Step 5 of Example 37 was replaced with meta-trifluoromethylbenzylamine, the same synthetic route as in Example 37 was adopted to prepare the title compound 67 (yellow solid).
LCMS: 428 [M+H]⁺
¹H NMR (400 MHz, DMSO-*d*₆) δ 11.82 (s, 1H), 8.55 (t, 1H), 7.77 (s, 1H), 7.58-7.53 (m, 2H), 7.47-7.43 (m, 2H), 7.39 (d, 1H), 7.26-7.21 (m, 2H), 6.91 (d, 1H), 4.33 (d, 2H), 4.27 (t, 2H), 2.66 (t, 2H), 2.43 (s, 3H).

### Example 41: synthesis of 4-(8-methyl-4-oxo-4,5-dihydro-3H-pyrimido[5,4-b]indol-3-yl)-N-(3-(trifluoromethyl)benzyl)butanami de (compound 68)

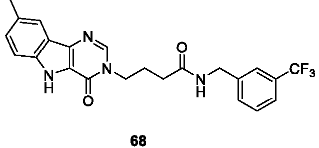

Except that 1-(2,3-dichlorophenyl)piperazine hydrochloride in Example 15 was replaced with meta-trifluoromethylbenzylamine, the same synthetic route as in Example 15 was adopted to prepare the title compound 68 (yellow solid).
LCMS: 443 [M+H]⁺
¹H NMR (400 MHz, DMSO-*d₆*) δ 11.98 (s, 1H), 8.47 (t, 1H), 8.24 (s, 1H), 7.76 (s, 1H), 7.61-7.50 (m, 4H), 7.41 (d, 1H), 7.28 (d, 1H), 4.32 (d, 2H), 4.10 (t, 2H), 2.44 (s, 3H), 2.23 (t, 2H), 2.04-1.94 (m, 2H).

### Example 42: synthesis of 3-(8-methyl-4-oxo-4,5-dihydro-3H-pyrimido[5,4-b]indol-3-yl)-N-(3-(3-(trifluoromethyl)phenyl)propyl )propanamide (compound 69)

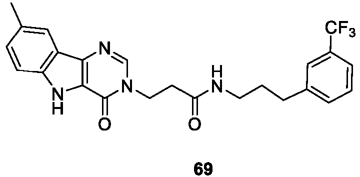

Except that 1b in Example 1 was replaced with 3-(3-(trifluoromethyl)phenyl)propan-1-amine, the same synthetic route as in Example 1 was adopted to prepare the title compound 69 (off-white solid).
LCMS: 457 [M+H]⁺
¹H NMR (400 MHz, DMSO-*d₆*) δ 11.97 (s, 1H), 8.15 (s, 1H), 8.00 (t, 1H), 7.71 (s, 1H), 7.49 (s, 2H), 7.43-7.36 (m, 3H), 7.26 (d, 1H), 4.27 (t, 2H), 3.02 (q, 2H), 2.61 (t, 2H), 2.53 (t, 2H), 2.42 (s, 3H), 1.66-1.56 (m, 2H).

### Example 43: synthesis of 3-(4-oxo-8-(trifluoromethyl)-4,5-dihydro-3H-pyrimido[5,4-b]indol-3-yl)-N-(3-(trifluoromethyl)benzyl )propanamide (compound 71)

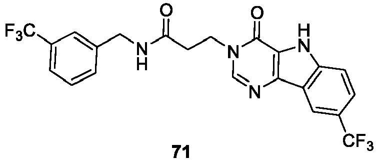

Except that 1-(3-(trifluoromethyl)phenyl)piperazine hydrochloride (5b) in Step 5 of Example 6 was replaced with meta-trifluoromethylbenzylamine, the same synthetic route as in Example 6 was adopted to prepare the title compound 71 (white solid).
LCMS: 483 [M+H]⁺
¹H NMR (400 MHz, DMSO-*d₆*) δ 12.62 (s, 1H), 8.58 (t, 1H), 8.30 (s, 1H), 8.28 (s, 1H), 7.77-7.70 (m, 2H), 7.51 (s, 2H), 7.42-7.40 (m, 2H), 4.34-4.31 (m, 4H), 2.73 (t, 2H).

### Example 44: synthesis of N-(2-(8-methyl-4-oxo-4,5-dihydro-3H-pyrimido[5,4-b]indol-3-yl)ethyl)-2-(3-(trifluoromethyl)phenyl)a cetamide (compound 72)

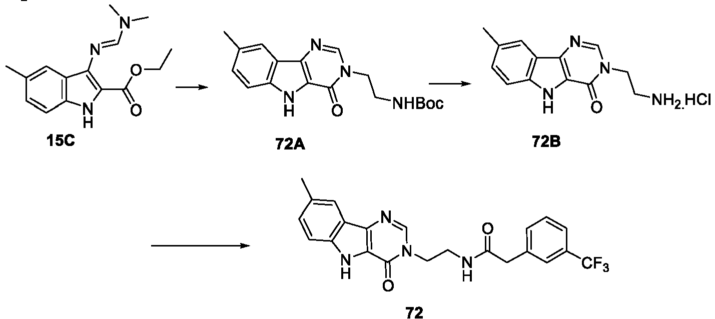

Step 1: Except that 4-aminobutanol in Step 1 of Example 29 was replaced with mono-Boc-ethylenediamine, the same synthetic method as in Step 1 of Example 29 was adopted to prepare the intermediate 72A (white solid).

Step 2: 72A (410 mg, 1.20 mmol, 1.0 eq.), EtOAc (4 mL) and 2M EtOAc/HCl solution (4 mL) were added to a reaction flask. The reaction solution was reacted at room temperature for 4 hours. After LCMS monitoring showed that the starting materials were completely reacted, the solvent was removed by rotary evaporation to obtain the off-white solid intermediate 72B (400 mg, yield: 90%).

Step 3: Except that 1b in Example 1 was replaced with meta-trifluoromethylphenylacetic acid and 1a was replaced with 72B, the same synthetic route as in Example 1 was adopted to prepare the title compound 72 (white solid).
LCMS: 429 [M+H]⁺
¹H NMR (400 MHz, DMSO-*d₆*) δ 11.98 (s, 1H), 8.29 (t, 1H), 8.03 (s, 1H), 7.76 (s, 1H), 7.55 (s, 2H), 7.47 (s, 2H), 7.41 (d, 1H), 7.28 (d, 1H), 4.12 (t, 2H), 3.51-3.43 (m, 4H), 2.45 (s, 3H).

### Example 45: synthesis of 3-(6-methyl-4-oxo-4,5-dihydro-3H-pyrimido[5,4-b]indol-3-yl)-N-(3-(trifluoromethyl)benzyl)propana mide (compound 73)

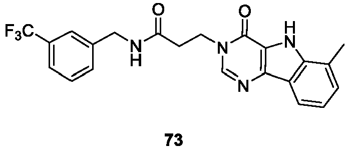

Except that 1-(3-(trifluoromethyl)phenyl)piperazine hydrochloride in the last step of Example 22 was replaced with 3-(trifluoromethyl)benzylamine, the same synthetic route as in Example 22 was adopted to prepare the title compound 73 (white solid).
LCMS: 429 [M+H]⁺
¹H NMR (400 MHz, DMSO-*d₆*) δ 12.02 (s, 1H), 8.59 (t, 1H), 8.24 (s, 1H), 7.85 (d, 1H), 7.61-7.53 (m, 2H), 7.47-7.43 (m, 2H), 7.29 (d, 1H), 7.17 (t, 1H), 4.43-4.32 (m, 4H), 2.76 (t, 2H), 2.60 (s, 3H).

### Example 46: synthesis of 3-(7-methyl-4-oxo-4,5-dihydro-3H-pyrimido[5,4-b]indol-3-yl)-N-(3-(trifluoromethyl)benzyl)propana mide (compound 74)

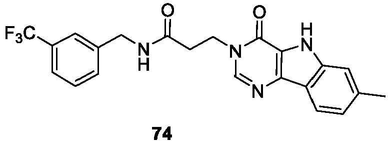

Except that 1-(2,4-dichlorophenyl)piperazine hydrochloride in Example 3 was replaced with 3-(trifluoromethyl)benzylamine, the same synthetic route as in Example 3 was adopted to prepare the title compound 74 (white solid).
LCMS: 429 [M+1];
¹H NMR (400 MHz, DMSO-*d₆*) δ 11.97 (s, 1H), 8.57 (t,1H), 8.17 (s, 1H), 7.86 (d, 1H), 7.53 (d, 2H), 7.40 (d, 2H), 7.30 (s, 1H), 7.06 (d, 1H), 4.42-4.19 (m, 4H), 2.71 (t, 2H), 2.45 (s, 3H).

### Example 47: synthesis of N-(3-bromobenzyl)-3-(8-methyl-4-oxo-4,5-dihydro-3H-pyrimido[5,4-b]indol-3-yl)propanamide (compound 75)

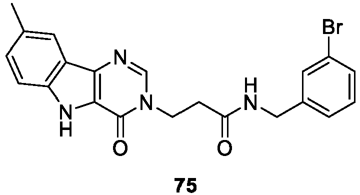

Except that 1b in Example 1 was replaced with 3-bromobenzylamine, the same synthetic route as in Example 1 was adopted to prepare the title compound 75 (white solid).
LCMS: 439, 441 [M+H]⁺
¹H NMR (400 MHz, DMSO-*d₆*) δ 11.97 (s, 1H), 8.50 (t, 1H), 8.17 (s, 1H), 7.76 (s, 1H), 7.41 (d, 1H), 7.37-7.34 (m, 2H), 7.28 (d, 1H), 7.19-7.03 (m, 2H), 4.29 (t, 2H), 4.22 (d, 2H), 2.71 (t, 2H), 2.44 (s, 3H).

### Example 48: synthesis of 3-(8-methyl-4-oxo-4,5-dihydro-3H-pyrimido[5,4-b]indol-3-yl)-N-(3-(trifluoromethyl)phenethyl)propa namide (compound 76)

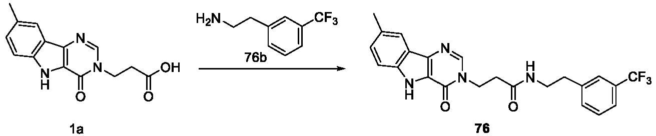

Except that 1b in Example 1 was replaced with 76b, the same synthetic route as in Example 1 was adopted to prepare the title compound 76 (white solid).
LCMS: 443 [M+H]⁺
¹H NMR (400 MHz, DMSO-*d₆*) δ 11.98 (s, 1H), 8.14 (s, 1H), 8.05 (t, 1H), 7.76 (s, 1H), 7.51 (s, 1H), 7.47-7.39 (m, 2H), 7.36-7.25 (m, 3H), 4.24 (t, 2H), 3.26 (q, 2H), 2.73 (t, 2H), 2.58 (t, 2H), 2.44 (s, 3H).

### Example 49: synthesis of 3-(9-methyl-4-oxo-4,5-dihydro-3H-pyrimido[5,4-b]indol-3-yl)-N-(3-(trifluoromethyl)benzyl)propana mide (compound 77)

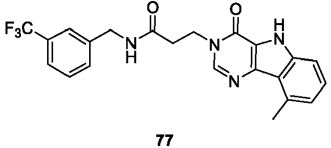

Except that 1-(3-(trifluoromethyl)phenyl)piperazine hydrochloride in the last step of Example 11 was replaced with 3-(trifluoromethyl)benzylamine, the same synthetic route as in Example 11 was adopted to prepare the title compound 77 (white solid).
LCMS: 429 [M+1];
¹H NMR (400 MHz, DMSO-*d₆*) δ 12.07 (s, 1H), 8.60 (t, 1H), 8.19 (s, 1H), 7.53 (d, 2H), 7.39 (s, 2H), 7.32 (d, 2H), 6.96 (s, 1H), 4.31 (t, 4H), 2.82 (s, 3H), 2.73 (t, 2H).

### Example 50: synthesis of 3-(8-bromo-4-oxo-4,5-dihydro-3H-pyrimido[5,4-b]indol-3-yl)-N-(3-(trifluoromethyl)benzyl)propana mide (compound 79)

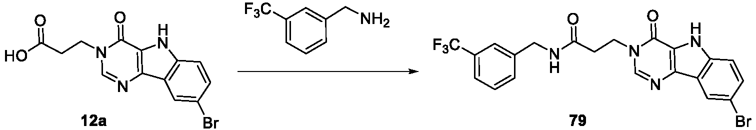

Except that 1b in Example 1 was replaced with meta-trifluoromethylbenzylamine and 1a was replaced with 12a, the same synthetic route as in Example 1 was adopted to prepare the title compound 79 (white solid).
LCMS [M+H]⁺: 493, 495
¹H NMR (400 MHz, DMSO-*d₆*) δ 12.35 (s, 1H), 8.57 (t, 1H), 8.11 (s, 1H), 7.58 (d, 1H), 7.53 (br, 2H), 7.49 (d, 1H), 7.42-7.41 (m, 2H), 4.33-4.28 (m, 4H), 2.72 (t, 2H).

### Example 51: synthesis of 3-(8-methoxy-4-oxo-4,5-dihydro-3H-pyrimido[5,4-b]indol-3-yl)-N-(3-(trifluoromethyl)benzyl)propan amide (compound 80)

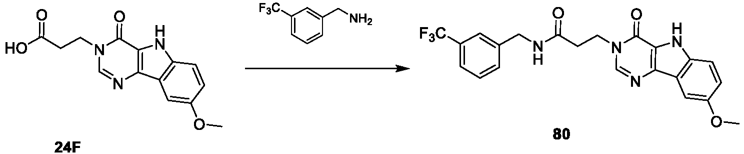

Except that compound 5b in Example 23 was replaced with meta-trifluoromethylbenzylamine, the same synthetic route as in Example 23 was adopted to prepare the title compound 80 (pale yellow solid).
LCMS: 445 [M+H]⁺
¹H NMR (400 MHz, DMSO-*d₆*) δ 11.98 (s, 1H), 8.59 (t, 1H), 8.20 (s, 1H), 7.60-7.54 (m, 2H), 7.48-7.42 (m, 4H), 7.14 (dd, 1H), 4.39-4.29 (m, 4H), 3.87 (s, 3H), 2.75 (t, 2H).

### Example 52: synthesis of N-(3-hydroxybenzyl)-3-(8-methyl-4-oxo-4,5-dihydro-3H-pyrimido[5,4-b]indol-3-yl)propanamide (compound 82)

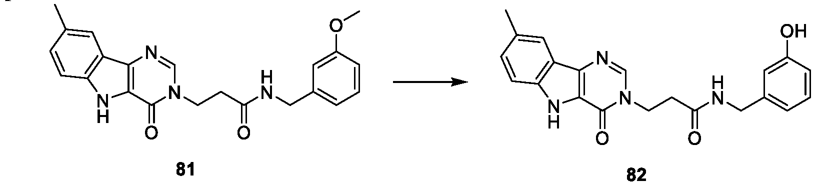

81 (200 mg, 0.52 mmol, 1.0 eq.) was dissolved in dichloromethane (2 mL). A solution of boron tribromide in dichloromethane (2.3 g, 1.54 mmol, 3.0 eq., 17%) was added dropwise under ice-bath, and the reaction was carried out at room temperature overnight. After filtration, the filter cake was washed with dichloromethane (2×10 mL). The filter cake was dissolved in ethyl acetate (10 mL) and saturated sodium bicarbonate solution (10 mL). The layers were separated, and the aqueous phase was extracted with ethyl acetate (2×20 mL). The combined organic phases were washed with saturated brine (20 mL), dried, filtered, and concentrated under reduced pressure to prepare the title compound 82 (white solid).
LCMS: 377 [M+H]⁺
¹H NMR (400 MHz, DMSO-*d₆*) δ 12.00 (s, 1H), 9.41 (s, 1H), 8.49 (t, 1H), 8.21 (s, 1H), 7.76 (s, 1H), 7.42 (d, 1H), 7.28 (d, 1H), 6.95 (t, 1H), 6.62 (s, 1H), 6.58 (d, 1H), 6.52 (d, 1H), 4.29 (t, 2H), 4.13 (d, 2H), 2.68 (t, 2H), 2.44 (s, 3H).

### Example 53: synthesis of methyl 3-((3-(8-methyl-4-oxo-4,5-dihydro-3H-pyrimido[5,4-b]indol-3-yl)propanamido)methyl)benzoate (compound 83)

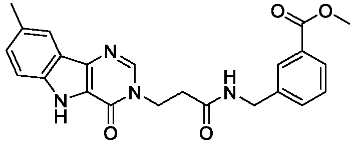

Except that 1b in Example 1 was replaced with methyl 3-(aminomethyl)benzoate, the same synthetic route as in Example 1 was adopted to prepare the title compound 83 (white solid).
LCMS: 419 [M+H]⁺
¹H NMR (400 MHz, DMSO-*d₆*) δ 11.97 (s, 1H), 8.56 (t, 1H), 8.16 (s, 1H), 7.82-7.74 (m, 3H), 7.43-7.26 (m, 4H), 4.33-4.26 (m, 4H), 3.79 (s, 3H), 2.70 (t, 2H), 2.44 (s, 3H).

### Example 54: synthesis of 3-((3-(8-methyl-4-oxo-4,5-dihydro-3H-pyrimido[5,4-b]indol-3-yl)propanamido)methyl)benzamide (compound 84)

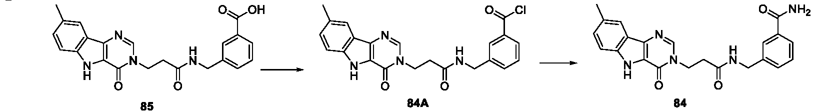

Step 1: Compound 85 (380 mg, 0.94 mmol, 1.0 eq.) and dichloromethane (5 mL) were added to a reaction flask. Under ice-bath, a solution of (COCl)₂ (240 mg, 1.88 mmol, 2.0 eq.) dissolved in 5 mL of dichloromethane water (2 mL) was added dropwise to the reaction solution. After the addition, the mixture was stirred at room temperature for two hours. The solvent was evaporated to obtain an orange-yellow solid, which was directly used in the next reaction (396 mg, yield: 100%).

Step 2: 84A (396 mg, 0.94 mmol, 1.0 eq.) and tetrahydrofuran (5 mL) were added to a reaction flask. Under ice-bath, ammonia water (5 mL, 40%) was added dropwise to the reaction solution. After the addition, the mixture was stirred at room temperature for half an hour. The organic phase was concentrated to obtain a crude product, which was purified by C18 reverse-phase column chromatography (eluent: 0.5% formic acid aqueous solution:MeOH = 10% - 80%). The target fraction was collected and concentrated to prepare the title compound 84 (white solid).
LCMS: 404 [M+H]⁺
¹H NMR (400 MHz, DMSO-*d₆*) δ 11.97 (s, 1H), 8.50 (t, 1H), 8.19 (s, 1H), 7.92 (s, 1H), 7.77-7.67 (m, 3H), 7.41 (d, 1H), 7.33-7.22 (m, 4H), 4.32-4.23 (m, 4H), 2.70 (t, 2H), 2.44 (s, 3H).

### Example 55: synthesis of 3-((3-(8-methyl-4-oxo-4,5-dihydro-3H-pyrimido[5,4-b]indol-3-yl)propanamido)methyl)benzoic acid (compound 85)

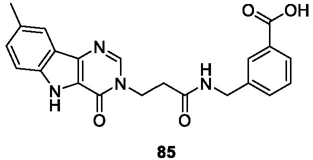

Compound 83 (150 mg, 0.358 mmol, 1.0 eq.), LiOH (34 mg, 1.43 mmol, 4.0 eq.), ethanol (10 mL) and water (2 mL) were added to a reaction flask and stirred at room temperature overnight. The pH was adjusted to 5 - 6 with 1.0 mol/L hydrochloric acid. After filtration, the filter cake was washed with water until neutral and dried to prepare the title compound 85 (white solid).
LCMS: 405 [M+H]⁺
¹H NMR (400 MHz, DMSO-*d₆*) δ 11.99 (s, 1H), 8.55 (t, 1H), 8.19 (s, 1H), 7.81-7.74 (m, 3H), 7.41 (d, 1H), 7.36-7.27 (m, 3H), 4.31-4.28 (m, 4H), 2.70 (t, 2H), 2.44 (s, 3H).

### Example 56: synthesis of N-(3-aminobenzyl)-3-(8-methyl-4-oxo-4,5-dihydro-3H-pyrimido[5,4-b]indol-3-yl)propanamide (compound 86) hydrochloride

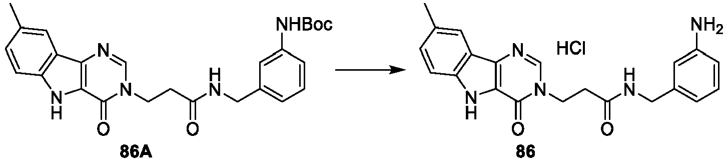

Except that 1b in Example 1 was replaced with meta-Boc-aminobenzylamine, the same synthetic route as in Example 1 was adopted to prepare the intermediate 86A (yellow solid).

The intermediate 86A (200 mg, 0.42 mmol, 1.0 eq.) was dissolved in 1,4-dioxane (10 mL). A solution of hydrogen chloride in dioxane (2 mL, 8 mmol, 20.0 eq., 4.0 M) was added, and the reaction was carried out at room temperature for 2 hours. After filtration, the filter cake was washed with ethyl acetate (2×10 mL) and dried at 45 °C to prepare the hydrochloride salt of compound 86 (yellow solid).
LCMS: 376 [M+H]⁺
¹H NMR (400 MHz, DMSO-*d₆*) δ 12.04 (s, 1H), 8.63 (t, 1H), 8.29 (s, 1H), 7.79 (s, 1H), 7.42 (d, 1H), 7.30 (m, 2H), 7.21-7.13 (m, 3H), 4.32 (t, 2H), 4.26 (d, 2H), 2.71 (t, 2H), 2.45 (s, 3H).

### Example 57: synthesis of 3-(8-methyl-4-oxobenzofuro[3,2-d]pyrimidin-3(4H)-yl)-N-(3-(trifluoromethyl)benzyl)propanamide (compound 87)

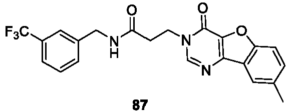

Except that 1-(3-(trifluoromethyl)phenyl)piperazine hydrochloride in the last step of Example 36 was replaced with 3-(trifluoromethyl)benzylamine, the same synthetic route as in Example 36 was adopted to prepare the title compound 87 (white solid).
LCMS: 430 [M+1];
¹H NMR (400 MHz, DMSO-*d₆*) δ 8.58 (t, 1H), 8.44 (s, 1H), 7.82 (s, 1H), 7.71 (d, 1H), 7.49 (d, 3H), 7.42 (s, 2H), 4.31 (d, 4H), 3.33 (s, 3H), 2.74 (d, 2H).

### Example 58: synthesis of 3-(8-methyl-4-oxo-4,5-dihydro-3H-pyrimido[5,4-b]indol-3-yl)-N-(3-sulfamoylbenzyl)propanamide (compound 88)

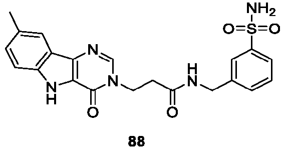

Except that 1b in Example 1 was replaced with 3-aminomethylbenzenesulfonamide, the same synthetic route as in Example 1 was adopted to prepare the title compound 88 (white solid).
LCMS: 440 [M+H]⁺
¹H NMR (400 MHz, DMSO-*d₆*) δ 12.00 (s, 1H), 8.61 (t, 1H), 8.18 (s, 1H), 7.77 (s, 1H), 7.70-7.60 (m, 2H), 7.42-7.21 (m, 6H), 4.43-4.19 (m, 4H), 2.71 (t, 2H), 2.44 (s, 3H).

### Example 59: synthesis of 3-(8-methyl-4-oxo-4,5-dihydro-3H-pyrimido[5,4-b]indol-3-yl)-N-(2-((3-(trifluoromethyl)phenyl)amin o)ethyl)propanamide (compound 89)

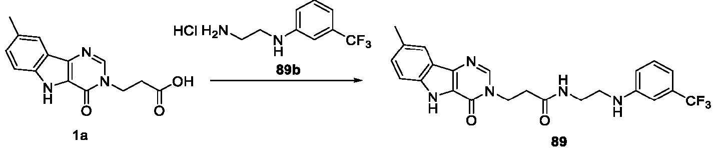

Except that compound 1b in Example 1 was replaced with 89b, the same synthetic route as in Example 1 was adopted to prepare the title compound 89 (white solid).
LCMS: 458 [M+H]⁺
¹H NMR (400 MHz, DMSO-*d₆*) δ 11.99 (s, 1H), 8.18 (s, 1H), 8.10 (t, 1H), 7.75 (s, 1H), 7.40 (d, 1H), 7.30-7.18 (m, 2H), 6.79-6.74 (m, 3H), 6.09 (t, 1H), 4.28 (t, 2H), 3.17 (q, 2H), 3.04 (q, 2H), 2.62 (t, 2H), 2.43 (s, 3H).

### Example 60: synthesis of 3-(8-methyl-4-oxo-4,5-dihydro-3H-pyrimido[5,4-b]indol-3-yl)-N-(2-(3-(trifluoromethyl)phenoxy)ethyl )propanamide (compound 90)

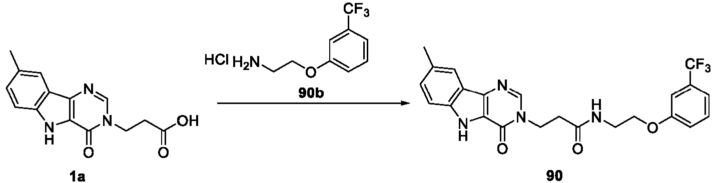

Except that 1b in Example 1 was replaced with 90b, the same synthetic route as in Example 1 was adopted to prepare the title compound 90 (white solid).
LCMS: 459 [M+H]⁺
¹H NMR (400 MHz, DMSO-*d₆*) δ 11.96 (s, 1H), 8.23 (t, 1H), 8.16 (s, 1H), 7.72 (s, 1H), 7.45-7.36 (m, 2H), 7.29-7.21 (m, 2H), 7.17 (s, 1H), 7.13 (d, 1H), 4.27 (t, 2H), 3.99 (t, 2H), 3.42-3-38 (m, 2H), 2.64 (t, 2H), 2.43 (s, 3H).

### Example 61: synthesis of N-methyl-3-(8-methyl-4-oxo-4,5-dihydro-3H-pyrimido[5,4-b]indol-3-yl)-N-(2-((3-(trifluoromethyl)ph enyl)amino)ethyl)propanamide (compound 91)

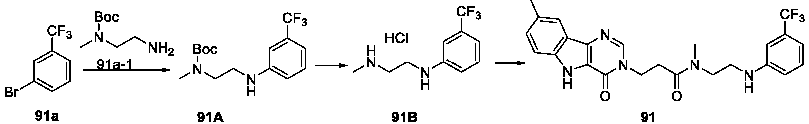

Step 1: 3-(trifluoromethyl)bromobenzene (1.125 g, 5.0 mmol, 1.0 eq.), compound 91a-1 (1.3 g, 7.5 mmol, 1.5 eq.), Pd₂(dba)₃ (0.915 g, 1.0 mmol, 0.2 eq.), X-phos (0.475 g, 1.0 mmol, 0.2 eq.), potassium phosphate (3.18 g, 15 mmol, 3.0 eq.) and 1,4-dioxane (30 mL) were added to a reaction flask. The flask was purged with nitrogen three times and stirred at 100 °C for 12 hours. After filtration, the filter cake was washed with ethyl acetate, concentrated, and purified by silica gel column chromatography (eluent: PE:EtOAc = 100:1 - 90:10). The target fraction was collected and concentrated to obtain the intermediate 91A as a burgundy oily liquid (1.0 g, yield: 62.9%).

Step 2: 91A (1.0 g, 3.1 mmol, 1.0 eq.), hydrochloric acid (2.0 mol/L solution in ethyl acetate, 12.4 mL, 12.4 mmol, 4.0 eq.) and ethyl acetate (10 mL) were added to a reaction flask and stirred at room temperature for 3 hours. After filtration, the filter cake was washed with ethyl acetate and dried to obtain the intermediate 91B as an off-white solid (0.8 g, yield: 100%).

Step 3: Except that 1b in Example 1 was replaced with 91B, the same synthetic route as in Example 1 was adopted to prepare the title compound 91 (white solid).
LCMS: 472 [M+H]⁺
¹H NMR (400 MHz, DMSO-*d₆*) δ 11.97-11.94 (m, 1H), 8.31-8.22 (m, 1H), 7.76 (s, 1H), 7.42-7.39 (m, 1H), 7.30-7.16 (m, 2H), 6.86-6.72 (m, 3H), 6.16-6.13 (m, 1H), 4.30-4.21 (m, 2H), 3.43-3.39 (m, 2H), 3.27-3.13 (m, 2H), 2.95 (s, 2H), 2.85-2.79 (m, 3H), 2.44 (s, 3H). Note: Affected by the chiral nitrogen, most of the hydrogen atoms showed two sets of peaks.

### Example 62: synthesis of N-methyl-N-(2-(methyl(3-(trifluoromethyl)phenyl)amino)ethyl)-3-(8-methyl-4-oxo-4,5-dihydro-3H-py rimido[5,4-b]indol-3-yl)propanamide (compound 92)

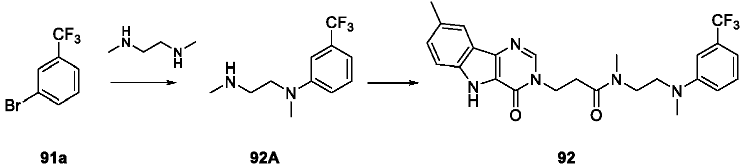

Step 1: Except that 91a-1 in Example 61 was replaced with N,N'-dimethylethylenediamine, the same synthetic method as in Step 1 of Example 61 was adopted to prepare compound 92A.

Step 2: Except that 1b in Example 1 was replaced with 92A, the same synthetic route as in Example 1 was adopted to prepare the title compound 92 (white solid).
LCMS: 486 [M+H]⁺
¹H NMR (400 MHz, DMSO-*d₆*) δ 11.96-11.93 (m, 1H), 8.30-8.19 (m, 1H), 7.76 (s, 1H), 7.43-7.39 (m, 1H), 7.30- 7.20 (m, 2H), 6.98-6.76(m, 3H), 4.26-4.21 (m, 2H), 3.56 - 3.34 (m, 4H), 2.93-2.87 (m, 5H), 2.84-2.69 (m, 3H), 2.44 (s, 3H). Note: Affected by the chiral nitrogen, most of the hydrogen atoms showed two sets of peaks.

### Example 63: synthesis of N-(2-(methyl(3-(trifluoromethyl)phenyl)amino)ethyl)-3-(8-methyl-4-oxo-4,5-dihydro-3H-pyrimido[5, 4-b]indol-3-yl)propanamide (compound 93)

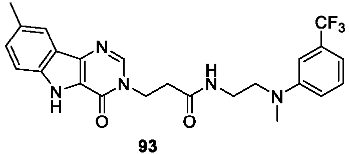

Except that 91a-1 in Example 61 was replaced with *tert-butyl* 2-(methylamino)ethylcarbamate, the same synthetic route as in Example 61 was adopted to prepare the title compound 93 (white solid).
LCMS: 472 [M+H]⁺
¹H NMR (400 MHz, DMSO-*d₆*) δ 11.98 (s, 1H), 8.16 (s, 1H), 8.10 (t, 1H), 7.75 (s, 1H), 7.40 (d, 1H), 7.31-7.26 (m, 2H), 6.92 (d, 1H), 6.88-6.82 (m, 2H), 4.24 (t, 2H), 3.36 (t, 2H), 3.17 (q, 2H), 2.84 (s, 3H), 2.56 (t, 2H), 2.43 (s, 3H).

### Example 64: synthesis of 8-methyl-3-(3-((3-(trifluoromethyl)benzyl)amino)propyl)-3,5-dihydro-4H-pyrimido[5,4-b]indol-4-one (Compound 94)

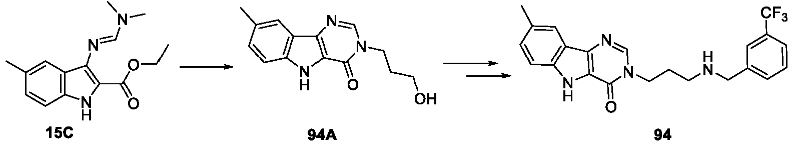

Step 1: Except that 4-aminobutanol in Step 1 of Example 29 was replaced with 3-amino-1-propanol, the same synthetic route as in Example 29 was adopted to prepare the intermediate 94A (yellow solid).

Step 2: Except that 1-(2,3-dichlorophenyl)piperazine hydrochloride in Example 31 was replaced with 3-(trifluoromethyl)benzylamine, the same synthetic route as in Example 31 was adopted to prepare the title compound 94 (white solid).
LCMS: 415 [M+H]⁺
¹H NMR (400 MHz, DMSO-*d₆*) δ 11.97 (s, 1H), 8.26 (s, 1H), 8.18 (s, 1H), 7.76 (d, 2H), 7.66 (d, 1H), 7.60 (d, 1H), 7.54 (t, 1H), 7.41 (d, 1H), 7.28 (d, 1H), 4.15 (t, 2H), 3.85 (s, 2H), 2.62 (t, 2H), 2.44 (s, 3H), 2.01-1.83 (m, 2H).

### Example 65: synthesis of 3-(trifluoromethyl)benzyl 3-(8-methyl-4-oxo-4,5-dihydro-3H-pyrimido[5,4-b]indol-3-yl)propanoate (compound 95)

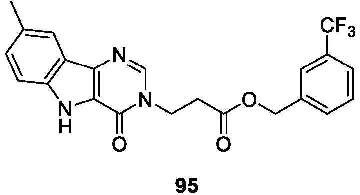

Except that 1b in Example 1 was replaced with meta-trifluoromethylbenzyl alcohol, the same synthetic route as in Example 1 was adopted to prepare the title compound 95 (off-white solid).
LCMS: 430 [M+H]⁺
¹H NMR (400 MHz, DMSO-*d₆*) δ 11.98 (s, 1H), 8.26 (s, 1H), 7.75 (s, 1H), 7.72 (s, 1H), 7.64 (t, 2H), 7.53 (t, 1H), 7.41 (d, 1H), 7.28 (d, 1H), 5.17 (s, 2H), 4.33 (t, 2H), 2.95 (t, 2H), 2.44 (s, 3H).

### Example 66: synthesis of 3-(8-methyl-4-oxo-4,5-dihydro-3H-pyrimido[5,4-b]indol-3-yl)-N-(2-((5,6,7,8-tetrahydronaphthalen-1-yl)amino)ethyl)propanamide (compound 129)

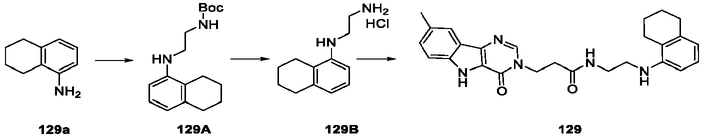

Step 1: 5,6,7,8-Tetrahydro-1-naphthylamine (129a) (2 g, 0.0136 mol, 1.0 eq.), 2-(BOC-amino)bromoethane (3.35 g, 0.0149 mol, 1.1 eq.), cesium carbonate (8.9 g, 0.0272 mol, 2.0 eq.) and anhydrous DMF (50 mL) were added to a 100 mL single-necked flask. Under nitrogen protection, the mixture was heated to 70 °C and reacted for 1 h. After the reaction was completed, the mixture was cooled to room temperature, diluted with a large amount of water, and extracted twice with DCM. The DCM layer was washed three times with saturated brine, separated, and dried over anhydrous sodium sulfate. The solvent was removed from the organic phase, and the crude product was purified by column chromatography (eluent: DCM:MeOH = 100:1 - 10:1) to obtain the intermediate 129A as a pale yellow oil (0.45 g, yield: 11.4%).

Step 2: 129A (0.15 g, 0.000517 mol, 1.0 eq.) and a solution of hydrogen chloride in 1,4-dioxane (2 mL) were added to a 50 mL single-necked flask and reacted at room temperature for 1 h. The solvent was removed by rotary evaporation to obtain the crude product 129B, which was directly used in the next step.

Step 3: Except that 1b in Example 1 was replaced with 129B, the same synthetic route as in Example 1 was adopted to prepare the title compound 129 (white solid).
LCMS: 444 [M+1]⁺
¹H NMR (400 MHz, DMSO-*d₆*): δ 12.00 (s, 1H), 8.23-8.13 (m, 2H), 7.76 (s, 1H), 7.42 (d, 1H), 7.29 (d, 1H), 6.85 (t, 1H), 6.31 (dd, 2H), 4.69 (s, 1H), 4.30 (t, 2H), 3.23 (q, 2H), 3.05 (q, 2H), 2.65 (t, 2H), 2.58 (t, 2H), 2.45 (s, 3H), 2.23 (t, 2H), 1.75-1.65 (m, 2H), 1.63-1.53 (m, 2H).

### Example 67: synthesis of N-(2-(methyl(5,6,7,8-tetrahydronaphthalen-1-yl)amino)ethyl)-3-(8-methyl-4-oxo-4,5-dihydro-3H-pyri mido[5,4-b]indol-3-yl)propanamide (compound 130)

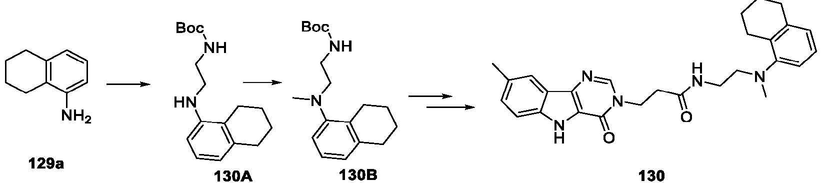

Except that the following step was added between Step 1 and Step 2 of the synthetic route in Example 66, the same synthetic route as in Example 66 was adopted to prepare the title compound 130 (white solid).

130A (0.15 g, 0.000517 mol, 1.0 eq.) and anhydrous DMF (3 mL) were added to a 100 mL three-necked flask. Under nitrogen protection, the mixture was cooled in an ice-bath. When the temperature dropped to 0 - 5 °C, NaH (0.025 g, 0.00062 mol, 1.2 eq., 60%) was added. After reacting for 1 h at this temperature, a solution of methyl iodide (0.088 g, 0.00062 mol, 1.2 eq.) in anhydrous DMF (1 mL) was added, and the mixture was reacted at room temperature for 1 h. After the reaction was completed, the mixture was diluted with a large amount of water, extracted with EA. The EA layer was washed three times with saturated brine, separated, and dried over anhydrous sodium sulfate. The solvent was removed from the organic phase, and the crude product was purified by column chromatography (eluent: DCM:MeOH = 100:1 - 10:1) to obtain the intermediate 130B as a pale yellow oil (0.13 g, yield: 82.8%).
LCMS: 458 [M+1]⁺
¹H NMR (400 MHz, DMSO-*d₆*): δ 11.98 (s, 1H), 8.37 (d, 2H), 7.77 (s, 1H), 7.43 (d, 1H), 7.30 (d, 1H), 6.89-6.83 (m, 1H), 6.45-6.30 (m, 2H), 4.32-4.24 (m, 2H), 3.53-3.44 (m, 2H), 3.23-3.18 (m, 2H), 2.96 (s, 2H), 2.86-2.81 (m, 3H), 2.60-2.58 (m, 2H), 2.45 (s, 3H), 2.28-2.27 (m, 2H), 1.73-1.72 (m, 2H), 1.59-1.58 (m, 2H).

### Example 68: synthesis of 3-(8-methyl-4-oxo-4,5-dihydro-3H-pyrimido[5,4-b]indol-3-yl)-N-(2-((5,6,7,8-tetrahydronaphthalen-2-yl)amino)ethyl)propanamide (compound 131)

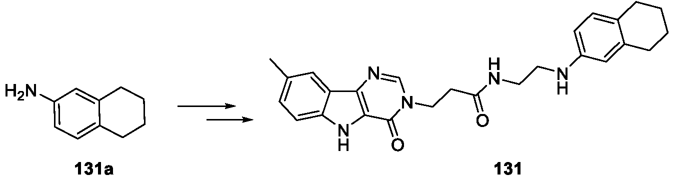

Except that 129a in Example 66 was replaced with 131a, the same synthetic route as in Example 66 was adopted to prepare the title compound 131 (white solid).
LCMS: 444 [M+1]⁺
¹H NMR (400 MHz, DMSO-*d₆*): δ 12.00 (s, 1H), 8.19 (s, 1H), 8.08 (s, 1H), 7.77 (s, 1H), 7.43 (d, 1H), 7.30 (d, 1H), 6.70 (d, 1H), 6.29 (d, 1H), 6.18 (s, 1H), 5.14 (s, 1H), 4.30 (t, 2H), 3.17-3.15 (m, 2H), 2.96 (s, 2H), 2.64 (t, 2H), 2.54 (s, 4H), 2.45 (s, 3H), 1.65 (s, 4H).

### Example 69: synthesis of 3-(8-methyl-4-oxo-4,5-dihydro-3H-pyrimido[5,4-b]indol-3-yl)-N-(2-(naphthalen-2-ylamino)ethyl)prop anamide (compound 132)

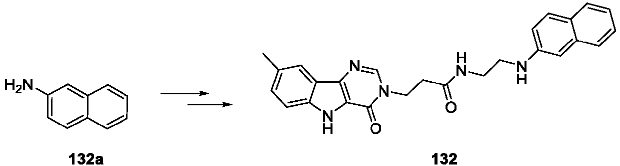

Except that 129a in Example 66 was replaced with 132a, the same synthetic route as in Example 66 was adopted to prepare the title compound 132 (white solid).
LCMS: 440 [M+1]⁺
¹H NMR (400 MHz, DMSO-*d₆*): δ 12.00 (s, 1H), 8.22 (s, 1H), 8.17 (t, 1H), 7.78 (s, 1H), 7.61 (d, 1H), 7.54 (dd, 2H), 7.43 (d, 1H), 7.33-7.24 (m, 2H), 7.10 (t, 1H), 6.90 (d, 1H), 6.71 (s, 1H), 5.86 (t, 1H), 4.31 (t, 2H), 3.28 (q, 2H), 3.12 (q, 2H), 2.66 (t, 2H), 2.44 (s, 3H).

### Example 70: synthesis of N-(2-(6-chloro-1H-pyrrolo[3,2-c]pyridin-1-yl)ethyl)-3-(8-methyl-4-oxo-4,5-dihydro-3H-pyrimido[5,4-b]indol-3-yl)propanamide (compound 133)

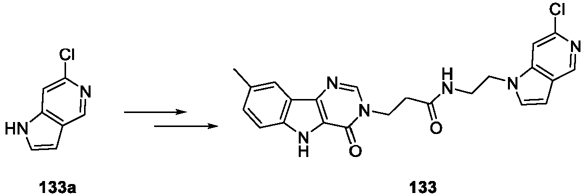

Except that 129a in Example 66 was replaced with 133a, the same synthetic route as in Example 66 was adopted to prepare the title compound 133 (white solid).
LCMS: 449 [M+1];
¹H NMR (400 MHz, DMSO-*d₆*) δ 11.99 (s, 1H), 8.52 (s, 1H), 8.14 (s, 1H), 8.08 (s, 1H), 7.76 (s, 1H), 7.61 (s, 1H), 7.41 (d, 1H), 7.27 (t, 2H), 6.37 (d, 1H), 4.19 (d, 4H), 3.34 (d, 4H), 2.44 (s, 3H).

### Example 71: synthesis of N-(imidazo[1,2-a]pyridin-2-ylmethyl)-3-(8-methyl-4-oxo-4,5-dihydro-3H-pyrimido[5,4-b]indol-3-yl)p ropanamide (compound 134)

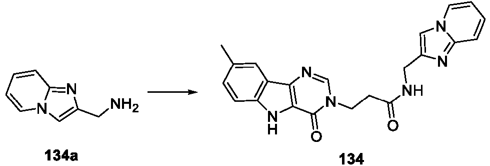

Except that 1b in Example 1 was replaced with 134a, the same synthetic route as in Example 1 was adopted to prepare the title compound 134 (off-white solid).
LCMS: 401 [M+1];
¹H NMR (400 MHz, DMSO-*d₆*) δ 12.00 (s, 1H), 8.49 (s, 1H), 8.30 (d,1H), 8.20 (s, 1H), 7.75 (s, 1H), 7.57 (s, 1H), 7.47-7.36 (m, 2H), 7.28 (d, 1H), 7.20-7.12 (m, 1H), 6.79 (t, 1H), 4.39-4.26 (m, 4H), 2.69 (t, 2H), 2.44 (s, 3H).

### Example 72: synthesis of N-(2-(6-chloro-1H-pyrrolo[3,2-b]pyridin-1-yl)ethyl)-3-(8-methyl-4-oxo-4,5-dihydro-3H-pyrimido[5,4-b]indol-3-yl)propanamide (compound 135)

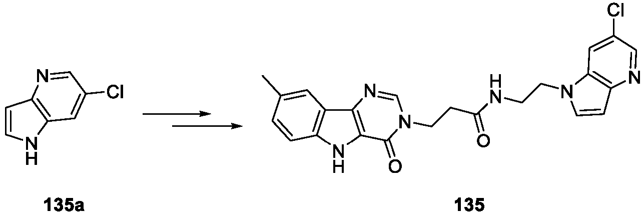

Except that 129a in Example 66 was replaced with 135a, the same synthetic route as in Example 66 was adopted to prepare the title compound 135 (white solid).
LCMS: 449 [M+1];
¹H NMR (400 MHz, DMSO-*d₆*) δ 12.00 (s, 1H), 8.27 (s, 1H), 8.16 (s, 1H), 8.07 (s, 2H), 7.76 (s, 1H), 7.42 (d, 2H), 7.28 (d, 1H), 6.32 (s, 1H), 4.21 (d, 4H), 3.33 (s, 4H), 2.43 (s, 3H).

### Example 73: synthesis of 8-methyl-3-(3-oxo-3-(4-(3-(trifluoromethyl)phenyl)piperazin-1-yl)propyl)pyrimido[5,4-b]quinolin-4(3 H)-one (Compound 138)

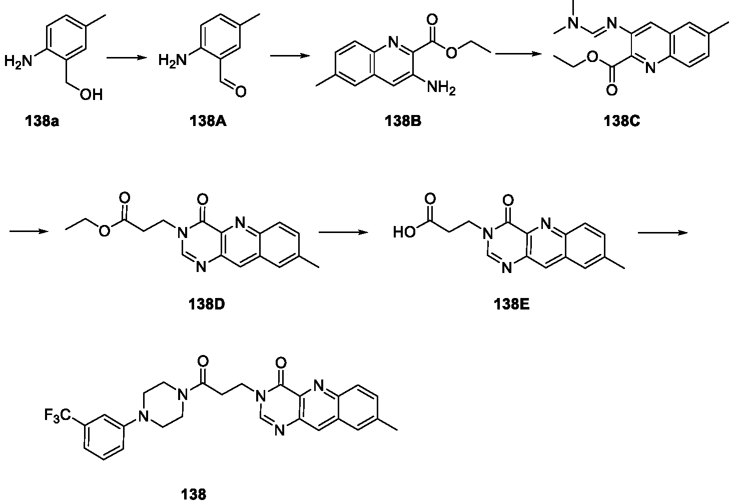

Step 1: 2-amino-5-methylbenzyl alcohol (5 g, 0.0365 mol, 1 eq.) (138a), manganese dioxide (3.2 g, 0.0365 mol, 1.0 eq.) and dichloromethane (100 mL) were added to a 250 mL three-necked flask. Under nitrogen protection, the mixture was heated to 50 °C and stirred for 3 hours. After the reaction was completed, the product was purified by silica gel column chromatography (dichloromethane:methanol = 10:1) and concentrated under reduced pressure to obtain the intermediate 138A as a yellow solid (4.5 g, yield: 91.3%).

Step 2: Methyl bromopyruvate (0.8 g, 0.0044 mol, 1 eq.) and ethanol (20 mL) were added to a 250 mL three-necked flask. Then pyridine (0.35 g, 0.0044 mol, 1 eq.) was added, and the mixture was stirred under reflux for 1 hour. After cooling to 20 °C, pyridine (1.6 g, 0.02 mol, 4.6 eq.) and 138A (0.6 g, 0.0044 mol, 1 eq) were added, and the reflux was continued for 4 hours. Then pyrrolidine (0.94 g, 0.0132 mol, 3.0 eq) was added, the mixture was heated to 90 °C and refluxed for 1 hour. After cooling to 20 °C, the product was purified by silica gel column chromatography (ethyl acetate:petroleum ether = 1:5) and concentrated under reduced pressure to obtain the intermediate 138B as a yellow liquid (0.86 g, yield: 85%).

Step 3: 138B (0.9 g, 0.0039 mol, 1 eq.)/DMF-DMA (1.8 mL) and DMF (10 mL) were added to a 250 mL three-necked flask and heated to 90 °C and stirred for 2 hours. Then ethyl 3-aminopropionate (1.3 g, 0.008 mol, 2 eq.) and ethanol (10 mL) were added, and the reflux was continued for 3 hours. After the reaction was completed, the product was purified by silica gel column chromatography (dichloromethane:methanol = 20:1) and concentrated to dryness under reduced pressure to obtain the intermediate 138C as a yellow solid (0.95 g, yield: 76.9%).

Step 4 - 6: Except that 3E in Example 3 was replaced with 138D and 1-(2,4-dichlorophenyl)piperazine hydrochloride was replaced with 1-(3-(trifluoromethyl)phenyl)piperazine, the same synthetic route as in Example 3 was adopted to prepare the title compound 138 (off-white solid).
LCMS: 496 [M+1]⁺
¹H NMR (400 MHz, DMSO-*d₆*): δ 8.59 (s, 1H), δ 8.44 (s, 1H), δ 8.09 (d, 1H), δ 7.90 (s, 1H), 7.71 (d, 1H), δ 7.38 (s, 1H), 7.18-7.13 (m, 2H), 7.05 (s, 1H), 4.23 (t, 2H), 3.57 (br, 4H), 3.21-3.16 (m, 4H), 2.93 (t, 2H), 2.53(s, 3H).

### Example 74: synthesis of methyl (2-(4-(3-(2-(trifluoromethyl)-10H-phenothiazin-10-yl)propyl)piperazin-1-yl)ethyl) succinate (compound 184)

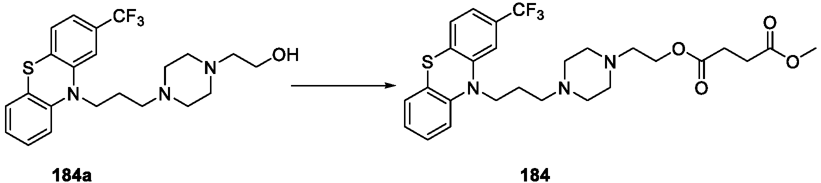

**184a** (100 mg, 0.23 mmol, 1.0 eq.) and dichloromethane (4 mL) were added to a reaction flask. Triethylamine (70 mg, 0.69 mmol, 3.0 eq) was added, and then methyl succinyl chloride (52 mg, 0.34 mmol, 1.5 eq) was added dropwise under ice-water bath. The reaction solution was warmed to room temperature and reacted for 4 hours. After LCMS monitoring showed that the starting materials were completely reacted, the reaction solution was filtered, concentrated, and separated by C18 reverse-phase column to obtain the title compound 184 (white solid) (84 mg, yield: 66%).
LCMS: 552 [M+H]⁺
¹H NMR (400 MHz, DMSO-*d₆*) δ 7.24-7.14 (m, 4H), 7.04 (s, 1H), 7.00 (t, 1H), 6.93 (d, 1H), 4.13 (t, 2H), 4.01 (t, 2H), 3.68 (s, 3H), 3.15-3.12 (m, 2H), 2.76 (t, 2H), 2.61 (br, 4H), 2.21-2.15 (m, 4H), 1.74-1.71 (m, 2H), 1.59-1.57 (m, 2H), 1.47 (br, 2H).

### Example 75: synthesis of 1-(3-(10H-phenothiazin-10-yl)propyl)piperidine-2-carboxamide (compound 185) formate

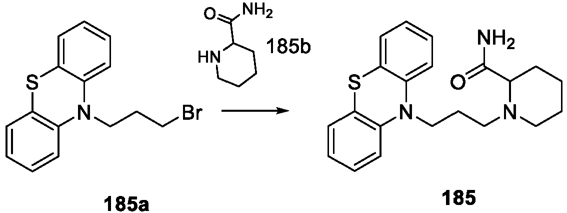

185a (100 mg, 0.31 mmol, 1.0 eq.) and N,N-dimethylformamide (4 mL) were added to a reaction flask. Potassium carbonate (130 mg, 0.93 mmol, 3.0 eq), 2-piperidinecarboxamide (185b) (54 mg, 0.37 mmol, 1.2 eq) and potassium iodide (10 mg, 0.038 mmol, 0.1 eq) were added. The reaction solution was reacted at 95 °C for 4 hours. After LCMS monitoring showed that the starting materials were completely reacted, the reaction solution was filtered and purified by C18 reverse-phase column to obtain the formate of the title compound 185 (white solid) (60 mg, yield: 52%).
LCMS: 368 [M+H]⁺
¹H NMR (400 MHz, DMSO-*d₆*) δ 8.16 (s, 1H), 7.20-7.11 (m, 4H), 7.03-6.90 (m, 4H), 3.96-3.92 (m, 1H), 3.79-3.75 (m, 1H), 3.00-2.97 (m, 1H), 2.69-2.52 (m, 2H), 2.31-2.29 (m, 1H), 2.02-1.97 (m, 1H), 1.86-1.82 (m, 2H), 1.68-1.43 (m, 5H), 1.24-1.19 (m, 1H).

### Example 76: synthesis of 1-(3-(10H-phenothiazin-10-yl)propyl)piperidin-2-one (Compound 186)

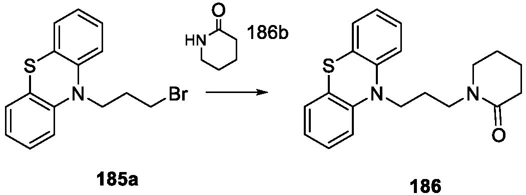

185a (100 mg, 0.31 mmol, 1.0 eq.) and N,N-dimethylformamide (4 mL) were added to a reaction flask. Sodium hydride (20 mg, 0.48 mmol, 1.5 eq) and piperidone (118b) (50 mg, 0.48 mmol, 1.5 eq) were added. The reaction solution was reacted at room temperature for 4 hours. After LCMS monitoring showed that the starting materials were completely reacted, the reaction was quenched with ice-water, extracted with ethyl acetate, dried, concentrated, and purified by silica gel column chromatography (DCM:MeOH = 20:1) to obtain the title compound 186 (gray liquid) (100 mg, yield: 94%).
LCMS: 339 [M+H]⁺
¹H NMR (400 MHz, DMSO-*d₆*) δ 7.23-7.11 (m, 4H), 7.00 (d, 2H), 6.93 (t, 2H), 3.84 (t, 2H), 3.35 (s, 1H), 3.31 (s, 1H), 3.19-3.09 (m, 2H), 2.19-2.08 (m, 2H), 1.94-1.81 (m, 2H), 1.66-1.54 (m, 4H).

### Example 77: synthesis of 1-(6-(2-(trifluoromethyl)-10H-phenothiazin-10-yl)hexyl)piperidin-2-one (Compound 187)

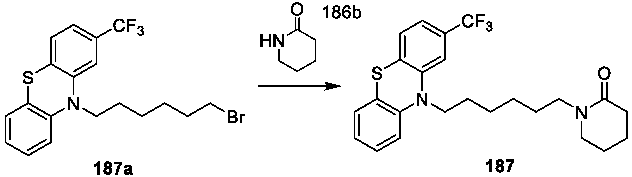

2-Piperidone (186b) (86 g, 0.87 mmol, 1.25 eq.) was dissolved in N,N-dimethylformamide (3 mL). Sodium hydride (45 mg, 1.12 mmol, 1.6 eq.) was slowly added under ice-bath, and the reaction was carried out for 0.5 h. Then a solution of 187a (300 mg, 0.70 mmol, 1.0 eq.) in N,N-dimethylformamide was added dropwise, and the reaction was carried out at room temperature for 4 h. The reaction was quenched with water (20 mL), extracted with ethyl acetate (3×20 mL). The combined organic phases were washed with saturated brine (20 mL), dried and concentrated to obtain a crude product, which was purified by reverse-phase column chromatography (eluent: 0.1% formic acid aqueous solution:MeOH = 9:1 - 2:8). The target fraction was collected and concentrated to obtain the title compound 187 (brown oily liquid) (200 mg, yield: 64%).
LCMS: 448 [M+H]⁺
¹H NMR (400 MHz, DMSO-*d₆*) δ 7.33 (d, 1H), 7.26-7.13 (m, 4H), 7.03 (d, 1H), 6.97 (t, 1H), 3.90 (t, 2H), 3.18-3.09 (m, 4H), 2.11 (t, 2H), 1.68-1.57 (m, 6H), 1.41-1.30 (m, 4H), 1.22-1.16 (m, 2H).

### Example 78: synthesis of N-(3-(10H-phenothiazin-10-yl)propyl)imidazo[1,2-a]pyridin-3-amine (compound 188) formate

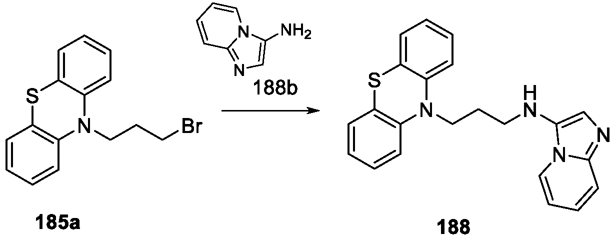

185a (383 mg, 1.2 mmol, 1.2 eq.) and acetonitrile (6 mL) were added to a reaction flask, followed by addition of potassium carbonate (415 mg, 3.0 mmol, 3.0 eq), imidazo[1,2-a]pyridin-3-amine (188b) (133 mg, 1.0 mmol, 1.0 eq) and potassium iodide (20 mg, 0.12 mmol, 0.1 eq). The reaction solution was reacted at 60 °C for 4 hours. After LCMS monitoring showed that the starting materials were completely reacted, the reaction solution was filtered, evaporated to dryness, and purified by C18 reverse-phase column to obtain the formate of the title compound 188 (white solid) (160 mg, yield: 36%).
LCMS: 373 [M+H]⁺
¹H NMR (400 MHz, DMSO-*d₆*) δ 8.48 (d, 1H), 8.39 (s, 1H), 7.83 (d, 1H), 7.63-7.47 (m, 1H), 7.33 (t, 1H), 7.27 (s, 1H), 7.16 (t, 4H), 7.04-6.88 (m, 4H), 6.06 (s, 1H), 4.43 (s, 2H), 3.92 (t, 2H), 2.29-2.15 (m, 2H).

### Example 79: synthesis of 2-(3-(10H-phenothiazin-10-yl)propyl)isoindolin-1-one (Compound 189)

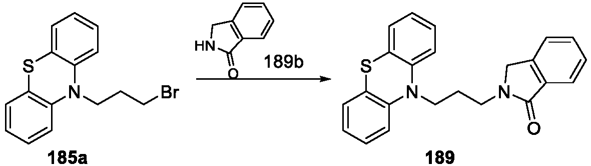

Except that 186b in Example 76 was replaced with 189b, the same synthetic route as in Example 76 was adopted to prepare the title compound 189 (white solid).
LCMS: 373 [M+H]⁺
¹H NMR (400 MHz, DMSO-*d₆*) δ 7.63 (d, 1H), 7.60-7.48 (m, 2H), 7.44 (t, 1H), 7.21-7.10 (m, 4H), 7.01 (d, 2H), 6.93 (t, 2H), 4.41 (s, 2H), 3.93 (t, 2H), 3.62 (t, 2H), 2.15-1.96 (m, 2H).

### Example 80: synthesis of 6-(3-(10H-phenothiazin-10-yl)propoxy)indoline-2-one (Compound 190)

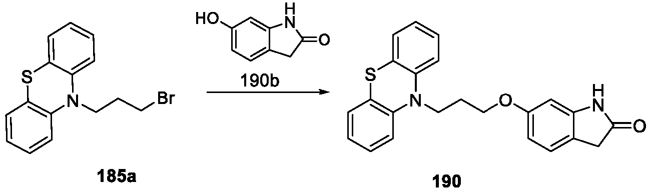

Except that 188b in Example 78 was replaced with 190b, the same synthetic route as in Example 78 was adopted to prepare the title compound 190 (white solid).
LCMS: 389 [M+H]⁺
¹H NMR (400 MHz, DMSO-*d₆*) δ 10.27 (s, 1H), 7.23-7.10 (m, 4H), 7.07 (d, 2H), 7.01 (d, 1H), 6.93 (t, 2H), 6.41 (d, 1H), 6.33 (s, 1H), 4.02 (d, 4H), 3.32 (d, 2H), 2.15-1.97 (m, 2H).

### Example 81: synthesis of methyl 1-(3-(10H-phenothiazin-10-yl)propyl)piperidin-2-carboxylate (compound 191) formate

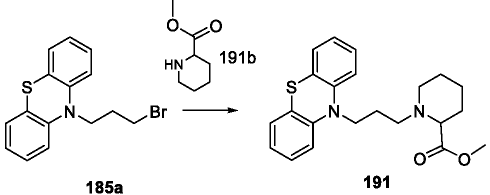

Except that 185b in Example 75 was replaced with 191b, the same synthetic route as in Example 75 was adopted to prepare the formate of the title compound 191 (white solid).
LCMS: 383 [M+H]⁺
¹H NMR (400 MHz, DMSO-*d₆*) δ 8.13 (s, 1H), 7.18 (t, 2H), 7.12 (d, 2H), 7.02 (d, 2H), 6.92 (t, 2H), 3.96-3.81 (m, 2H), 3.54 (s, 3H), 3.14 (s, 1H), 2.92-2.78 (m, 1H), 2.61-2.50 (m, 1H), 2.42-2.36 (m, 1H), 2.24-2.20 (m, 1H), 1.79-1.72 (m, 2H), 1.62-1.54 (m, 2H), 1.44-1.32 (m, 4H).

### Example 82: synthesis of 1-(3-(10H-phenothiazin-10-yl)propyl)piperidin-2-carboxylic acid (compound 192)

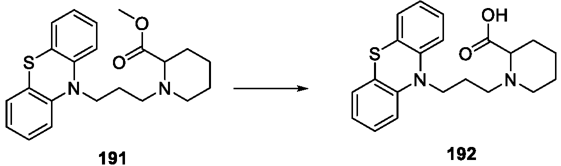

191 (100 mg, 0.26 mmol, 1.0 eq.), methanol (3 mL)/water (1 mL) were added to a reaction flask, followed by addition of lithium hydroxide (60 mg, 1.3 mmol, 5.0 eq). The reaction solution was reacted at 45 °C for 12 hours. After LCMS monitoring showed that the starting materials were completely reacted, the reaction solution was evaporated to dryness, the pH of the concentrated solution was adjusted to 3 - 5, evaporated to dryness, and then purified by C18 reverse-phase column to obtain the title compound 192 (gray solid) (40 mg, yield: 42%).
LCMS: 369 [M+H]⁺
¹H NMR (400 MHz, DMSO-*d₆*) δ 8.30 (s, 1H), 7.19 (t, 2H), 7.14 (d, 2H), 7.04 (d, 2H), 6.93 (t, 2H), 3.95-3.82 (m, 4H), 3.11-3.05 (m, 2H), 2.97-2.94 (m, 1H), 2.73-2.70 (m, 1H), 1.93-1.90 (m, 2H), 1.79-1.74 (m, 1H), 1.64-1.62 (m, 1H), 1.51 (br, 2H), 1.34 (br, 1H).

### Example 83: synthesis of 1-(6-(6-(trifluoromethyl)-2,3-dihydro-4H-benzo[b][1,4]thiazin-4-yl)hexyl)piperidin-2-one (Compound 193)

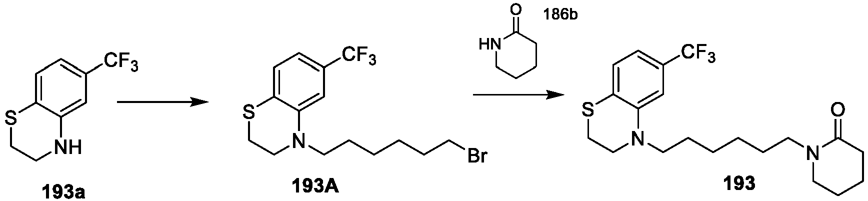

Step 1: 193a (600 mg, 2.73 mmol, 1.0 eq.), DMF (10 mL), K₂CO₃ (1.13 g, 8.19 mmol, 3.0 eq.) and 1,6-dibromohexane (660 mg, 2.73 mmol, 1.0 eq) were added to a reaction flask. The mixture was stirred at 90 °C overnight. After the reaction solution was filtered, it was purified by column chromatography (eluent: 0.1% formic acid aqueous solution:MeOH = 4:6). The target fraction was collected and concentrated under reduced pressure to obtain the white solid 193A (200 mg, yield: 19%).

Step 2: 193A (200 mg, 0.53 mmol, 1.0 eq.) and DMF (2 mL) were added to a reaction flask. The temperature was lowered to 0 °C, and after purging with nitrogen, sodium hydride (38 mg, 1.59 mmol, 3.0 eq.) was added. After stirring at room temperature for 30 min, piperidin-2-one (186b) (79 mg, 0.80 mmol, 1.5 eq) was added. The mixture was stirred at room temperature for 2 hours. Water was added dropwise to quench the reaction. After extraction with ethyl acetate, the organic phase was concentrated and purified by column chromatography (eluent: 0.1% formic acid aqueous solution:MeOH = 55:45). The target fraction was collected and concentrated under reduced pressure to obtain the title compound 193 (white solid) (64 mg, yield: 30%).
LCMS: 401 [M+H]⁺
¹H NMR (400 MHz, DMSO-*d₆*) δ 7.15 (d, 1H), 6.85-6.78 (m, 2H), 3.65-3.61 (m, 2H), 3.27-3.22 (m, 4H), 3.13-3.06 (m, 2H), 2.20 (t, 2H), 1.75-1.65 (m, 4H), 1.62-1.42 (m, 5H), 1.40-1.24 (m, 5H).

### Example 84: synthesis of 1-(3-(6-(trifluoromethyl)-2,3-dihydro-4H-benzo[b][1,4]thiazin-4-yl)propyl)piperidin-2-one (Compound 194)

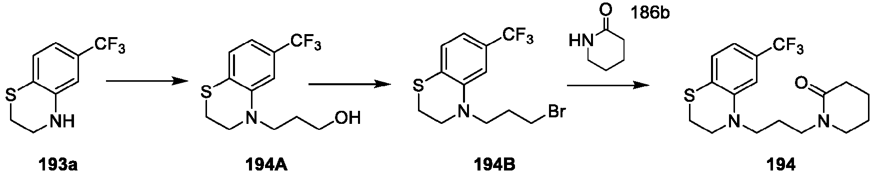

Step 1: Except that 1,6-dibromohexane in Step 1 of Example 83 was replaced with 3-bromopropan-1-ol, the same synthetic route as in Step 1 of Example 83 was adopted to prepare compound 194A (white solid).

Step 2: 194A (200 mg, 0.72 mmol, 1.0 eq.) and 40% hydrobromic acid aqueous solution were added to a reaction flask and refluxed with stirring at 100 °C overnight. The reaction solution was concentrated under reduced pressure to obtain the white solid 194B (150 mg, yield: 62%).

Step 3: Except that 193A in Step 2 of Example 83 was replaced with 194B, the same synthetic route as in Step 2 of Example 83 was adopted to prepare the title compound 194 (white solid).
LCMS: 359 [M+H]⁺
¹H NMR (400 MHz, DMSO-*d₆*) δ 7.16 (d, 1H), 6.85-6.81 (m, 2H), 3.64-3.61 (m, 2H), 3.37-3.32 (m, 6H), 3.13-3.10 (m, 2H), 2.23 (t, 2H), 1.80-1.70 (m, 6H).

### Example 85: synthesis of 4-(6-(2-oxopiperidin-1-yl)hexyl)-6-(trifluoromethyl)-2H-benzo[b][1,4]thiazin-3(4H)-one (Compound 195)

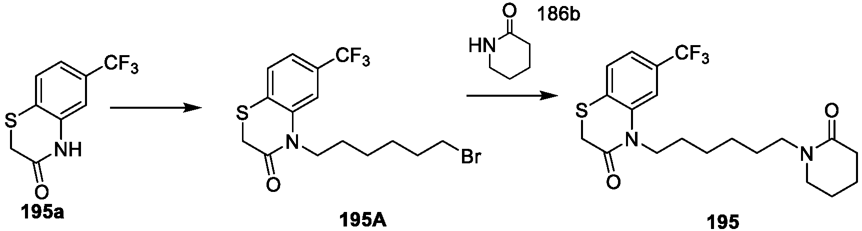

Except that 193a in Example 83 was replaced with 195a, the same synthetic route as in Example 83 was adopted to prepare compound 195 (white solid).
LCMS: 415 [M+H]⁺
¹H NMR (400 MHz, DMSO-*d₆*) δ 7.69 (d, 1H), 7.59 (s, 1H), 7.42 (d, 1H), 4.09 (t, 2H), 3.60 (s, 2H), 3.26-3.19 (m, 4H), 2.19 (t, 2H), 1.72-1.68 (m, 4H), 1.51-1.47 (m, 2H), 1.42-1.38 (m, 2H), 1.28-1.21 (m, 4H).

### Example 86: synthesis of 4-(3-(2-oxopiperidin-1-yl)propyl)-6-(trifluoromethyl)-2H-benzo[b][1,4]thiazin-3(4H)-one (Compound 196)

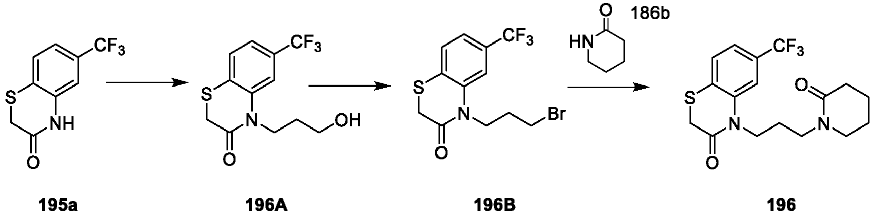

Step 1: Except that 1,6-dibromohexane in Step 1 of Example 83 was replaced with 3-bromopropan-1-ol and 193a was replaced with 195a, the same synthetic route as in Step 1 of Example 83 was adopted to prepare compound 196A.

Step 2: Except that 194A in Step 2 of Example 84 was replaced with 196A, the same synthetic route as in Step 2 of Example 84 was adopted to prepare compound 196B.

Step 3: Except that 193A in Step 2 of Example 83 was replaced with 196B, the same synthetic route as in Step 2 of Example 83 was adopted to prepare the title compound 196 (white solid).
LCMS: 373 [M+H]⁺
¹H NMR (400 MHz, CD₃OD) δ 7.64 (d, 1H), 7.55 (s, 1H), 7.39 (d, 1H), 4.13 (t, 2H), 3.53 (s, 2H), 3.45 (t, 2H), 3.41-3.32 (m, 2H), 2.39 (t, 2H), 1.93-1.83 (m, 6H).

### Example 87: synthesis of 2-(3-(6-(trifluoromethyl)-2,3-dihydro-4H-benzo[b][1,4]thiazin-4-yl)propyl)isoindolin-1-one (Compound 197)

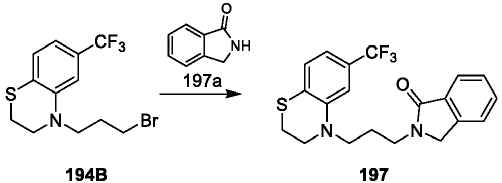

Except that 193A in Step 2 of Example 83 was replaced with 194B and 186b was replaced with 197a, the same synthetic route as in Step 2 of Example 83 was adopted to prepare the title compound 197 (white solid).
LCMS: 393 [M+H]⁺
¹H NMR (400 MHz, DMSO-*d₆*) δ 7.71 (d, 1H), 7.63-7.62 (m, 2H), 7.52 (dd, 1H), 7.16 (d, 1H), 6.83-7.81 (m, 2H), 4.54 (s, 2H), 3.67-3.61 (m, 4H), 3.45-3.39 (t, 2H), 3.14-3.10 (m, 2H), 1.98-1.85 (m, 2H).

### Example 88: synthesis of 7-(4-(2-(trifluoromethyl)-10H-phenothiazin-10-yl)butoxy)-3,4-dihydroquinolin-2(1H)-one (Compound 205)

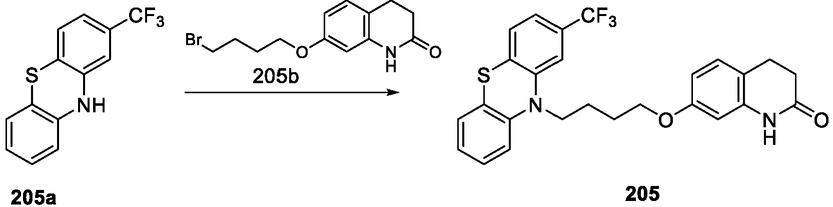

205a (200 mg, 0.75 mmol, 1.0 eq.) and N,N-dimethylformamide (4 mL) were added to a reaction flask. Then sodium hydride (60 mg, 1.50 mmol, 2.0 eq) was added. After the reaction solution was reacted at room temperature for 30 minutes, 205b (268 mg, 0.90 mmol, 1.2 eq.) was added. The reaction solution was reacted at room temperature for 4 hours. After LCMS monitoring showed that the starting materials were substantially reacted completely, the reaction was quenched by adding ice water, extracted with ethyl acetate, dried and concentrated. The residue was purified by silica gel column chromatography (DCM:MeOH = 20:1). The target fraction was collected and concentrated under reduced pressure to obtain the title compound (off-white solid) (250 mg, yield: 69%).
LCMS: 485 [M+H]⁺
¹H NMR (400 MHz, CDCl₃) δ 7.93 (s, 1H), 7.21-7.12 (m, 4H), 7.03-6.94 (m, 3H), 6.91 (d, 1H), 6.44 (dd, 1H), 6.21 (d, 1H), 3.99-3.92 (m, 4H), 2.89 (t, 2H), 2.60 (t, 2H), 2.0-1.88 (m, 4H).

### Example 89: synthesis of 7-((5-(2-(trifluoromethyl)-10H-phenothiazin-10-yl)pentyl)oxy)-3,4-dihydroquinolin-2(1H)-one (Compound 206)

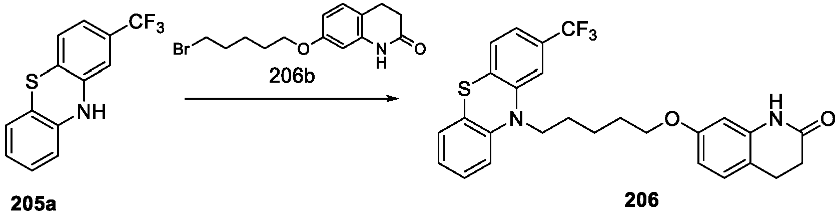

205a (0.294 g, 1.1 mmol, 1.1 eq.) and DMF (10 mL) were added to a reaction flask. NaH (52 mg, 1.3 mmol, 1.3 eq.) was added in batches under an ice bath. After the addition was completed, the mixture was stirred for 15 minutes, and then 206b (0.312 g, 1.0 mmol, 1.0 eq.) was added and stirred for 3 hours. The reaction was quenched by adding water under an ice bath and concentrated to obtain a crude product. The crude product was separated and purified by a silica gel column (eluent: PE:EtOAc = 1:1). The target fraction was collected and concentrated to dryness to obtain the title compound 206 (white solid) (85 mg, yield: 57.8%).
LCMS: 499 [M+H]⁺
¹H NMR (400 MHz, DMSO-*d₆*) δ 9.93 (s, 1H), 7.33 (d, 1H), 7.24-7.19 (m, 3H), 7.15 (d, 1H), 7.04 (d, 1H), 6.99-6.95 (m, 2H), 6.41-6.37 (m, 2H), 3.94 (t, 2H), 3.80 (t, 2H), 2.76 (t, 2H), 2.37 (t, 2H), 1.75-1.63 (m, 4H), 1.55-1.44 (m, 2H).

### Example 90: synthesis of 7-((6-(2-(trifluoromethyl)-10H-phenothiazin-10-yl)hexyl)oxy)-3,4-dihydroquinolin-2(1H)-one (Compound 207)

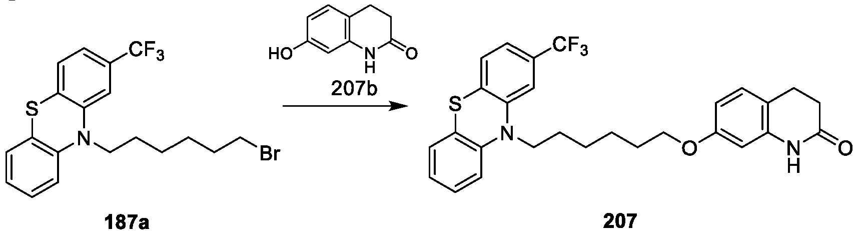

Compound 187a (100 mg, 0.23 mmol, 1.0 eq.) was dissolved in N,N-dimethylformamide (1 mL) and water (0.1 mL). Anhydrous potassium carbonate (42 mg, 0.30 mmol, 1.3 eq.) and 7-hydroxy-3,4-dihydroquinolin-2(1H)-one (207b) (46 mg, 0.28 mmol, 1.2 eq.) were added. The mixture was heated to 35 °C and reacted overnight. The reaction was diluted with water (20 mL), extracted with ethyl acetate (2 × 20 mL). The combined organic phases were washed with saturated brine (20 mL), dried and concentrated to obtain a crude product. The obtained crude product was purified by C18 reverse-phase column chromatography (eluent: 0.1% formic acid aqueous solution:MeOH = 1:9 - 9:1). The target fraction was collected and concentrated to obtain the title compound as a white solid (60 mg, yield: 50%).
LCMS: 513 [M+H]⁺
¹H NMR (400 MHz, DMSO-*d₆*) δ 9.94 (s, 1H), 7.32 (d, 1H), 7.25-7.17 (m, 3H), 7.15 (d, 1H), 7.04 (d, 1H), 6.97 (m, 2H), 6.41-6.34 (m, 2H), 3.92 (t, 2H), 3.80 (t, 2H), 2.74 (t, 2H), 2.38 (t, 2H), 1.72-1.56 (m, 4H), 1.37 (m, 4H).

### Example 91: synthesis of 3-(3-oxo-3-(4-(3-(trifluoromethyl)phenyl)piperazin-1-yl)propyl)benzo[4,5]thieno[3,2-d]pyrimidin-4(3 H)-one

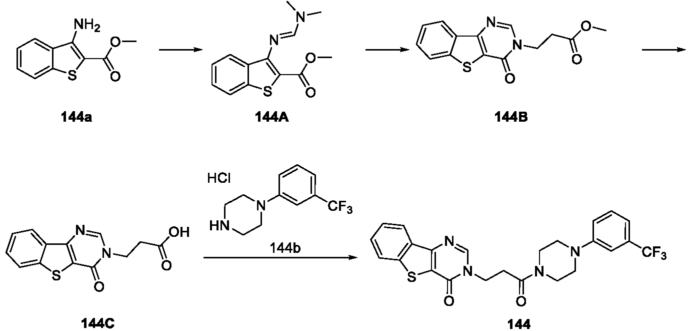

Step 1: 144a (5 g, 24.13 mmol, 1.0 eq) was added to DMF (50 mL), and then DMF-DMA (11.5 g, 96.50 mmol, 4.0 eq) was added. The mixture was stirred at 80 °C for 3 hours. The reaction solution was diluted with water, extracted with ethyl acetate. The organic phase was dried, filtered and evaporated to dryness to obtain a brown oil 144A (6 g, yield: 94.8%).

Step 2: 144A (6 g, 22.87 mmol, 1.0 eq) was added to MeOH (100 mL), and then methyl 3-aminopropionate hydrochloride (6.39 g, 45.74 mmol, 2.0 eq) was added. The mixture was stirred at 70 °C for 3 hours. The reaction solution was cooled to room temperature, and a large amount of solid precipitated. The reaction was filtered, and the filter cake was dried to obtain a brown solid 144B (4.1 g, yield: 62.17%).

Step 3: 144B (2 g, 6.94 mmol, 1.0 eq) was added to THF/MeOH/H₂O (60 mL/20 mL/20 mL), and then LiOH (498 mg, 20.81 mmol, 3.0 eq) was added. The mixture was stirred at 25 °C for 3 hours. The reaction solution was diluted with water, and the pH was adjusted to 6 - 7 with 1M hydrochloric acid aqueous solution until a large amount of solid precipitated. The reaction was filtered, and the filter cake was dried to obtain a yellow solid 144C (1.2 g, yield: 63.0%).

Step 4: 144C (150 mg, 0.547 mmol, 1.0 eq), 1-(3-(trifluoromethyl)phenyl)piperazine hydrochloride (144b) (175 mg, 0.656 mmol, 1.2 eq) and DIPEA (353 mg, 2.73 mmol, 5.0 eq) were added to DMF (3 mL), and then HATU (312 mg, 0.821 mmol, 1.5 eq) was added. The mixture was stirred at 25 °C for 2 hours. The reaction solution was purified by a C18 reverse-phase chromatography column (eluent: 0.1% formic acid aqueous solution:MeOH = 1:20 - 9:1). The target fraction was collected and freeze-dried to obtain an off-white solid 144 (37 mg, yield: 13.9%).
LCMS: 487 [M+H]⁺
¹H NMR (400 MHz, DMSO-*d₆*) δ 8.72 (s, 1H), 8.28 (d, 1H), 8.18 (d, 1H), 7.73-7.69 (m, 1H), 7.66-7.62 (m, 1H), 7.46-7.42 (m, 1H), 7.24-7.20 (m, 2H), 7.12-7.10 (m, 1H), 4.35 (t, 2H), 3.64-3.60 (m, 4H), 3.36-3.21 (m, 4H), 2.99 (t, 2H).

### Example 92: synthesis of 3-(3-(4-(2,3-dichlorophenyl)piperazin-1-yl)-3-oxopropyl)benzo[4,5]thieno[3,2-d]pyrimidin-4(3H)-one

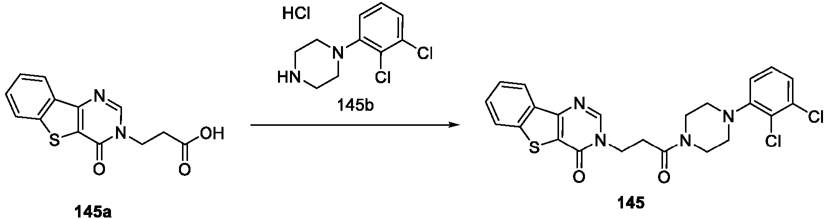

Except that 1-(3-(trifluoromethyl)phenyl)piperazine hydrochloride (144b) in Step 4 of Example 91 was replaced with 1-(2,3-dichlorophenyl)piperazine hydrochloride (145b), the same synthetic route as in Example 91 was adopted to prepare the title compound 145 (off-white solid).
LCMS: 487 [M+H]+
¹H NMR (400 MHz, DMSO-*d₆*) δ 8.72 (s, 1H), 8.29 (d, 1H), 8.20 (d, 1H), 7.73-7.70 (m, 1H), 7.66-7.63 (m, 1H), 7.35-7.27 (m, 2H), 7.09-7.06 (m, 1H), 4.35 (t, 2H), 3.64-3.62 (m, 4H), 3.00-2.92 (m, 6H).

### Example 93: synthesis of 3-(3-(4-(4-fluorobenzyl)piperidin-1-yl)-3-oxopropyl)benzo[4,5]thieno[3,2-d]pyrimidin-4(3H)-one

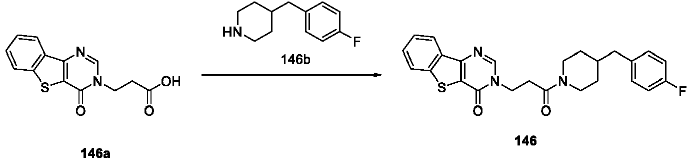

Except that 1-(3-(trifluoromethyl)phenyl)piperazine hydrochloride (144b) in Step 4 of Example 91 was replaced with 4-(4-fluorobenzyl)piperidine (146b), the same synthetic route as in Example 91 was adopted to prepare the title compound 146 (off-white solid).
LCMS: 450 [M+H]⁺
¹H NMR (400 MHz, DMSO-*d₆*) δ 8.67 (s, 1H), 8.28 (d, 1H), 8.20 (d, 1H), 7.73-7.69 (m, 1H), 7.66-7.62 (m, 1H), 7.14-7.06 (m, 4H), 4.38-4.28 (m, 3H), 3.82-3.78 (m, 1H), 2.96-2.81 (m, 3H), 2.49-2.35 (m, 3H), 1.76-1.64 (m, 1H), 1.55-1.50 (m, 2H), 0.98-0.87 (m, 2H).

### Example 94: synthesis of 3-(3-(4-hydroxy-4-(3-(trifluoromethyl)phenyl)piperidin-1-yl)-3-oxopropyl)benzo[4,5]thieno[3,2-d]pyr imidin-4(3H)-one

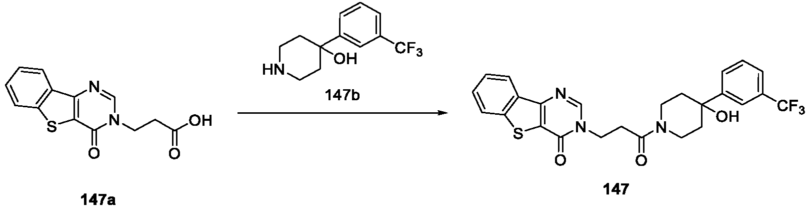

Except that 1-(3-(trifluoromethyl)phenyl)piperazine hydrochloride (144b) in Step 4 of Example 91 was replaced with 4-(3-(trifluoromethyl)phenyl)piperidin-4-ol (147b), the same synthetic route as in Example 91 was adopted to prepare the title compound 147 (off-white solid).
LCMS: 502 [M+H]+
¹H NMR (400 MHz, DMSO-*d₆*) δ 8.72 (s, 1H), 8.29 (d, 1H), 8.19 (d, 1H), 7.86 (s, 1H), 7.73-7.58 (m, 4H), 7.50 (t, 1H), 5.41 (s, 1H), 4.41-4.34 (m, 3H), 3.81-3.78 (m, 1H), 3.45-3.41 (m, 1H), 3.05-2.90 (m, 3H), 1.99-1.91 (m, 1H), 1.82-1.75 (m, 1H), 1.63-1.59 (m, 2H).

### Example 95: synthesis of 8-fluoro-3-(3-oxo-3-(4-(3-(trifluoromethyl)phenyl)piperazin-1-yl)propyl)benzofuro[3,2-d]pyrimidin-4 (3H)-one

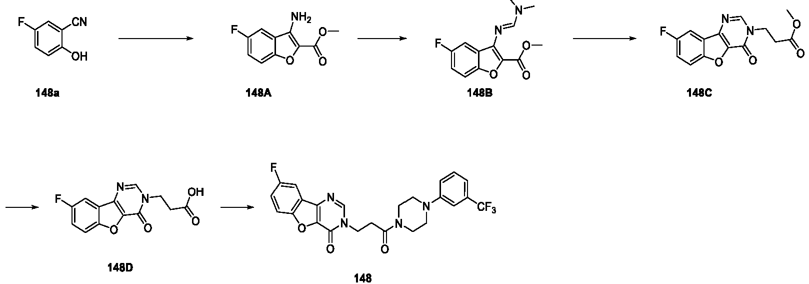

Step 1: 148a (2 g, 0.0146 mol, 1.0 eq.), methyl bromoacetate (2.68 g, 0.0175 mol, 1.2 eq.), potassium carbonate (4 g, 0.0292 mol, 2.0 eq.), and DMF (20 mL) were added to a 100 mL single-necked flask. The reaction was carried out at 60 °C for 16 h. After the reaction was completed, it was cooled to room temperature and filtered. The filter cake was washed with ethyl acetate. A large amount of water was added to the filtrate, and it was extracted twice with ethyl acetate. The ethyl acetate phase was washed three times with saturated brine, dried, and the solvent was removed to obtain a brown oil 148A. The crude product was directly used in the next step.

Step 2: 148A (crude product, 0.0146 mol, 1.0 eq.), DMF-DMA (5.22 g, 0.0438 mol, 3.0 eq.), and DMF (40 mL) were added to a 100 mL single-necked flask. The reaction was carried out at 60 °C for 3 h. After the reaction was completed, it was cooled to room temperature. A large amount of water was added to the reaction solution, and it was extracted twice with ethyl acetate. The ethyl acetate phase was washed three times with saturated brine, dried, and the solvent was removed to obtain a brown oil 148B. The crude product was directly used in the next step.

Step 3: 148B (crude product, 0.0146 mol, 1.0 eq.), methyl 3-aminopropionate hydrochloride (4.08 g, 0.0292 mol, 2.0 eq.), and acetonitrile (40 mL) were added to a 100 mL single-necked flask. The reaction was refluxed for 16 h. The mixture was stirred and cooled. After being cooled to room temperature, it was further cooled in an ice-bath and filtered. The filter cake was washed with methanol and dried to obtain a white solid 148C (1.43 g, yield: 33.7%).

Step 4: 148C (1.43 g, 0.00493 mol, 1.0 eq.), THF (12 mL), and water (3 mL) were added to a 100 mL single-necked flask. It was cooled to 0 - 5 °C in an ice-bath, and LiOH (0.236 g, 0.00986 mol, 2.0 eq.) was added. The reaction was maintained in the ice-bath for 1 h. After the reaction was completed, the pH was adjusted to 3 - 4 with dilute hydrochloric acid in the ice-bath. A large amount of solid precipitated. The reaction was filtered, and the filter cake was washed with water and dried to obtain a white solid 148D (1.12 g, yield: 82.2%).

Step 5: 148D (0.15 g, 0.000543 mol, 1.0 eq.), 1-(3-(trifluoromethyl)phenyl)piperazine hydrochloride (144b) (0.22 g, 0.000815 mol, 1.5 eq.), HATU (0.31 g, 0.00815 mol, 1.5 eq.), anhydrous DMF (5 mL), and DIPEA (0.35 g, 0.00272 mol, 5.0 eq.) were added to a 100 mL single-necked flask. The reaction was carried out at room temperature for 1 h. The reaction solution was subjected to reverse-phase column chromatography (eluent: 0.5‰ formic acid aqueous solution : methanol = 100:0 - 10:90) to collect the product fraction. The methanol was evaporated to dryness, and the residue was freeze-dried to obtain a pale yellow solid 148 (0.16 g, yield: 21.4%).
LCMS: 489 [M+1]⁺
¹H NMR (400 MHz, DMSO-*d₆*): δ 8.63 (s, 1H), 7.92 (dd, 1H), 7.86 (dd, 1H), 7.58-7.52 (m, 1H), 7.43 (t, 1H), 7.22-7.20 (m, 1H), 7.16 (m, 1H), 7.09 (d,1H), 4.35 (t, 2H), 3.61-3.56 (m, 4H), 3.25-3.18 (m, 4H), 2.96 (t, 2H).

### Example 96: synthesis of 3-(3-(4-(2,3-dichlorophenyl)piperazin-1-yl)-3-oxopropyl)-8-fluorobenzofuro[3,2-d]pyrimidin-4(3H)-o ne

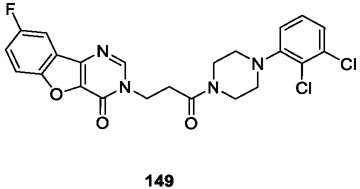

Except that 1-(3-(trifluoromethyl)phenyl)piperazine hydrochloride (144b) in Step 5 of Example 95 was replaced with 1-(2,3-dichlorophenyl)piperazine hydrochloride (145b), the same synthetic route as in Example 95 was adopted to prepare the title compound 149 (white solid).
LCMS: 489 [M+1]⁺
¹H NMR (400 MHz, DMSO-*d₆*): δ 8.64 (s, 1H), 7.93 (dd, 1H), 7.88-7.85 (m, 1H), 7.58-7.53 (m, 1H), 7.33-7.27 (m, 2H), 7.09-7.07 (m, 1H), 4.35 (t, 2H), 3.61-3.57 (m, 4H), 2.96-2.90 (m, 6H).

### Example 97: synthesis of 8-fluoro-3-(3-(4-(4-fluorobenzyl)piperidin-1-yl)-3-oxopropyl)benzofuro[3,2-d]pyrimidin-4(3H)-one

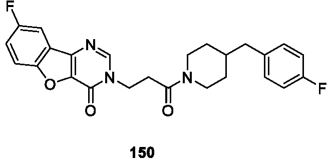

Except that 1-(3-(trifluoromethyl)phenyl)piperazine hydrochloride (144b) in Step 5 of Example 95 was replaced with 4-(4-fluorobenzyl)piperidine (146b), the same synthetic route as in Example 95 was adopted to prepare the title compound 150 (white solid).
LCMS: 452 [M+1]⁺
¹H NMR (400 MHz, DMSO-*d₆*): δ 8.60 (s, 1H), 7.93 (dd, 1H), 7.88 (dd, 1H), 7.58-7.53 (m, 1H), 7.15-7.11 (m, 2H), 7.09-7.04 (m, 2H), 4.36-4.32 (m, 1H), 4.31 (t, 2H), 3.79-3.75 (m, 1H), 2.91-2.77 (m, 3H), 2.50-2.33 (m, 3H), 1.74-1.65 (m, 1H), 1.55-1.50 (m, 2H), 1.02-0.86 (m, 2H).

### Example 98: synthesis of 8-fluoro-3-(3-(4-hydroxy-4-(3-(trifluoromethyl)phenyl)piperidin-1-yl)-3-oxopropyl)benzofuro[3,2-d]p yrimidin-4(3H)-one

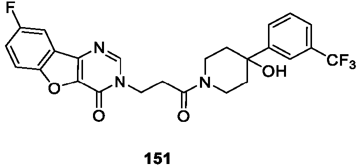

Except that 1-(3-(trifluoromethyl)phenyl)piperazine hydrochloride (144b) in Example 95 was replaced with 4-(3-(trifluoromethyl)phenyl)piperidin-4-ol (147b), the same synthetic route as in Example 95 was adopted to prepare the title compound 151 (white solid).
LCMS: 504 [M+1]⁺
¹H NMR (400 MHz, DMSO-*d₆*): δ 8.64 (s, 1H), 7.92 (dd, 1H), 7.88 (dd, 1H), 7.82 (br, 1H), 7.73 (d, 1H), 7.59-7.50 (m, 3H), 5.38 (s, 1H), 4.38-4.32 (m, 3H), 3.77-3.74 (m, 1H), 3.43-3.36 (m, 1H), 3.01-2.86 (m, 3H), 1.97-1.90 (m, 1H), 1.81-1.74 (m, 1H), 1.61-1.58 (m, 2H).

### Example 99: synthesis of 3-(3-(4-(2,3-dichlorophenyl)piperazin-1-yl)-3-oxopropyl)-8-fluoro-3,5-dihydro-4H-pyrimido[5,4-b]ind ol-4-one formate

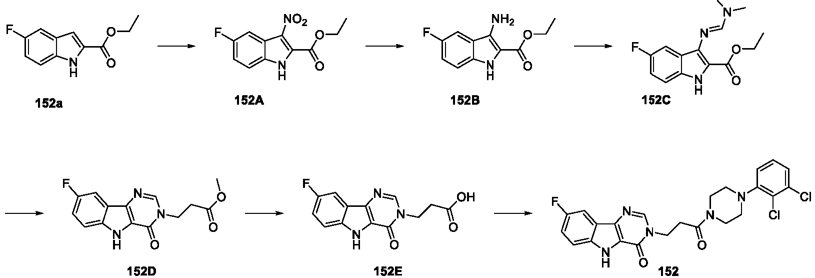

Step 1: 152a (5 g, 24.1 mmol, 1.0 eq) was dissolved in DCM (30 mL). Then a mixed solution of 65% HNO₃ and acetic acid (2.34 g of 65% HNO₃/15 g of acetic acid) was slowly added dropwise to the system. After the addition was completed, the mixture was stirred at 40 °C for 16 h. After the reaction was completed, the pH of the system was adjusted to 8 - 9 with a saturated sodium bicarbonate solution at 0 °C. Then it was extracted with water (100 mL) and dichloromethane (50 mL×2). The organic phase was evaporated to dryness and subjected to flash column chromatography (PE:EA = 1:1) to obtain a yellow solid 152A (3 g, yield: 49.3%).

Step 2: 152A (3 g) was dissolved in MeOH (50 mL). Then Pd/C (300 mg, 0.1 w/w) was added. The mixture was stirred at room temperature under a hydrogen atmosphere for 16 h. After the reaction was completed, the mixture was filtered. The filtrate was evaporated to dryness and subjected to flash column chromatography (PE:EA = 1:1) to obtain a yellow solid 152B (2.23 g, yield: 84.3%).

Step 3: 152B (2.23 g, 10.0 mmol, 1.0 eq) was dissolved in DMF (30 mL). Then DMF-DMA (5.96 g, 50.0 mmol, 5.0 eq) was added. The mixture was stirred at 80 °C for 16 h. After the reaction was completed, it was extracted with water (100 mL) and dichloromethane (50 mL×2). The organic phase was evaporated to dryness to obtain a red oil 152C (3 g).

Step 4: 152C (3 g, 10.83 mmol, 1.0 eq) was dissolved in methanol (30 mL). Then methyl 3-aminopropionate (1.6 g, 16.24 mmol, 1.5 eq) was added. The mixture was stirred at 70 °C for 16 h. After the reaction was completed, it was filtered to obtain a white solid 152D (1 g, yield: 31.9%).

Step 5: 152D (1 g) was added to THF/H₂O (10 mL/3 mL). Then 10% sodium hydroxide (3 mL) was added. The mixture was stirred at room temperature for 16 h. After the reaction was completed, the mixture was evaporated to dryness, and the pH of the system was adjusted to 5 - 6. The mixture was filtered to obtain a white solid 152E (0.7 g, yield 73.0%).

Step 6: 152E (200 mg, 0.72 mmol, 1.0 eq), 145b (290 mg, 1.08 mmol, 1.5 eq), HATU (550 mg, 1.45 mmol, 2.0 eq), and DIPEA (233 mg, 1.81 mmol, 2.5 eq) were added to DCM (5 mL). The mixture was stirred at room temperature for 16 h. After the reaction was completed, the mixture was extracted with water (20 mL) and dichloromethane (10 mL×2). The organic phase was evaporated to dryness and purified by a reverse-phase column (0.1% FA/H₂O:MeOH = 30:70). The obtained preparation solution was freeze-dried to obtain the formate salt of compound 152 as a yellow solid (40 mg, yield 11.3%).
LCMS: 488 [M+H]⁺
¹H NMR (400 MHz, DMSO-*d₆*) δ 12.19 (s, 1H), 8.35 (s, 1H), 7.71 (dd, 1H), 7.56-7.52 (m, 1H), 7.41 (t, 1H), 7.36-7.30 (m, 1H), 7.21 (d, 1H), 7.16 (s, 1H), 7.08 (d, 1H), 4.32 (t, 2H), 3.62-3.57 (m, 4H),3.23-3.17 (m, 4H), 2.93 (t, 2H).

### Example 100: synthesis of 8-fluoro-3-(3-oxo-3-(4-(3-(trifluoromethyl)phenyl)piperazin-1-yl)propyl)-3,5-dihydro-4H-pyrimido[5, 4-b]indol-4-one

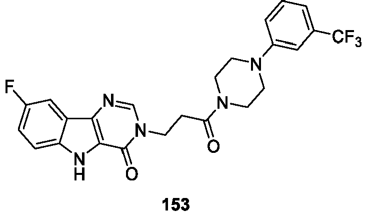

Except that 145b in Step 6 of Example 99 was replaced with 1-(3-(trifluoromethyl)phenyl)piperazine (144b), the same synthetic route as in Example 99 was adopted to prepare the title compound 153 (white solid).
LCMS: 488 [M+H]⁺
¹H NMR (400 MHz, DMSO-*d₆*) δ 12.20 (s, 1H), 8.36 (s, 1H), 7.70 (dd, 1H), 7.56-7.52 (m, 1H), 7.41 (t, 1H), 7.36-7.31 (m, 1H), 7.21-7.16 (m, 2H), 7.08 (d, 1H), 4.32 (t, 2H), 3.62-3.57(m, 4H), 3.23-3.17 (m, 4H), 2.93 (t, 2H).

### Example 101: synthesis of 8-fluoro-3-(3-(4-(4-fluorobenzyl)piperidin-1-yl)-3-oxopropyl)-3,5-dihydro-4H-pyrimido[5,4-b]indol-4-one

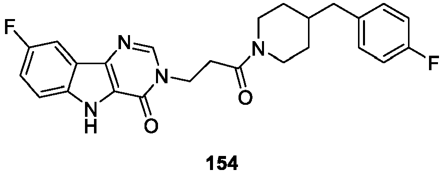

Except that 145b in Step 6 of Example 99 was replaced with 146b, the same synthetic route as in Example 99 was adopted to prepare the title compound 154 (white solid).
LCMS: 451 [M+H]⁺
¹H NMR (400 MHz, DMSO-*d₆*) δ 12.22 (s, 1H), 8.30 (s, 1H), 7.72 (dd, 1H), 7.57-7.54 (m, 1H), 7.37-7.31 (m, 1H), 7.10-7.02 (m, 4H), 4.34 (d, 1H), 4.29-4.26 (t, 2H), 3.77(d, 1H), 2.94-2.83 (m, 2H), 2.81-2.73 (m, 1H), 2.45-2.38 (m, 2H), 2.34-2.29 (m, 1H), 1.72-1.61 (m, 1H), 1.53-1.44 (m, 2H), 0.92-0.83 (m, 2H).

### Example 102: synthesis of 3-(4-fluorobenzyl)piperidin-1-yl)-3-oxopropyl-8-methyl-3,5-dihydro-4H-pyrimido[5,4-b]indol-4-one

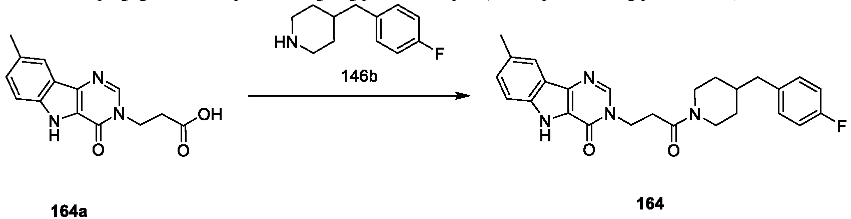

165a (100 mg, 0.37 mmol, 1.0 eq.), DMF (2 mL), and HATU (169 mg, 0.44 mmol, 1.2 eq.) were added to a reaction flask and stirred at room temperature for 30 min. Then 4-(4-fluorobenzyl)piperidine (146b) (79 mg, 0.41 mmol, 1.1 eq) and DIPEA (143 mg, 1.11 mmol, 3.0 eq) were added. The mixture was stirred at room temperature overnight. The reaction solution was purified by column chromatography (eluent: 0.1% formic acid aqueous solution : MeOH = 44%). The target fraction was collected and concentrated under reduced pressure to obtain a white solid 164 (49 mg, yield: 29.70%).
LCMS: 447 [M+H]⁺
¹H NMR (400 MHz, DMSO-*d₆*) δ 11.98 (s, 1H), 8.29 (s, 1H), 7.81 (s, 1H), 7.46 (d, 1H), 7.34 - 7.29 (dd, 1H), 7.10-7.06 (m, 4H), 4.37 (d, 1H), 4.34-4.27 (m, 2H), 3.80 (d, 1H), 2.98-2.87 (m, 2H), 2.87-2.55 (m, 2H), 2.48 (s, 3H), 2.43-2.31 (m, 2H), 1.69 (s, 1H), 1.57-1.44 (m, 2H), 0.95 - 0.84 (m, 2H).

### Example 103: synthesis of 3-(3-(4-hydroxy-4-(3-(trifluoromethyl)phenyl)piperidin-1-yl)-3-oxopropyl)-8-methyl-3,5-dihydro-4H-pyrimido[5,4-b]indol-4-one

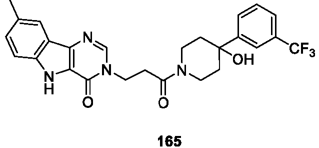

Except that 4-(4-fluorobenzyl)piperidine (146b) in Example 102 was replaced with 4-(3-(trifluoromethyl)phenyl)piperidin-4-ol (147b), the same synthetic route as in Example 102 was adopted to prepare the title compound 165 (white solid).
LCMS: 499 [M+H]
¹H NMR (400 MHz, DMSO-*d₆*) δ 11.98 (s, 1H), 8.34 (s, 1H), 7.83 (d, 2H), 7.61 (dd, 2H), 7.46 (t, 2H), 7.32 (dd, 1H), 5.38 (s, 1H), 4.45-4.30 (m, 3H), 3.79 (d, 1H), 3.41 (t, 1H), 3.05-2.85 (m, 3H), 2.49 (s, 3H), 1.95-1.71 (m, 2H), 1.60 (t, 2H).

### Example 104: synthesis of 3-(3-(4-((2,3-dichlorophenyl)(methyl)amino)piperidin-1-yl)-3-oxopropyl)-8-methyl-3,5-dihydro-4H-p yrimido[5,4-b]indol-4-one

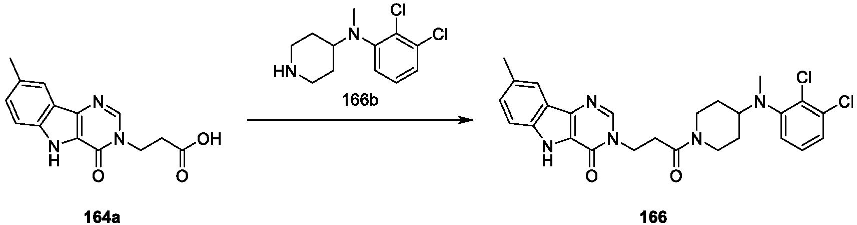

Except that 4-(4-fluorobenzyl)piperidine (146b) in Example 102 was replaced with N-(2,3-dichlorophenyl)-N-methylpiperidin-4-amine (166b), the same synthetic route as in Example 102 was adopted to prepare the title compound 166 (white solid).
LCMS: 513 [M+H]⁺
¹H NMR (400 MHz, DMSO-*d₆*) δ 11.99 (s, 1H), 8.32 (s, 1H), 7.80 (s, 1H), 7.45 (d, 1H), 7.33-7.27 (m, 3H), 7.19-7.14 (m, 1H), 4.41 (d, 1H), 4.31 (t, 2H), 3.87 (d, 1H), 3.31-3.24 (m, 1H), 3.02-2.91 (m, 2H), 2.88-2.80 (m, 1H), 2.60-2.55 (m, 1H), 2.53-2.51 (m, 3H), 2.48 (s, 3H), 1.65-1.61 (m, 2H), 1.50-1.47 (m, 2H).

### Example 105: synthesis of 8-methyl-3-(3-oxo-3-(4-(pyridin-3-yl)piperazin-1-yl)propyl)-3,5-dihydro-4H-pyrimido[5,4-b]indol-4-o ne formate

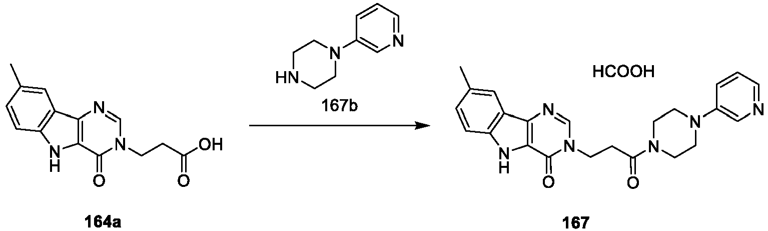

Except that 4-(4-fluorobenzyl)piperidine (146b) in Example 102 was replaced with 1-(pyridin-3-yl)piperazine (167b), the same synthetic route as in Example 102 was adopted to prepare the formate salt of compound 167 (white solid).
LCMS: 417 [M+H]⁺
¹H NMR (400 MHz, DMSO-*d₆*) δ 11.97 (s, 1H), 8.34 (s, 1H), 8.31 (s, 1H), 8.17 (s, 1H), 8.05-8.00 (m, 1H), 7.78 (s, 1H), 7.44 (d, 1H), 7.34-7.29 (m, 2H), 7.24-7.19 (m, 1H), 4.33 (t, 2H), 3.64-3.59 (m, 4H), 3.21-3.16 (m, 4H), 2.95 (t, 2H), 2.47 (s, 3H).

### Example 106: synthesis of 8-methyl-3-(3-(4-(4-(methylsulfonyl)phenyl)piperazin-1-yl)-3-oxopropyl)-3,5-dihydro-4H-pyrimido[5, 4-b]indol-4-one

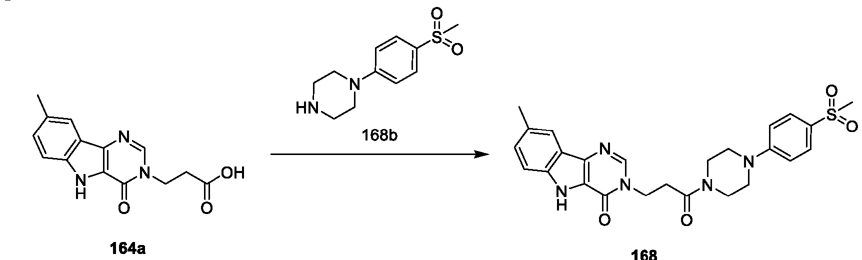

Except that 4-(4-fluorobenzyl)piperidine (146b) in Example 102 was replaced with 1-(4-(methylsulfonyl)phenyl)piperazine (168b), the same synthetic route as in Example 102 was adopted to prepare the title compound 168 (white solid).
LCMS: 494 [M+H]⁺
¹H NMR (400 MHz, DMSO-*d₆*) δ 11.96 (s, 1H), 8.35 (s, 1H), 7.79 (s, 1H), 7.70 (d, 2H), 7.44 (d, 1H), 7.33-7.29 (dd, 1H), 7.07 (d, 2H), 4.34 (t, 2H), 3.66-3.58 (m, 4H), 3.41-3.37 (m, 4H), 3.10 (s, 3H), 2.95 (t, 2H), 2.47 (s, 3H).

### Example 107: synthesis of 8-methyl-3-(3-oxo-3-(4-(4-(piperidin-1-ylsulfonyl)phenyl)piperazin-1-yl)propyl)-3,5-dihydro-4H-pyri mido[5,4-b]indol-4-one

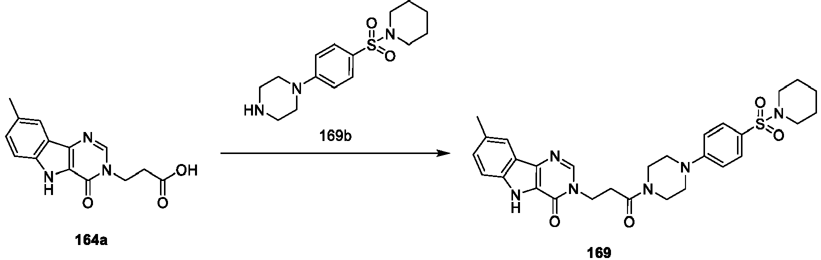

Except that 4-(4-fluorobenzyl)piperidine (146b) in Example 102 was replaced with 1-(4-(piperidin-1-ylsulfonyl)phenyl)piperazine (169b), the same synthetic route as in Example 102 was adopted to prepare the title compound 169 (white solid).
LCMS: 563 [M+H]+
¹H NMR (400 MHz, DMSO-*d₆*) δ 11.97 (s, 1H), 8.35 (s, 1H), 7.79 (s, 1H), 7.52 (d, 2H), 7.45 (d, 1H), 7.33-7.29(m, 1H), 7.06 (d, 2H), 4.34 (t, 2H), 3.65-3.60 (m, 4H), 3.39-3.35 (m, 4H), 2.95 (t, 2H), 2.83 (t, 4H), 2.47 (s, 3H), 1.57-1.52 (m, 4H), 1.38-1.34 (m, 2H).

### Example 108: synthesis of 8-methyl-3-(3-oxo-3-(4-(3-(piperidin-1-ylsulfonyl)phenyl)piperazin-1-yl)propyl)-3,5-dihydro-4H-pyri mido[5,4-b]indol-4-one

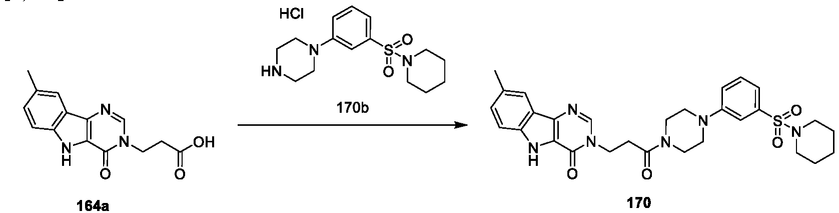

Except that 4-(4-fluorobenzyl)piperidine (146b) in Example 102 was replaced with 1-(3-(piperidin-1-ylsulfonyl)phenyl)piperazine hydrochloride (170b), the same synthetic route as in Example 102 was adopted to prepare the title compound 170 (white solid).
LCMS: 563 [M+1]⁺
¹H NMR (400 MHz, DMSO-*d₆*) δ 11.97 (s, 1H), 8.35 (s, 1H), 7.79 (s, 1H), 7.49-7.43 (m, 2H), 7.32-7.26 (m, 2H), 7.14-7.11 (m, 2H), 4.34 (t, 2H), 3.63 (m, 4H), 3.24 (m, 4H), 2.97-2.88 (m, 6H), 2.47 (s, 3H), 1.58-1.52 (m, 4H), 1.40-1.36 (m, 2H).

### Example 109: synthesis of 3-(4-(3-(8-methyl-4-oxo-4,5-dihydro-3H-pyrimido[5,4-b]indol-3-yl)propanoyl)piperazin-1-yl)benzonit rile

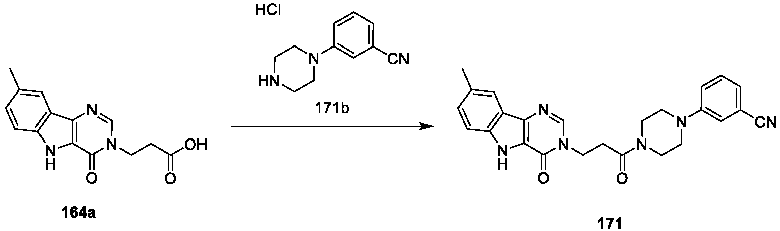

Except that 4-(4-fluorobenzyl)piperidine (146b) in Example 102 was replaced with 3-cyanophenylpiperazine hydrochloride (171b), the same synthetic route as in Example 102 was adopted to prepare the title compound 171 (white solid).
LCMS: 441 [M+1]⁺
¹H NMR (400 MHz, DMSO-*d₆*) δ 11.97 (s, 1H), 8.34 (s, 1H), 7.78 (s, 1H), 7.45-7.37 (m, 2H), 7.33-7.25 (m, 3H), 7.19 (d, 1H), 4.33 (t, 2H), 3.62-3.57 (m, 4H), 3.25-3.20 (m, 4H), 2.95 (t, 2H), 2.53-2.52 (m, 3H).

### Example 110: synthesis of 8-methyl-3-(3-(4-(3-nitrophenyl)piperazin-1-yl)-3-oxopropyl)-3,5-dihydro-4H-pyrimido[5,4-b]indol-4 -one

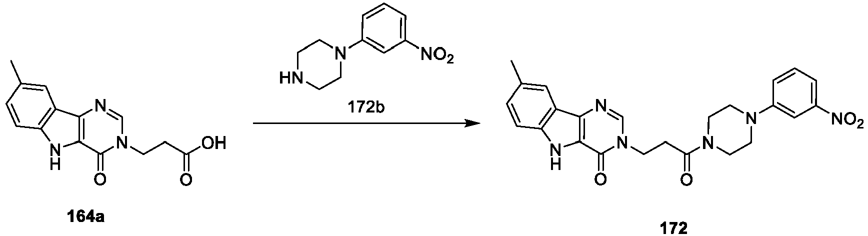

Except that 4-(4-fluorobenzyl)piperidine (146b) in Example 102 was replaced with 3-nitrophenylpiperazine (172b), the same synthetic route as in Example 102 was adopted to prepare the title compound 172 (yellow solid).
LCMS: 461 [M+1]⁺
¹H NMR (400 MHz, DMSO-*d₆*) δ 11.96 (s, 1H), 8.34 (s, 1H), 7.77 (s, 1H), 7.64-7.60 (m, 2H), 7.50-7.38 (m, 3H), 7.31 (d, 1H), 4.34 (t, 2H), 3.63 (m, 4H), 3.33-3.27 (m, 4H), 2.96 (t, 2H), 2.46 (s, 3H).

### Example 111: synthesis of 3-(3-(4-(3-chlorobenzoyl)piperazin-1-yl)-3-oxopropyl)-8-methyl-3,5-dihydro-4H-pyrimido[5,4-b]indol -4-one

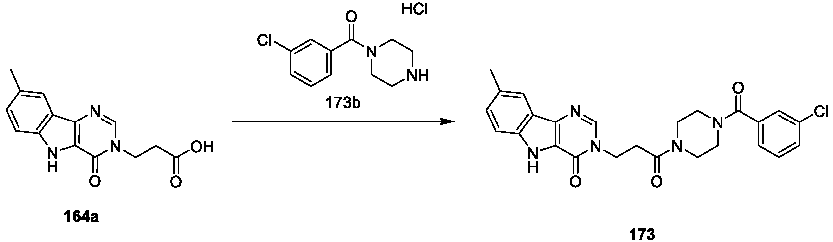

Except that 4-(4-fluorobenzyl)piperidine (146b) in Example 102 was replaced with (3-chlorophenyl)(piperazin-1-yl)methanone hydrochloride (173b), the same synthetic route as in Example 102 was adopted to prepare the title compound 173 (white solid).
LCMS: 478 [M+1]⁺
¹H NMR (400 MHz, DMSO-*d₆*) δ 11.97 (s, 1H), 8.34 (s, 1H), 7.79 (s, 1H), 7.56-7.54 (m, 1H), 7.52-7.50 (m, 2H), 7.48-7.44 (m, 1H), 7.39-7.37 (m, 1H), 7.32-7.30 (m, 1H), 4.32 (t, 2H), 3.54 (m, 8H), 2.92 (s, 2H), 2.48 (s, 3H).

### Example 112: synthesis of 3-(3-(4-isobutylpiperazin-1-yl)-3-oxopropyl)-8-methyl-3,5-dihydro-4H-pyrimido[5,4-b]indol-4-one

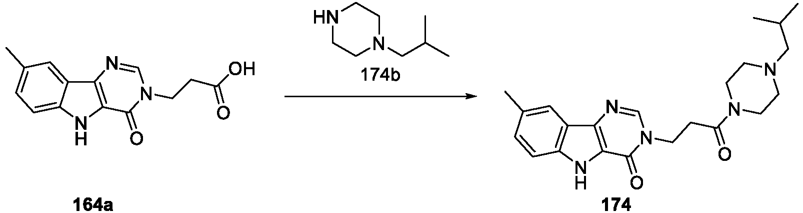

Except that 4-(4-fluorobenzyl)piperidine (146b) in Example 102 was replaced with 1-isobutylpiperazine (174b), the same synthetic route as in Example 102 was adopted to prepare the title compound 174 (white solid).
LCMS: 396 [M+H]⁺
¹H NMR (400 MHz, DMSO-*d₆*) δ 11.94 (s, 1H), 8.29 (s, 1H), 7.77 (s, 1H), 7.42 (d, 1H), 7.28 (dd, 1H), 4.28 (t, 2H), 3.44-3.41 (m, 4H), 2.85 (t, 2H), 2.45(s, 3H), 2.22 (t, 4H), 1.99-1.95 (m, 2H), 1.74-1.67 (m, 1H), 0.83 (s, 3H), 0.81 (s, 3H).

### Example 113: synthesis of 3-(3-(4-isobutyrylpiperazin-1-yl)-3-oxopropyl)-8-methyl-3,5-dihydro-4H-pyrimido[5,4-b]indol-4-one

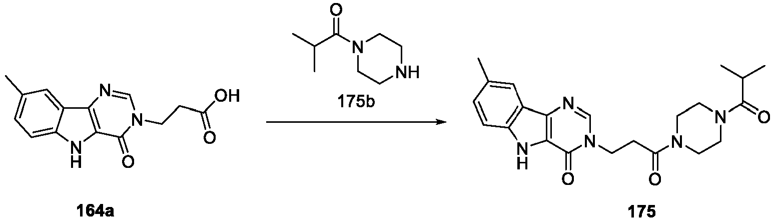

Except that 4-(4-fluorobenzyl)piperidine (146b) in Example 102 was replaced with 2-methyl-1-(piperazin-1-yl)propan-1-one (175b), the same synthetic route as in Example 102 was adopted to prepare the title compound 175 (white solid).
LCMS: 410 [M+H]⁺
¹H NMR (400 MHz, DMSO-*d₆*) δ 11.94 (s, 1H), 8.31 (s, 1H), 7.77 (s, 1H), 7.42 (d, 1H), 7.30-7.27 (m, 1H), 4.29 (t, 2H), 3.51-3.32 (m, 8H), 2.91-2.81 (m, 3H), 2.45 (s, 3H), 0.98 (s, 3H), 0.97 (s, 3H).

### Example 114: synthesis of 8-chloro-3-(3-(4-(2,3-dichlorophenyl)piperazin-1-yl)-3-oxopropyl)-3,5-dihydro-4H-pyrimido[5,4-b]in dol-4-one

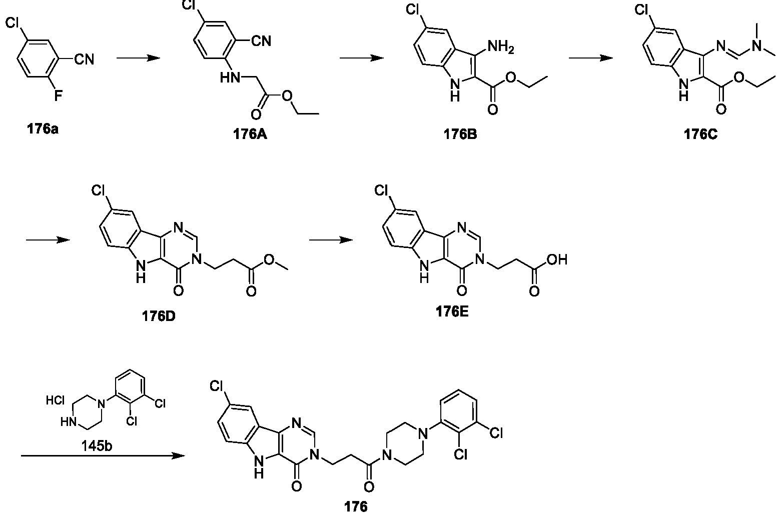

**Step 1:** 176a (5.0 g, 32.14 mmol, 1.0 eq.) was dissolved in acetonitrile (50 mL). DIPEA (20.8 g, 160.71 mmol, 5.0 eq.) and glycine ethyl ester hydrochloride (6.7 g, 48.21 mmol, 1.5 eq.) were added, and the temperature was raised to 85 °C for reaction for 3 days (the reaction was still incomplete and was directly processed). The reaction solution was filtered, the solid was washed with acetonitrile (2×20 mL), the filtrate was concentrated under reduced pressure, and the residue was separated and purified by silica gel column normal phase chromatography (eluent: petroleum ether: ethyl acetate = 67:33) to obtain a yellow solid 176A (2.1 g, yield: 27.37%).

**Step 2:** 176A (1.6 g, 6.70 mmol, 1.0 eq.) was dissolved in acetonitrile (20 mL), potassium carbonate (5.6 g, 40.22 mmol, 6.0 eq.) was added, and the temperature was raised to 85 °C for reaction for 9 days. The reaction solution was filtered, the solid was washed with ethyl acetate (2×20 mL), and the filtrate was concentrated to dryness under reduced pressure to obtain a brown solid 176B (1.2 g, yield: 75.00%).

**Step 3:** 176B (1.1 g, 4.61 mmol, 1.0 eq.) was dissolved in N,N-dimethylformamide (5 mL), N,N-dimethylformamide dimethyl acetal (5.5 g, 46.09 mmol, 10.0 eq.) was added, and the temperature was raised to 60 °C for reaction overnight. The reaction was concentrated to dryness under reduced pressure to obtain a brown solid 176C (1.3 g, yield: 96.02%).

**Step 4:** 176C (1.3 g, 4.43 mmol, 1.0 eq.) was dissolved in methanol (20 mL), methyl 3-aminopropionate hydrochloride (2.5 g, 17.70 mmol, 4.0 eq.) was added, and the temperature was raised to 70 °C for reaction overnight. The reaction was concentrated under reduced pressure, and the residue was separated and purified by silica gel column normal phase chromatography (eluent: dichloromethane: methanol = 90:10) to obtain a pale yellow solid 176D (670 mg, yield: 47.69%).

**Step 5:** 176D (670 mg, 2.19 mmol, 1.0 eq.) was dissolved in methanol (10 mL), lithium hydroxide (210 mg, 8.77 mmol, 4.0 eq.) and water (2 mL) were added, and the reaction was carried out at room temperature overnight. The solvent was removed by concentration under reduced pressure, the residue was dissolved in water (10 mL), the pH was adjusted to 5 with diluted hydrochloric acid, filtered, the filter cake was washed with water (2×10 mL), and dried to obtain a pale yellow solid 176E (580 mg, yield: 90.73%).

**Step 6:** 176E (100 mg, 0.34 mmol, 1.0 eq.), HATU (156 mg, 0.41 mmol, 1.2 eq.), and N,N-dimethylformamide (3 mL) were added to a reaction flask and reacted at room temperature for 0.5 h. Then 1-(2,3-dichlorophenyl)piperazine hydrochloride (145b) (110 mg, 0.41 mmol, 1.2 eq.) and DIPEA (164 mg, 1.27 mmol, 3.7 eq.) were added and reacted at room temperature overnight. The reaction solution was filtered, the filter cake was washed with dichloromethane (2×10 mL), and dried to obtain a white solid 176 (60 mg, yield: 34.67%).
LCMS: 504 [M+H]⁺
¹H NMR (400 MHz, DMSO-*d₆*) δ 12.37 (s, 1H), 8.40 (s, 1H), 8.01 (d, 1H), 7.57 (d, 1H), 7.49 (dd, 1H), 7.37-7.24 (m, 2H), 7.05 (d, 1H), 4.35 (t, 2H), 3.69-3.54 (m, 4H), 2.97-2.88 (m, 6H).

### Example 115: synthesis of 8-chloro-3-(3-oxo-3-(4-(3-(trifluoromethyl)phenyl)piperazin-1-yl)propyl)-3,5-dihydro-4H-pyrimido[5, 4-b]indol-4-one

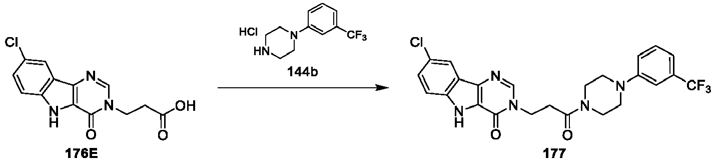

Except that 145b in Step 6 of Example 114 was replaced with 1-(3-trifluoromethylphenyl)piperazine hydrochloride (144b), the same synthetic route as in Example 114 was adopted to prepare the title compound 177 (white solid).
LCMS: 504 [M+H]⁺
¹H NMR (400 MHz, DMSO-*d₆*) δ 12.37 (s, 1H), 8.42 (s, 1H), 8.01 (d, 1H), 7.58 (d, 1H), 7.50 (dd, 1H), 7.45 (t, 1H), 7.24 (d, 1H), 7.20 (s, 1H), 7.12 (d, 1H), 4.35 (t, 2H), 3.68-3.57 (m, 4H), 3.28-3.20 (m, 4H), 2.97 (t, 2H).

### Example 116: synthesis of 8-chloro-3-(3-(4-hydroxy-4-(3-(trifluoromethyl)phenyl)piperidin-1-yl)-3-oxopropyl)-3,5-dihydro-4H-pyrimido[5,4-b]indol-4-one

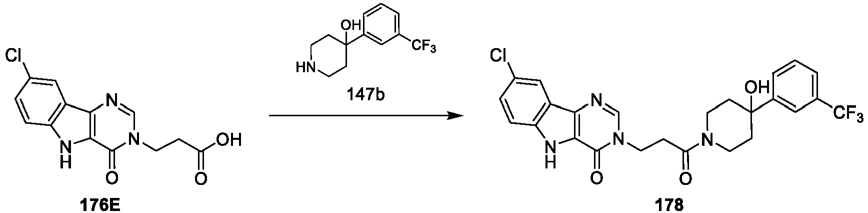

Except that 145b in Step 6 of Example 114 was replaced with 4-(3-trifluoromethyl)phenyl-4-piperidinol (147b), the same synthetic route as in Example 114 was adopted to prepare the title compound 178 (yellow solid).
LCMS: 519 [M+H]+
¹H NMR (400 MHz, DMSO-*d₆*) δ 12.35 (s, 1H), 8.39 (s, 1H), 8.00 (d, 1H), 7.83 (s, 1H), 7.65 (d, 1H), 7.60-7.53 (m, 2H), 7.51-7.44 (m, 2H), 5.39 (s, 1H), 4.44-4.28 (m, 3H), 3.82-3.70 (m, 1H), 3.44-3.37 (m, 1H), 3.02-2.84 (m, 3H), 1.93-1.65 (m, 2H), 1.57 (t, 2H).

### Example 117: synthesis of 8-chloro-3-(3-(4-(4-fluorobenzyl)piperidin-1-yl)-3-oxopropyl)-3,5-dihydro-4H-pyrimido[5,4-b]indol-4 -one

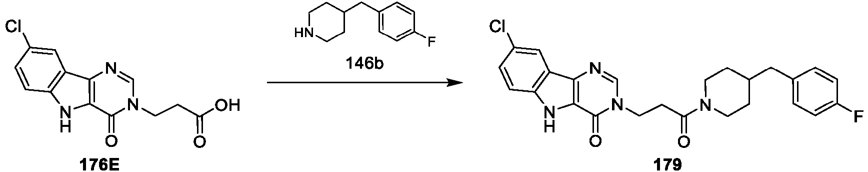

Except that 145b in Step 6 of Example 114 was replaced with 4-(4'-fluorobenzyl)piperidine (146b), the same synthetic route as in Example 114 was adopted to prepare the title compound 179 (yellow solid).
LCMS: 467 [M+H]+
¹H NMR (400 MHz, DMSO-*d₆*) δ 12.37 (s, 1H), 8.35 (s, 1H), 8.01 (d, 1H), 7.58 (d, 1H), 7.49 (dd, 1H), 7.12-7.05 (m, 4H), 4.37 (d, 1H), 4.30 (t, 2H), 3.79 (d, 1H), 2.96-2.75 (m, 3H), 2.50-2.30 (m, 3H), 1.74-1.60 (m, 1H), 1.56-1.43 (m, 2H), 0.95-0.83 (m, 2H).

### Example 118: synthesis of 9-methyl-3-(3-oxo-3-(4-(3-(trifluoromethyl)phenyl)piperazin-1-yl)propyl)-3,5-dihydro-2H-chromeno[ 4,3-d]pyrimidin-2-one

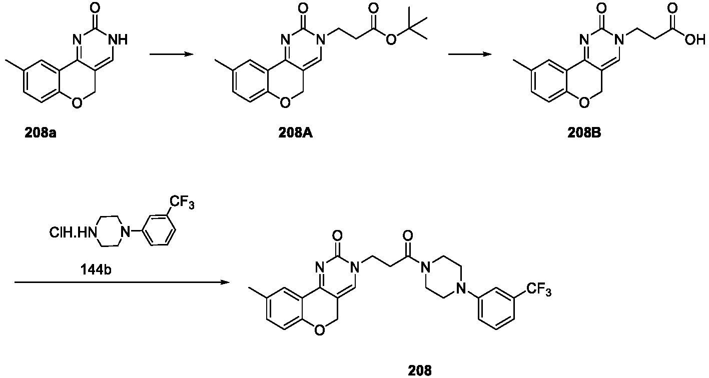

Step 1: 208a (500 mg, 2.33 mmol, 1.0 eq) and potassium carbonate (968 mg, 7.00 mmol, 3.0 eq) were added to DMF (5 mL). Then *tert-butyl* bromopropionate (976 mg, 4.67 mmol, 2.0 eq) was added, and the reaction was carried out at 40 °C for 16 hours. The reaction was diluted with water, extracted with ethyl acetate, the organic phase was dried, filtered, and evaporated to obtain a crude product, which was purified by a normal phase column (petroleum ether: ethyl acetate = 5% - 60%). The target fraction was collected and freeze-dried to obtain a white solid 208A (700 mg, yield: 87.5%).

Step 2: 208A (700 mg, 2.04 mmol, 1.0 eq) was added to 4 M HCl/1,4-dioxane (10 mL) and stirred at 25 °C for 3 hours. The reaction solution was evaporated to obtain a yellow solid 208B (550 mg, yield: 93.9%).

Step 3: 208B (200 mg, 0.699 mmol, 1.0 eq), 144b (279 mg, 1.05 mmol, 1.5 eq), and DIPEA (451 mg, 3.49 mmol, 5.0 eq) were added to DMF (3 mL). Then HATU (399 mg, 1.05 mmol, 1.5 eq) was added, and the mixture was stirred at 25 °C for 16 hours. The reaction solution was purified by a C18 reverse-phase column (eluent: 0.1% formic acid aqueous solution: MeOH = 1:20 - 9:1). The target fraction was collected and freeze-dried to obtain a white solid 208 (77 mg, yield: 22.1%).
LCMS: 499 [M+H]⁺
¹H NMR (400 MHz, DMSO-*d₆*) δ 8.22 (s, 1H), 7.83 (d, 1H), 7.43 (t, 1H), 7.33 (dd, 1H), 7.24-7.19 (m, 2H), 7.10 (d, 1H), 6.94 (d, 1H), 5.02 (s, 2H), 4.08 (t, 2H), 3.63-3.58 (m, 4H), 3.26-3.19 (m, 4H), 2.91 (t, 2H), 2.32 (s, 3H).

### Example 119: synthesis of 3-(3-(4-(2,3-dichlorophenyl)piperazin-1-yl)propyl)-9-methyl-5,6-dihydrobenzo[h]quinazolin-4(3H)-on e

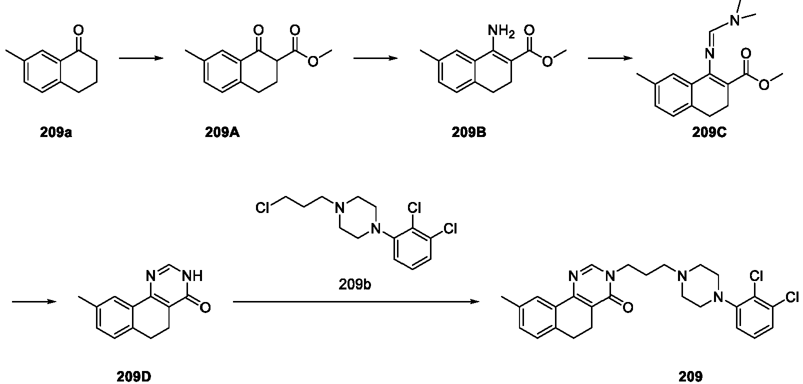

Step 1: 209a (3.2 g, 20.0 mmol, 1.0 eq) was added to anhydrous THF (30 ml), then 60% NaH (2.0 g, 50.0 mmol, 2.5 eq) was added. The mixture was stirred at 0 °C under nitrogen protection for 0.5 h. DMC (20 ml) was added to the system with a syringe, and then the mixture was stirred at 70 °C for 16 h. After the reaction was completed, methanol was added to quench the reaction. The mixture was evaporated and purified by a normal phase column (eluent: (petroleum ether: ethyl acetate = 1% - 10%)). The obtained organic phase was evaporated to give a colorless oily liquid 209A (3.0 g, yield: 68.8%).

Step 2: 209A (2.0 g, 9.16 mmol, 1.0 eq) and ammonium acetate (3.5 g, 45.82 mmol, 5.0 eq) were added to methanol (20 mL). The mixture was stirred at 30 °C for 16 h. After the reaction solution was evaporated, saturated sodium bicarbonate solution (50 ml) and ethyl acetate (20 ml * 2) were added for extraction. The organic phase was evaporated to give a white oil 209B (1.8 g, yield: 89.6%).

Step 3: 209B (1.8 g, 8.29 mmol, 1.0 eq) and DMF-DMA (4.9 g, 41.47 mmol, 5.0 eq) were added to DMF (20 mL). The mixture was stirred at 80 °C for 24 h. Water (100 ml) and ethyl acetate (30 ml * 2) were added, and the organic phase was evaporated to give a blue oily liquid 209C (2 g, yield: 88.6%).

Step 4: 209C (2 g, 7.34 mmol, 1.0 eq) was added to formamide (20 mL) and stirred at 200 °C for 1 h. After the reaction was completed, it was cooled to room temperature. Water (100 ml) was added to precipitate a solid, which was filtered to give a brown solid 209D (0.9 g, yield: 57.7%).

Step 5: 209D (500 mg, 2.36 mmol, 1.0 eq), 209b (724 mg, 2.36 mmol, 1.0 eq), and potassium carbonate (651 mg, 4.72 mmol, 2.0 eq) were added to acetonitrile (5 mL). The mixture was stirred at 70 °C for 4 h. After the reaction solution was evaporated, water (30 ml) was added to precipitate a solid, which was filtered to give 200 mg of a white solid. The white solid was purified by reverse-phase preparative chromatography (0.1% FA). The obtained preparative solution was concentrated and freeze-dried to give a white solid 209 (20 mg, yield: 1.75%).
LCMS: 483[M+H]⁺
¹H NMR (400 MHz, CDCl₃) δ 8.23 (s, 1H), 7.98 (s, 1H), 7.22-7.15 (m, 4H), 6.97-6.94 (m, 1H), 4.12 (t, 2H), 3.11 (s, 4H), 2.91 (s, 4H), 2.70 (s, 4H), 2.55 (s, 2H), 2.43 (s, 3H), 2.10 (t, 2H).

### Example 120: synthesis of 8-methoxy-3-(3-(4-(3-(trifluoromethyl)phenyl)piperazin-1-yl)propyl)-3,5-dihydro-4H-pyrrolo[2,3-c]q uinolin-4-one

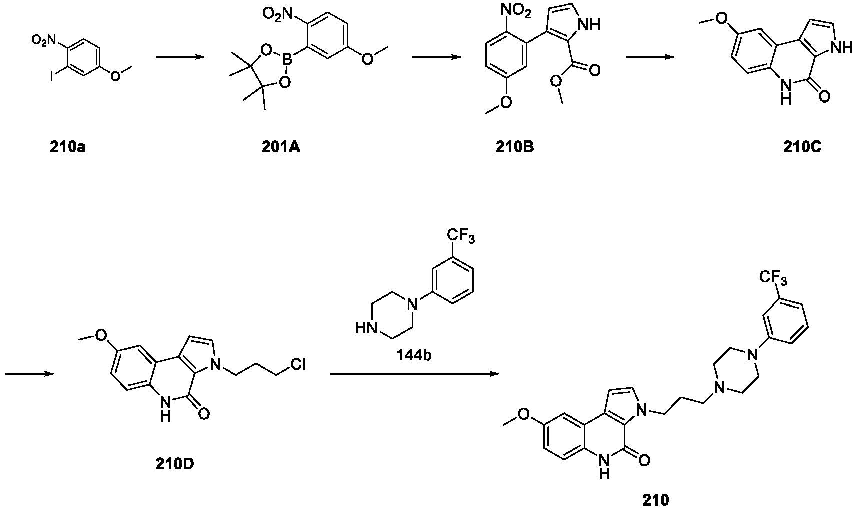

Step 1: Bis(pinacolato)diboron (2.5 g, 0.01 mol, 2.0 eq.), 201a (1.4 g, 0.005 mol, 1.0 eq.), DMSO (30 mL), sodium carbonate (1.1 g, 0.01 mol, 2.0 eq.), and tetrakis(triphenylphosphine)palladium (0.3 g, 0.00026 mol, 2.6%) were added to a 250 mL single-necked flask. Under nitrogen protection, the temperature was raised to 80 °C and the reaction was carried out for 6 h. The reaction solution was concentrated and then purified by silica gel column chromatography. The product was eluted with ethyl acetate:petroleum ether = 1:2 and concentrated to give a pale white liquid 210A (1.8 g, yield: 64.5%).

Step 2: Methyl 3-bromopyrrole-2-carboxylate (1.62 g, 0.0064 mol, 1.0 eq.), 210A (1.8 g, 0.0064 mol, 1.0 eq.), potassium carbonate (4.2 g, 0.03 mol, 5.0 eq.), Pd(dppf)₂Cl₂ (0.6 g, 0.00074 mol, 11.6%), dioxane (60 mL), and water (10 mL) were added to a 250 mL single-necked flask. Under nitrogen protection, the temperature was raised to 100 °C and the reaction was carried out for 3 h. After the reaction was completed, it was purified by silica gel column chromatography. The product was eluted with petroleum ether:ethyl acetate = 2:1 and concentrated to give a yellow solid 210B (2.1 g, yield: 100%).

Step 3: 210B (2.1 g, 0.0064 mol, 1.0 eq.), Pd/C (0.5 g, 25% wt.), and methanol (50 mL) were added to a 250 mL single-necked flask. Under hydrogen protection, the reaction was carried out at room temperature for 6 h. After the reaction was completed, it was filtered. The organic phase was purified by silica gel column chromatography. The product was eluted with dichloromethane:methanol = 10:1 and concentrated to give a pale white liquid 210C (0.21 g, yield: 15.3%).

Step 4: 210C (0.21 g, 0.001 mol, 1.0 eq.), acetonitrile (50 mL), and potassium carbonate (0.42 g, 0.003 mol, 3 eq.) were added to a 250 mL single-necked flask. Under nitrogen protection, 1-chloro-3-bromopropane (0.45 g, 0.003 mol, 3.0 eq.) was added, and the reaction was carried out at 60 °C for 3 h. After the reaction was completed, it was purified by silica gel column chromatography. The product was eluted with dichloromethane:methanol = 10:1 and concentrated to give an off-white solid 210D (0.2 g, yield: 68.8%).

Step 5: 210D (0.2 g, 0.0007 mol, 1.0 eq.), acetonitrile (10 mL), potassium carbonate (0.28 g, 0.002 mol, 3 eq.), and 3-trifluorophenylpiperazine (144b) (0.46 g, 0.002 mol, 3 eq.) were added to a 250 mL single-necked flask. Under nitrogen protection, the reaction was carried out at 80 °C for 16 h. After the reaction was completed, it was purified by silica gel column chromatography. The product was eluted with dichloromethane:methanol = 10:1 and concentrated to give a crude product, which was then purified by preparative liquid chromatography and concentrated to give an off-white solid 210 (0.02 g, yield: 6%).
LCMS: 485 [M+1]⁺
¹H NMR (400 MHz, CD₃OD): δ 7.54-7.49 (m, 3H), 7.38-7.32(m, 3H),7.26 (d, 1H), 7.09 (dd, 1H), 7.01 (d, 1H), 4.81-4.77 (t, 2H), 3.96-3.93 (m, 5H), 3.75 (b, 2H), 3.29-3.25 (m, 6H), 2.49-2.42 (m, 2H).

### Example 121: synthesis of 3-(3-(4-(2,3-dichlorophenyl)piperazin-1-yl)propyl)-9-methylpyrimido[5,4-c]quinolin-4(3H)-one formate

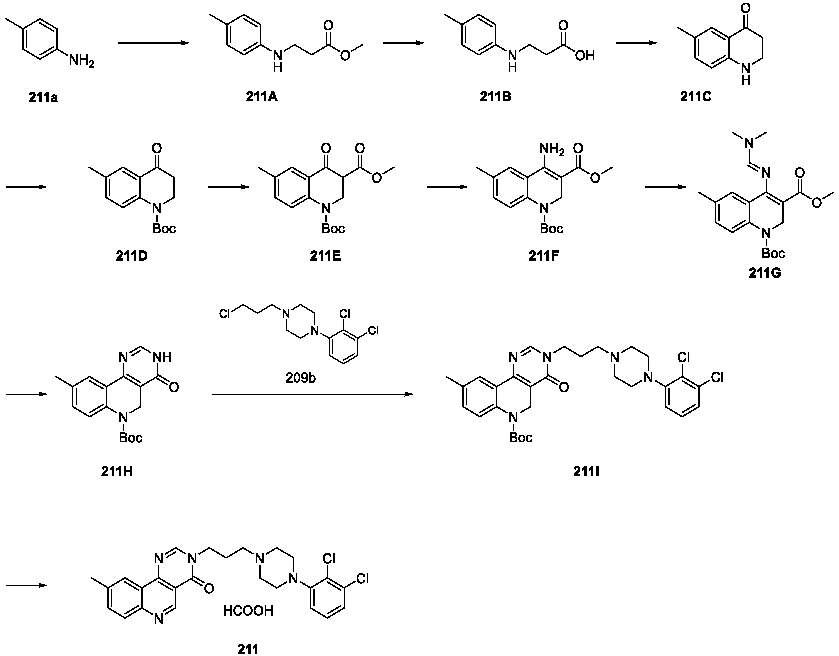

Step 1: 211a (10 g, 93.32 mmol, 1.0 eq) was added to AcOH (20 mL), then methyl 3-bromopropionate (8.03 g, 93.32 mmol, 1.0 eq) was added, and the mixture was stirred at 70 °C for 4 h. The reaction solution was diluted with water, extracted with dichloromethane. The organic phase was dried, filtered, and evaporated to give a crude product, which was purified by a normal phase column (petroleum ether: ethyl acetate = 5% - 10%)). The target fraction was collected and evaporated to give a yellow solid 211A (15.3 g, yield: 84.8%).

Step 2: 211A (15.3 g, 79.18 mmol, 1.0 eq) was added to MeOH/H₂O (150 mL/50 mL), then NaOH (4.75 g, 118.7 mmol, 1.5 eq) was added, and the mixture was stirred at 25 °C for 2 h. The reaction solution was diluted with water and washed with ethyl acetate. The aqueous phase was adjusted to pH = 5 - 6 with saturated citric acid aqueous solution, extracted with ethyl acetate. The organic phase was dried, filtered, and evaporated to give a yellow oil 211B (14.1 g, yield: 99.3%).

Step 3: 211B (14.1 g, 78.68 mmol, 1.0 eq) was added to PPA (50 mL) and stirred at 130 °C for 3 h. The reaction solution was poured into a saturated sodium carbonate aqueous solution, adjusted to pH = 7 - 8, and extracted with ethyl acetate. The organic phase was dried, filtered, and evaporated to give a crude product, which was purified by a normal phase column (petroleum ether: ethyl acetate = 5% - 50%)). The target fraction was collected and evaporated to give a yellow solid 211C (4.2 g, yield: 33.1%).

Step 4: 211C (4.2 g, 26.05 mmol, 1.0 eq) and DMAP (4.77 g, 39.08 mmol, 1.5 eq) were added to DCM (50 mL), then Boc₂O (11.37 g, 52.11 mmol, 2.0 eq) was added, and the mixture was stirred at 25 °C for 16 h. The reaction solution was evaporated to give a crude product, which was purified by a normal phase column (petroleum ether: ethyl acetate = 1% - 20%)). The target fraction was collected and evaporated to give a white solid 211D (6 g, yield: 88.1%).

Step 5: 211D (6 g, 22.96 mmol, 1.0 eq) was added to THF (60 mL). Then 60% wt. NaH (2.3 g, 57.40 mmol, 2.5 eq, in mineral oil) was added in batches at 0 °C. After stirring at 25 °C for 0.5 h, dimethyl carbonate (3.1 g, 34.4 mmol, 1.5 eq) was added, and the mixture was stirred at 70 °C under nitrogen protection for 2 h. The reaction solution was diluted with water, extracted with ethyl acetate. The organic phase was dried, filtered, and evaporated to give a crude product, which was purified by a normal phase column (petroleum ether: ethyl acetate = 1% - 10%)). The target fraction was collected and evaporated to give a yellow solid 211E (6 g, yield: 81.8%).

Step 6: 211E (2.5 g, 7.83 mmol, 1.0 eq) was added to MeOH (30 mL), then NH₄OAc (3.02 g, 39.14 mmol, 5.0 eq) was added, and the mixture was stirred at 80 °C for 16 h. The reaction solution was evaporated to give a crude product, which was purified by a normal phase column (petroleum ether: ethyl acetate = 1% - 15%)). The target fraction was collected and evaporated to give a white solid 211F (2 g, yield: 80.2%).

Step 7: 211F (2 g, 6.28 mmol, 1.0 eq) was added to DMF (20 mL), then DMF-DMA (3.74 g, 31.41 mmol, 5.0 eq) was added, and the mixture was stirred at 80 °C for 16 h. The reaction solution was diluted with water, extracted with ethyl acetate. The organic phase was dried, filtered, and evaporated to give a black solid 211G (2.1 g, yield: 89.5%).

Step 8: 211G (700 mg, 1.88 mmol, 1.0 eq) was added to MeOH (10 mL), then NH₄OAc (1.44 g, 18.8 mmol, 10.0 eq) was added, and the mixture was stirred at 80 °C for 16 h. The reaction solution was evaporated to give a crude product, which was purified by a normal phase column (dichloromethane: methanol = 1% - 10%)). The target fraction was collected and evaporated to give a white solid 211H (580 mg, yield: 98.7%).

Step 9: 211H (300 mg, 0.957 mmol, 1.0 eq) and potassium carbonate (265 mg, 1.91 mmol, 2.0 eq) were added to ACN (5 mL), then 209b (442 mg, 1.44 mmol, 1.5 eq) was added, and the mixture was stirred at 50 °C for 16 h. The reaction solution was evaporated to give a crude product, which was purified by a normal phase column (petroleum ether: ethyl acetate = 5% - 100%)). The target fraction was collected and evaporated to give a white solid 211I (440 mg, yield: 78.6%).

Step 10: 211I (440 mg, 0.75 mmol, 1.0 eq) was added to DCM (6 mL), then TFA (3 mL) was added, and the mixture was stirred at 25 °C for 1 h. The reaction solution was evaporated to give a crude product, which was purified by a C18 reverse-phase column (eluent: 0.1% formic acid aqueous solution: MeOH = 1:20 - 9:1). The target fraction was collected and freeze-dried to give the formate salt of compound 211 as a white solid (85 mg, yield: 23.4%).
LCMS: 482 [M+H]+
¹H NMR (400 MHz, DMSO-*d₆*) δ 9.40 (s, 1H), 8.86 (s, 1H), 8.59 (s, 1H), 8.20 (s, 1H), 8.03 (d, 1H), 7.82-7.80 (m, 1H), 7.28-7.26 (m, 1H), 7.18 (t, 1H), 6.79-6.77 (m, 1H), 4.18 (t, 2H), 2.84-2.72 (m, 4H), 2.61 (s, 3H), 2.50-2.47 (m, 6H), 2.02-1.95 (m, 2H).

### Example 122: synthesis of 9-methyl-3-(3-oxo-3-(4-(3-(trifluoromethyl)phenyl)piperazin-1-yl)propyl)pyrimido[5,4-c]quinolin-4(3 H)-one

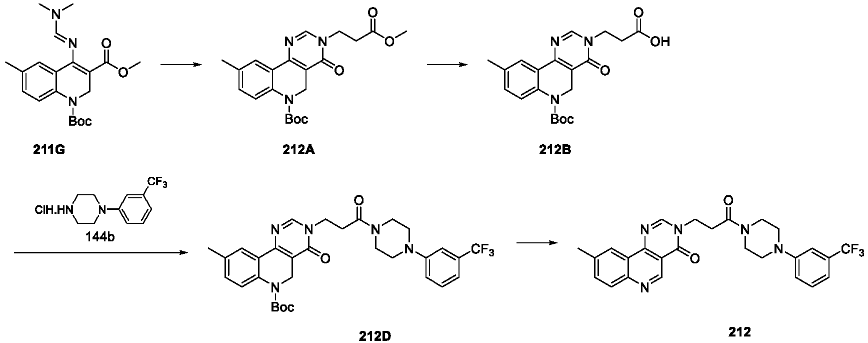

Step 1: 211G (500 mg, 1.34 mmol, 1.0 eq) was added to MeOH (10 mL), then methyl 3-aminopropionate hydrochloride (374 mg, 2.68 mmol, 2.0 eq) was added, and the mixture was stirred at 70 °C for 16 h. The reaction solution was evaporated to give a crude product, which was purified by a normal phase column (petroleum ether: ethyl acetate = 5% - 50%). The target fraction was collected and evaporated to give a yellow solid 212A (200 mg, yield: 37.4%).

Step 2: 212A (200 mg, 0.5 mmol, 1.0 eq) was added to THF/H₂O (8 mL/2 mL), then LiOH (30 mg, 1.25 mmol, 2.5 eq) was added, and the mixture was stirred at 0 °C for 2 h. The reaction solution was diluted with water, adjusted to pH = 5 - 6 with 1 M hydrochloric acid aqueous solution, and extracted with ethyl acetate. The organic phase was dried, filtered, and evaporated to give a white solid 212B (190 mg, yield: 98.4%).

Step 3: 212B (190 mg, 0.493 mmol, 1.0 eq), 144b (197 mg, 0.739 mmol, 1.5 eq), and DIPEA (319 mg, 2.46 mmol, 5.0 eq) were added to DMF (2 mL), then HATU (281 mg, 0.739 mmol, 1.5 eq) was added, and the mixture was stirred at 25 °C for 16 h. The reaction solution was diluted with water, extracted with ethyl acetate. The organic phase was dried, filtered, and evaporated to give a crude product, which was purified by a normal phase column (dichloromethane: methanol = 1% - 5%). The target fraction was collected and evaporated to give a yellow solid 212D (200 mg, yield: 67.8%).

Step 4: 212D (200 mg, 0.335 mmol, 1.0 eq) was added to 4 M HCl/1,4-dioxane (3 mL) and stirred at 25 °C for 2 h. The reaction solution was evaporated to give a crude product, which was purified by a C18 reverse-phase column (eluent: 0.1% formic acid aqueous solution: MeOH = 1:20 - 9:1). The target fraction was collected and freeze-dried to give a white solid 212 (28 mg, yield: 16.8%).
LCMS: 496 [M+H]⁺
¹H NMR (400 MHz, DMSO-*d₆*) δ 9.40 (s, 1H), 8.90 (s, 1H), 8.59 (s, 1H), 8.05 (s, 1H), 7.84-7.81 (m, 1H), 7.44 (t, 1H), 7.24-7.19 (m, 2H), 7.10 (d, 1H), 4.33 (t, 2H), 3.64-3.59 (m, 4H), 3.28-3.21 (m, 4H), 3.00 (t, 2H), 2.62 (s, 3H).

### Example 123: synthesis of 3-(3-(4-(2,3-dichlorophenyl)piperazin-1-yl)propyl)-9-methyl-3,5-dihydro-2H-chromeno[4,3-d]pyrimid in-2-one

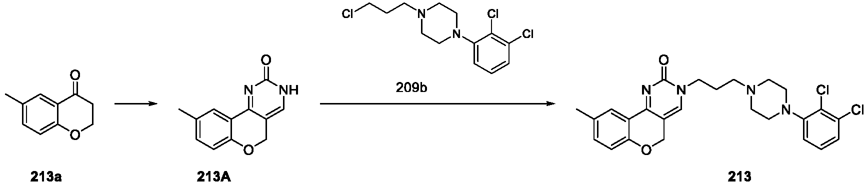

Step 1: 213a (4.5 g, 27.72 mmol, 1.0 eq), urea (1.84 g, 30.51 mmol, 1.1 eq), and triethyl orthoformate (4.11 g, 27.72 mmol, 1.0 eq) were added to MeOH (5 mL), then methanesulfonic acid (15 drops) was added, and the mixture was stirred at 130 °C for 6 h. The reaction solution was diluted with methanol, filtered, and the filter cake was evaporated to give a yellow solid 213A (3.3 g, yield: 55.5%).

Step 2: 213A (500 mg, 2.33 mmol, 1.0 eq) and potassium carbonate (968 mg, 7.00 mmol, 3.0 eq) were added to DMF (5 mL), then 209b (862 mg, 2.80 mmol, 1.2 eq) was added, and the mixture was stirred at 50 °C for 24 h. The reaction solution was diluted with water, extracted with ethyl acetate. The organic phase was dried, filtered, and evaporated to give a crude product, which was purified by a normal phase column (dichloromethane: methanol = 1% - 10%), and then purified by a C18 reverse-phase column (eluent: 0.1% formic acid aqueous solution: MeOH = 1:20 - 9:1). The target fraction was collected and freeze-dried to give a white solid 213 (155 mg, yield: 13.6%).
LCMS: 485 [M+H]⁺
¹H NMR (400 MHz, DMSO-*d₆*) δ 8.20 (s, 1H), 7.87-7.86 (m, 1H), 7.36-7.01 (m, 3H), 7.0 (d, 1H), 6.96 (d, 1H), 5.04 (s, 2H), 3.94 (t, 2H), 3.00-2.86 (m, 4H), 2.54-2.53 (m, 4H), 2.44 (t, 2H), 2.34 (s, 3H), 1.95-1.88 (m, 2H).

### Example 125: synthesis of 9-methyl-3-(3-oxo-3-(4-(3-(trifluoromethyl)phenyl)piperazin-1-yl)propyl)-5,6-dihydrobenzo[h]quinaz olin-4(3H)-one

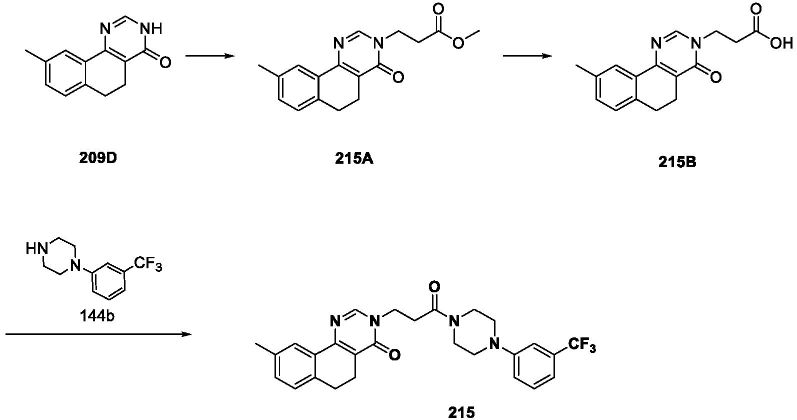

Step 1: 209D (0.50 g, 2.36 mmol, 1.0 eq) was added to anhydrous DMF (30 mL), then potassium carbonate (0.73 g, 4.7 mmol, 2.0 eq) and methyl 3-bromopropionate (1 g, 5.62 mmol, 2.4 eq) were added. The mixture was stirred at 80 °C for 4 h. After the reaction was completed, it was cooled to room temperature, and water (100 mL) was added until the system became turbid. The white filter cake was obtained by filtration and dried to give 215A (0.48 g, yield: 68.2%).

Step 2: 215A (0.48 g, 1.60 mmol, 1.0 eq) was dissolved in THF (3 mL), then water (1 mL) and LiOH (116 mg, 4.80 mmol, 3.0 eq) were added to the system. The mixture was stirred at 30 °C for 16 h. After the reaction was completed, the system was evaporated, water (10 mL) was added, and the system was adjusted to pH = 5 - 6, extracted with DCM (5 mL * 2). The organic phase was evaporated to give a white solid 215B (0.45 g, yield: 98.9%).

Step 3: 215B (0.40 g, 1.40 mmol, 1.0 eq), DIPEA (0.45 g, 3.5 mmol, 2.5 eq), HATU (1.1 g, 2.80 mmol), and 144b (0.39 g, 1.70 mmol, 1.2 eq) were added to DMF (5 mL) and stirred at 30 °C for 16 h. After the reaction was completed, water (20 ml) and ethyl acetate (10 ml * 2) were added for extraction. The organic phase was evaporated, purified by reverse-phase preparative chromatography (0.1% FA), concentrated, and freeze-dried to give a white solid 215 (0.05 g, yield: 7.19%).
LCMS: 497 [M+H]⁺
¹H NMR (400 MHz, DMSO-*d₆*) δ 8.52 (s, 1H), 7.89 (s, 1H), 7.44 (t, 1H), 7.25-7.20 (m, 4H), 7.11 (d, 1H), 4.17 (t, 2H), 3.64-3.58 (m, 4H), 3.27-3.20 (m, 4H), 2.91 (t, 2H), 2.81 (t, 2H), 2.70 (t, 2H), 2.35 (s, 3H).

### Example 126: synthesis of 3-(3-(4-(2,3-dichlorophenyl)piperazin-1-yl)propyl)-8-methoxy-3,5-dihydro-4H-imidazo[4,5-c]quinolin -4-one formate

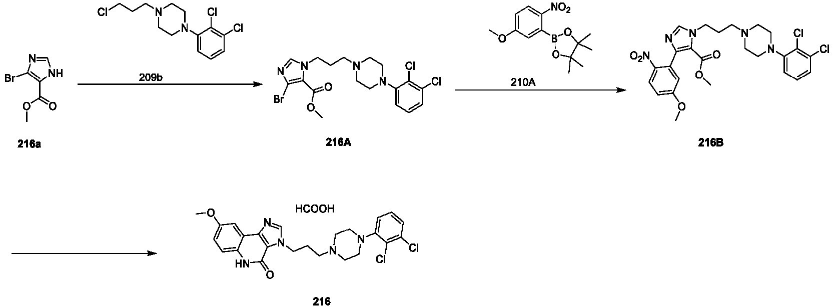

Step 1: 216a (0.8 g, 0.0039 mol, 1.5 eq), 209b (0.8 g, 0.0026 mol, 1.0 eq), cesium carbonate (1.7 g, 0.0052 mol, 2.0 eq), and DMF (10 mL) were added to a 100 mL three-necked flask and stirred at 80 °C overnight. After the reaction was completed, it was purified by silica gel column chromatography. The product was eluted with MeOH:DCM = 10% and concentrated to give a yellow oil 216A (1.2 g, yield 79.56%).

Step 2: 216A (1.14 g, 0.0024 mol, 1.0 eq), 210A (1.0 g, 0.0036 mol, 1.5 eq), Pd-118 (0.15 g, 0.00024 mol, 0.1 eq), potassium carbonate (0.66 g, 0.0048 mol, 2.0 eq), 1,4-dioxane (20 mL), and H₂O (5 mL) were added to a 250 mL three-necked flask. Under nitrogen protection, the mixture was stirred at 100 °C overnight. After the reaction was completed, it was purified by column chromatography. The product was eluted with MeOH:DCM = 7% and concentrated under reduced pressure to give a brown oil 216B (1.14 g, yield: 86.83%).

Step 3: 216B (0.5 g, 0.0009 mol, 1.0 eq), zinc powder (0.6 g, 0.009 mol, 10.0 eq), saturated NH₄Cl aqueous solution (0.5 mL), and EtOH (5 mL) were added to a 100 mL three-necked flask and stirred at 80 °C overnight. After the reaction was completed, it was purified by a reverse-phase column. The product was eluted with methanol/water and freeze-dried to give the formate salt of compound 216 as a yellow solid (0.052 g, yield: 5.91%).
LCMS: 486 [M+1]+
¹H NMR (400 MHz, DMSO-*d₆*) δ 11.55 (s, 1H), 8.29 (s, 1H), 8.21 (s, 1H), 7.54 (d, 1H), 7.39 (d, 1H), 7.32-7.30 (m, 2H), 7.11-7.05 (m, 2H), 4.54 (t, 2H), 3.86 (s, 3H), 3.42 (m, 3H), 3.02-2.95 (m, 5H), 2.37 (t, 2H), 2.16-2.01 (m, 2H).

### Example 127: synthesis of 3-(4-(4-(2,3-dichlorophenyl)piperazin-1-yl)-4-oxobutyl)-9-methylpyrimido[5,4-c]quinolin-4(3H)-one

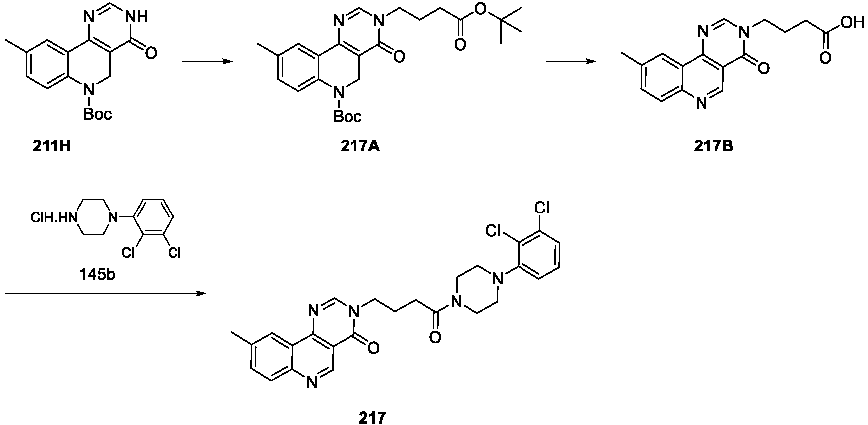

**Step 1:** 211H (180 mg, 0.574 mmol, 1.0 eq) was added to ACN (2 mL), then potassium carbonate (238 mg, 1.72 mmol, 3.0 eq) and tert-butyl 4-bromobutyrate (192 mg, 0.862 mmol, 1.5 eq) were added, and the mixture was stirred at 40 °C for 16 h. Then the reaction solution was evaporated to give a crude product, which was purified by a normal phase column (eluent: (petroleum ether:ethyl acetate = 5% - 40%)). The target fraction was collected and evaporated to give a yellow oil 217A (220 mg, yield: 84.0%).

**Step 2:** 217A (220 mg, 0.483 mmol, 1.0 eq) was added to 4 M HCl/1,4-dioxane (5 mL) and stirred at 25 °C for 1 h. The reaction solution was evaporated to give a yellow solid 217B (140 mg, yield: 97.5%).

**Step 3:** 217B (70 mg, 0.236 mmol, 1.0 eq), 145b (82 mg, 0.305 mmol, 1.3 eq), and DIPEA (152 mg, 1.18 mmol, 5.0 eq) were added to DMF (2 mL), then HATU (135 mg, 0.354 mmol, 1.5 eq) was added, and the mixture was stirred at 25 °C for 2 h. The reaction solution was purified by a C18 reverse-phase column (eluent: 0.1% formic acid aqueous solution: MeOH = 1:20 - 9:1). The target fraction was collected and freeze-dried to give an off-white solid 217 (14 mg, yield: 11.6%).
LCMS: 510 [M+H]⁺
¹H NMR (400 MHz, DMSO-*d₆*) δ 9.38 (s, 1H), 8.80 (s, 1H), 8.56 (s, 1H), 8.02 (d, 1H), 7.81-7.78 (m, 1H), 7.33-7.29 (m, 2H), 7.11-7.09 (m, 1H), 4.12 (t, 2H), 3.58-3.54 (m, 4H), 3.00-2.92 (m, 2H), 2.87-2.85 (m, 2H), 2.58 (s, 3H), 2.50-2.47 (m, 2H), 2.08-2.01 (m, 2H).

### Example 128: synthesis of 3-(4-(4-(4-fluorobenzyl)piperidin-1-yl)-4-oxobutyl)-9-methylpyrimido[5,4-c]quinolin-4(3H)-one

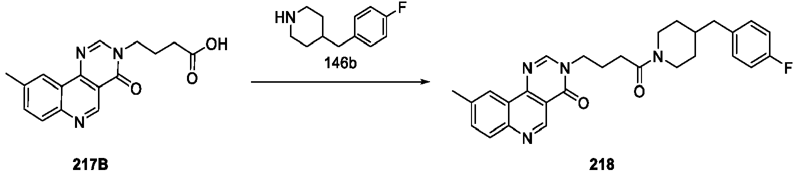

Except that 145b in Step 3 of Example 127 was replaced with 4-(4-fluorobenzyl)piperidine (146b), the same synthetic route as in Example 127 was adopted to prepare the title compound 218 (off-white solid).
LCMS: 473 [M+H]⁺
¹H NMR (400 MHz, DMSO-*d₆*) δ 9.44 (s, 1H), 8.82 (s, 1H), 8.63 (s, 1H), 8.09 (d, 1H), 7.87-7.84 (m, 1H), 7.23-7.19 (m, 2H), 7.15-7.10 (m, 2H), 4.31-4.28 (m, 1H), 4.15 (t, 2H), 3.86-3.83 (m, 1H), 2.95-2.89 (m, 1H), 2.65 (s, 3H), 2.51-2.38 (m, 5H), 2.11-2.01 (m, 2H), 1.79-1.68 (m, 1H), 1.60-1.47 (m, 2H), 1.14-1.04 (m, 1H), 0.93-0.82 (m, 1H).

### Example 129: synthesis of 3-(4-(4-(2,3-dichlorophenyl)piperazin-1-yl)-4-oxobutyl)-9-methyl-3,5-dihydro-2H-chromeno[4,3-d]py rimidin-2-one

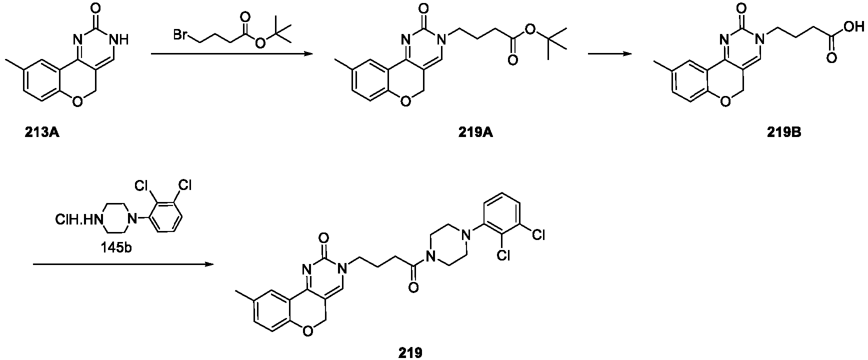

Step 1: 213A (600 mg, 2.80 mmol, 1.0 eq) was added to DMF (10 mL). Then, potassium carbonate (1.16 g, 8.40 mmol, 3.0 eq) and tert-butyl 4-bromobutyrate (937 mg, 4.20 mmol, 1.5 eq) were added. After being stirred at 40 °C for 16 hours, the reaction solution was diluted with water and extracted with ethyl acetate. The organic phase was evaporated to obtain a crude product, which was purified by a normal-phase column (eluent: (petroleum ether: ethyl acetate = 5% - 80%)). The target fraction was collected and evaporated to obtain a yellow solid 219A (690 mg, yield: 69.1%).

Step 2: 219A (690 mg, 1.94 mmol, 1.0 eq) was added to 4M HCl/1,4-dioxane (10 mL) and stirred at 25 °C for 2 hours. The reaction solution was evaporated to obtain a yellow solid 219B (550 mg, yield: 94.6%).

Step 3: 219B (150 mg, 0.499 mmol, 1.0 eq), 145b (160 mg, 0.599 mmol, 1.2 eq), and DIPEA (323 mg, 2.50 mmol, 5.0 eq) were added to DMF (3 mL). Then, HATU (285 mg, 0.749 mmol, 1.5 eq) was added. After being stirred at 25 °C for 2 hours, the reaction solution was purified by a C18 reverse-phase column (eluent: 0.1% formic acid aqueous solution: MeOH = 1:20 - 9:1). The target fraction was collected and freeze-dried to obtain an off-white solid 219 (169 mg, yield: 65.9%).
LCMS: 513 [M+H]⁺
¹H NMR (400 MHz, DMSO-*d₆*) δ 8.17 (s, 1H), 7.88-7.87 (m, 1H), 7.38-7.34 (m, 3H), 7.18-7.16 (m, 1H), 6.97 (d, 1H), 5.06 (s, 2H), 3.94 (t, 2H), 3.69-3.60 (m, 4H), 3.02-2.96 (m, 4H), 2.48 (m, 2H), 2.35 (s, 3H), 2.04-1.97 (m, 2H).

### Example 130: synthesis of 3-(4-(4-(4-fluorobenzyl)piperidin-1-yl)-4-oxobutyl)-9-methyl-3,5-dihydro-2H-chromeno[4,3-d]pyrimi din-2-one

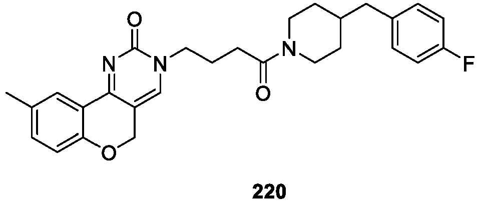

Except that 145b in Step 3 of Example 129 was replaced with 4-(4-fluorobenzyl)piperidine (146b), the same synthetic route as in Example 129 was adopted to prepare the title compound 220 (off-white solid).
LCMS: 476 [M+H]⁺
¹H NMR (400 MHz, DMSO-*d₆*) δ 8.12 (s, 1H), 7.86-7.85 (m, 1H), 7.36-7.34 (m, 1H), 7.22-7.18 (m, 2H), 7.13-7.09 (m, 2H), 6.95 (d, 1H), 5.03 (s, 2H), 4.37-4.34 (m, 1H), 3.90-3.79 (m, 3H), 2.94-2.88 (m, 1H), 2.49-2.33 (m, 8H), 1.96-1.89 (m, 2H), 1.77-1.67 (m, 1H), 1.58-1.52 (m, 2H), 1.13-0.94 (m, 2H).

### Example 131: synthesis of 3-(3-oxo-3-(4-(3-(trifluoromethyl)phenyl)piperazin-1-yl)propyl)-3,5,6,7-tetrahydro-4H-benzo[6,7]cycl ohepta[1,2-d]pyrimidin-4-one

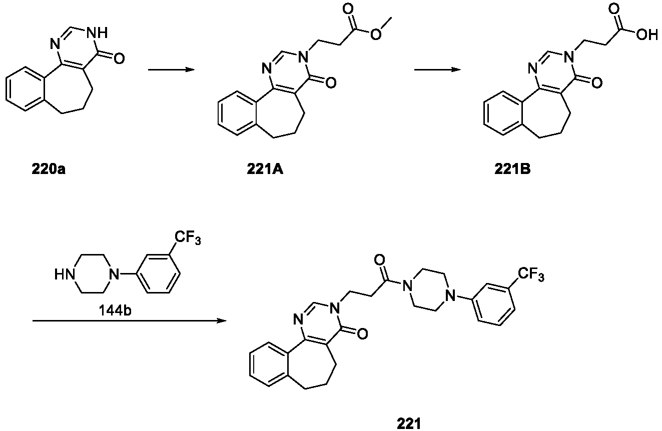

Step 1: 220D (200 mg, 0.94 mmol, 1.0 eq), methyl 3-bromopropionate (224 mg, 1.13 mmol, 1.2 eq), and potassium carbonate (325 mg, 2.36 mmol, 2.5 eq) were dissolved in ACN (10 mL). The mixture was stirred at 70 °C for 16 hours. After the reaction was completed, the reaction solution was evaporated and purified by flash column chromatography (PE:EA = 1:1) to obtain a white oil 221A (250 mg, yield: 89.1%).

Step 2: 221A (250 mg) was dissolved in THF/H₂O (5 mL/2 mL). Then, LiOH (50 mg, 0.2 w/w) was added. The mixture was stirred at room temperature for 16 hours. After the reaction was completed, the mixture was filtered. The filtrate was evaporated and purified by flash column chromatography (PE:EA = 1:1) to obtain a white solid 221B (150 mg, yield: 62.9%).

Step 3: 221B (150 mg, 0.52 mmol, 1.0 eq), 144b (181 mg, 0.79 mmol, 1.5 eq), HATU (400 mg, 1.05 mmol, 2.0 eq), and DIPEA (169 mg, 1.31 mmol, 2.5 eq) were dissolved in DCM (5 mL). The mixture was stirred at room temperature for 16 hours. After the reaction was completed, the mixture was extracted with water (20 mL) and dichloromethane (5 mL * 2). The organic phase was evaporated and purified by a reverse-phase column (0.1% FA/H₂O:MeOH = 30:70). The resulting preparative solution was freeze-dried to obtain a white solid 221 (100 mg, yield 38.7%).
LCMS: 497 [M+H]⁺
1H NMR (400 MHz, DMSO-*d₆*) δ 8.50 (s, 1H), 7.59-7.56 (m, 1H), 7.43 (t, 1H), 7.39-7.35 (m, 2H), 7.34-7.30 (m, 1H), 7.24 (dd, 1H), 7.18 (s, 1H), 7.19 (dd, 1H), 4.16 (t, 2H), 3.64-3.56(m, 4H), 3.25-3.19 (m, 4H), 2.91(t, 2H), 2.48-2.46 (m, 2H), 2.33-2.30(m, 2H), 2.17-2.12 (m, 2H).

### Example 132: synthesis of 3-(3-(4-(2,3-dichlorophenyl)piperazin-1-yl)propyl)-3,5,6,7-tetrahydro-4H-benzo[6,7]cyclohepta[1,2-d] pyrimidin-4-one formate

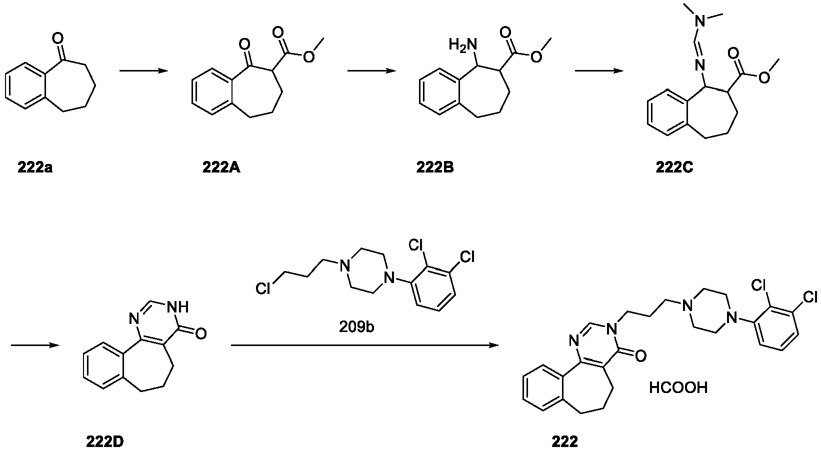

Step 1: 222a (10 g, 62.4 mmol, 1.0 eq) was dissolved in THF (100 mL). The system was cooled to 0 °C, and then 60% NaH was added. The mixture was stirred at room temperature for 30 minutes under nitrogen protection. Finally, DMC (30 mL) was added, and the system was stirred at room temperature for 16 hours. After the reaction was completed, it was quenched with MeOH (100 mL). The mixture was evaporated and purified by flash column chromatography (PE:EA = 1:1) to obtain a yellow oil 222A (13 g, yield: 95.4%).

Step 2: 222A (3 g, 13.74 mmol, 1.0 eq) and ammonium acetate (4.2 g, 54.98 mmol) were dissolved in MeOH (50 mL). The mixture was stirred at room temperature for 16 hours. After the reaction was completed, the mixture was evaporated. The reaction was extracted with water (50 mL) and dichloromethane (20 mL * 2). The organic phase was evaporated to obtain a white oil 222B (3 g, yield: 99.6%).

Step 3: 222B (3 g, 13.8 mmol, 1.0 eq) was dissolved in DMF (30 mL). Then, DMF-DMA (6.58 g, 55.256 mmol, 4.0 eq) was added. The mixture was stirred at 80 °C for 16 hours. After the reaction was completed, the mixture was extracted with water (100 mL) and dichloromethane (50 mL * 2). The organic phase was evaporated to obtain a red oil 222C (3.76 g, crude yield: 99.2%).

Step 4: 222C (3.76 g) was dissolved in formamide (20 mL). The mixture was stirred at 200 °C for 1 hour. After the reaction was completed, the mixture was extracted with water (50 mL) and dichloromethane (20 mL * 2). The organic phase was evaporated and purified by flash column chromatography (DCM:MeOH = 20:1) to obtain a yellow solid 222D (1.2 g, yield: 41.2%).

Step 5: 222D (200 mg, 0.94 mmol, 1.0 eq), 209b (347 mg, 1.13 mmol, 1.2 eq), and potassium carbonate (325 mg, 2.36 mmol, 2.5 eq) were added to acetonitrile (5 mL). The mixture was stirred at 70 °C for 16 hours. After the reaction was completed, the mixture was evaporated. The reaction was extracted with water (20 mL) and dichloromethane (10 mL * 2). The organic phase was evaporated and purified by reverse-phase column chromatography (0.05% FA/H₂O:MeOH = 30:70). The product was freeze-dried to obtain the formate salt of compound 222 as a white solid (90 mg, yield 19.8%).
LCMS: 483 [M+H]⁺
¹H NMR (400 MHz, DMSO-*d₆*) δ 8.47 (s, 1H), 8.21 (s, 1H), 7.62-7.60 (m, 1H), 7.39-7.36 (m, 2H), 7.34-7.31 (m, 1H), 7.30-7.27 (m, 2H), 7.10-7.06 (m, 1H), 4.01 (t, 2H), 2.95(s, 5H), 2.56-2.53 (m, 5H), 2.44 (t, 2H), 2.33 (t, 2H), 2.18-2.13 (m, 2H), 1.95-1.90 (m, 2H).

### Example 133: synthesis of 3-(4-(3-(4-fluorobenzyl)piperidin-1-yl)-4-oxobutyl)-9-methyl-5,6-dihydrobenzo[h]quinazolin-4(3H)-o ne

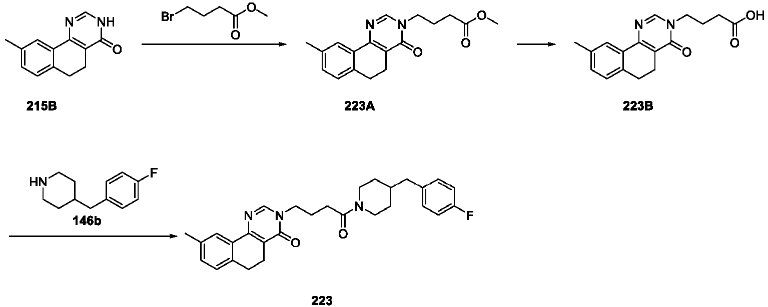

Step 1: 215B (500 mg, 2.35 mmol, 1.0 eq), methyl 4-bromobutyrate (639 mg, 3.53 mmol, 1.5 eq), and potassium carbonate (813 mg, 5.89 mmol, 2.5 eq) were dissolved in ACN (10 mL). The mixture was stirred at 70 °C for 16 hours. After the reaction was completed, the reaction solution was evaporated and purified by flash column chromatography (PE:EA = 1:1) to obtain a yellow oil 223A (600 mg, yield: 81.7%).

Step 2: 223A (600 mg) was dissolved in THF/H₂O (10 mL/3 mL). Then, LiOH (120 mg, 0.2 w/w) was added. The mixture was stirred at room temperature for 16 hours. After the reaction was completed, the mixture was filtered. The filtrate was evaporated and purified by flash column chromatography (PE:EA = 1:1) to obtain a white solid 223B (500 mg, yield: 87.2%).

Step 3: 223B (200 mg, 0.67 mmol, 1.0 eq), 146b (194 mg, 1.00 mmol, 1.5 eq), HATU (510 mg, 1.34 mmol, 2.0 eq), and DIPEA (216 mg, 1.67 mmol, 2.5 eq) were dissolved in DCM (5 mL). The mixture was stirred at room temperature for 16 hours. After the reaction was completed, the mixture was extracted with water (20 mL) and dichloromethane (5 mL * 2). The organic phase was evaporated and purified by a reverse-phase column (0.1% FA/H₂O:MeOH = 30:70). The resulting preparative solution was freeze-dried to obtain a yellow solid 223 (50 mg, yield 15.7%).
LCMS: 474 [M+H]⁺
¹H NMR (400 MHz, DMSO-*d₆*) δ 8.39 (s, 1H), 7.88 (s, 1H), 7.20-7.15 (m, 4H), 7.11-7.06 (m, 2H), 4.33 (dd, 1H), 3.93 (t, 2H), 3.79 (d, 1H), 2.90 (t, 1H), 2.83-2.79 (m, 2H), 2.70-2.66 (m, 2H), 2.47-2.41 (m, 3H), 2.36-2.32 (m, 5H), 1.94-1.89 (m, 2H), 1.72-1.68 (m, 1H), 1.53(t, 2H), 1.13-0.88 (m, 2H).

### Example 134: synthesis of 3-(3-(4-(2,3-dichlorophenyl)piperazin-1-yl)-3-oxopropyl)-8-methoxy-3,5-dihydro-4H-imidazo[4,5-c]q uinolin-4-one

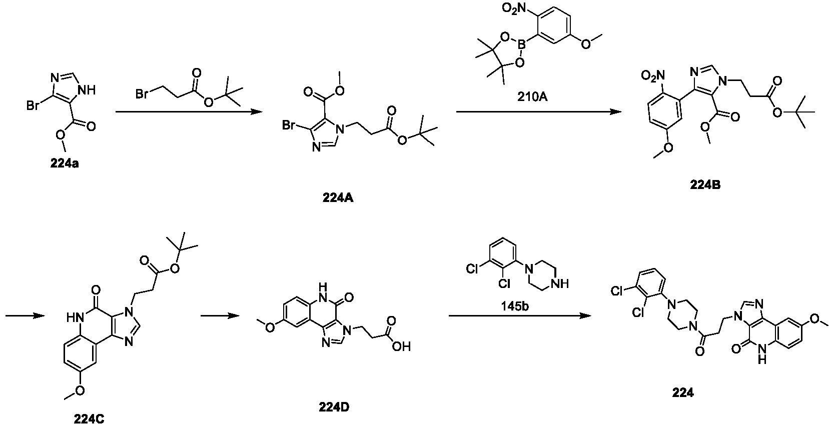

Step 1: 224a (1.11 g, 0.0054 mol, 1.0 eq.), tert-butyl 4-bromopropionate (1.7 g, 0.0108 mol, 2.0 eq.), cesium carbonate (3.53 g, 0.0108 mol, 2.0 eq.), DIPEA (1 mL), and DMF (20 mL) were added to a 250 mL three-necked flask and stirred at 80 °C overnight. After the reaction was completed, the product was purified by column chromatography and eluted with EtOAc:PE = 20:80. After concentration under reduced pressure, a yellow oil 224A (1.1 g, yield: 61.45%) was obtained.

Step 2: 224A (1.1 g, 0.0033 mol, 1.0 eq.), 210A (1.38 g, 0.0049 mol, 1.5 eq.), Pd-118 (0.2 g, 0.00033 mol, 0.1 eq.), potassium carbonate (0.91 g, 0.0066 mol, 2.0 eq.), dioxane (20 mL), and water (5 mL) were added to a 250 mL three-necked flask and stirred at 100 °C overnight under nitrogen protection. After the reaction was completed, the product was purified by silica gel column chromatography and eluted with EtOAc:PE = 20:80. After concentration, a yellow oil 224B (1.24 g, yield 93.23%) was obtained.

Step 3: 224B (1.24 g, 0.003 mol, 1.0 eq.), zinc powder (1.95 g, 0.03 mol, 10.0 eq.), saturated aqueous NH₄Cl solution (1.2 mL), and EtOH (10 mL) were added to a 100 mL three-necked flask and stirred at 85 °C overnight. After the reaction was completed, the mixture was filtered, and the filtrate was evaporated to obtain a crude product 224C (0.5 g).

Step 4: 224C (0.5 g) and HCl/1,4-dioxane solution (1M, 10 mL) were added to a 50 mL three-necked flask and stirred at room temperature overnight. After the reaction was completed, the product was purified by a reverse-phase column and eluted with MeOH/H₂O. The target fraction was collected and evaporated to obtain a white solid 224D (0.1 g, yield: 27.03%).

Step 5: 224D (0.1 g, 0.35 mmol, 1.0 eq.), 2,3-dichlorophenylpiperazine hydrochloride (145b) (0.096 g, 0.42 mmol, 1.2 eq.), HATU (0.16 g, 0.42 mmol, 1.2 eq.), DIPEA (0.3 mL), and DCM (5 mL) were added to a 100 mL three-necked flask and stirred at room temperature overnight. After the reaction was completed, the product was purified by reverse-phase column chromatography and eluted with MeOH/H₂O. The target fraction was collected and freeze-dried to obtain a white solid 224 (0.014 g, yield: 8.05%).
LCMS: 500 [M+1]⁺
¹H NMR (400 MHz, DMSO-*d₆*) δ 11.58 (s, 1H), 8.29 (s, 1H), 7.52 (d, 1H), 7.40-7.28 (m, 3H), 7.11-7.05 (m, 2H), 4.71 (t, 2H), 3.86 (s, 3H), 3.64-3.61 (m, 4H), 3.07 (t, 2H), 2.92-2.91 (m, 4H).

### Example 135: synthesis of 3-(3-(4-(4-fluorobenzyl)piperidin-1-yl)-3-oxopropyl)-9-methyl-5,6-dihydrobenzo[h]quinazolin-4(3H)-one

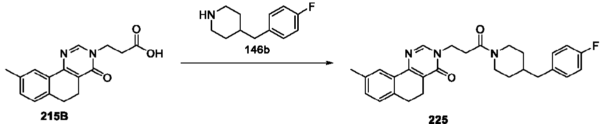

Except that 144b in Step 3 of Example 125 was replaced with 146b, the same synthetic route as in Example 125 was adopted to prepare the title compound 225 (white solid).
LCMS: 460 [M+H]⁺
¹H NMR (400 MHz, DMSO-*d₆*) δ 8.45 (s, 1H), 7.88 (s, 1H), 7.19-7.17 (m, 2H), 7.14-7.04 (m, 4H), 4.34 (d, 1H), 4.10 (t, 2H), 3.77 (d, 1H), 2.92-2.87 (m, 1H), 2.82-2.72 (m, 4H), 2.68-2.65 (m, 2H), 2.47-2.40 (m, 3H), 2.34(s, 3H), 1.74-1.64 (m,1H), 1.52 (d, 2H), 1.01-0.85 (m, 2H).

### Example 136: synthesis of 3-(8-methyl-4-oxopyrimido[5,4-b]quinolin-3(4H)-yl)-N-(3-(trifluoromethyl)benzyl)propanamide

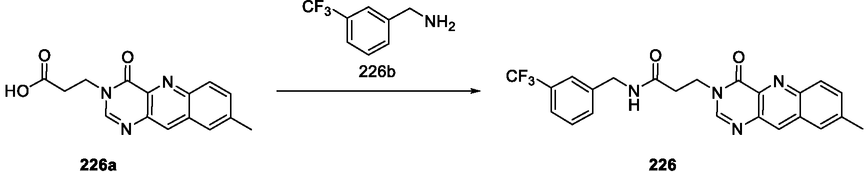

226a (0.28 g, 0.001 mol, 1 eq.), 3-(trifluoromethyl)benzylamine (226b) (0.17 g, 0.001 mol, 1 eq.), HATU (0.76 g, 0.002 mol, 2 eq.), DIPEA (0.26 g, 0.002 mol, 2 eq.), and dichloromethane (50 mL) were added to a 250 mL three-necked flask, and the temperature was lowered to 0 °C. The reaction was carried out for 2 hours. After the reaction was completed, the reaction was quenched with a saturated sodium bicarbonate solution, and the mixture was extracted twice with dichloromethane. The organic phase was dried over anhydrous sodium sulfate, concentrated, and then purified by preparative liquid chromatography. The product was eluted with methanol/water, concentrated under reduced pressure, and then freeze-dried to obtain an off-white solid 226 (0.06 g, yield: 13.6%).
LCMS: 441 [M+1]+
¹H NMR (400 MHz, DMSO-*d₆*): δ 8.59 (t, 2H), δ 8.30 (s, 1H), δ 8.12 (d, 1H), δ 7.92 (s, 1H), 7.72 (d, 1H), δ 7.50 (s, 2H),7.43-7.38 (m, 2H), 4.41 (d, 2H),4.26(t, 2H),2.75(t, 2H), 2.54 (s, 3H).

### Example 137: synthesis of 3-(4-(4-(2,3-dichlorophenyl)piperazin-1-yl)-4-oxobutyl)-8-methoxy-3,5-dihydro-4H-imidazo[4,5-c]qui nolin-4-one

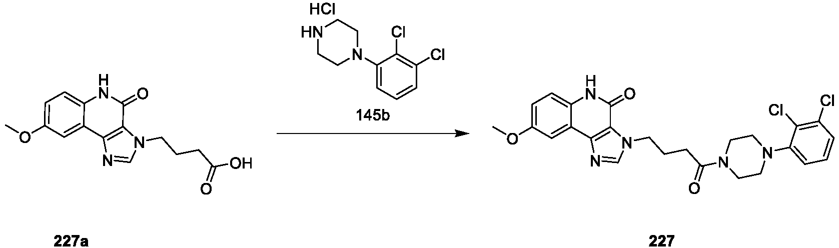

227a (0.23 g, 0.681 mmol, 1.0 eq.), 1-(2,3-dichlorophenyl)piperazine hydrochloride (145b) (0.27 g, 1.02 mmol, 1.5 eq.), HATU (0.39 g, 1.02 mmol, 1.5 eq.), anhydrous DMF (5 mL), and DIPEA (0.44 g, 3.41 mmol, 5.0 eq.) were added to a 100 mL single-necked flask, and the reaction was carried out at room temperature for 1 h. The reaction solution was subjected to reverse-phase column chromatography (eluent: 0.5‰ formic acid in water: methanol = 10:90) to collect the product fraction. The methanol was evaporated, and a solid precipitated. The solid was filtered, washed with water, and dried under reduced pressure to obtain a light-brown solid 227 (0.21 g, yield: 59.9%).
LCMS: 514 [M+1]⁺
¹H NMR (400 MHz, DMSO-*d₆*): 11.57 (s, 1H), 8.18 (s, 1H), 7.51 (d, 1H), 7.37-7.29 (m, 3H), 7.10-7.05 (m, 2H), 4.50 (t, 2H), 3.83 (s, 3H), 3.58-3.53 (m, 4H), 2.92-2.87 (m, 4H), 2.38 (t, 2H), 2.16-2.09 (m, 2H).

### Example 138: synthesis of 3-(4-oxo-4-(4-(3-(trifluoromethyl)phenyl)piperazin-1-yl)butyl)-3,5,6,7-tetrahydro-4H-benzo[6,7]cyclo hepta[1,2-d]pyrimidin-4-one

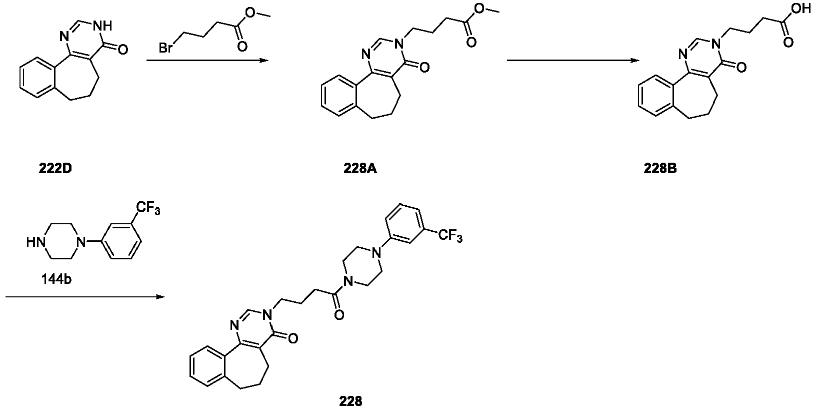

Step 1: 222D (200 mg, 0.94 mmol, 1.0 eq.), methyl 4-bromobutyrate (240 mg, 1.13 mmol, 1.2 eq.), and potassium carbonate (325 mg, 2.36 mmol, 2.5 eq.) were dissolved in ACN (10 mL), and the mixture was stirred at 70 °C for 16 h. After the reaction was completed, the reaction solution was evaporated and purified by flash column chromatography (PE:EA = 1:1) to obtain a white oil 228A (150 mg, yield: 51.1%).

Step 2: 228A (150 mg) was dissolved in THF/H₂O (5 mL/2 mL), and then LiOH (50 mg, 0.2 w/w) was added. The mixture was stirred at room temperature for 16 h. After the reaction was completed, the mixture was filtered, and the filtrate was evaporated and purified by flash column chromatography (PE:EA = 1:1) to obtain a white solid 228B (120 mg, yield: 83.3%).

Step 3: 228B (120 mg, 0.40 mmol, 1.0 eq.), 1-(3-(trifluoromethyl)phenyl)piperazine (144b) (138 mg, 0.60 mmol, 1.5 eq.), HATU (305 mg, 0.80 mmol, 2.0 eq.), and DIPEA (129 mg, 1.00 mmol, 2.5 eq.) were dissolved in DCM (5 mL), and the mixture was stirred at room temperature for 16 h. After the reaction was completed, the mixture was extracted with water (20 mL) and dichloromethane (5 mL * 2). The organic phase was evaporated and purified by a reverse-phase column (0.1% FA/H₂O:MeOH = 30:70). The resulting preparation was freeze-dried to obtain a white solid 228 (40 mg, yield 19.6%).
LCMS: 511 [M+H]⁺
¹H NMR (400 MHz, DMSO-*d₆*) δ 8.43 (s, 1H), 7.61-7.59 (m, 1H), 7.45-7.41 (m, 1H), 7.39-7.36 (m, 2H), 7.33-7.31 (m, 1H), 7.24 (d, 1H), 7.18 (s, 1H), 7.09 (d, 1H), 3.98 (t, 2H), 3.61-3.58(m, 4H), 3.28-3.25 (m, 2H), 3.21-3.19 (m, 2H), 2.55-2.53 (m, 2H), 2.46(t, 2H), 2.31(t, 2H), 2.17-2.09 (m, 2H), 2.01-1.94 (m, 2H).

### Example 139: synthesis of 8-methoxy-2-(4-oxo-4-(4-(3-(trifluoromethyl)phenyl)piperazin-1-yl)butyl)-1,2,3,5-tetrahydro-4H-pyrr olo[3,4-c]quinolin-4-one

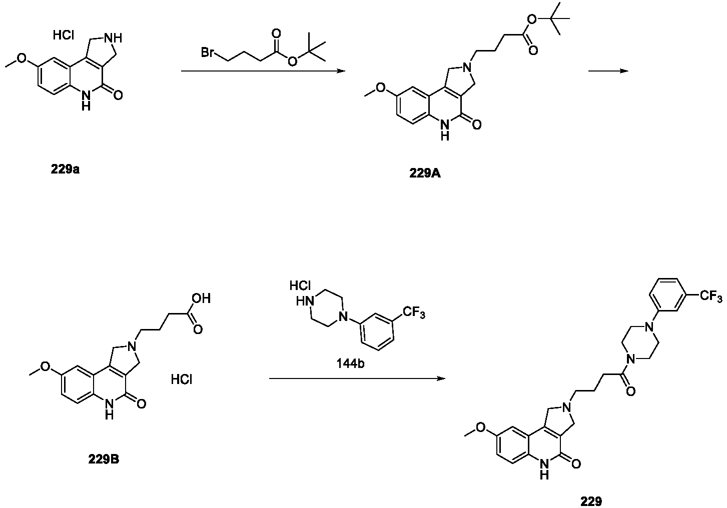

Step 1: 229a (0.2 g, 0.791 mmol, 1.0 eq.), *tert-butyl* 4-bromobutyrate (0.26 g, 1.19 mmol, 1.5 eq.), triethylamine (0.4 g, 3.96 mmol, 5.0 eq.), and acetonitrile (15 mL) were added to a 100 mL single-necked flask and reacted at 60 °C for 24 h. After the reaction was completed, the crude product was purified by column chromatography (eluent: DCM:MeOH = 96:4). The product fraction was collected and concentrated to obtain a light-brown solid 229A (0.4 g, a preliminarily purified crude product, which was directly used in the next step).

Step 2: 229A (0.4 g, 0.791 mmol, 1.0 eq.) and a solution of HCl in 1,4-dioxane (5 mL, 4 mol/L) were added to a 100 mL single-necked flask and reacted at room temperature for 1 h. After the reaction was completed, the mixture was filtered, and the filter cake was washed with 1,4-dioxane to obtain an off-white solid 229B (0.18 g, yield 67.1%).

Step 3: 229B (0.18 g, 0.571 mmol, 1.0 eq.), 1-(3-trifluoromethylphenyl)piperazine hydrochloride (144b) (0.21 g, 0.797 mmol, 1.5 eq.), HATU (0.3 g, 0.797 mmol, 1.5 eq.), anhydrous DMF (5 mL), and DIPEA (0.34 g, 2.66 mmol, 5.0 eq.) were added to a 100 mL single-necked flask and reacted at room temperature for 1 h. The reaction solution was preliminarily purified by reverse-phase column chromatography (eluent: 0.5‰ formic acid in water: methanol = 51:49). The resulting crude product was purified by preparative chromatography (eluent: 0.5‰ formic acid in water: methanol = 44:56). The product fraction was collected, the methanol was evaporated, and the residue was freeze-dried to obtain an off-white solid 229 (0.08 g, yield: 27.1%).
LCMS: 515 [M+1]⁺
¹H NMR (400 MHz, CDCl₃): 11.79 (s, 1H), 7.38-7.34 (m, 2H), 7.15-7.10 (m, 3H), 7.07-7.05 (m, 1H), 6.77 (d, 1H), 4.41 (s, 2H), 4.23 (s, 2H), 3.85 (s, 3H), 3.82-3.80 (m, 2H), 3.69-3.67 (m, 2H), 3.24-3.21 (m, 4H), 3.04 (t, 2H), 2.57 (t, 2H), 2.10-2.04 (m, 2H).

### Example 140: synthesis of 2-(3-(4-(2,3-dichlorophenyl)piperazin-1-yl)-3-oxopropyl)-8-methoxy-1,2,3,5-tetrahydro-4H-pyrrolo[3, 4-c]quinolin-4-one

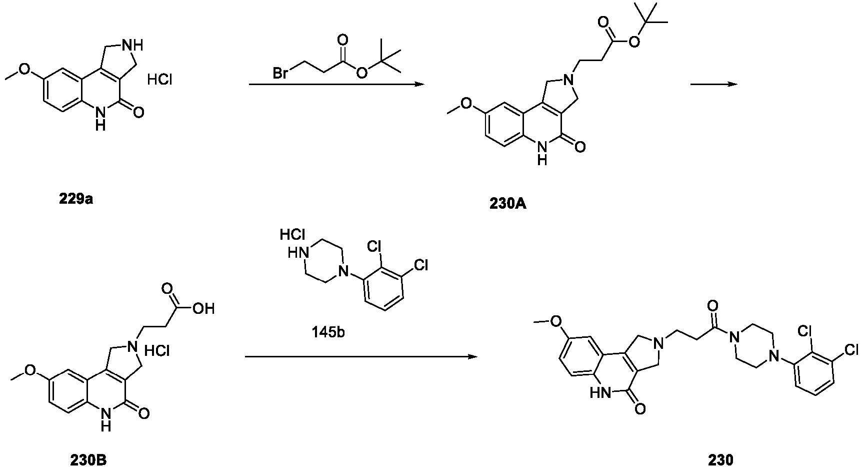

Step 1: 229a (crude product, 0.665 mmol, 1.0 eq.), tert-butyl 3-bromopropionate (0.21 g, 0.998 mmol, 1.5 eq.), triethylamine (0.27 g, 2.66 mmol, 4.0 eq.), and acetonitrile (15 mL) were added to a 100 mL single-necked flask and reacted at 60 °C for 24 h. After the reaction was completed, the crude product was purified by column chromatography (eluent: DCM:MeOH = 95:5). The product fraction was collected and concentrated to obtain a light-brown solid 230A (0.33 g, a preliminarily purified crude product, which was directly used in the next step).

Step 2: 230A (0.33 g, 0.665 mmol, 1.0 eq.) and a solution of HCl in 1,4-dioxane (5 mL, 4 mol/L) were added to a 100 mL single-necked flask and reacted at room temperature for 1 h. After the reaction was completed, the mixture was concentrated to obtain a crude product of light-yellow solid 230B, which was directly used in the next step.

Step 3: 230B (crude product, 0.831 mmol, 1.0 eq.), 1-(2,3-dichlorophenyl)piperazine hydrochloride (145b) (0.33 g, 1.25 mmol, 1.5 eq.), HATU (0.48 g, 1.25 mmol, 1.5 eq.), anhydrous DMF (5 mL), and DIPEA (0.54 g, 4.16 mmol, 5.0 eq.) were added to a 100 mL single-necked flask and reacted at room temperature for 1 h. The reaction solution was preliminarily purified by reverse-phase column chromatography (eluent: 0.5‰ formic acid in water: methanol = 55:45). The resulting crude product was purified by preparative chromatography (eluent: 1‰ formic acid in water: methanol = 43:57). The product fraction was collected, the methanol was evaporated, and the residue was freeze-dried to obtain a light-yellow solid 230 (0.05 g, yield: 11.9%).
LCMS: 501 [M+1]⁺
¹H NMR (400 MHz, DMSO-*d₆*): 11.62 (s, 1H), 7.35-7.30 (m, 3H), 7.16-7.13 (m, 2H), 6.96 (d, 1H), 4.21-4.20 (m, 2H), 3.88-3.86 (m, 2H), 3.79 (s, 3H), 3.70-3.63 (m, 4H), 3.01-2.93 (m, 6H), 2.68-2.64 (m, 2H).

### Example 141: synthesis of 3-(4-(4-(2,3-dichlorophenyl)piperazin-1-yl)-4-oxobutyl)-8-methoxy-3,5-dihydro-4H-imidazo[4,5-c]qui nolin-4-one

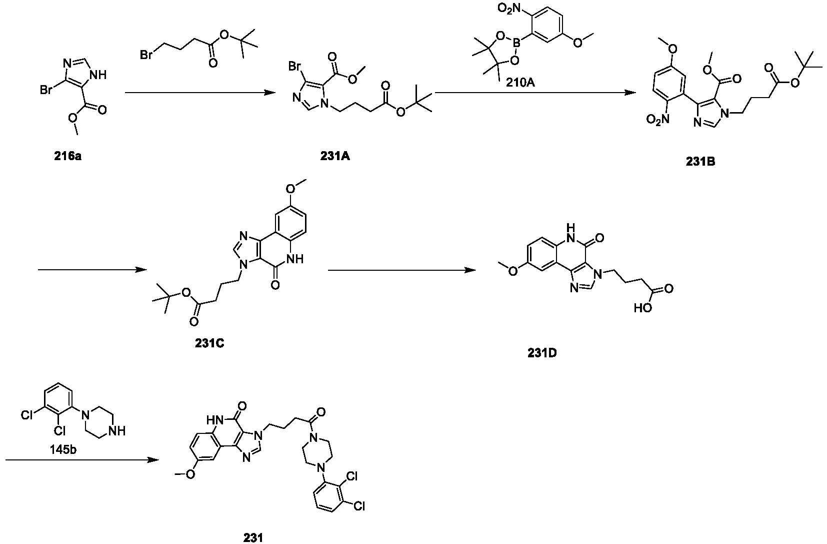

Step 1: 216a (1.11 g, 0.0054 mol, 1.0 eq.), *tert-butyl* 4-bromobutyrate (1.7 g, 0.0108 mol, 2.0 eq.), cesium carbonate (3.53 g, 0.0108 mol, 2.0 eq.), DIPEA (1 mL), and DMF (20 mL) were added to a 250 mL three-necked flask and stirred at 80 °C overnight. After the reaction was completed, the product was purified by column chromatography and eluted with EtOAc:PE = 20:80. After concentration under reduced pressure, a yellow oil 231A (1.1 g, yield: 61.45%) was obtained.

Step 2: 231A (1.1 g, 0.0033 mol, 1.0 eq.), 201A (1.38 g, 0.0049 mol, 1.5 eq.), Pd-118 (0.2 g, 0.00033 mol, 0.1 eq.), potassium carbonate (0.91 g, 0.0066 mol, 2.0 eq.), dioxane (20 mL), and water (5 mL) were added to a 250 mL three-necked flask and stirred at 100 °C overnight under nitrogen protection. After the reaction was completed, the product was purified by silica gel column chromatography and eluted with EtOAc:PE = 20:80. After concentration, a yellow oil 231B (1.24 g, yield 93.23%) was obtained.

Step 3: 231B (1.24 g, 0.003 mol, 1.0 eq.), zinc powder (1.95 g, 0.03 mol, 10.0 eq.), saturated aqueous NH₄Cl solution (1.2 mL), and EtOH (10 mL) were added to a 100 mL three-necked flask and stirred at 85 °C overnight. After the reaction was completed, the mixture was filtered, and the filtrate was evaporated to obtain a crude product 231C (0.5 g).

Step 4: 231C (0.5 g) and HCl/1,4-dioxane solution (1M, 10 mL) were added to a 50 mL three-necked flask and stirred at room temperature overnight. After the reaction was completed, the product was purified by a reverse-phase column and eluted with MeOH/H₂O. The target fraction was collected and evaporated to obtain a white solid 231D (0.1 g, yield: 27.03%).

Step 5: 231D (0.1 g, 0.35 mmol, 1.0 eq.), 2,3-dichlorophenylpiperazine hydrochloride (145b) (0.096 g, 0.42 mmol, 1.2 eq.), HATU (0.16 g, 0.42 mmol, 1.2 eq.), DIPEA (0.3 mL), and DCM (5 mL) were added to a 100 mL three-necked flask and stirred at room temperature overnight. After the reaction was completed, the product was purified by reverse-phase column chromatography and eluted with MeOH/H₂O. The target fraction was collected and freeze-dried to obtain a white solid 231 (0.008 g, yield: 4.44%).
LCMS: 514 [M+1]+
¹H NMR (500 MHz, DMSO-*d₆*) δ 11.57 (s, 1H), 8.27 (s, 1H), 7.50 (d, 1H), 7.36-7.29 (m, 3H), 7.10-7.05 (m, 2H), 4.50 (t, 2H), 3.83 (s, 3H), 3.57-3.54 (m, 4H), 2.92-2.88 (m, 4H), 2.38 (t, 2H), 2.15-2.09 (m, 2H).

### Example 142: synthesis of 2-methyl-N-(3-(4-(3-trifluoromethylphenyl)piperazin-1-yl)propyl)-6,6a,7,8,9,10-hexahydrobenzo[b]py rido[1,2-d][1,4]oxazine-7-carboxamide formate

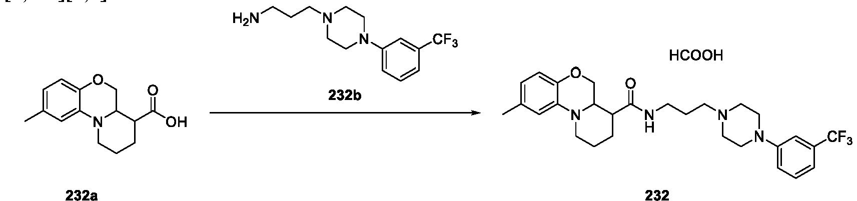

232a (50 mg, 0.20 mmol, 1.0 eq.), DMF (2 mL), and HATU (93 mg, 0.24 mmol, 1.2 eq.) were added to a reaction flask and stirred at room temperature for 30 min. Then, 3-(4-(3-trifluoromethylphenyl)piperazin-1-yl)propylamine (232b) (72 mg, 0.22 mmol, 1.1 eq) and DIPEA (78 mg, 0.61 mmol, 3.0 eq) were added. The mixture was stirred at room temperature overnight. The reaction solution was purified by column chromatography (eluent: 0.1% formic acid aqueous solution: MeOH = 46%). The target fraction was collected and concentrated under reduced pressure to obtain a white solid 232 (40 mg, yield: 38.31%).
LCMS: 517 [M+H]+
¹H NMR (400 MHz, DMSO-*d₆*) δ 8.16 (s, 1H), 8.03 (t, 0.73 H), 7.77 (t, 0.23 H), 7.41 (t, 1H), 7.26-7.11 (m, 2H), 7.06 (d, 1H), 6.81 (s, 0.25 H), 6.71 (s, 0.76 H), 6.56 (t, 1H), 6.47 (d, 0.25 H), 6.38 (d, 0.77 H), 4.06-3.99 (m, 1H), 3.92-3.78 (m, 2H), 3.35-2.94 (m, 8H), 2.63-2.54 (m, 4H), 2.47 (t, 1H), 2.40-2.31 (m, 2H), 2.17 (s, 3H), 1.87-1.79 (m, 1H), 1.71-1.57 (m, 4H), 1.54-1.43 (m, 1H).

### Example 143: synthesis of N-(2-4-(2,3-dichlorophenyl)piperazin-1-yl)ethyl)-2-methyl-6,6a,7,8,9,10-hexahydrobenzo[b]pyrido[1, 2-d][1,4]oxazine-7-carboxamide

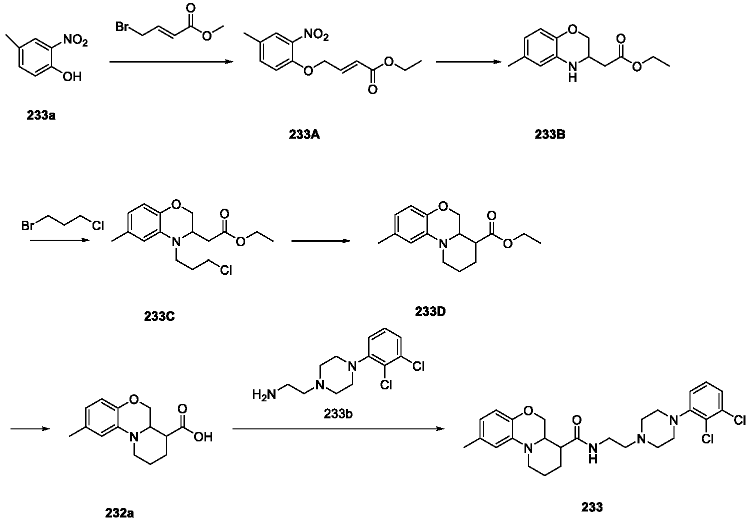

Step 1: 233a (4.50 g, 29.41 mmol, 1.0 eq.), MeCN (100 mL), methyl 4-bromobut-2-enoate (11.35 g, 58.82 mmol, 2.0 eq.), potassium carbonate (12.18 g, 88.24 mmol, 3.0 eq), and potassium iodide (976 mg, 5.88 mmol, 0.2 eq) were added to a reaction flask and stirred at 85 °C overnight. After the reaction solution was filtered, the filter cake was washed with ethyl acetate. Silica gel was added to the filtrate. After concentration, the residue was purified by column chromatography (eluent: ethyl acetate: petroleum ether = 45%). The target fraction was collected and concentrated under reduced pressure to obtain a white solid 233A (7.3 g, yield: 93.66%).

Step 2: 233A (7.30 g, 27.55 mmol, 1.0 eq.), acetic acid (100 mL), iron powder (9.26 g, 165.28 mmol, 6.0 eq.), potassium carbonate (12.18 g, 88.24 mmol, 3.0 eq), and potassium iodide (976 mg, 5.88 mmol, 0.2 eq) were added to a reaction flask and stirred at 120 °C for 30 minutes. After the reaction solution was cooled, it was extracted three times with ethyl acetate. The organic phase was washed with an aqueous sodium bicarbonate solution and then concentrated to obtain a crude product of black oil 233B (10.0 g).

Step 3: 233B (1.26 g, 5.36 mmol, 1.0 eq.), DMF (10 mL), DIPEA (2.07 g, 16.09 mmol, 3.0 eq.), and 1-bromo-3-chloropropane (1.68 g, 10.72 mmol, 2.0 eq) were added to a reaction flask and stirred at 80 °C overnight. After the reaction solution was cooled, water was added. It was extracted three times with ethyl acetate. The organic phase was washed with saturated sodium chloride, dried over anhydrous sodium sulfate, and then silica gel was added. After concentration, the residue was purified by column chromatography (eluent: ethyl acetate: petroleum ether = 12%). The target fraction was collected and concentrated under reduced pressure to obtain a white solid 233C (635 mg, yield: 39.87%).

Step 4: 233C (635 mg, 2.042 mmol, 1.0 eq.) and anhydrous tetrahydrofuran (20 mL) were added to a reaction flask. Nitrogen was purged. After the system was cooled to -78 °C, LiHMDS (1M) (13.2 mL, 6.5 eq) was added dropwise, and the reaction was allowed to proceed for 4 h with natural warming. After the reaction solution was quenched by dropwise addition of water, it was extracted three times with ethyl acetate. The organic phase was washed with saturated sodium chloride, dried over anhydrous sodium sulfate, and then silica gel was added. After concentration, the residue was purified by column chromatography (eluent: ethyl acetate: petroleum ether = 20%). The target fraction was collected and concentrated under reduced pressure to obtain a white solid 233D (300 mg, yield: 47.24%).

Step 5: 233D (300 mg, 1.09 mmol, 1.0 eq.), tetrahydrofuran (3 mL), water (1 mL), and lithium hydroxide (79 mg, 3.27 mmol, 3.0 eq.) were added to a reaction flask and stirred at room temperature overnight. After the reaction solution was concentrated, water (5 mL) was added. 2M HCl was added dropwise until no more white solid precipitated. After filtration and drying, a white solid 232a (250 mg, yield: 92.77%) was obtained.

Step 6: 232a (50 mg, 0.20 mmol, 1.0 eq.), DMF (2 mL), and HATU (93 mg, 0.24 mmol, 1.2 eq.) were added to a reaction flask and stirred at room temperature for 30 min. Then, 2-(4-(2,3-dichlorophenyl)piperazin-1-yl)ethan-1-amine (233b) (123 mg, 0.44 mmol, 1.2 eq) and DIPEA (78 mg, 0.61 mmol, 3.0 eq) were added. The mixture was stirred at room temperature overnight. The reaction solution was purified by column chromatography (eluent: 0.1% formic acid aqueous solution: MeOH = 46%). The target fraction was collected and concentrated under reduced pressure to obtain a white solid 233 (30 mg, yield: 29.58%).
LCMS: 503 [M+H]⁺
¹H NMR (400 MHz, DMSO-*d₆*) δ 7.97 (t, 1H), 7.37-7.25 (m, 2H), 7.14 (dd, 1H), 6.71 (s, 1H), 6.54 (d, 1H), 6.39 (d, 1H), 4.16(dd, 1H), 3.91-3.84 (m, 2H), 3.35-3.30 (m, 2H), 3.16-3.13 (m, 2H), 2.98 (br, 4H), 2.61-2.54 (m, 4H), 2.43 (t, 2H), 2.24-2.19 (m, 1H), 2.17 (s, 3H), 1.86-1.80 (m, 1H), 1.72-1.60 (m, 2H), 1.54-1.44 (m, 1H).

### Example 144: synthesis of N-(3-(4-(2,3-dichlorophenyl)piperazin-1-yl)propyl)-2-methyl-6,6a,7,8,9,10-hexahydrobenzo[b]pyrido[ 1,2-d][1,4]oxazine-7-carboxamide formate

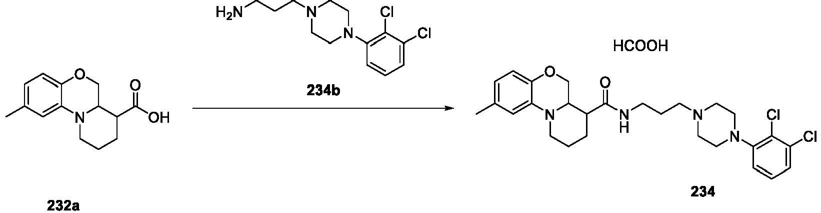

Except that 233b in Step 6 of Example 143 was replaced with 234b, the same synthetic route as in Example 143 was adopted to prepare the title compound 234 (white solid).
LCMS: 517 [M+H]+
¹H NMR (400 MHz, DMSO-*d₆*) δ 8.14 (s, 1H), 8.05 (t, 1H), 7.31 (d, 2H), 7.17-7.11 (m, 1H), 6.81 (s, 0.23 H), 6.72 (s, 0.74 H), 6.58 (d, 1H), 6.55 (d, 0.24 H), 6.39 (d, 0.75 H), 4.02 (dd, 1H), 3.91-3.82 (m, 2H), 3.22-3.08 (m, 4H), 3.06-2.96 (m, 4H), 2.67-2.57 (m, 6H), 2.48-2.43 (m, 1H), 2.17 (s, 3H), 1.87-1.76 (m, 1H), 1.72-1.57 (m, 4H), 1.55-1.46 (m, 1H).

### Example 145: synthesis of 2-(3-(4-(2,3-dichlorophenyl)piperazin-1-yl)propyl)-8-methoxy-1,2,3,5-tetrahydro-4H-pyrrolo[3,4-c]qu inolin-4-one

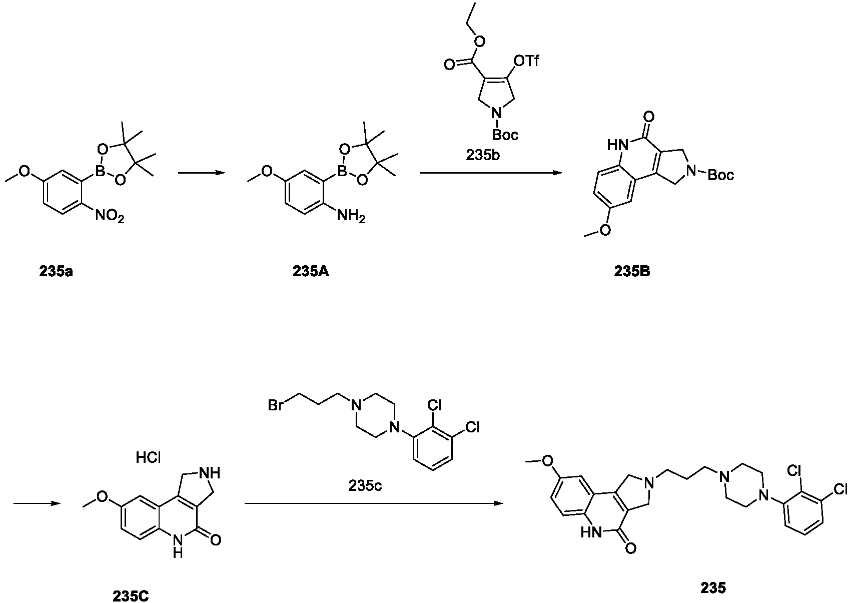

Step 1: 235a (2 g, 7.17 mmol, 1.0 eq.), 10% Pd/C (0.3 g, 15% w/w), and ethyl acetate (80 mL) were added to a 250 mL single-necked flask. Hydrogen was introduced at atmospheric pressure, and the reaction was allowed to proceed at room temperature for 16 h. After the reaction was completed, the mixture was filtered. The filter cake was washed with ethyl acetate. The filtrate was concentrated to obtain 235A. The crude product was directly used in the next step.

Step 2: 235A (crude product, 7.71 mmol, 1.5 eq.), 235b (2 g, 5.14 mmol, 1.0 eq.), Pd(dppf)Cl₂-DCM (0.84 g, 1.03 mmol, 0.2 eq.), sodium carbonate (1.63 g, 15.4 mmol, 3.0 eq.), 1,4-dioxane (100 mL), and water (10 mL) were added to a 250 mL single-necked flask. The reaction was carried out at 100 °C for 16 h under nitrogen protection. After the reaction was completed, the mixture was filtered. The filter cake was washed with DCM. The filtrate was concentrated. The crude product was subjected to silica gel column chromatography (eluent: DCM:MeOH = 97:3). The product fraction was collected, and the solvent was evaporated to obtain a brown solid 235B (0.7 g, yield: 43.0%).

Step 3: 235B (0.7 g, 2.22 mmol, 1.0 eq.) and a solution of HCl in 1,4-dioxane (7 mL, 4 mol/L) were added to a 100 mL single-necked flask and reacted at room temperature for 1 h. After the reaction was completed, the mixture was filtered. The filter cake was washed with 1,4-dioxane and dried to obtain a light-brownish-yellow solid 235C (0.45 g, yield: 80.2%).

Step 4: 235C (0.25 g, 0.989 mmol, 1.0 eq.), 1-(3-bromopropyl)-4-(2,3-dichlorophenyl)piperazine (235c) (0.7 g, 1.98 mmol, 2.0 eq.), triethylamine (0.5 g, 4.95 mmol, 5.0 eq.), and acetonitrile (15 mL) were added to a 100 mL single-necked flask and reacted at 60 °C for 16 h. After the reaction was completed, the mixture was filtered. The filter cake was washed with acetonitrile. The filter cake was added to DMSO (10 mL), stirred for 30 min, and then filtered. The filter cake was washed successively with DMSO and methanol and dried to obtain an off-white solid 235 (0.12 g, yield: 24.9%).
LCMS: 487 [M+1]⁺
¹H NMR (400 MHz, CDCl₃): 11.81 (s, 1H), 7.44 (d, 1H), 7.22-7.16 (m, 3H), 7.03 (dd, 1H), 6.83-6.82 (m, 1H), 4.30-4.28 (m, 2H), 4.16-4.14 (m, 2H), 3.90 (s, 3H), 3.17 (br, 4H), 2.93 (t, 2H), 2.77-2.64 (m, 6H), 1.97-1.92 (m, 2H).

### Experimental Examples

### Experimental Example 1. Pharmacodynamic Experiment (TPK Enzyme Activity Test)

### 1.1 Experimental Purpose

The purpose of this experimental example was to test the promoting effect of compounds on TPK enzyme activity and evaluate the in vitro activity of the compounds based on EC₅₀ and Eₘₐₓ.

### 1.2 Experimental Method

### 1.2.1 Experimental Materials

| **Reagent (Equipment) Name** | **Supplier Company** | **Catalog Number** |
|---|---|---|
| Tris-HCl (Trizma hydrochloride) | VETEC | V900312-500G |
| EDTA Disodium Salt | Sigma | 27285-500G-R |
| 2-Mercaptoethanol | Sigma | M6250-100ML |
| MgSO₄ | Sigma | 63136-250G-F |
| ATP | Sigma | A26209-5G |
| TM (Thiamine Hydrochloride) | Sigma | T4625-100G |
| NaOH | Sigma | S8045-500G |
| HCl | Sinopharm/Shanghai Reagent | 10011018 |
| 72% Perchloric Acid | Sinopharm | 20181207 |
| Distilled Water | Watson's | 400 mL/bottle |
| High-speed Low-temperature Centrifuge | eppendorf | 5430R |
| Constant-temperature Water Bath | Shanghai Bilon Instrument Co., Ltd. | DK-8D |
| High performance Liquid Chromatograph | Shimadzu | Agilent 1260 |

### 1.2.2 Experimental Procedures

1.1) The constant-temperature water-bath shaker was pre-heated half an hour in advance at a pre-heating temperature of 37 °C.
1.2) The stock solution of the compound to be tested was taken out, allowed to thaw at room temperature, and then diluted to the required concentration.
1.3) The required reagents were placed on ice to thaw.
2.1) For each reaction system, TPK enzyme solution, Tris-HCl buffer solution, and ATP solution were added. The concentration of ATP used was 1 - 500 mM. The above three solutions were formulated into a homogeneous mixture.
2.2) The mixture of the above three solutions was dispensed into centrifuge tubes. Then, the compound at the required concentration was added to each centrifuge tube. Finally, thiamine solution (1 - 100 µM) was added. After the addition of the above system was completed, the centrifuge tubes were tightly capped, inserted into a floating board, and placed in the constant-temperature water-bath shaker that had been pre-heated to 37 °C for incubation for 0.5 - 2.0 hours.
2.3) After the incubation was completed, perchloric acid stop solution was added and mixed well. Samples were taken into 1.5 mL centrifuge tubes and stored at -20 °C.
3.1) The samples stored at -20 °C were taken out, thawed at room temperature, and then subjected to derivatization treatment. The samples were placed in centrifuge tubes, potassium ferricyanide derivatization reagent was added, and finally phosphoric acid stop solution was added to stop the reaction.
3.2) The derivatized samples were placed in sample vials for liquid chromatography analysis, and the contents of TDP/thiamine were detected by high performance liquid chromatography. TPK enzyme activity = TDP (nM)/mg protein/min.

### 1.3 Experimental Results

The effects of the compounds of the present invention on TPK enzyme activity at different concentrations were determined according to the above procedures. The measured Eₘₐₓ (calculated with the effect of DMSO on TPK enzyme activity as 100%) and EC₅₀ data are shown in the following table.

| No. | Eₘₐₓ |
|---|---|
| 1 | 159.05% |
| 2 | 149.05% |
| 3 | 136.16% |
| 4 | 128.44% |
| 5 | 138.59% |
| 6 | 208.02% |
| 7 | 148.14% |
| 9 | 146.86% |
| 10 | 156.79% |
| 11 | 180.24% |
| 12 | 205.25% |
| 13 | 123.92% |
| 14 | 151.79% |
| 15 | 145.07% |
| 16 | 144.27% |
| 18 | 159.34% |
| 19 | 144.66% |
| 20 | 121.29% |
| 22 | 207.37% |
| 23 | 120.64% |
| 26 | 165.78% |
| 27 | 121.61% |
| 28 | 123.61% |
| 31 | 151.21% |
| 32 | 122.96% |
| 33 | 122.60% |
| 34 | 166.87% |
| 35 | 125.54% |
| 38 | 144.59% |
| 39 | 139.19% |
| 40 | 125.32% |
| 41 | 140.58% |
| 42 | 156.19% |
| 43 | 136.64% |
| 44 | 151.99% |
| 45 | 158.23% |
| 46 | 122.50% |
| 47 | 135.30% |
| Formate salt of 48 | 210.48% |
| 49 | 136.09% |
| Diformate salt of 52 | 195.12% |
| 53 | 162.02% |
| 54 | 169.90% |
| 55 | 158.92% |
| 57 | 158.96% |
| 58 | 146.75% |
| 59 | 190.24% |
| 61 | 171.66% |
| 63 | 148.14% |
| 66 | 137.59% |
| 67 | 187.37% |
| 69 | 168.03% |
| 71 | 141.74% |
| 72 | 122.42% |
| 77 | 121.84% |
| 81 | 122.53% |
| 82 | 123.15% |
| 83 | 125.92% |
| 87 | 146.31% |
| 88 | 124.42% |
| 89 | 131.59% |
| 91 | 139.63% |
| 92 | 160.24% |
| 93 | 192.41% |
| 97 | 177.65% |
| 113 | 120.49% |
| 118 | 121.59% |
| 129 | 156.45% |
| 130 | 209.89% |
| 131 | 192.65% |
| 132 | 163.08% |
| 134 | 124.84% |
| 138 | 146.39% |
| 139 | 197.96% |
| 140 | 187.35% |
| 141 | 203.96% |
| 142 | 223.56% |
| 144 | 161.38% |
| 146 | 158.92% |
| 147 | 160.16% |
| 148 | 150.52% |
| 150 | 170.69% |
| 151 | 188.64% |
| 153 | 130.73% |
| 154 | 144.27% |
| 164 | 172.61% |
| 165 | 156.73% |
| 166 | 119.46% |
| 169 | 146.74% |
| 170 | 151.12% |
| 184 | 176.21% |
| 186 | 175.98% |
| 187 | 191.25% |
| 189 | 179.75% |
| 190 | 164.09% |
| Formate salt of 191 | 130.85% |
| 193 | 177.82 % |
| 194 | 139.09 % |
| 195 | 135.82 % |
| 197 | 132.38 % |
| Dihydrochloride salt of 198 | 132.31 % |
| 199 | 203.82 % |
| 200 | 168.10 % |
| 204 | 186.22% |
| 205 | 137.51% |
| 206 | 164.79% |
| 207 | 129.32% |
| 210 | 166.19% |
| 213 | 174.20% |
| 214 | 137.60% |
| 215 | 172.99% |
| 217 | 153.22% |
| 218 | 153.86% |
| 220 | 157.21% |
| 221 | 154.34% |
| Formate salt of 222 | 143.31% |
| 223 | 152.22% |
| 225 | 172.73% |
| 228 | 147.52% |
| 231 | 137.36% |
| Formate salt of 232 | 135.64% |
| 235 | 137.39% |

| No. | EC₅₀ (µM) |
|---|---|
| 1 | 0.11 |
| 2 | 2.048 |
| 3 | 1.784 |
| 5 | 2.29 |
| 6 | 1.032 |
| 7 | 2.319 |
| 9 | 0.7683 |
| 10 | 1.15 |
| 11 | 1.075 |
| 14 | 2.444 |
| 15 | 0.4632 |
| 16 | 0.36 |
| 19 | 1.999 |
| 26 | 2.616 |
| 31 | 1.21 |
| 34 | 1.374 |
| 42 | 3.955 |
| 44 | 3.298 |
| 45 | 1.223 |
| Formate salt of 48 | 1.06 |
| Diformate salt of 52 | 1.276 |
| 53 | 1.582 |
| 54 | 1.679 |
| 55 | 10.56 |
| 57 | 1.191 |
| 59 | 1.384 |
| 61 | 1.208 |
| 67 | 1.203 |
| 91 | 3.72 |
| 92 | 3.555 |
| 97 | 1.007 |
| 129 | 1.165 |
| 130 | 1.358 |
| 138 | 1.162 |
| 139 | 0.95 |
| 141 | 4.069 |
| 142 | 9.044 |
| 144 | 1.956 |
| 146 | 1.908 |
| 147 | 7.716 |
| 148 | 1.987 |
| 150 | 1.99 |
| 151 | 5.983 |
| 153 | 3.17 |
| 154 | 2.60 |
| 164 | 2.32 |
| 165 | 2.49 |
| 166 | 0.4951 |
| 169 | 2.144 |
| 170 | 2.37 |
| 186 | 2.66 |
| 187 | 1.132 |
| 189 | 1.201 |
| 190 | 1.107 |
| Formate salt of 191 | 5.6 |
| 193 | 7.487 |
| Dihydrochloride salt of 198 | 3.366 |
| 199 | 0.49 |
| 200 | 0.002936 |
| 204 | 0.1071 |
| 206 | 1.453 |
| 210 | 1.80 |
| 213 | 0.5455 |
| 214 | 2.376 |
| 215 | 1.126 |
| 217 | 1.47 |
| 218 | 0.6171 |
| 220 | 3.886 |
| 221 | 1.65 |
| Formate salt of 222 | 0.50 |
| 223 | 1.33 |
| 225 | 0.93 |
| 228 | 2.134 |
| 231 | 2.262 |
| Formate salt of 232 | 1.812 |
| 235 | 0.5719 |

Various modifications of the invention in addition to those described herein will become apparent to those skilled in the art from the foregoing description. Such modifications are intended to fall within the scope of the appended claims. Each reference, including all patents, applications, journal articles, books and any other disclosure, referred to herein is hereby incorporated by reference in its entirety.

## Claims

1. A method for the prophylaxis or treatment of neurodegenerative diseases or alleviating symptoms of neurodegenerative diseases, which comprises administering to a subject in need thereof a prophylactically or therapeutically effective amount of a thiamine pyrophosphokinase (TPK) agonist;
preferably, the neurodegenerative disease is Alzheimer's disease;
more preferably, the Alzheimer's disease is one in which the subject has decreased TPK enzyme activity, decreased TPK expression level, and/or decreased TDP level;
wherein the TPK agonist is a compound of Formula (I)-I, or a pharmaceutically acceptable salt, ester, stereoisomer, polymorph, solvate, N-oxide, isotopically labeled compound, metabolite, or prodrug thereof: 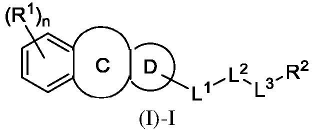
preferably, the TPK agonist is a compound of Formula (I), or a pharmaceutically acceptable salt, ester, stereoisomer, polymorph, solvate, N-oxide, isotopically labeled compound, metabolite, or prodrug thereof: 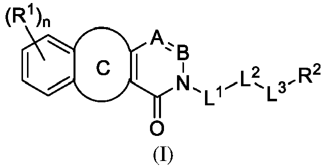
wherein:
A and B are each independently CR³ or N;
ring C and ring D are each independently C₃₋₁₀ hydrocarbon ring (e.g., C₃₋₆ hydrocarbon ring), 3- to 10-membered heterocycle, C₆₋₁₀ aromatic ring or 5- to 14-membered heteroaromatic ring; preferably, ring C is 5- to 6-membered heteroaromatic ring;
L¹, L² and L³ are each independently absent or are selected from the group consisting of -O-, -C(=O)-, -C(=O)O-, -NR-, -C(=O)NR-, -(S=O)NR-, -S(=O)₂NR-, -S-, -S(=O)-, -S(=O)₂-, -C₁₋₆ alkylene-, -C₂₋₆ alkenylene-, -C₂₋₆ alkynylene-, -C₃₋₆ cyclic hydrocarbylene-, -(3- to 10-membered heterocyclylene)-, -C₆₋₁₀ arylene-, -(5- to 14-membered heteroarylene)-, -W-C₁₋₆ alkylene-, -C₁₋₆ alkylene-W- and -W-C₁₋₆ alkylene-W'-, wherein the alkylene group is optionally further interrupted by one or more W; provided that at least one of L¹, L² and L³ is present;
W and W', at each occurrence, are each independently selected from the group consisting of -O-, -C(=O)-, -C(=O)O-, -NR-, -C(=O)NR-, -(S=O)NR-, -S(=O)₂NR-, -S-, -S(=O)- and -S(=O)₂-;
R¹, at each occurrence, is each independently selected from the group consisting of halogen, -OH, -NH₂, -CN, -NO₂, C₁₋₆ alkyl, haloC₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-OR^{a}, -C₁₋₆ alkylene-NR^{a}R^{b} and -O-C₁₋₆ alkylene-NR^{a}R^{b};
R² and R³, at each occurrence, are each independently selected from the group consisting of H, halogen, -OH, -NH₂, -CN, -NO₂, C₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-OR^{a}, -C₁₋₆ alkylene-NR^{a}R^{b} and -O-C₁₋₆ alkylene-NR^{a}R^{b};
R, R^{a} and R^{b}, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl;
n is 0, 1, 2, 3 or 4; preferably, n is 0, 1 or 2;
the above alkyl, alkylene, alkenyl, alkenylene, alkynyl, alkynylene, cyclic hydrocarbyl, cyclic hydrocarbylene, hydrocarbon ring, heterocyclyl, heterocyclylene, heterocycle, aryl, arylene, aromatic ring, heteroaryl, heteroarylene, heteroaromatic ring and aralkyl, at each occurrence, are each optionally substituted with one or more substituents independently selected from the group consisting of: halogen, -OH, =O, -NH₂, -CN, -NO₂, C₁₋₆ alkyl, haloC₁₋₆ alkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{c}, -OC(=O)R^{c}, -C(=O)OR^{c}, -OR^{c}, -SR^{c}, -S(=O)R^{c}, -S(=O)₂R^{c}, -S(=O)₂NR^{c}R^{d}, -NR^{c}R^{d}, -C(=O)NR^{c}R^{d}, -NR^{c}-C(=O)R^{d}, -NR^{c}-C(=O)OR^{d}, -NR^{c}-S(=O)₂-R^{d}, -NR^{c}-C(=O)-NR^{c}R^{d}, -C₁₋₆ alkylene-OR^{c}, -C₁₋₆ alkylene-NR^{c}R^{d} and -O-C₁₋₆ alkylene-NR^{c}R^{d}, the alkyl, alkylene, cyclic hydrocarbyl, heterocyclyl, aryl, heteroaryl and aralkyl are further optionally substituted with one or more substituents independently selected from the group consisting of: halogen, -OH, =O, -C(=O)O-tert-butyl, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -O-C₁₋₆ alkyl and -C₁₋₆ alkylene-O-C₁₋₆ alkyl; and
R^{c} and R^{d}, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl, the alkyl, cyclic hydrocarbyl, heterocyclyl, aryl, heteroaryl and aralkyl are further optionally substituted with one or more substituents independently selected from the group consisting of: halogen, -OH, =O, -C(=O)O-tert-butyl, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl and -C₁₋₆ alkylene-O-C₁₋₆ alkyl.

2. The method according to claim 1, wherein the TPK agonist is a compound of Formula (II), or a pharmaceutically acceptable salt, ester, stereoisomer, polymorph, solvate, N-oxide, isotopically labeled compound, metabolite, or prodrug thereof: 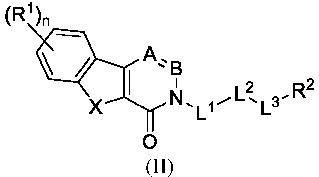 wherein:
X is -C(R)₂-, -NR-, -O- or -S-; preferably, X is -NR-, -O- or -S-; more preferably, X is -NH-, -N(CH₃)-, -O- or -S-; and
each of the remaining groups is as defined in claim 1.

3. The method according to claim 1, wherein

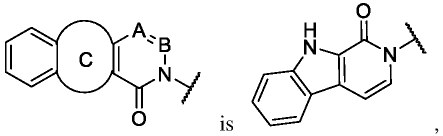

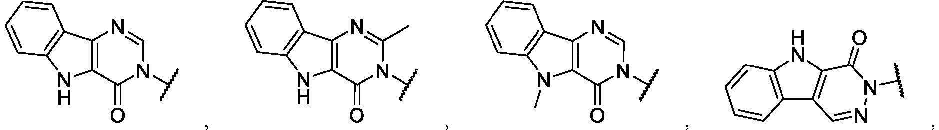

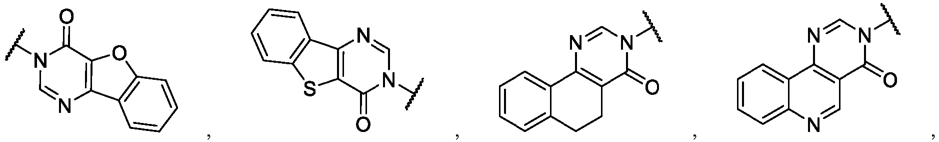

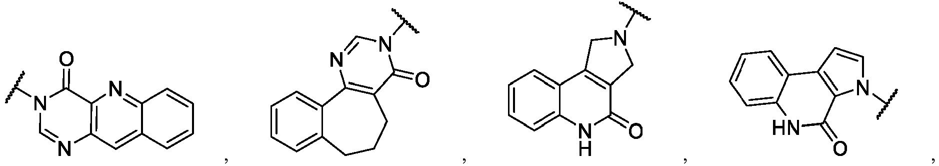

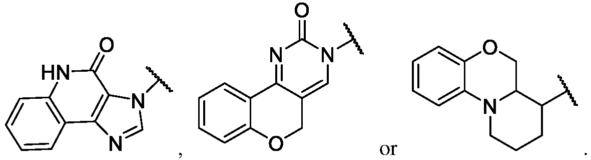

4. The method according to any one of claims 1 to 3, wherein R³, at each occurrence, is each independently H or C₁₋₆ alkyl (preferably methyl);
preferably, A and B are each independently CH, CCH₃ or N; and
more preferably, A is N and B is CH.

5. The method according to any one of claims 1 to 4, wherein L¹ and L³ are each independently absent or are -C(=O)-, -N(CH₃)-, -C₁₋₆ alkylene-, -W-C₁₋₆ alkylene- or -C₁₋₆ alkylene-W-, the alkylene group is optionally further interrupted by one or more W, and
W is -O-, -C(=O)-, -C(=O)O-, -NH-, -N(CH₃)-, -C(=O)NH- or -C(=O)N(CH₃)-.

6. The method according to any one of claims 1 to 5, wherein L² is absent or is -(3- to 10-membered heterocyclylene)-;
preferably, L² is absent or is piperazinylene or piperidinylene.

7. The method according to any one of claims 1 to 6, wherein 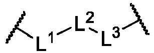 is

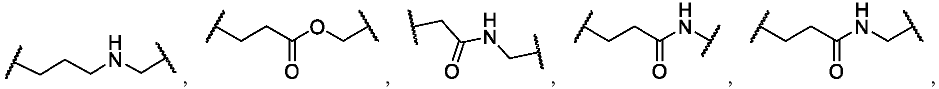

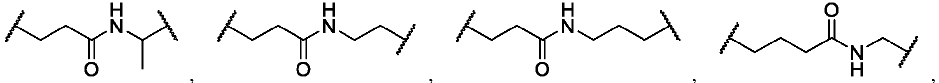

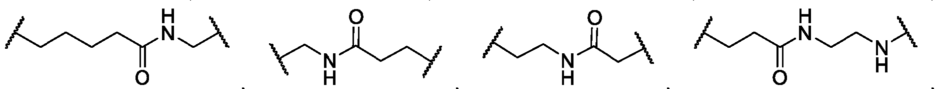

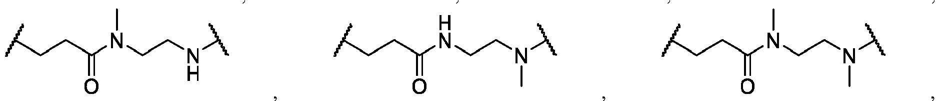

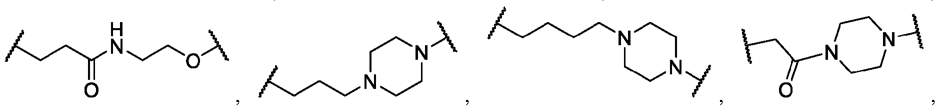

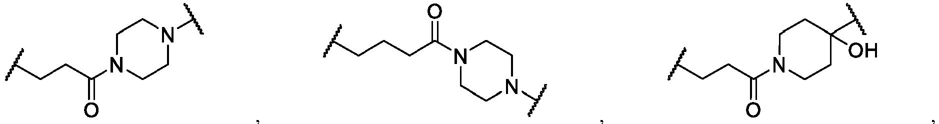

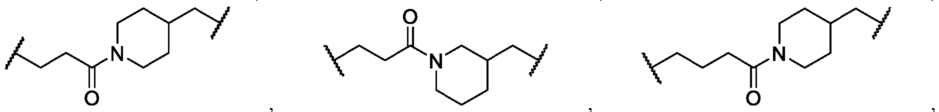

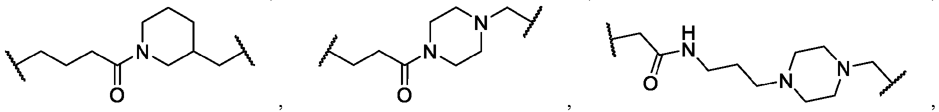

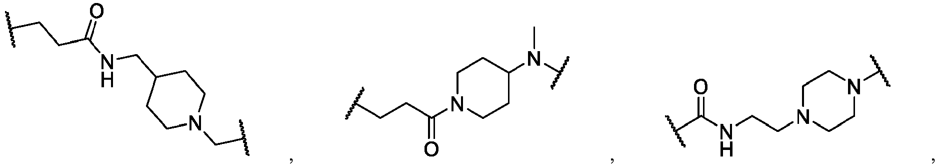

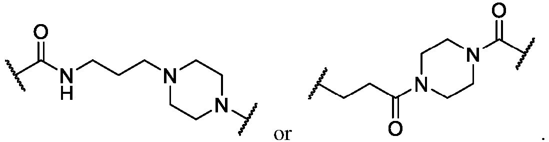

8. The method according to any one of claims 1 to 7, wherein R¹, at each occurrence, is each independently selected from the group consisting of halogen, -CN, C₁₋₆ alkyl, haloC₁₋₆ alkyl and C₁₋₆ alkoxy;
preferably, R¹, at each occurrence, is each independently selected from the group consisting of -F, -Cl, -Br, -CN, -CH₃, -CF₃ and -OCH₃.

9. The method according to any one of claims 1 to 8, wherein R² is independently selected from the group consisting of H, C₁₋₆ alkyl, C₆₋₁₀ aryl and 5- to 14-membered heteroaryl; the groups are optionally substituted with one or more substituents independently selected from the group consisting of: halogen, -OH, -NH₂, -CN, -NO₂, C₁₋₆ alkyl, haloC₁₋₆ alkyl, -N(C₁₋₆ alkyl)₂, C₁₋₆ alkoxy, haloC₁₋₆ alkoxy, -C(=O)-C₁₋₆ alkyl, -C(=O)OH, -C(=O)O-C₁₋₆ alkyl, -S-C₁₋₆ alkyl, -S(=O)₂-C₁₋₆ alkyl, -S(=O)₂-(3- to 10-membered heterocyclyl), -S(=O)₂NH₂ and -C(=O)NH₂;
preferably, R² is independently selected from the group consisting of isopropyl,

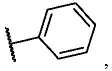

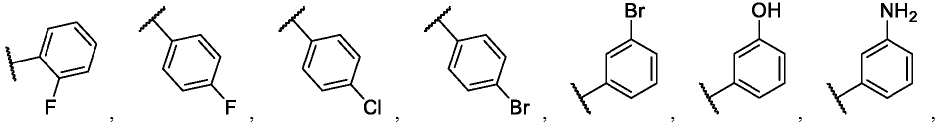

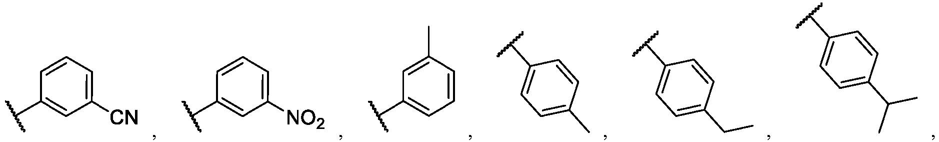

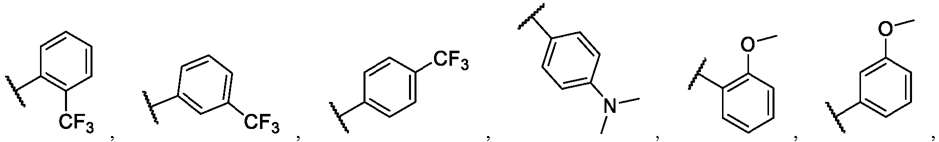

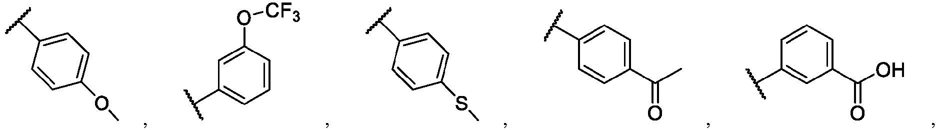

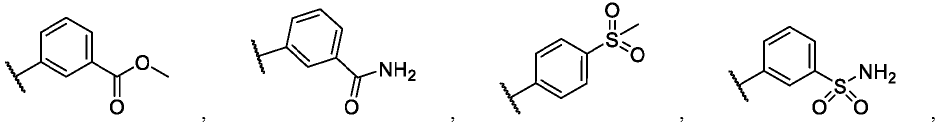

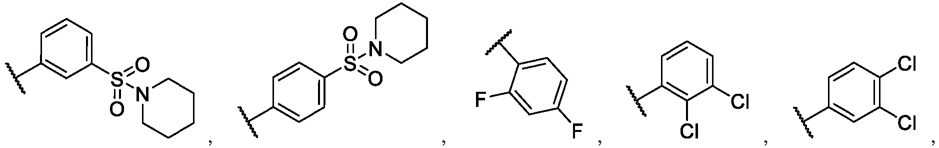

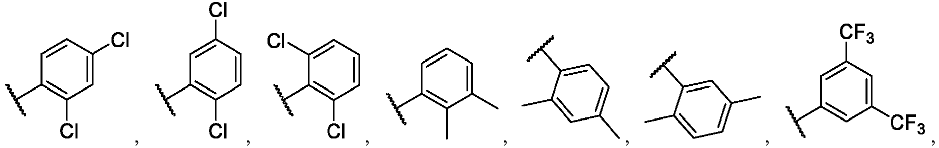

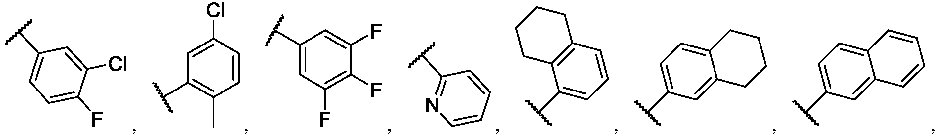

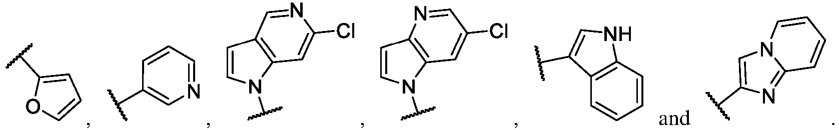

10. The method according to any one of claims 1 to 9, wherein the TPK agonist is a compound of Formula (III), or a pharmaceutically acceptable salt, ester, stereoisomer, polymorph, solvate, N-oxide, isotopically labeled compound, metabolite, or prodrug thereof: 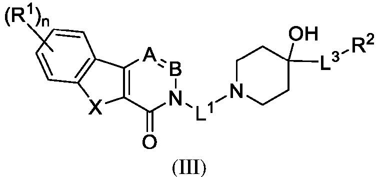 wherein each group is as defined in any one of claims 1 to 9.

11. A method for the prophylaxis or treatment of neurodegenerative diseases or alleviating symptoms of neurodegenerative diseases, which comprises administering to a subject in need thereof a prophylactically or therapeutically effective amount of a thiamine pyrophosphokinase (TPK) agonist;
preferably, the neurodegenerative disease is Alzheimer's disease;
more preferably, the Alzheimer's disease is one in which the subject has decreased TPK enzyme activity, decreased TPK expression level, and/or decreased TDP level;
wherein the TPK agonist is a compound of Formula (IV), or a pharmaceutically acceptable salt, ester, stereoisomer, polymorph, solvate, N-oxide, isotopically labeled compound, metabolite, or prodrug thereof: 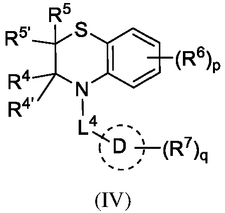
wherein:
ring D is absent or is C₃₋₆ hydrocarbon ring, 3- to 10-membered heterocycle, C₆₋₁₀ aromatic ring or 5-to 14-membered heteroaromatic ring;
L⁴ is selected from the group consisting of -O-, -C(=O)-, -C(=O)O-, -NR'-, -C(=O)NR'-, -(S=O)NR'-, -S(=O)₂NR'-, -S-, -S(=O)-, -S(=O)₂-, -C₁₋₆ alkylene-, -C₂₋₆ alkenylene-, -C₂₋₆ alkynylene-, -C₃₋₆ cyclic hydrocarbylene-, -(3- to 10-membered heterocyclylene)-, -C₆₋₁₀ arylene-, -(5- to 14-membered heteroarylene)-, -U-C₁₋₆ alkylene-, -C₁₋₆ alkylene-U-, -U-C₁₋₆ alkylene-U'- and -C₁₋₆ alkylene-U-C₁₋₆ alkylene-, wherein the alkylene group is optionally further interrupted by one or more U;
U and U', at each occurrence, are each independently selected from the group consisting of -O-, -C(=O)-, -C(=O)O-, -NR'-, -C(=O)NR'-, -(S=O)NR'-, -S(=O)₂NR'-, -S-, -S(=O)- and -S(=O)₂-;
R⁴, R^{4'}, R⁵, R^{5'}, R⁶ and R⁷, at each occurrence, are each independently selected from the group consisting of H, halogen, -OH, -NH₂, -CN, -NO₂, C₁₋₆ alkyl, haloC₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{e}, -OC(=O)R^{e}, -C(=O)OR^{e}, -ORS^{e}, -SR^{e}, -S(=O)R^{e}, -S(=O)₂R^{e}, -S(=O)₂NR^{e}R^{f}, -NR^{e}R^{f}, -C(=O)NR^{e}R^{f}, -NR^{e}-C(=O)R^{f}, -NR^{e}-C(=O)OR^{f}, -NR^{e}-S(=O)₂-R^{f}, -NR^{e}-C(=O)-NR^{e}R^{f}, -C₁₋₆ alkylene-OR^{e}, -C₁₋₆ alkylene-NR^{e}R^{f}, -O-C₁₋₆ alkylene-NR^{e}R^{f} and -C₁₋₆ alkylene-OC(=O)-C₁₋₆ alkylene-C(=O)OR^{e}; or, R⁴ and R^{4'} or R⁵ and R^{5'} together form =O; or, R⁴, R^{4'}, R⁵, R^{5'} together with the group to which they are attached form C₃₋₆ hydrocarbon ring, 3- to 10-membered heterocycle, C₆₋₁₀ aromatic ring or 5- to 14-membered heteroaromatic ring;
R', R^{e} and R^{f}, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl;
p and q are each independently 1, 2, 3 or 4; preferably, p and q are each independently 1 or 2; provided that when ring D is absent, q is 1;
the above alkyl, alkylene, alkenyl, alkenylene, alkynyl, alkynylene, cyclic hydrocarbyl, cyclic hydrocarbylene, hydrocarbon ring, heterocyclyl, heterocyclylene, heterocycle, aryl, arylene, aromatic ring, heteroaryl, heteroarylene, heteroaromatic ring and aralkyl, at each occurrence, are each optionally substituted with one or more substituents independently selected from the group consisting of: halogen, -OH, =O, -NH₂, -CN, -NO₂, C₁₋₆ alkyl, haloC₁₋₆ alkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{g}, -OC(=O)R^{g}, -C(=O)OR^{g}, -OR^{g}, -SR^{g}, -S(=O)R^{g}, -S(=O)₂R^{g}, -S(=O)₂NR^{g}R^{h}, -NR^{g}R^{h}, -C(=O)NR^{g}R^{h}, -NR^{g}-C(=O)R^{h}, -NR^{g}-C(=O)OR^{h}, -NR^{g}-S(=O)₂-R^{h}, -NR^{g}-C(=O)-NR^{g}R^{h}, -C₁₋₆ alkylene-OR^{g}, -C₁₋₆ alkylene-NR^{g}R^{h} and -O-C₁₋₆ alkylene-NR^{g}R^{h}, the alkyl, alkylene, cyclic hydrocarbyl, heterocyclyl, aryl, heteroaryl and aralkyl are further optionally substituted with one or more substituents independently selected from the group consisting of: halogen, -OH, =O, -C(=O)O-tert-butyl, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -O-C₁₋₆ alkyl and -C₁₋₆ alkylene-O-C₁₋₆ alkyl; and
R^{g} and R^{h}, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl, the alkyl, cyclic hydrocarbyl, heterocyclyl, aryl, heteroaryl and aralkyl are further optionally substituted with one or more substituents independently selected from the group consisting of: halogen, -OH, =O, -C(=O)O-tert-butyl, -NH₂, -CN, -NO₂, C₁₋₆ alkyl, C₁₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl and -C₁₋₆ alkylene-O-C₁₋₆ alkyl.

12. The method according to claim 11, wherein the TPK agonist is a compound of Formula (V), or a pharmaceutically acceptable salt, ester, stereoisomer, polymorph, solvate, N-oxide, isotopically labeled compound, metabolite, or prodrug thereof: 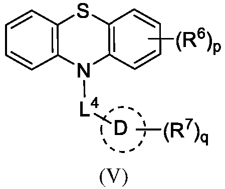 wherein each group is as defined in claim 11.

13. The method according to claim 11 or 12, wherein L⁴ is selected from the group consisting of -(CH₂)₂-, -(CH₂)₃-, -(CH₂)₆-, -(CH₂)₃-NH-, -(CH₂)₃-O-, -(CH₂)₄-O-, -(CH₂)₅-O-, -(CH₂)₆-O-, -C(=O)-CH₂-, -C(=O)-(CH₂)₂-, -(CH₂)₂-C(=O)NH-(CH₂)₂- and -CH₂-CH(OH)-CH₂-NH-.

14. The method according to any one of claims 11 to 13, wherein 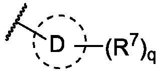 is -CN, -NH₂,

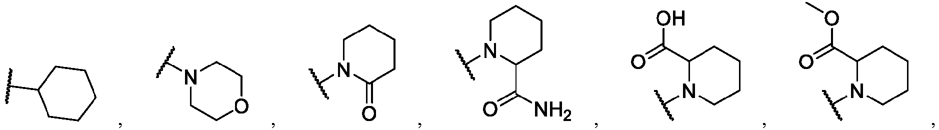

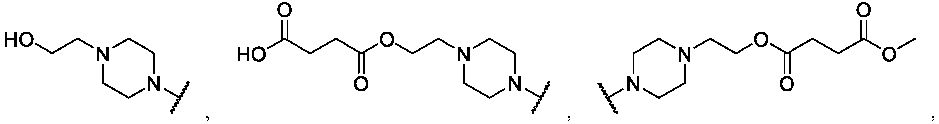

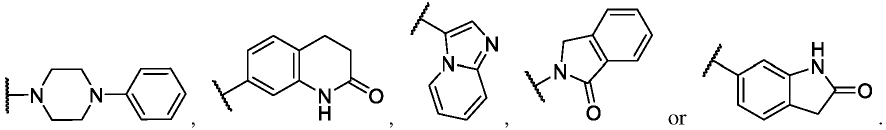

15. A method for the prophylaxis or treatment of neurodegenerative diseases or alleviating symptoms of neurodegenerative diseases, which comprises administering to a subject in need thereof a prophylactically or therapeutically effective amount of a thiamine pyrophosphokinase (TPK) agonist;
preferably, the neurodegenerative disease is Alzheimer's disease;
more preferably, the Alzheimer's disease is one in which the subject has decreased TPK enzyme activity, decreased TPK expression level, and/or decreased TDP level;
wherein the TPK agonist is selected from the following compounds, or a pharmaceutically acceptable salt, ester, stereoisomer, polymorph, solvate, N-oxide, isotopically labeled compound, metabolite, or prodrug thereof:
| **No.** | **Structure** |
|---|---|
| **1** | 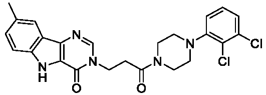 |
| **2** | 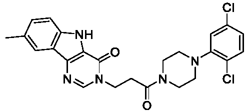 |
| **3** | 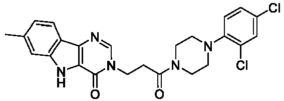 |
| **4** | 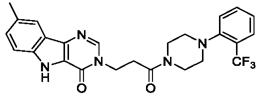 |
| **5** | 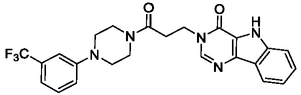 |
| **6** | 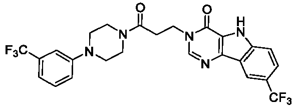 |
| **7** | 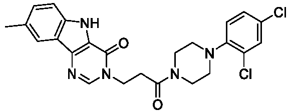 |
| **8** | 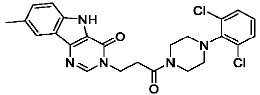 |
| **9** | 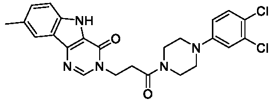 |
| **10** | 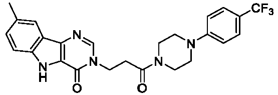 |
| **11** | 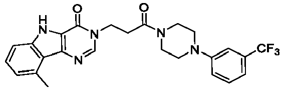 |
| **12** | 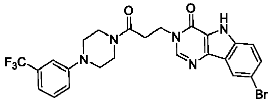 |
| **13** | 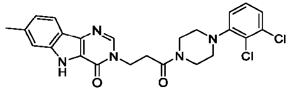 |
| **14** | 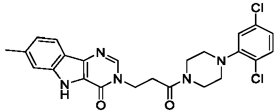 |
| **15** | 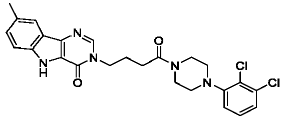 |
| **16** | 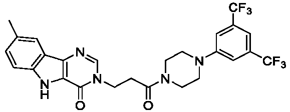 |
| **17** | 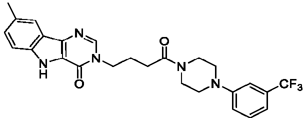 |
| **18** | 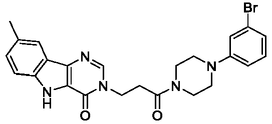 |
| **19** | 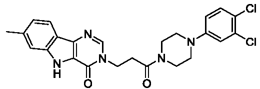 |
| **20** | 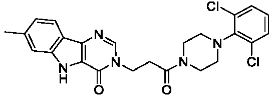 |
| **22** | 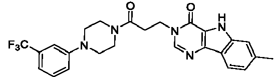 |
| **23** | 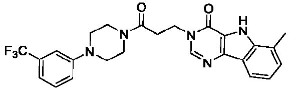 |
| **24** | 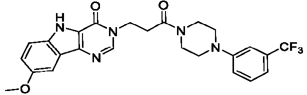 |
| **25** | 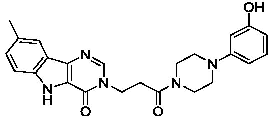 |
| **26** | 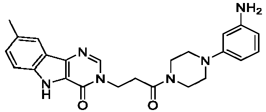 |
| **27** | 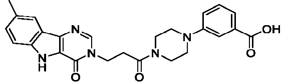 |
| **28** | 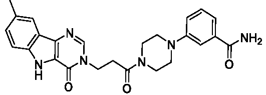 |
| **29** | 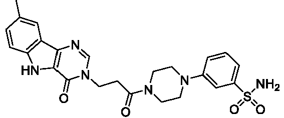 |
| **30** | 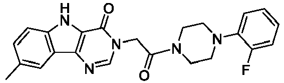 |
| **31** | 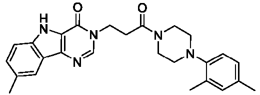 |
| **32** | 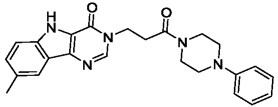 |
| **33** | 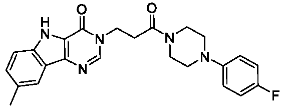 |
| **34** | 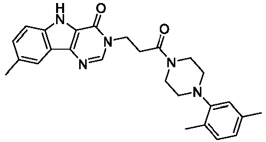 |
| **35** | 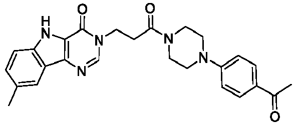 |
| **36** | 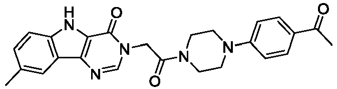 |
| **37** | 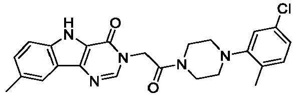 |
| **38** | 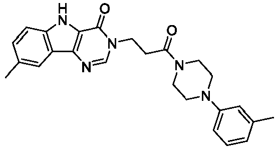 |
| **39** | 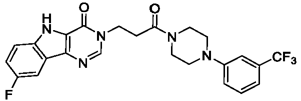 |
| **40** | 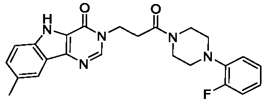 |
| **41** | 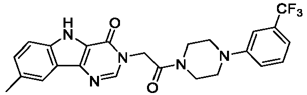 |
| **42** | 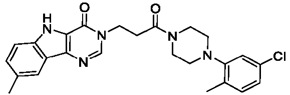 |
| **43** | 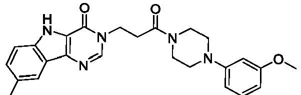 |
| **44** | 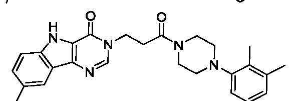 |
| **45** | 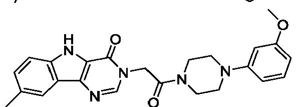 |
| **46** | 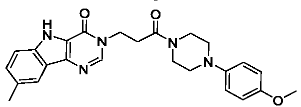 |
| **47** | 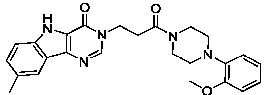 |
| **48** | 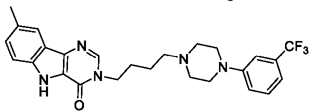 |
| **49** | 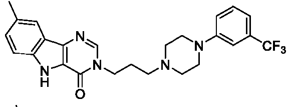 |
| **50** | 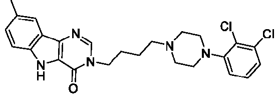 |
| **51** | 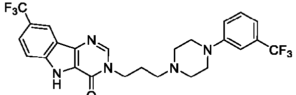 |
| **52** | 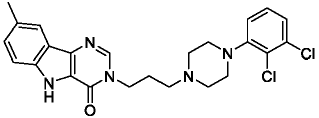 |
| **53** | 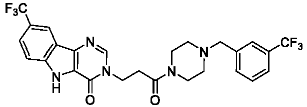 |
| **54** | 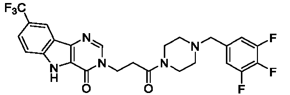 |
| **55** | 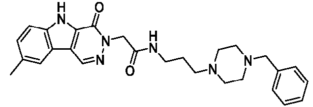 |
| **56** | 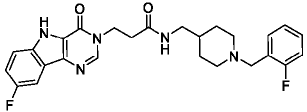 |
| **57** | 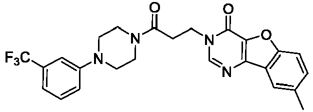 |
| **58** | 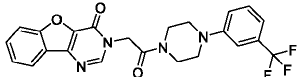 |
| **59** | 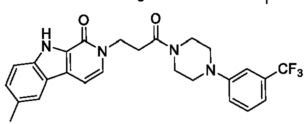 |
| **60** | 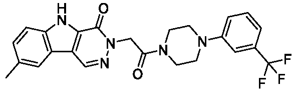 |
| **61** | 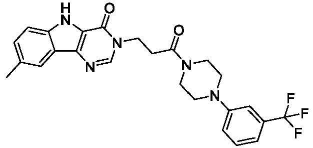 |
| **62** | 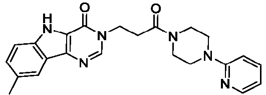 |
| **63** | 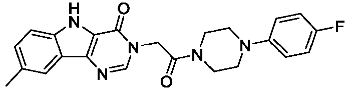 |
| **64** | 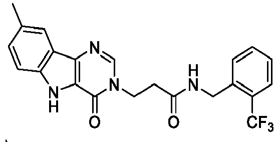 |
| **65** | 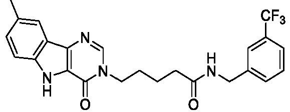 |
| **66** | 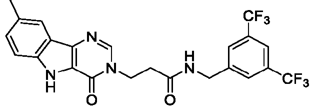 |
| **67** | 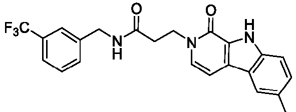 |
| **68** | 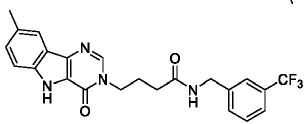 |
| **69** | 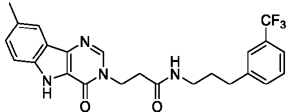 |
| **70** | 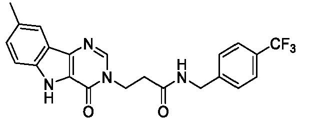 |
| **71** | 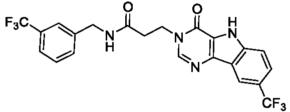 |
| **72** | 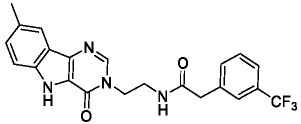 |
| **73** | 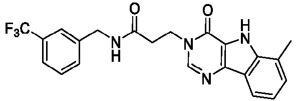 |
| **74** | 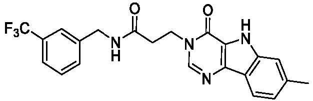 |
| **75** | 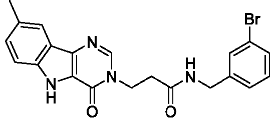 |
| **76** | 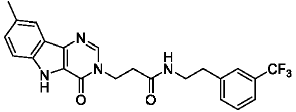 |
| **77** | 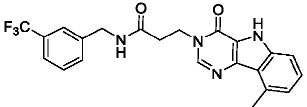 |
| **78** | 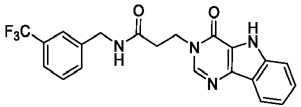 |
| **79** | 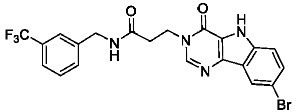 |
| **80** | 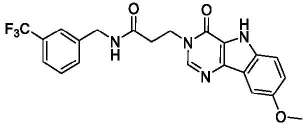 |
| **81** | 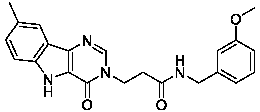 |
| **82** | 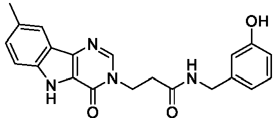 |
| **83** | 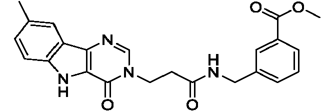 |
| **84** | 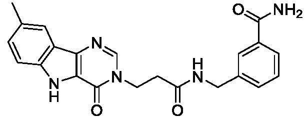 |
| **85** | 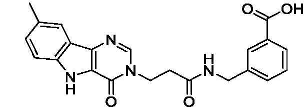 |
| **86** | 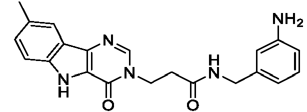 |
| **87** | 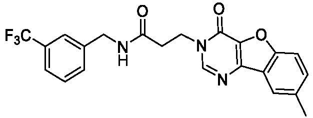 |
| **88** | 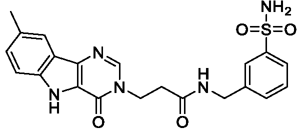 |
| **89** | 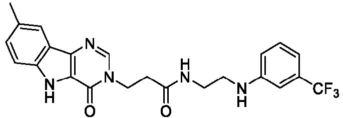 |
| **90** | 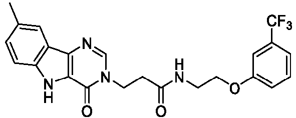 |
| **91** | 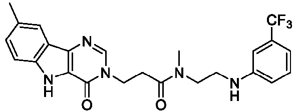 |
| **92** | 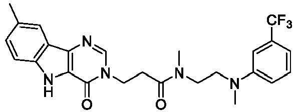 |
| **93** | 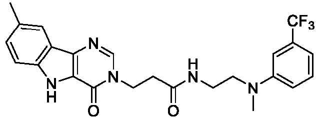 |
| **94** | 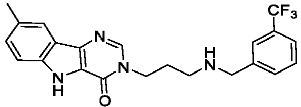 |
| **95** | 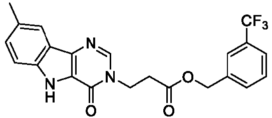 |
| **96** | 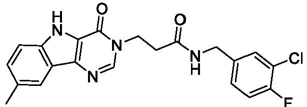 |
| **97** | 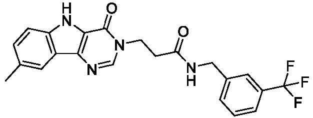 |
| **98** | 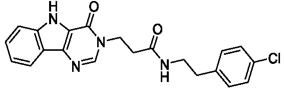 |
| **99** | 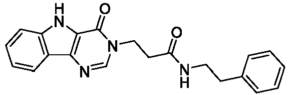 |
| **100** | 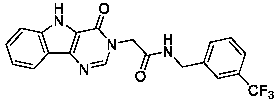 |
| **101** | 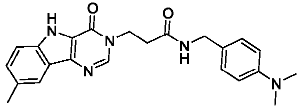 |
| **102** | 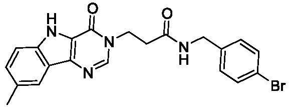 |
| **103** | 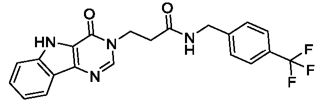 |
| **104** | 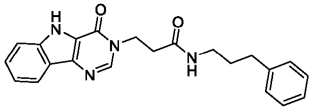 |
| **105** | 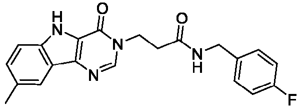 |
| **106** | 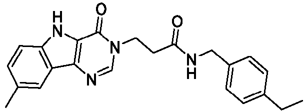 |
| **107** | 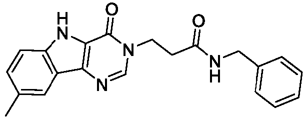 |
| **108** | 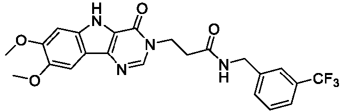 |
| **109** | 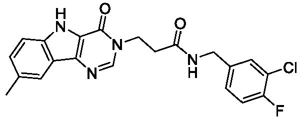 |
| **110** | 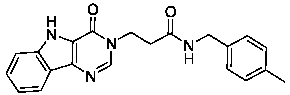 |
| **111** | 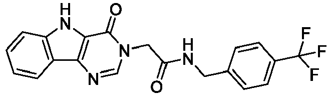 |
| **112** | 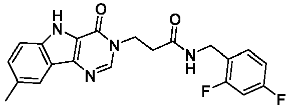 |
| **113** | 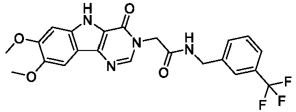 |
| **114** | 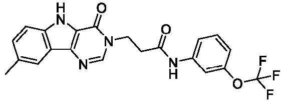 |
| **115** | 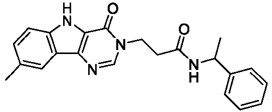 |
| **116** | 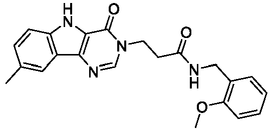 |
| **117** | 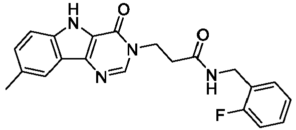 |
| **118** | 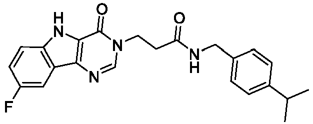 |
| **119** | 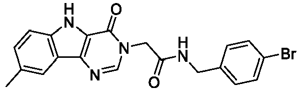 |
| **120** | 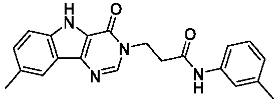 |
| **121** | 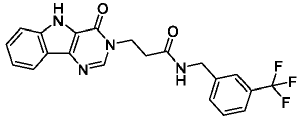 |
| **122** | 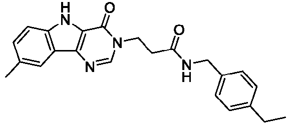 |
| **123** | 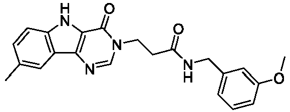 |
| **124** | 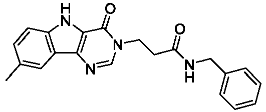 |
| **125** | 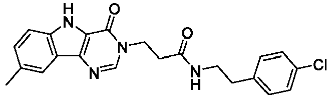 |
| **126** | 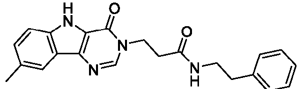 |
| **127** | 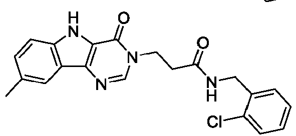 |
| **128** | 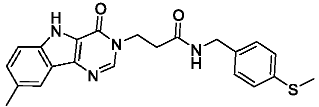 |
| **129** | 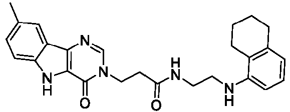 |
| **130** | 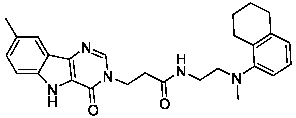 |
| **131** | 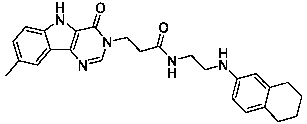 |
| **132** | 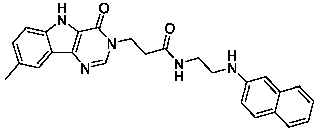 |
| **133** | 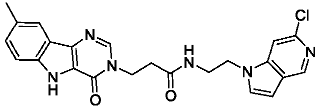 |
| **134** | 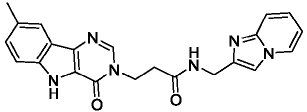 |
| **135** | 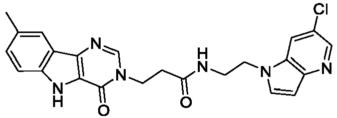 |
| **136** | 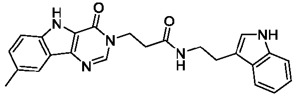 |
| **137** | 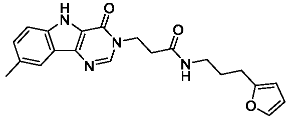 |
| **138** | 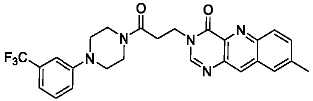 |
| **139** | 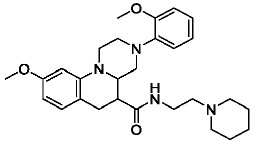 |
| **140** | 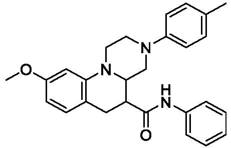 |
| **141** | 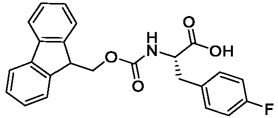 |
| **142** | 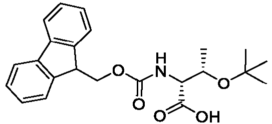 |
| **143** | 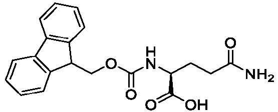 |
| **144** | 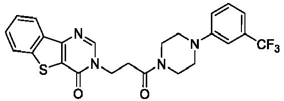 |
| **145** | 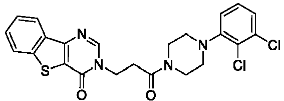 |
| **146** | 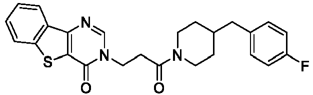 |
| **147** | 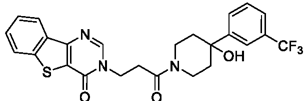 |
| **148** | 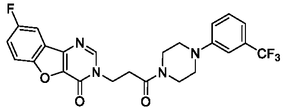 |
| **149** | 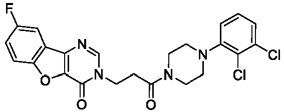 |
| **150** | 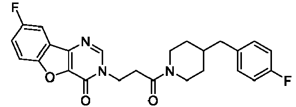 |
| **151** | 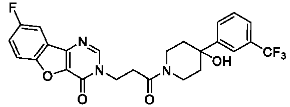 |
| **152** | 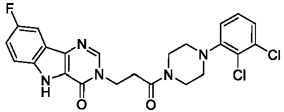 |
| **153** | 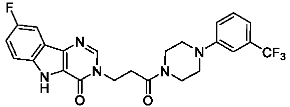 |
| **154** | 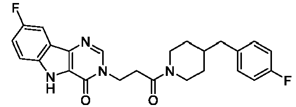 |
| **155** | 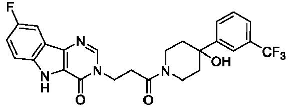 |
| **156** | 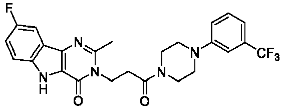 |
| **157** | 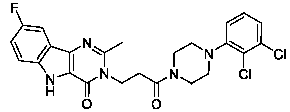 |
| **158** | 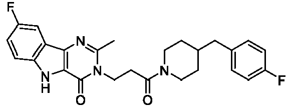 |
| **159** | 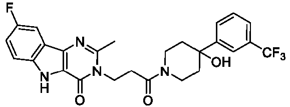 |
| **160** | 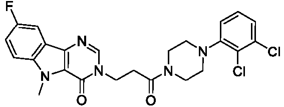 |
| **161** | 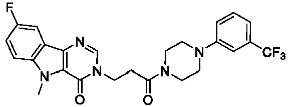 |
| **162** | 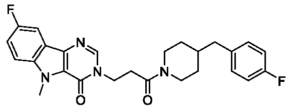 |
| **163** | 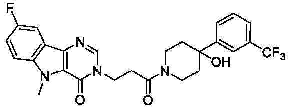 |
| **164** | 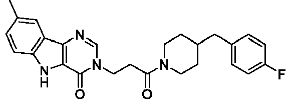 |
| **165** | 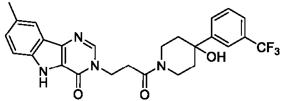 |
| **166** | 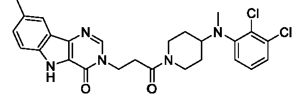 |
| **167** | 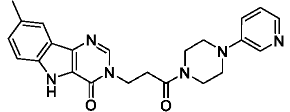 |
| **168** | 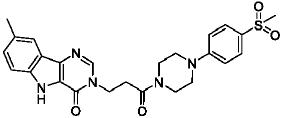 |
| **169** | 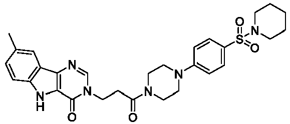 |
| **170** | 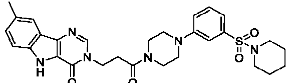 |
| **171** | 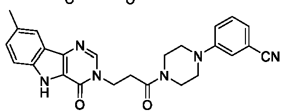 |
| **172** | 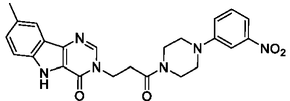 |
| **173** | 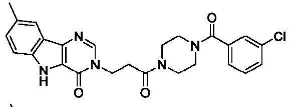 |
| **174** | 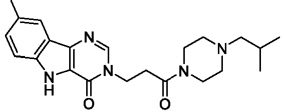 |
| **175** | 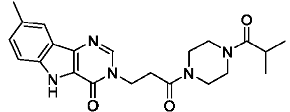 |
| **176** | 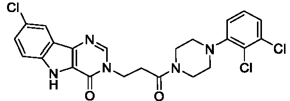 |
| **177** | 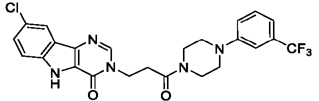 |
| **178** | 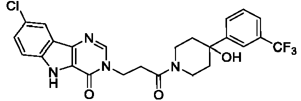 |
| **179** | 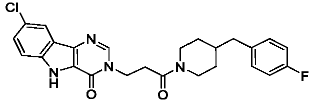 |
| **180** | 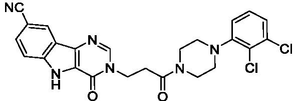 |
| **181** | 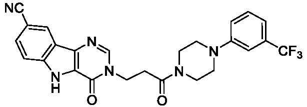 |
| **182** | 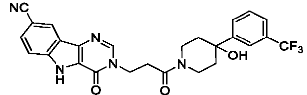 |
| **183** | 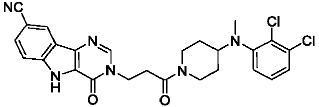 |
| **184** | 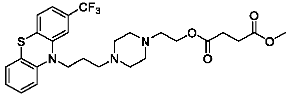 |
| **185** | 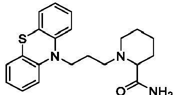 |
| **186** | 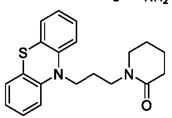 |
| **187** | 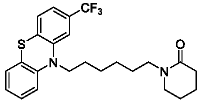 |
| **188** | 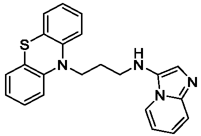 |
| **189** | 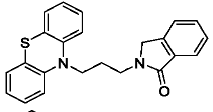 |
| **190** | 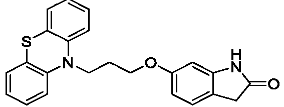 |
| **191** | 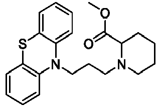 |
| **192** | 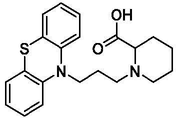 |
| **193** | 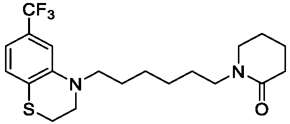 |
| **194** | 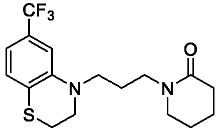 |
| **195** | 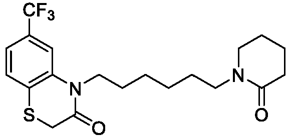 |
| **196** | 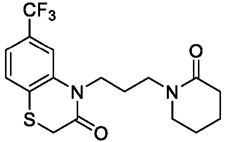 |
| **197** | 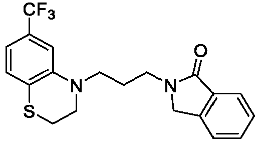 |
| **198** | 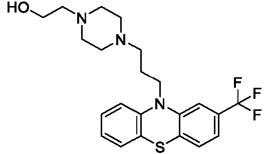 |
| **199** | 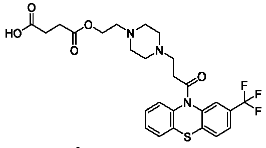 |
| **200** | 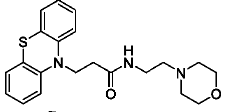 |
| **201** | 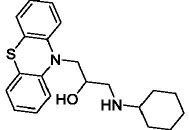 |
| **202** | 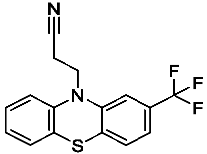 |
| **203** | 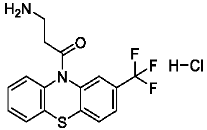 |
| **204** | 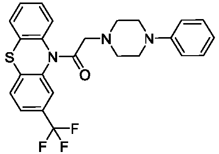 |
| **205** | 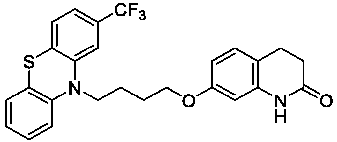 |
| **206** | 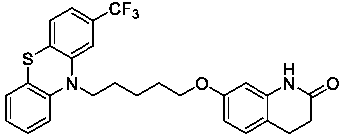 |
| **207** | 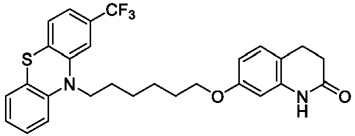 |
| **208** | 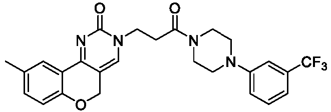 |
| **209** | 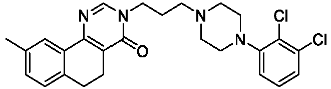 |
| **210** | 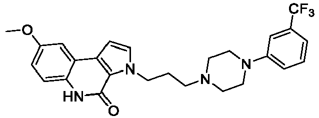 |
| **211** | 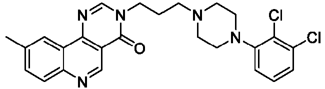 |
| **212** | 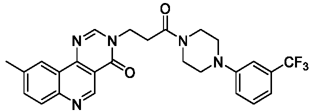 |
| **213** | 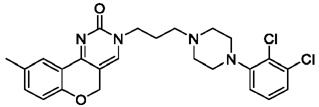 |
| **215** | 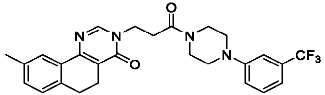 |
| **216** | 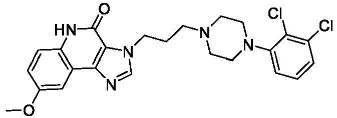 |
| **217** | 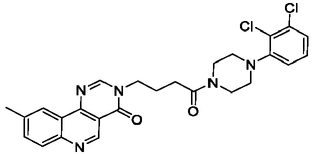 |
| **218** | 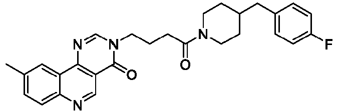 |
| **219** | 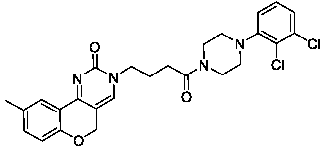 |
| **220** | 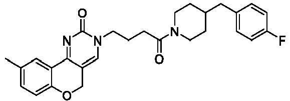 |
| **221** | 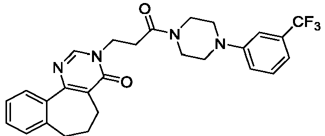 |
| **222** | 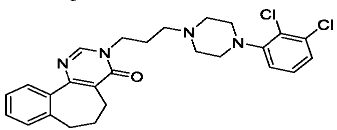 |
| **223** | 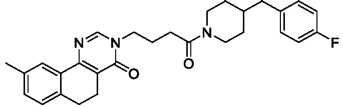 |
| **224** | 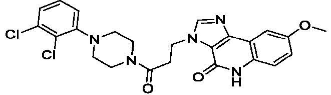 |
| **225** | 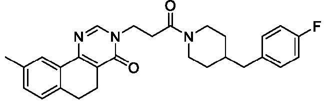 |
| **226** | 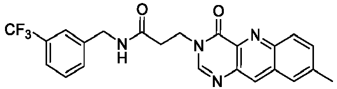 |
| **227** | 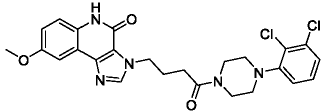 |
| **228** | 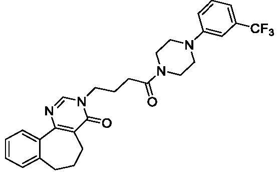 |
| **229** | 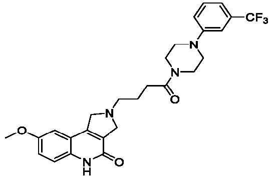 |
| **230** | 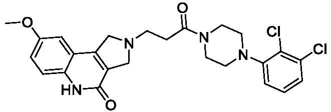 |
| **231** | 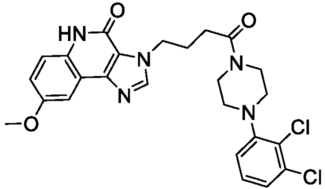 |
| **232** | 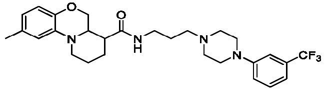 |
| **233** | 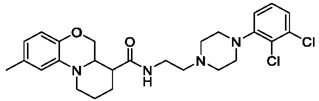 |
| **234** | 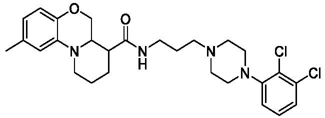 |
| **235** | 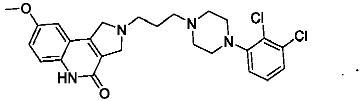 |

16. The method according to any one of claims 1 to 15, wherein the TPK agonist is administered in an amount of about 0.005 mg/day to about 5000 mg/day, e.g., in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

17. The method according to any one of claims 1 to 16, wherein the TPK agonist is administered in an amount of about 1 ng/kg to about 200 mg/kg, about 1 µg/kg to about 100 mg/kg or about 1 mg/kg to about 50 mg/kg body weight per day, e.g., is administered in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg or about 300 mg/kg body weight per day.

18. The method according to any one of claims 1 to 17, wherein the daily dose of the TPK agonist is administered at one time or is administered in two, three or four doses.

19. The method according to any one of claims 1 to 18, wherein the TPK agonist is administered continuously for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days or at least 50 days.

20. The method according to any one of claims 1 to 19, wherein the TPK agonist is administered for one or more (e.g., 1, 2, 3, 4, 5, 6, 7, 8, 9 or 10) courses of treatment, wherein each course of treatment lasts for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days or at least 50 days; and the interval between every two courses of treatment is 0, 1, 2, 3, 4, 5, 6, 7, 8, 9, 10 days, two weeks, three weeks, or four weeks.

21. The method according to any one of claims 1 to 20, wherein the TPK agonist is administered through injection (e.g., intravenous, intraarterial, subcutaneous, intraperitoneal, intramuscular injection, including dripping), or transdermal administration, or is administered via oral, buccal, nasal, transmucosal, or topical route, as an ophthalmic formulation, or via inhalation.

22. The method according to any one of claims 1 to 21, wherein the TPK agonist is administered in a dosage form selected from the group consisting of tablet, capsule, lozenge, hard candy, powder, spray, cream, salve, suppository, gel, paste, lotion, ointment, aqueous suspensions, injectable solution, elixir, and syrup.

23. The method according to any one of claims 1 to 22, wherein the method improves the following pathophysiological manifestations in the subject: abnormal cognitive behavior, neurodegenerative changes (e.g., progressive synaptic/neuronal loss and brain atrophy), β-amyloid deposition, abnormal phosphorylation of Tau and the resulting neurofibrillary tangles, glial cell activation and inflammation, and/or impaired cerebral glucose metabolism.

24. The method according to any one of claims 1 to 23, further comprising administering one or more additional therapeutic agents.

25. A compound, or a pharmaceutically acceptable salt, ester, stereoisomer, polymorph, solvate, N-oxide, isotopically labeled compound, metabolite, or prodrug thereof, the compound has the structure of Formula (III): 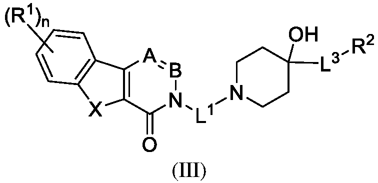
wherein each group is as defined in any one of claims 1 to 9;
provided that -L³-R² is not H and methyl.

26. A compound, or a pharmaceutically acceptable salt, ester, stereoisomer, polymorph, solvate, N-oxide, isotopically labeled compound, metabolite, or prodrug thereof, wherein the compound is selected from the group consisting of:
| **No.** | **Structure** |
|---|---|
| **1** | 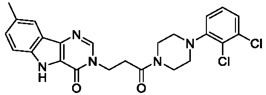 |
| **2** | 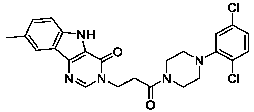 |
| **3** | 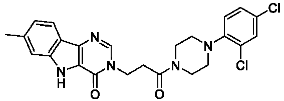 |
| **4** | 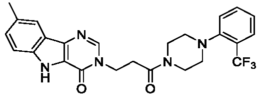 |
| **5** | 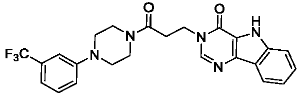 |
| **6** | 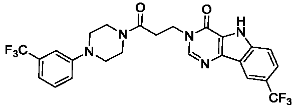 |
| **7** | 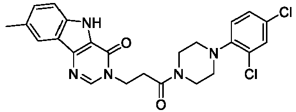 |
| **8** | 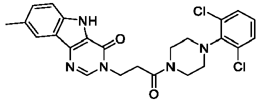 |
| **9** | 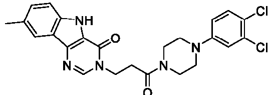 |
| **10** | 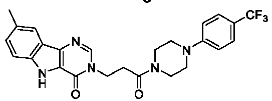 |
| **11** | 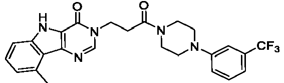 |
| **12** | 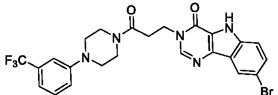 |
| **13** | 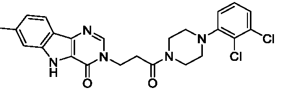 |
| **14** | 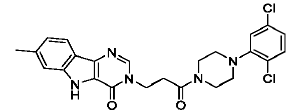 |
| **15** | 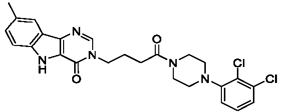 |
| **16** | 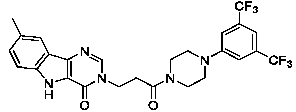 |
| **17** | 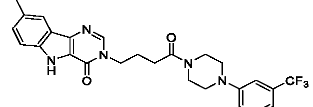 |
| **18** | 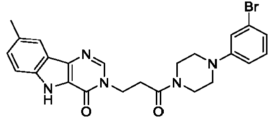 |
| **19** | 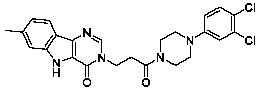 |
| **20** | 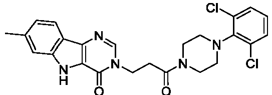 |
| **22** | 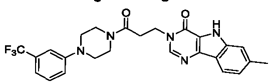 |
| **23** | 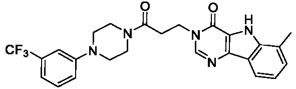 |
| **24** | 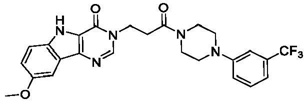 |
| **25** | 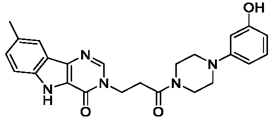 |
| **26** | 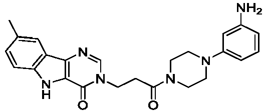 |
| **27** | 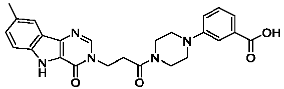 |
| **28** | 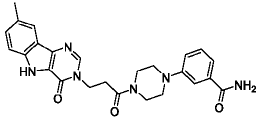 |
| **29** | 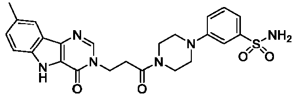 |
| **48** | 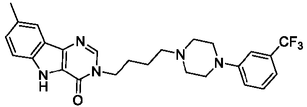 |
| **49** | 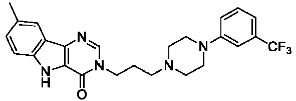 |
| **50** | 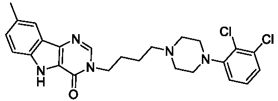 |
| **51** | 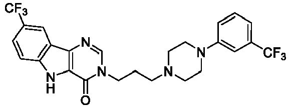 |
| **52** | 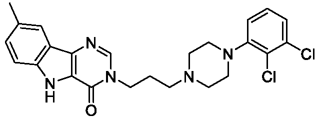 |
| **53** | 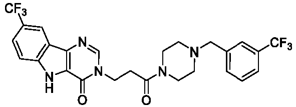 |
| **54** | 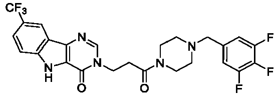 |
| **57** | 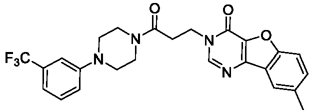 |
| **59** | 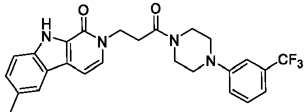 |
| **65** | 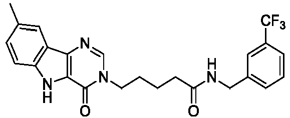 |
| **66** | 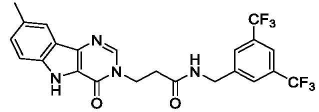 |
| **67** | 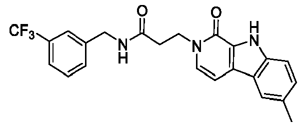 |
| **68** | 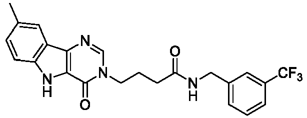 |
| **69** | 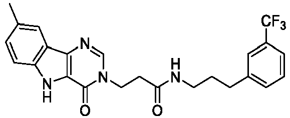 |
| **71** | 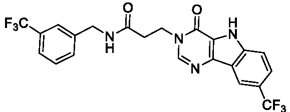 |
| **72** | 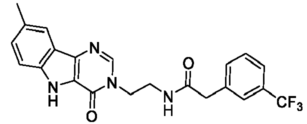 |
| **73** | 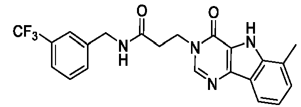 |
| **74** | 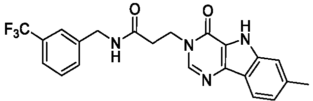 |
| **75** | 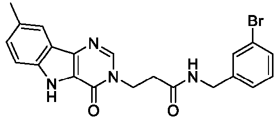 |
| **76** | 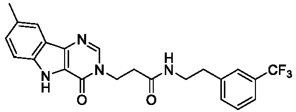 |
| **77** | 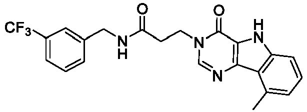 |
| **79** | 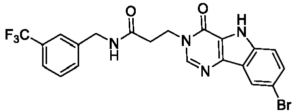 |
| **80** | 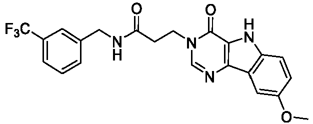 |
| **82** | 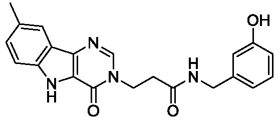 |
| **83** | 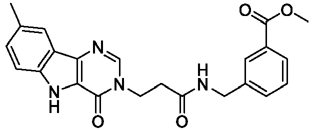 |
| **84** | 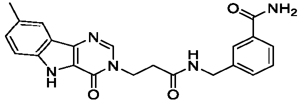 |
| **85** | 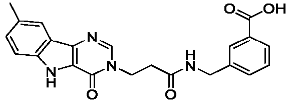 |
| **86** | 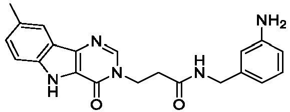 |
| **87** | 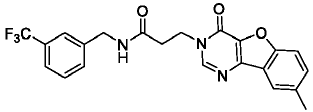 |
| **88** | 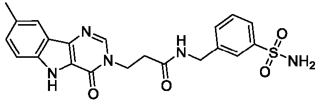 |
| **89** | 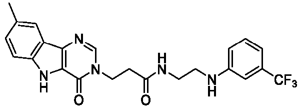 |
| **90** | 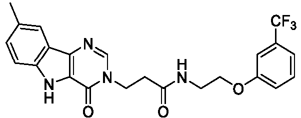 |
| **91** | 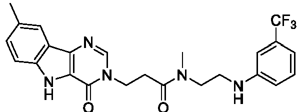 |
| **92** | 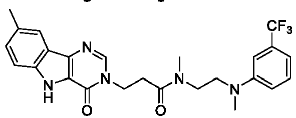 |
| **93** | 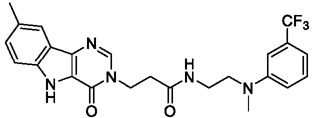 |
| **94** | 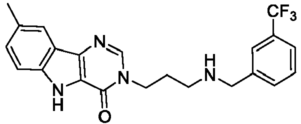 |
| **95** | 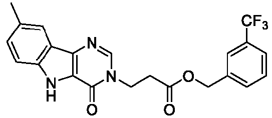 |
| **129** | 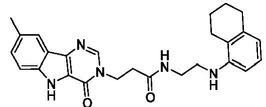 |
| **130** | 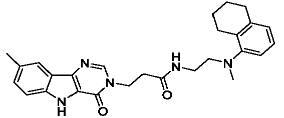 |
| **131** | 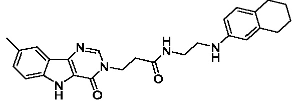 |
| **132** | 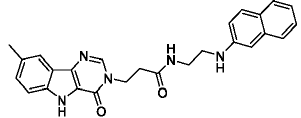 |
| **133** | 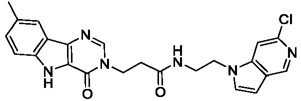 |
| **134** | 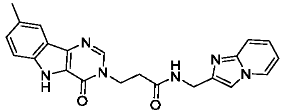 |
| **135** | 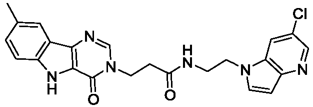 |
| **138** | 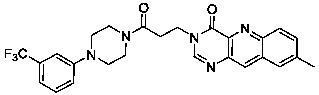 |
| **144** | 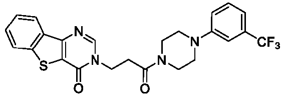 |
| **145** | 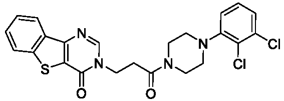 |
| **146** | 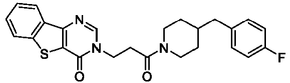 |
| **147** | 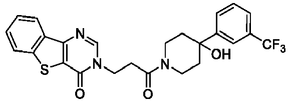 |
| **148** | 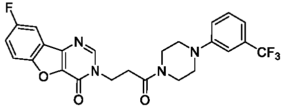 |
| **149** | 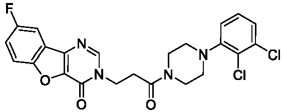 |
| **150** | 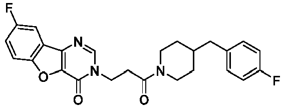 |
| **151** | 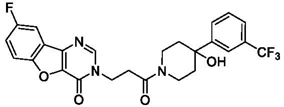 |
| **152** | 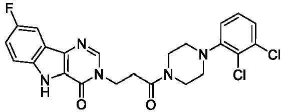 |
| **153** | 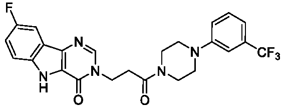 |
| **154** | 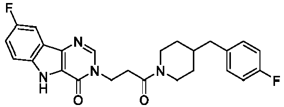 |
| **155** | 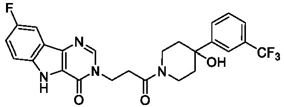 |
| **156** | 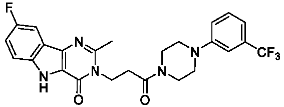 |
| **157** | 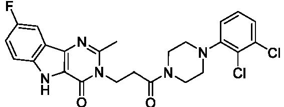 |
| **158** | 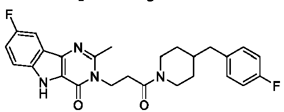 |
| **159** | 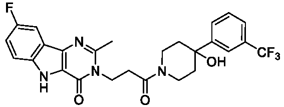 |
| **160** | 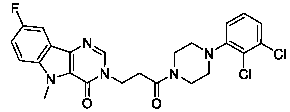 |
| **161** | 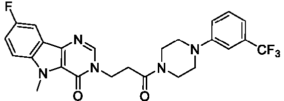 |
| **162** | 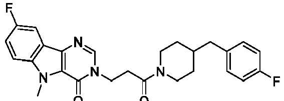 |
| **163** | 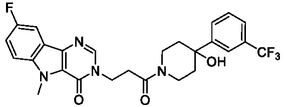 |
| **164** | 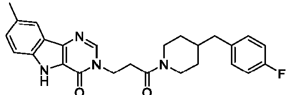 |
| **165** | 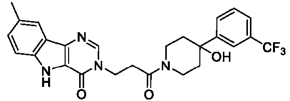 |
| **166** | 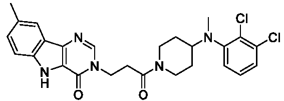 |
| **167** | 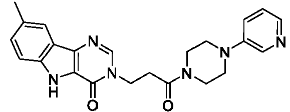 |
| **168** | 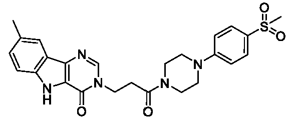 |
| **169** | 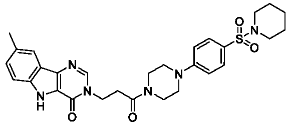 |
| **170** | 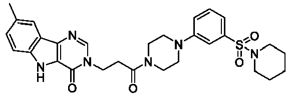 |
| **171** | 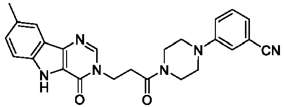 |
| **172** | 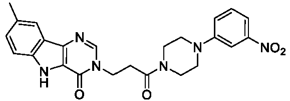 |
| **173** | 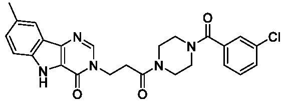 |
| **174** | 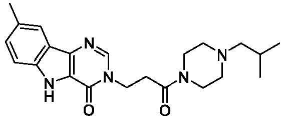 |
| **175** | 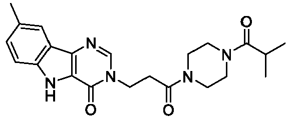 |
| **176** | 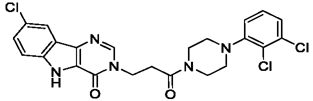 |
| **177** | 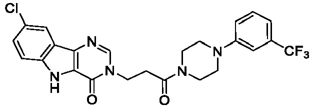 |
| **178** | 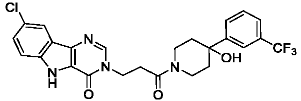 |
| **179** | 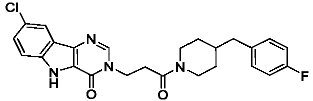 |
| **180** | 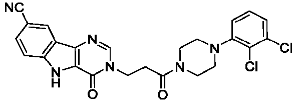 |
| **181** | 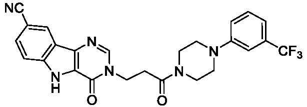 |
| **182** | 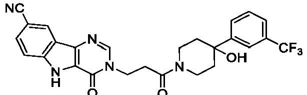 |
| **183** | 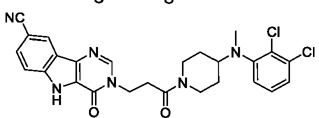 |
| **184** | 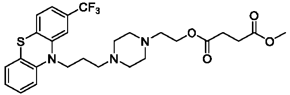 |
| **185** | 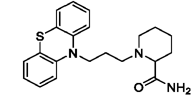 |
| **186** | 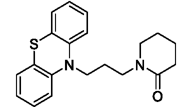 |
| **187** | 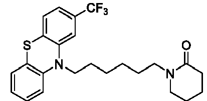 |
| **188** | 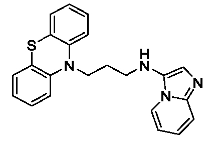 |
| **189** | 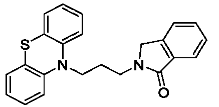 |
| **190** | 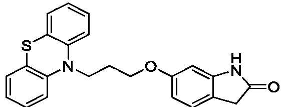 |
| **191** | 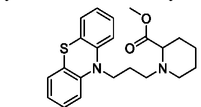 |
| **192** | 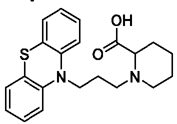 |
| **193** | 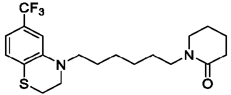 |
| **194** | 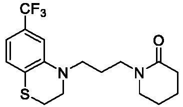 |
| **195** | 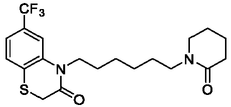 |
| **196** | 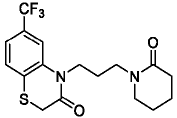 |
| **197** | 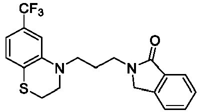 |
| **205** | 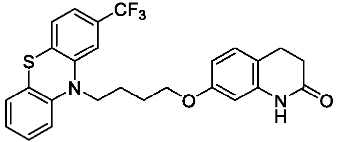 |
| **206** | 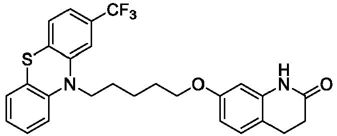 |
| **207** | 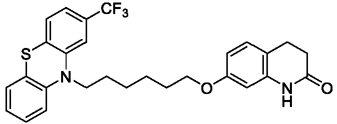 |
| **208** | 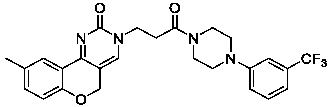 |
| **209** | 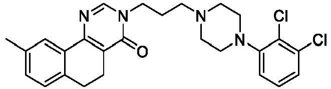 |
| **210** | 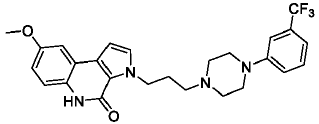 |
| **211** | 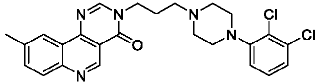 |
| **212** | 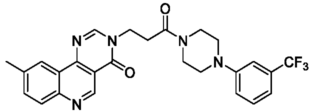 |
| **213** | 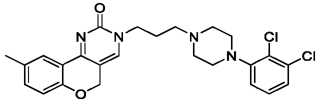 |
| **215** | 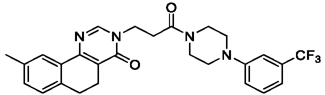 |
| **216** | 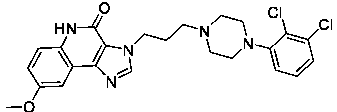 |
| **217** | 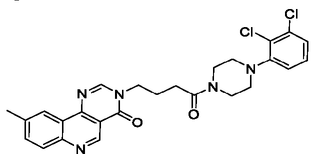 |
| **218** | 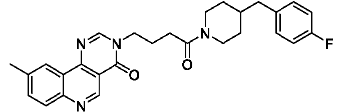 |
| **219** | 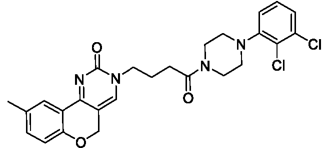 |
| **220** | 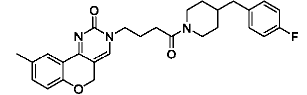 |
| **221** | 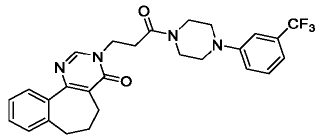 |
| **222** | 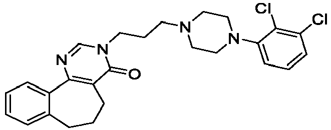 |
| **223** | 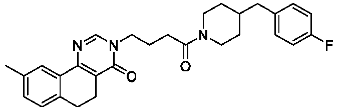 |
| **224** | 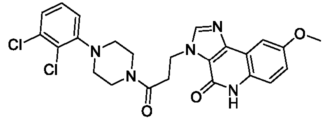 |
| **225** | 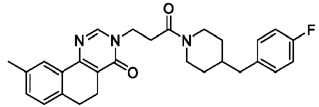 |
| **226** | 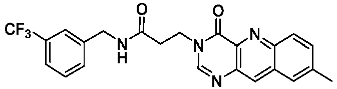 |
| **227** | 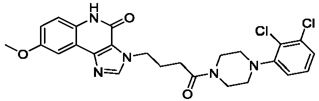 |
| **228** | 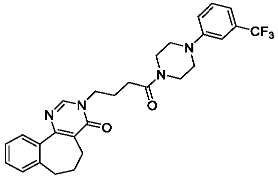 |
| **229** | 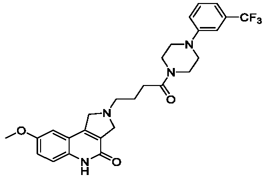 |
| **230** | 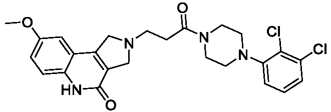 |
| **231** | 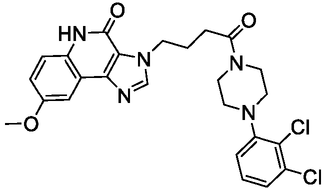 |
| **232** | 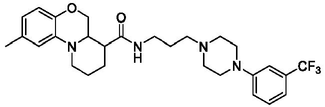 |
| **233** | 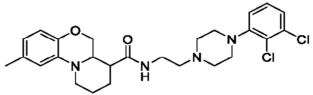 |
| **234** | 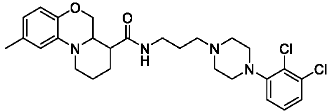 |
| **235** | 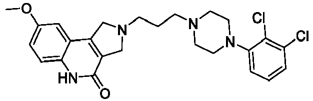 . |
